# EUROPEAN PATENT SPECIFICATION

(11) **EP 2 496 598 B1**
(45) Date of publication and mention of the grant of the patent: **02.08.2017**
(21) Application number: 10775936.7
(22) Date of filing: 27.10.2010
(51) Int. Cl.: C07K 14/31, C07K 14/705, C07K 16/32, C07K 16/28

(54) **HER3 BINDING POLYPEPTIDES**
HER3-BINDENDE POLYPEPTIDE
POLYPEPTIDES DE LIAISON DE HER3

(30) Priority: 04.11.2009 EP 09175025
(43) Date of publication of application: 12.09.2012
(73) Proprietor: Affibody AB, 171 69 Solna (SE)
(72) Inventor: FREJD, Fredrik, S-112 20 Stockholm (SE); GUNNERIUSSON, Elin, S-133 36 Saltsjöbaden (SE); KRONQVIST, Nina, S-187 63 Täby (SE); LÖFBLOM, John, S-141 37 Huddinge (SE); STÅHL, Stefan, S-111 40 Stockholm (SE)
(74) Representative: Awapatent AB
(86) International application number: PCT/SE2010/051164
(87) International publication number: WO 2011/056124

(56) References cited:
- WO-A1-2005/003156
- WO-A1-2007/065635
- WO-A1-2009/019117
- WO-A2-2008/100624
- FRIEDMAN ET AL: "Directed Evolution to Low Nanomolar Affinity of a Tumor-Targeting Epidermal Growth Factor Receptor-Binding Affibody Molecule", JOURNAL OF MOLECULAR BIOLOGY, LONDON, GB, vol. 376, no. 5, 4 January 2008 (2008-01-04), pages 1388-1402, XP022483411, ISSN: 0022-2836, DOI: DOI:10.1016/J.JMB.2007.12.060
- ORLOVA ANNA ET AL: "Update: affibody molecules for molecular imaging and therapy for cancer", CANCER BIOTHERAPY AND RADIOPHARMACEUTICALS, LIEBERT, US, vol. 22, no. 5, 1 October 2007 (2007-10-01), pages 573-584, XP002488606, DOI: DOI:10.1089/CBR.2006.004-U
- GRÖNWALL C ET AL: "Engineered affinity proteins-Generation and applications", JOURNAL OF BIOTECHNOLOGY, ELSEVIER SCIENCE PUBLISHERS, AMSTERDAM, NL, vol. 140, no. 3-4, 25 March 2009 (2009-03-25), pages 254-269, XP026073227, ISSN: 0168-1656, DOI: DOI:10.1016/J.JBIOTEC.2009.01.014 [retrieved on 2009-02-02]
- NORD KARIN ET AL: "A combinatorial library of an alpha-helical bacterial receptor domain", PROTEIN ENGINEERING, OXFORD UNIVERSITY PRESS, SURREY, GB, vol. 8, no. 6, 1 January 1995 (1995-01-01) , pages 601-608, XP002190390, ISSN: 0269-2139

### SEQUENCE LISTING

<110> Affibody AB
<120> Polypeptides
<130> 21048625
<160> 1009
<170> PatentIn version 3.5
<210> 1
   <211> 29
   <212> PRT
   <213> Artificial Sequence
<220>
   <223> Engineered polypeptide
<400> 1
   Glu Lys Val Ala Ala Thr Gly Glu Ile Trp Asp Leu Pro Asn Leu Asn
   1               5                   10                  15
   Thr Arg Gln Lys Asn Ala Phe Ile Gly Ser Leu Trp Asp
               20                  25
<210> 2
   <211> 29
   <212> PRT
   <213> Artificial Sequence
<220>
   <223> Engineered polypeptide
<400> 2
   Glu Trp Arg Trp Ala Ala His Glu Ile Trp Asp Leu Pro Asn Leu Asn
   1               5                   10                  15
   Val Tyr Gln Arg Ala Ala Phe Ile Arg Ser Leu Glu Asp
               20                  25
<210> 3
   <211> 29
   <212> PRT
   <213> Artificial Sequence
<220>
   <223> Engineered polypeptide
<400> 3
   Glu Arg Arg Leu Ala Ser Val Glu Ile Trp Glu Leu Pro Asn Leu Asn
   1               5                   10                  15
   Ala Val Gln Lys Ser Ala Phe Ile Ser Ser Leu Phe Asp
               20                  25
<210> 4
   <211> 29
   <212> PRT
   <213> Artificial Sequence
<220>
   <223> Engineered polypeptide
<400> 4
   Glu Val Lys Glu Ala Arg Phe Glu Ile Trp Asp Leu Pro Asn Leu Asn
   1               5                   10                  15
   Arg Thr Gln Lys His Ala Phe Ile Val Ser Leu Glu Asp
               20                  25
<210> 5
   <211> 29
   <212> PRT
   <213> Artificial Sequence
<220>
   <223> Engineered polypeptide
<400> 5
   Glu Trp Lys Gly Ala Ala Gly Glu Ile Trp Asn Leu Pro Asn Leu Asn
   1               5                   10                  15
   Val Ser Gln Arg Val Ala Phe Ile Gly Ser Leu Ala Asp
               20                  25
<210> 6
   <211> 29
   <212> PRT
   <213> Artificial Sequence
<220>
   <223> Engineered polypeptide
<400> 6
   Glu Lys Tyr Lys Ala Ala His Glu Ile Trp Glu Leu Pro Asn Leu Asn
   1 5 10 15
   Arg Asp Gln Arg Ala Ala Phe Ile Thr Ser Leu Thr Asp
   20 25
<210> 7
   <211> 29
   <212> PRT
   <213> Artificial Sequence
<220>
   <223> Engineered polypeptide
<400> 7
   Glu Arg Ser Leu Ala Ser Phe Glu Ile Trp Glu Leu Pro Asn Leu Asn
   1 5 10 15
   Pro Lys Gln Lys Ala Ala Phe Ile Val Ser Leu Phe Asp
   20 25
<210> 8
   <211> 29
   <212> PRT
   <213> Artificial Sequence
<220>
   <223> Engineered polypeptide
<400> 8
   Glu Trp Lys Arg Ala Ser Trp Glu Ile Trp Glu Leu Pro Asn Leu Asn
   1 5 10 15
   Asn Ala Gln Lys Arg Ala Phe Ile Ser Ser Leu Ser Asp
   20 25
<210> 9
   <211> 29
   <212> PRT
   <213> Artificial Sequence
<220>
   <223> Engineered polypeptide
<400> 9
   Glu Lys Tyr Lys Ala Met Thr Glu Ile Trp Ile Leu Pro Asn Leu Asn
   1 5 10 15
   Gln Arg Gln Lys Val Ala Phe Ile Gly Ser Leu Asp Asp
   20 25
<210> 10
   <211> 29
   <212> PRT
   <213> Artificial Sequence
<220>
   <223> Engineered polypeptide
<400> 10
   Glu Trp Arg Gly Ala Ala Gly Glu Ile Trp Ala Leu Pro Asn Leu Asn
   1               5                   10                  15
   Asn Arg Gln Lys Gly Ala Phe Ile Glu Ser Leu Pro Asp
               20                  25
<210> 11
   <211> 29
   <212> PRT
   <213> Artificial Sequence
<220>
   <223> Engineered polypeptide
<400> 11
   Glu Arg Trp Glu Ala Thr Val Glu Ile Trp Asp Leu Pro Asn Leu Asn
   1               5                   10                  15
   Arg Asn Gln Lys Ala Ala Phe Ile Ala Ser Leu Gln Asp
               20                  25
<210> 12
   <211> 29
   <212> PRT
   <213> Artificial Sequence
<220>
   <223> Engineered polypeptide
<400> 12
   Glu Lys Tyr Asn Ala Tyr Ala Glu Ile Trp Leu Leu Pro Asn Leu Asn
   1               5                   10                  15
   Arg Tyr Gln Lys Ala Ala Phe Ile Gly Ser Leu Ser Asp
               20                  25
<210> 13
   <211> 29
   <212> PRT
   <213> Artificial Sequence
<220>
   <223> Engineered polypeptide
<400> 13
   Glu Arg Ser Val Ala Gln Lys Glu Ile Trp Glu Leu Pro Asn Leu Asn
   1               5                   10                  15
   Arg Trp Gln Ala Gly Ala Phe Ile Lys Ser Leu Tyr Asp
               20                  25
<210> 14
   <211> 29
   <212> PRT
   <213> Artificial Sequence
<220>
   <223> Engineered polypeptide
<400> 14
   Glu Lys Met Asp Ala Met Gly Glu Ile Trp Asp Leu Pro Asn Leu Asn
   1               5                   10                  15
   Arg Gly Gln Ala Ser Ala Phe Ile Ala Ser Leu Gln Asp
               20                  25
<210> 15
   <211> 29
   <212> PRT
   <213> Artificial Sequence
<220>
   <223> Engineered polypeptide
<400> 15
   Glu Lys Arg Asn Ala Gln Val Glu Ile Trp Thr Leu Pro Asn Leu Asn
   1               5                   10                  15
   Ser Lys Gln Arg Ala Ala Phe Ile Lys Ser Leu Tyr Asp
               20                  25
<210> 16
   <211> 29
   <212> PRT
   <213> Artificial Sequence
<220>
   <223> Engineered polypeptide
<400> 16
   Glu Arg Arg Asp Ala Arg Phe Glu Ile Trp Glu Leu Pro Asn Leu Asn
   1               5                   10                  15
   Lys Tyr Gln Arg Ala Ala Phe Ile Ser Ser Leu Asp Asp
               20                  25
<210> 17
   <211> 29
   <212> PRT
   <213> Artificial Sequence
<220>
   <223> Engineered polypeptide
<400> 17
   Glu Arg Ser Met Ala Arg Phe Glu Ile Trp Glu Leu Pro Asn Leu Asn
   1               5                   10                  15
   Arg Gly Gln Lys Ser Ala Phe Ile Ala Ser Leu Glu Asp
               20                  25
<210> 18
   <211> 29
   <212> PRT
   <213> Artificial Sequence
<220>
   <223> Engineered polypeptide
<400> 18
   Glu Trp His Gly Ala Ala Ser Glu Ile Trp Glu Leu Pro Asn Leu Asn
   1               5                   10                  15
   Lys Ser Gln Lys Ser Ala Phe Ile Lys Ser Leu Pro Asp
               20                  25
<210> 19
   <211> 29
   <212> PRT
   <213> Artificial Sequence
<220>
   <223> Engineered polypeptide
<400> 19
   Glu Trp Lys Gln Ala Ala Glu Glu Ile Trp Asp Leu Pro Asn Leu Asn
   1               5                   10                  15
   Arg Arg Gln Ala Gly Ala Phe Ile Thr Ser Leu Gln Asp
               20                  25
<210> 20
   <211> 29
   <212> PRT
   <213> Artificial Sequence
<220>
   <223> Engineered polypeptide
<400> 20
   Glu Lys Val Gln Ala Ser Glu Glu Ile Trp Asn Leu Pro Asn Leu Asn
   1               5                   10                  15
   Arg Arg Gln Arg Ala Ala Phe Ile Gly Ser Leu Tyr Asp
               20                  25
<210> 21
   <211> 29
   <212> PRT
   <213> Artificial Sequence
<220>
   <223> Engineered polypeptide
<400> 21
   Glu Arg Tyr Ser Ala Thr Val Glu Ile Trp Asp Leu Pro Asn Leu Asn
   1               5                   10                  15
   Thr Leu Gln Lys Ser Ala Phe Ile Gly Ser Leu Gln Asp
               20                  25
<210> 22
   <211> 29
   <212> PRT
   <213> Artificial Sequence
<220>
   <223> Engineered polypeptide
<400> 22
   Glu Arg Tyr Ser Ala Tyr Tyr Glu Ile Trp Gln Leu Pro Asn Leu Asn
   1               5                   10                  15
   Arg Ile Gln Lys Ala Ala Phe Ile Ser Ser Leu Gln Asp
               20                  25
<210> 23
   <211> 29
   <212> PRT
   <213> Artificial Sequence
<220>
   <223> Engineered polypeptide
<400> 23
   Glu Arg Tyr Arg Ala Tyr Phe Glu Ile Trp Gln Leu Pro Asn Leu Asn
   1 5 10 15
   Arg Leu Gln Lys Ala Ala Phe Ile Ser Ser Leu Glu Asp
   20 25
<210> 24
   <211> 29
   <212> PRT
   <213> Artificial Sequence
<220>
   <223> Engineered polypeptide
<400> 24
   Glu Lys Tyr Lys Ala Tyr Gly Glu Ile Trp Gln Leu Pro Asn Leu Asn
   1 5 10 15
   Arg Val Gln Lys Ala Ala Phe Ile Ala Ser Leu Ser Asp
   20 25
<210> 25
   <211> 29
   <212> PRT
   <213> Artificial Sequence
<220>
   <223> Engineered polypeptide
<400> 25
   Glu Arg Lys Gln Ala Thr Phe Glu Ile Trp Glu Leu Pro Asn Leu Asn
   1 5 10 15
   Gln Arg Gln Lys Ala Ala Phe Ile Lys Ser Leu Trp Asp
   20 25
<210> 26
   <211> 29
   <212> PRT
   <213> Artificial Sequence
<220>
   <223> Engineered polypeptide
<400> 26
   Glu Trp Lys Ile Ala Ala Gly Glu Ile Trp Gln Leu Pro Asn Leu Asn
   1               5                   10                  15
   Arg His Gln Lys Gly Ala Phe Ile Ser Ser Leu Tyr Asp
               20                  25
<210> 27
   <211> 29
   <212> PRT
   <213> Artificial Sequence
<220>
   <223> Engineered polypeptide
<400> 27
   Glu Lys Tyr Lys Ala Tyr Val Glu Ile Trp Gln Leu Pro Asn Leu Asn
   1               5                   10                  15
   Arg Tyr Gln Arg Ala Ala Phe Ile Gly Ser Leu Gln Asp
               20                  25
<210> 28
   <211> 29
   <212> PRT
   <213> Artificial Sequence
<220>
   <223> Engineered polypeptide
<400> 28
   Glu Arg Lys Ala Ala Thr Phe Glu Ile Trp Glu Leu Pro Asn Leu Asn
   1               5                   10                  15
   Arg Ile Gln Lys Ala Ala Phe Ile Ala Ser Leu Phe Asp
               20                  25
<210> 29
   <211> 29
   <212> PRT
   <213> Artificial Sequence
<220>
   <223> Engineered polypeptide
<400> 29
   Glu Lys Tyr Lys Ala Tyr Gly Glu Ile Trp Gln Leu Pro Asn Leu Asn
   1               5                   10                  15
   Arg Ile Gln Lys Ala Ala Phe Ile Ser Ser Leu Gln Asp
               20                  25
<210> 30
   <211> 29
   <212> PRT
   <213> Artificial Sequence
<220>
   <223> Engineered polypeptide
<400> 30
   Glu Lys Arg Ile Ala Thr Trp Glu Ile Trp Gln Leu Pro Asn Leu Asn
   1               5                   10                  15
   Gln His Gln Lys Ala Ala Phe Ile Ser Ser Leu Trp Asp
               20                  25
<210> 31
   <211> 29
   <212> PRT
   <213> Artificial Sequence
<220>
   <223> Engineered polypeptide
<400> 31
   Glu Lys Tyr Lys Ala Tyr Val Glu Ile Trp Gln Leu Pro Asn Leu Asn
   1               5                   10                  15
   Arg Gly Gln Lys Ala Ala Phe Ile Ser Ser Leu Gln Asp
               20                  25
<210> 32
   <211> 29
   <212> PRT
   <213> Artificial Sequence
<220>
   <223> Engineered polypeptide
<400> 32
   Glu Arg Tyr Leu Ala Tyr Tyr Glu Ile Trp Gln Leu Pro Asn Leu Asn
   1               5                   10                  15
   Arg Thr Gln Lys Ala Ala Phe Ile Gly Ser Leu Gln Asp
                       20                  25
<210> 33
   <211> 29
   <212> PRT
   <213> Artificial Sequence
<220>
   <223> Engineered polypeptide
<400> 33
   Glu Arg Tyr Ile Ala Tyr Trp Glu Ile Trp Gln Leu Pro Asn Leu Asn
   1               5                   10                  15
   Arg Arg Gln Lys Ala Ala Phe Ile Ser Ser Leu Gln Asp
               20                  25
<210> 34
   <211> 29
   <212> PRT
   <213> Artificial Sequence
<220>
   <223> Engineered polypeptide
<400> 34
   Glu Lys Tyr Arg Ala Tyr Gly Glu Ile Trp Gln Leu Pro Asn Leu Asn
   1               5                   10                  15
   Arg Gly Gln Lys Ala Ala Phe Ile Ala Ser Leu Gln Asp
               20                  25
<210> 35
   <211> 29
   <212> PRT
   <213> Artificial Sequence
<220>
   <223> Engineered polypeptide
<400> 35
   Glu Lys Tyr Thr Ala Tyr Phe Glu Ile Trp Gln Leu Pro Asn Leu Asn
   1               5                   10                  15
   Val Arg Gln Lys Ala Ala Phe Ile Ser Ser Leu Gln Asp
               20                  25
<210> 36
   <211> 29
   <212> PRT
   <213> Artificial Sequence
<220>
   <223> Engineered polypeptide
<400> 36
   Glu Arg Tyr Ser Ala Tyr Tyr Glu Ile Trp Gln Leu Pro Asn Leu Asn
   1               5                   10                  15
   Val Arg Gln Lys Ala Ala Phe Ile Gly Ser Leu Gln Asp
               20                  25
<210> 37
   <211> 29
   <212> PRT
   <213> Artificial Sequence
<220>
   <223> Engineered polypeptide
<400> 37
   Glu Lys Tyr Ala Ala Tyr Gly Glu Ile Trp Gln Leu Pro Asn Leu Asn
   1               5                   10                  15
   Arg Ser Gln Lys Ala Ala Phe Ile Gly Ser Leu Ser Asp
               20                  25
<210> 38
   <211> 29
   <212> PRT
   <213> Artificial Sequence
<220>
   <223> Engineered polypeptide
<400> 38
   Glu Arg Lys Arg Ala Thr Val Glu Ile Trp Glu Leu Pro Asn Leu Asn
   1               5                   10                  15
   Arg Leu Gln Arg Gly Ala Phe Ile Ala Ser Leu Trp Asp
               20                  25
<210> 39
   <211> 29
   <212> PRT
   <213> Artificial Sequence
<220>
   <223> Engineered polypeptide
<400> 39
   Glu Trp Lys Gln Ala Ser Phe Glu Ile Trp Glu Leu Pro Asn Leu Asn
   1               5                   10                  15
   Arg Leu Gln Lys Ala Ala Phe Ile Gly Ser Leu Ala Asp
               20                  25
<210> 40
   <211> 29
   <212> PRT
   <213> Artificial Sequence
<220>
   <223> Engineered polypeptide
<400> 40
   Glu Arg Lys His Ala Thr Val Glu Ile Trp Glu Leu Pro Asn Leu Asn
   1               5                   10                  15
   Arg Val Gln Lys Ala Ala Phe Ile Ser Ser Leu Trp Asp
               20                  25
<210> 41
   <211> 29
   <212> PRT
   <213> Artificial Sequence
<220>
   <223> Engineered polypeptide
<400> 41
   Glu Lys Tyr Lys Ala Tyr Val Glu Ile Trp Gln Leu Pro Asn Leu Asn
   1               5                   10                  15
   Gln Arg Gln Lys Ala Ala Phe Ile Gly Ser Leu Ala Asp
               20                  25
<210> 42
   <211> 29
   <212> PRT
   <213> Artificial Sequence
<220>
   <223> Engineered polypeptide
<400> 42
   Glu Lys Tyr Val Ala Tyr Gly Glu Ile Trp Gln Leu Pro Asn Leu Asn
   1               5                   10                  15
   Arg Thr Gln Lys Ala Ala Phe Ile Ser Ser Leu Ser Asp
               20                  25
<210> 43
   <211> 29
   <212> PRT
   <213> Artificial Sequence
<220>
   <223> Engineered polypeptide
<400> 43
   Glu Arg Tyr Ile Ala Tyr Tyr Glu Ile Trp Gln Leu Pro Asn Leu Asn
   1 5 10 15
   Arg Tyr Gln Lys Ala Ala Phe Ile Ala Ser Leu Ser Asp
   20 25
<210> 44
   <211> 29
   <212> PRT
   <213> Artificial Sequence
<220>
   <223> Engineered polypeptide
<400> 44
   Glu Lys Tyr Asn Ala Tyr Gly Glu Ile Trp Gln Leu Pro Asn Leu Asn
   1 5 10 15
   Val Arg Gln Lys Ala Ala Phe Ile Ser Ser Leu Ala Asp
   20 25
<210> 45
   <211> 29
   <212> PRT
   <213> Artificial Sequence
<220>
   <223> Engineered polypeptide
<400> 45
   Glu Arg Lys Ala Ala Thr Val Glu Ile Trp Glu Leu Pro Asn Leu Asn
   1 5 10 15
   Arg Val Gln Lys Ala Ala Phe Ile Lys Ser Leu Phe Asp
   20 25
<210> 46
   <211> 29
   <212> PRT
   <213> Artificial Sequence
<220>
   <223> Engineered polypeptide
<400> 46
   Glu Arg Tyr Val Ala Tyr Tyr Glu Ile Trp Glu Leu Pro Asn Leu Asn
   1 5 10 15
   Gln Arg Gln Lys Ala Ala Phe Ile Gly Ser Leu Gln Asp
   20 25
<210> 47
   <211> 29
   <212> PRT
   <213> Artificial Sequence
   <220>
   <223> Engineered polypeptide
<400> 47
   Glu Arg Tyr Arg Ala Tyr Tyr Glu Ile Trp Gln Leu Pro Asn Leu Asn
   1               5                   10                  15
   Gln Arg Gln Lys Ala Ala Phe Ile Gly Ser Leu Ser Asp
               20                  25
<210> 48
   <211> 29
   <212> PRT
   <213> Artificial Sequence
<220>
   <223> Engineered polypeptide
<400> 48
   Glu Trp Lys Ser Ala Ala Phe Glu Ile Trp Glu Leu Pro Asn Leu Asn
   1               5                   10                  15
   Arg Leu Gln Lys Ala Ala Phe Ile Arg Ser Leu Ser Asp
               20                  25
<210> 49
   <211> 29
   <212> PRT
   <213> Artificial Sequence
<220>
   <223> Engineered polypeptide
<400> 49
   Glu Arg Lys Gln Ala Thr Phe Glu Ile Trp Glu Leu Pro Asn Leu Asn
   1               5                   10                  15
   Arg His Gln Lys Gly Ala Phe Ile Ala Ser Leu Trp Asp
               20                  25
<210> 50
   <211> 29
   <212> PRT
   <213> Artificial Sequence
<220>
   <223> Engineered polypeptide
<400> 50
   Glu Trp Lys Ile Ala Ala Gly Glu Ile Trp Gln Leu Pro Asn Leu Asn
   1               5                   10                  15
   Arg Arg Gln Lys Ala Ala Phe Ile Ala Ser Leu Ser Asp
               20                  25
<210> 51
   <211> 29
   <212> PRT
   <213> Artificial Sequence
<220>
   <223> Engineered polypeptide
<400> 51
   Glu Arg Tyr Leu Ala Tyr Tyr Glu Ile Trp Gln Leu Pro Asn Leu Asn
   1               5                   10                  15
   Arg Arg Gln Lys Ala Ala Phe Ile Gly Ser Leu Gln Asp
               20                  25
<210> 52
   <211> 29
   <212> PRT
   <213> Artificial Sequence
<220>
   <223> Engineered polypeptide
<400> 52
   Glu Arg Tyr His Ala Tyr Tyr Glu Ile Trp Glu Leu Pro Asn Leu Asn
   1               5                   10                  15
   Arg Leu Gln Lys Ala Ala Phe Ile Ser Ser Leu Ser Asp
               20                  25
<210> 53
   <211> 29
   <212> PRT
   <213> Artificial Sequence
<220>
   <223> Engineered polypeptide
<400> 53
   Glu Lys Tyr Lys Ala Tyr Ala Glu Ile Trp Gln Leu Pro Asn Leu Asn
   1 5 10 15
   Arg Thr Gln Lys Ala Ala Phe Ile Ser Ser Leu Gln Asp
   20 25
<210> 54
   <211> 29
   <212> PRT
   <213> Artificial Sequence
<220>
   <223> Engineered polypeptide
<400> 54
   Glu Arg Tyr Lys Ala Tyr Tyr Glu Ile Trp Gln Leu Pro Asn Leu Asn
   1 5 10 15
   Arg Gln Gln Lys Gly Ala Phe Ile Ser Ser Leu Gln Asp
   20 25
<210> 55
   <211> 29
   <212> PRT
   <213> Artificial Sequence
<220>
   <223> Engineered polypeptide
<400> 55
   Glu Arg Lys Leu Ala Thr Phe Glu Ile Trp Glu Leu Pro Asn Leu Asn
   1 5 10 15
   Thr Ser Gln Lys Gly Ala Phe Ile Ser Ser Leu Trp Asp
   20 25
<210> 56
   <211> 29
   <212> PRT
   <213> Artificial Sequence
<220>
   <223> Engineered polypeptide
<400> 56
   Glu Arg Tyr Val Ala Tyr Tyr Glu Ile Trp Gln Leu Pro Asn Leu Asn
   1 5 10 15
   Val Gln Gln Lys Ala Ala Phe Ile Ala Ser Leu Gln Asp
   20 25
<210> 57
   <211> 29
   <212> PRT
   <213> Artificial Sequence
<220>
   <223> Engineered polypeptide
<400> 57
   Glu Arg Ser Ile Ala Ser Val Glu Ile Trp Glu Leu Pro Asn Leu Asn
   1               5                   10                  15
   Gln Arg Gln Lys Gly Ala Phe Ile Ala Ser Leu Trp Asp
               20                  25
<210> 58
   <211> 29
   <212> PRT
   <213> Artificial Sequence
<220>
   <223> Engineered polypeptide
<400> 58
   Glu Arg Lys Gln Ala Ala Val Glu Ile Trp Glu Leu Pro Asn Leu Asn
   1               5                   10                  15
   Val Arg Gln Lys Ala Ala Phe Ile Arg Ser Leu Phe Asp
               20                  25
<210> 59
   <211> 29
   <212> PRT
   <213> Artificial Sequence
<220>
   <223> Engineered polypeptide
<400> 59
   Glu Lys Lys Ile Ala Ala Phe Glu Ile Trp Glu Leu Pro Asn Leu Asn
   1               5                   10                  15
   Gln Trp Gln Lys Ala Ala Phe Ile Ala Ser Leu Phe Asp
               20                  25
<210> 60
   <211> 29
   <212> PRT
   <213> Artificial Sequence
<220>
   <223> Engineered polypeptide
<400> 60
   Glu Arg Lys Ile Ala Thr Val Glu Ile Trp Glu Leu Pro Asn Leu Asn
   1               5                   10                  15
   Arg Ile Gln Lys Gly Ala Phe Ile Ala Ser Leu Trp Asp
               20                  25
<210> 61
   <211> 29
   <212> PRT
   <213> Artificial Sequence
<220>
   <223> Engineered polypeptide
<400> 61
   Glu Lys Tyr Arg Ala Ala Gly Glu Ile Trp Glu Leu Pro Asn Leu Asn
   1               5                   10                  15
   Val Leu Gln Lys Ala Ala Phe Ile Lys Ser Leu Trp Asp
               20                  25
<210> 62
   <211> 29
   <212> PRT
   <213> Artificial Sequence
<220>
   <223> Engineered polypeptide
<400> 62
   Glu Arg Lys Ile Ala Ala Val Glu Ile Trp Glu Leu Pro Asn Leu Asn
   1               5                   10                  15
   Arg Tyr Gln Lys Ala Ala Phe Ile Lys Ser Leu Phe Asp
               20                  25
<210> 63
   <211> 29
   <212> PRT
   <213> Artificial Sequence
<220>
   <223> Engineered polypeptide
<400> 63
   Glu Lys Arg Thr Ala Thr Trp Glu Ile Trp Gln Leu Pro Asn Leu Asn
   1               5                   10                  15
   Gln Arg Gln Arg Ala Ala Phe Ile Gly Ser Leu Trp Asp
               20                  25
<210> 64
   <211> 29
   <212> PRT
   <213> Artificial Sequence
<220>
   <223> Engineered polypeptide
<400> 64
   Glu Arg Lys Thr Ala Thr Val Glu Ile Trp Glu Leu Pro Asn Leu Asn
   1               5                   10                  15
   Arg Arg Gln Lys Ala Ala Phe Ile Ala Ser Leu Trp Asp
               20                  25
<210> 65
   <211> 29
   <212> PRT
   <213> Artificial Sequence
<220>
   <223> Engineered polypeptide
<400> 65
   Glu Arg Lys Gln Ala Ala Val Glu Ile Trp Glu Leu Pro Asn Leu Asn
   1               5                   10                  15
   Arg Ile Gln Lys Gly Ala Phe Ile Lys Ser Leu Trp Asp
               20                  25
<210> 66
   <211> 29
   <212> PRT
   <213> Artificial Sequence
<220>
   <223> Engineered polypeptide
<400> 66
   Glu Arg Tyr Ile Ala Tyr Gly Glu Ile Trp Gln Leu Pro Asn Leu Asn
   1               5                   10                  15
   Arg Arg Gln Lys Ala Ala Phe Ile Gly Ser Leu Ser Asp
               20                  25
<210> 67
   <211> 29
   <212> PRT
   <213> Artificial Sequence
<220>
   <223> Engineered polypeptide
<400> 67
   Glu Arg Tyr Arg Ala Tyr Tyr Glu Ile Trp Gln Leu Pro Asn Leu Asn
   1               5                   10                  15
   Arg Thr Gln Lys Val Ala Phe Ile Gly Ser Leu Gln Asp
               20                  25
<210> 68
   <211> 29
   <212> PRT
   <213> Artificial Sequence
<220>
   <223> Engineered polypeptide
<400> 68
   Glu Arg Arg Leu Ala Tyr Trp Glu Ile Trp Gln Leu Pro Asn Leu Asn
   1               5                   10                  15
   Thr Thr Gln Lys Ala Ala Phe Ile Gly Ser Leu Gln Asp
               20                  25
<210> 69
   <211> 29
   <212> PRT
   <213> Artificial Sequence
<220>
   <223> Engineered polypeptide
<400> 69
   Glu Arg Arg Arg Ala Tyr Tyr Glu Ile Trp Gln Leu Pro Asn Leu Asn
   1               5                   10                  15
   Arg Ile Gln Lys Ala Ala Phe Ile Gly Ser Leu Gln Asp
               20                  25
<210> 70
   <211> 29
   <212> PRT
   <213> Artificial Sequence
<220>
   <223> Engineered polypeptide
<400> 70
   Glu Arg Arg Leu Ala Tyr Tyr Glu Ile Trp Glu Leu Pro Asn Leu Asn
   1 5 10 15
   Val Ala Gln Lys Ala Ala Phe Ile Gly Ser Leu Gln Asp
   20 25
<210> 71
   <211> 29
   <212> PRT
   <213> Artificial Sequence
<220>
   <223> Engineered polypeptide
<400> 71
   Glu Arg Tyr Leu Ala Tyr Tyr Glu Ile Trp Glu Leu Pro Asn Leu Asn
   1 5 10 15
   Val Arg Gln Lys Ala Ala Phe Ile Gly Ser Leu Gln Asp
   20 25
<210> 72
   <211> 29
   <212> PRT
   <213> Artificial Sequence
<220>
   <223> Engineered polypeptide
<400> 72
   Glu Arg Tyr Ala Ala Tyr Tyr Glu Ile Trp Gln Leu Pro Asn Leu Asn
   1 5 10 15
   Ser Arg Gln Lys Ala Ala Phe Ile Gly Ser Leu Ser Asp
   20 25
<210> 73
   <211> 29
   <212> PRT
   <213> Artificial Sequence
<220>
   <223> Engineered polypeptide
<400> 73
   Glu Arg Tyr Leu Ala Tyr Tyr Glu Ile Trp Gln Leu Pro Asn Leu Asn
   1 5 10 15
   Gln Leu Gln Lys Ala Ala Phe Ile Gly Ser Leu Ala Asp
   20 25
<210> 74
   <211> 29
   <212> PRT
   <213> Artificial Sequence
<220>
   <223> Engineered polypeptide
<400> 74
   Glu Arg Tyr His Ala Tyr Tyr Glu Ile Trp Gln Leu Pro Asn Leu Asn
   1               5                   10                  15
   Ser Trp Gln Lys Ala Ala Phe Ile Gly Ser Leu Gln Asp
               20                  25
<210> 75
   <211> 29
   <212> PRT
   <213> Artificial Sequence
<220>
   <223> Engineered polypeptide
<400> 75
   Glu Arg Tyr His Ala Tyr Tyr Glu Ile Trp Gln Leu Pro Asn Leu Asn
   1               5                   10                  15
   Ser Ala Gln Lys Ala Ala Phe Ile Gly Ser Leu Gln Asp
               20                  25
<210> 76
   <211> 29
   <212> PRT
   <213> Artificial Sequence
<220>
   <223> Engineered polypeptide
<400> 76
   Glu Arg Tyr Glu Ala Tyr Tyr Glu Ile Trp Gln Leu Pro Asn Leu Asn
   1               5                   10                  15
   Ser Val Gln Lys Ala Ala Phe Ile Gly Ser Leu Gln Asp
               20                  25
<210> 77
   <211> 29
   <212> PRT
   <213> Artificial Sequence
<220>
   <223> Engineered polypeptide
<400> 77
   Glu Arg Tyr His Ala Tyr Tyr Glu Ile Trp Gln Leu Pro Asn Leu Asn
   1 5 10 15
   Arg Arg Gln Lys Ala Ala Phe Ile Gly Ser Leu Gln Asp
   20 25
<210> 78
   <211> 29
   <212> PRT
   <213> Artificial Sequence
<220>
   <223> Engineered polypeptide
<400> 78
   Glu Arg Tyr Thr Ala Tyr Tyr Glu Ile Trp Gln Leu Pro Asn Leu Asn
   1 5 10 15
   Arg Ala Gln Lys Ala Ala Phe Ile Gly Ser Leu Gln Asp
   20 25
<210> 79
   <211> 29
   <212> PRT
   <213> Artificial Sequence
<220>
   <223> Engineered polypeptide
<400> 79
   Glu Arg Tyr Ser Ala Tyr Tyr Glu Ile Trp Gln Leu Pro Asn Leu Asn
   1 5 10 15
   Arg Ala Gln Lys Ala Ala Phe Ile Gly Ser Leu Ser Asp
   20 25
<210> 80
   <211> 29
   <212> PRT
   <213> Artificial Sequence
<220>
   <223> Engineered polypeptide
<400> 80
   Glu Arg Tyr Ser Ala Tyr Tyr Glu Ile Trp Glu Leu Pro Asn Leu Asn
   1 5 10 15
   Arg Ala Gln Lys Ala Ala Phe Ile Gly Ser Leu Ser Asp
   20 25
<210> 81
   <211> 29
   <212> PRT
   <213> Artificial Sequence
<220>
   <223> Engineered polypeptide
<400> 81
   Glu Arg Tyr His Ala Tyr Tyr Glu Ile Trp Gln Leu Pro Asn Leu Asn
   1               5                   10                  15
   Arg Ala Gln Lys Ala Ala Phe Ile Gly Ser Leu Tyr Asp
               20                  25
<210> 82
   <211> 29
   <212> PRT
   <213> Artificial Sequence
<220>
   <223> Engineered polypeptide
<400> 82
   Glu Arg Tyr Ser Ala Tyr Val Glu Ile Trp Gln Leu Pro Asn Leu Asn
   1               5                   10                  15
   Arg Gly Gln Lys Ala Ala Phe Ile Gly Ser Leu Tyr Asp
               20                  25
<210> 83
   <211> 29
   <212> PRT
   <213> Artificial Sequence
<220>
   <223> Engineered polypeptide
<400> 83
   Glu Arg Tyr Ser Ala Tyr Phe Glu Ile Trp Gln Leu Pro Asn Leu Asn
   1               5                   10                  15
   Arg Leu Gln Lys Ala Ala Phe Ile Gly Ser Leu Gln Asp
               20                  25
<210> 84
   <211> 29
   <212> PRT
   <213> Artificial Sequence
<220>
   <223> Engineered polypeptide
<400> 84
   Glu Arg Tyr Asn Ala Tyr Phe Glu Ile Trp Gln Leu Pro Asn Leu Asn
   1               5                   10                  15
   Arg Tyr Gln Lys Ser Ala Phe Ile Gly Ser Leu Gln Asp
               20                  25
<210> 85
   <211> 29
   <212> PRT
   <213> Artificial Sequence
<220>
   <223> Engineered polypeptide
<400> 85
   Glu Arg Tyr Ala Ala Tyr Phe Glu Ile Trp Gln Leu Pro Asn Leu Asn
   1               5                   10                  15
   Val Ala Gln Lys Ala Ala Phe Ile Gly Ser Leu Gln Asp
               20                  25
<210> 86
   <211> 29
   <212> PRT
   <213> Artificial Sequence
<220>
   <223> Engineered polypeptide
<400> 86
   Glu Arg Tyr Gln Ala Tyr Phe Glu Ile Trp Gln Leu Pro Asn Leu Asn
   1               5                   10                  15
   Gln Trp Gln Lys Ala Ala Phe Ile Gly Ser Leu Gln Asp
               20                  25
<210> 87
   <211> 29
   <212> PRT
   <213> Artificial Sequence
<220>
   <223> Engineered polypeptide
<400> 87
   Glu Arg Tyr Lys Ala Tyr Trp Glu Ile Trp Gln Leu Pro Asn Leu Asn
   1 5 10 15
   Gln Gly Gln Lys Ala Ala Phe Ile Gly Ser Leu Gln Asp
   20 25
<210> 88
   <211> 29
   <212> PRT
   <213> Artificial Sequence
<220>
   <223> Engineered polypeptide
<400> 88
   Glu Arg Tyr Ile Ala Tyr Gly Glu Ile Trp Gln Leu Pro Asn Leu Asn
   1 5 10 15
   Val Gly Gln Lys Ala Ala Phe Ile Gly Ser Leu Gln Asp
   20 25
<210> 89
   <211> 29
   <212> PRT
   <213> Artificial Sequence
<220>
   <223> Engineered polypeptide
<400> 89
   Glu Arg Tyr Arg Ala Tyr Val Glu Ile Trp Gln Leu Pro Asn Leu Asn
   1 5 10 15
   Arg Val Gln Lys Ala Ala Phe Ile Gly Ser Leu Gln Asp
   20 25
<210> 90
   <211> 29
   <212> PRT
   <213> Artificial Sequence
<220>
   <223> Engineered polypeptide
<400> 90
   Glu Arg Tyr Lys Ala Tyr Val Glu Ile Trp Gln Leu Pro Asn Leu Asn
   1 5 10 15
   Arg Thr Gln Lys Ala Ala Phe Ile Gly Ser Leu Gln Asp
   20 25
<210> 91
   <211> 29
   <212> PRT
   <213> Artificial Sequence
<220>
   <223> Engineered polypeptide
<400> 91
   Glu Lys Tyr Glu Ala Tyr Gly Glu Ile Trp Glu Leu Pro Asn Leu Asn
   1 5 10 15
   Arg Ser Gln Lys Ala Ala Phe Ile Gly Ser Leu Glu Asp
   20 25
<210> 92
   <211> 29
   <212> PRT
   <213> Artificial Sequence
<220>
   <223> Engineered polypeptide
<400> 92
   Glu Lys Tyr Gln Ala Tyr Gly Glu Ile Trp Gln Leu Pro Asn Leu Asn
   1 5 10 15
   Arg Ser Gln Lys Ala Ala Phe Ile Thr Ser Leu Glu Asp
   20 25
<210> 93
   <211> 29
   <212> PRT
   <213> Artificial Sequence
<220>
   <223> Engineered polypeptide
<400> 93
   Glu Lys Tyr Ala Ala Tyr Gly Glu Ile Trp Leu Leu Pro Asn Leu Asn
   1 5 10 15
   Arg Ile Gln Lys Ala Ala Phe Ile Gly Ser Leu Gln Asp
   20 25
<210> 94
   <211> 29
   <212> PRT
   <213> Artificial Sequence
   <220>
   <223> Engineered polypeptide
<400> 94
   Glu Lys Tyr Lys Ala Tyr Gly Glu Ile Trp Glu Leu Pro Asn Leu Asn
   1               5                   10                  15
   Arg Arg Gln Lys Val Ala Phe Ile Ser Ser Leu Ser Asp
               20                  25
<210> 95
   <211> 29
   <212> PRT
   <213> Artificial Sequence
<220>
   <223> Engineered polypeptide
<400> 95
   Glu Lys Tyr Lys Ala Tyr Gly Glu Ile Trp Glu Leu Pro Asn Leu Asn
   1               5                   10                  15
   Arg Ser Gln Lys Ala Ala Phe Ile Ala Ser Leu Ser Asp
               20                  25
<210> 96
   <211> 29
   <212> PRT
   <213> Artificial Sequence
<220>
   <223> Engineered polypeptide
<400> 96
   Glu Lys Tyr Lys Ala Tyr Gly Glu Ile Trp Gln Leu Pro Asn Leu Asn
   1               5                   10                  15
   Gln Arg Gln Lys Ala Ala Phe Ile Ala Ser Leu Ser Asp
               20                  25
<210> 97
   <211> 29
   <212> PRT
   <213> Artificial Sequence
<220>
   <223> Engineered polypeptide
<400> 97
   Glu Lys Tyr Lys Ala Tyr Gly Glu Ile Trp Gln Leu Pro Asn Leu Asn
   1 5 10 15
   Arg Ser Gln Lys Ala Ala Phe Ile Ser Ser Leu Gln Asp
   20 25
<210> 98
   <211> 29
   <212> PRT
   <213> Artificial Sequence
<220>
   <223> Engineered polypeptide
<400> 98
   Glu Lys Tyr Lys Ala Tyr Gly Glu Ile Trp Gln Leu Pro Asn Leu Asn
   1 5 10 15
   Arg Arg Gln Lys Ala Ala Phe Ile Ser Ser Leu Ala Asp
   20 25
<210> 99
   <211> 29
   <212> PRT
   <213> Artificial Sequence
<220>
   <223> Engineered polypeptide
<400> 99
   Glu Lys Tyr Asn Ala Tyr Gly Glu Ile Trp Gln Leu Pro Asn Leu Asn
   1 5 10 15
   Arg Gly Gln Lys Ala Ala Phe Ile Ser Ser Leu Ser Asp
   20 25
<210> 100
   <211> 29
   <212> PRT
   <213> Artificial Sequence
<220>
   <223> Engineered polypeptide
<400> 100
   Glu Lys Tyr Val Ala Tyr Ala Glu Ile Trp Gln Leu Pro Asn Leu Asn
   1 5 10 15
   Arg Val Gln Lys Ala Ala Phe Ile Gly Ser Leu Ser Asp
   20 25
<210> 101
   <211> 29
   <212> PRT
   <213> Artificial Sequence
<220>
   <223> Engineered polypeptide
<400> 101
   Glu Lys Tyr Gln Ala Tyr Gly Glu Ile Trp Gln Leu Pro Asn Leu Asn
   1 5 10 15
   Arg Val Gln Lys Ala Ala Phe Ile Gly Ser Leu Ser Asp
   20 25
<210> 102
   <211> 29
   <212> PRT
   <213> Artificial Sequence
<220>
   <223> Engineered polypeptide
<400> 102
   Glu Lys Tyr His Ala Tyr Gly Glu Ile Trp Gln Leu Pro Asn Leu Asn
   1 5 10 15
   Arg Ile Gln Lys Ala Ala Phe Ile Ala Ser Leu Gln Asp
   20 25
<210> 103
   <211> 29
   <212> PRT
   <213> Artificial Sequence
<220>
   <223> Engineered polypeptide
<400> 103
   Glu Lys Tyr Thr Ala Tyr Val Glu Ile Trp Gln Leu Pro Asn Leu Asn
   1 5 10 15
   Arg Tyr Gln Arg Ala Ala Phe Ile Ala Ser Leu Ser Asp
   20 25
<210> 104
   <211> 29
   <212> PRT
   <213> Artificial Sequence
<220>
   <223> Engineered polypeptide
<400> 104
   Glu Lys Tyr Lys Ala Tyr Val Glu Ile Trp Gln Leu Pro Asn Leu Asn
   1               5                   10                  15
   Arg Gly Gln Lys Ala Ala Phe Ile Gly Ser Leu Ser Asp
               20                  25
<210> 105
   <211> 29
   <212> PRT
   <213> Artificial Sequence
<220>
   <223> Engineered polypeptide
<400> 105
   Glu Lys Tyr Val Ala Tyr Val Glu Ile Trp Gln Leu Pro Asn Leu Asn
   1               5                   10                  15
   Arg Thr Gln Lys Ala Ala Phe Ile Ala Ser Leu Glu Asp
               20                  25
<210> 106
   <211> 29
   <212> PRT
   <213> Artificial Sequence
<220>
   <223> Engineered polypeptide
<400> 106
   Glu Lys Tyr Ile Ala Tyr Val Glu Ile Trp Gln Leu Pro Asn Leu Asn
   1               5                   10                  15
   Arg Thr Gln Lys Ala Ala Phe Ile Ala Ser Leu Ser Asp
               20                  25
<210> 107
   <211> 29
   <212> PRT
   <213> Artificial Sequence
<220>
   <223> Engineered polypeptide
<400> 107
   Glu Lys Tyr Lys Ala Tyr Ala Glu Ile Trp Gln Leu Pro Asn Leu Asn
   1               5                   10                  15
   Arg Ala Gln Lys Ala Ala Phe Ile Gly Ser Leu Tyr Asp
               20                  25
<210> 108
   <211> 29
   <212> PRT
   <213> Artificial Sequence
<220>
   <223> Engineered polypeptide
<400> 108
   Glu Lys Tyr Thr Ala Tyr Gly Glu Ile Trp Gln Leu Pro Asn Leu Asn
   1               5                   10                  15
   Val Arg Gln Lys Ala Ala Phe Ile Ser Ser Leu Ser Asp
               20                  25
<210> 109
   <211> 29
   <212> PRT
   <213> Artificial Sequence
<220>
   <223> Engineered polypeptide
<400> 109
   Glu Lys Tyr Thr Ala Tyr Gly Glu Ile Trp Gln Leu Pro Asn Leu Asn
   1               5                   10                  15
   Gln Arg Gln Lys Ala Ala Phe Ile Ser Ser Leu Gln Asp
               20                  25
<210> 110
   <211> 29
   <212> PRT
   <213> Artificial Sequence
<220>
   <223> Engineered polypeptide
<400> 110
   Glu Lys Tyr Thr Ala Tyr Phe Glu Ile Trp Gln Leu Pro Asn Leu Asn
   1               5                   10                  15
   Gln Gly Gln Lys Ala Ala Phe Ile Gly Ser Leu Gln Asp
               20                  25
<210> 111
   <211> 29
   <212> PRT
   <213> Artificial Sequence
<220>
   <223> Engineered polypeptide
<400> 111
   Glu Lys Tyr Gln Ala Tyr Val Glu Ile Trp Gln Leu Pro Asn Leu Asn
   1 5 10 15
   Gln Arg Gln Lys Ala Ala Phe Ile Gly Ser Leu Ala Asp
   20 25
<210> 112
   <211> 29
   <212> PRT
   <213> Artificial Sequence
   <220>
   <223> Engineered polypeptide
<400> 112
   Glu Lys Tyr Ala Ala Tyr Val Glu Ile Trp Gln Leu Pro Asn Leu Asn
   1 5 10 15
   Gln Arg Gln Lys Ala Ala Phe Ile Gly Ser Leu Gln Asp
   20 25
<210> 113
   <211> 29
   <212> PRT
   <213> Artificial Sequence
<220>
   <223> Engineered polypeptide
<400> 113
   Glu Lys Tyr Val Ala Tyr Val Glu Ile Trp Gln Leu Pro Asn Leu Asn
   1 5 10 15
   Thr Ser Gln Lys Ala Ala Phe Ile Gly Ser Leu Gln Asp
   20 25
<210> 114
   <211> 29
   <212> PRT
   <213> Artificial Sequence
<220>
   <223> Engineered polypeptide
<400> 114
   Glu Lys Tyr Gln Ala Tyr Ala Glu Ile Trp Gln Leu Pro Asn Leu Asn
   1               5                   10                  15
   Val Arg Gln Lys Ser Ala Phe Ile Gly Ser Leu Gln Asp
               20                  25
<210> 115
   <211> 29
   <212> PRT
   <213> Artificial Sequence
<220>
   <223> Engineered polypeptide
<400> 115
   Glu Lys Tyr Gln Ala Tyr Tyr Glu Ile Trp Glu Leu Pro Asn Leu Asn
   1               5                   10                  15
   Val Gln Gln Lys Ser Ala Phe Ile Thr Ser Leu Gln Asp
               20                  25
<210> 116
   <211> 29
   <212> PRT
   <213> Artificial Sequence
<220>
   <223> Engineered polypeptide
<400> 116
   Glu Lys Tyr His Ala Tyr Tyr Glu Ile Trp Gln Leu Pro Asn Leu Asn
   1               5                   10                  15
   Val His Gln Lys Ala Ala Phe Ile Arg Ser Leu Ala Asp
               20                  25
<210> 117
   <211> 29
   <212> PRT
   <213> Artificial Sequence
<220>
   <223> Engineered polypeptide
<400> 117
   Glu Lys Tyr Gln Ala Tyr Tyr Glu Ile Trp Gln Leu Pro Asn Leu Asn
   1 5 10 15
   Val Ala Gln Lys Ala Ala Phe Ile Arg Ser Leu Ser Asp
   20 25
<210> 118
   <211> 29
   <212> PRT
   <213> Artificial Sequence
<220>
   <223> Engineered polypeptide
<400> 118
   Glu Lys Tyr Thr Ala Tyr Tyr Glu Ile Trp Gln Leu Pro Asn Leu Asn
   1 5 10 15
   Arg Thr Gln Lys Ala Ala Phe Ile Gly Ser Leu Ser Asp
   20 25
<210> 119
   <211> 29
   <212> PRT
   <213> Artificial Sequence
<220>
   <223> Engineered polypeptide
<400> 119
   Glu Arg Tyr Arg Ala Tyr Tyr Glu Ile Trp Gln Leu Pro Asn Leu Asn
   1 5 10 15
   Arg Gln Gln Lys Gly Ala Phe Ile Ala Ser Leu Gln Asp
   20 25
<210> 120
   <211> 29
   <212> PRT
   <213> Artificial Sequence
<220>
   <223> Engineered polypeptide
<400> 120
   Glu Lys Tyr Lys Ala Tyr Tyr Glu Ile Trp Gln Leu Pro Asn Leu Asn
   1 5 10 15
   Arg Ser Gln Lys Gly Ala Phe Ile Gly Ser Leu Gln Asp
   20 25
<210> 121
   <211> 29
   <212> PRT
   <213> Artificial Sequence
<220>
   <223> Engineered polypeptide
<400> 121
   Glu Lys Tyr Ile Ala Tyr Tyr Glu Ile Trp Gln Leu Pro Asn Leu Asn
   1               5                   10                  15
   Arg Ser Gln Lys Gly Ala Phe Ile Gly Ser Leu Gln Asp
               20                  25
<210> 122
   <211> 29
   <212> PRT
   <213> Artificial Sequence
<220>
   <223> Engineered polypeptide
<400> 122
   Glu Arg Tyr Lys Ala Tyr Tyr Glu Ile Trp Gln Leu Pro Asn Leu Asn
   1               5                   10                  15
   Arg Gln Gln Lys Ala Ala Phe Ile Gly Ser Leu Ala Asp
               20                  25
<210> 123
   <211> 29
   <212> PRT
   <213> Artificial Sequence
<220>
   <223> Engineered polypeptide
<400> 123
   Glu Arg Tyr Leu Ala Tyr Tyr Glu Ile Trp Gln Leu Pro Asn Leu Asn
   1               5                   10                  15
   Gln Phe Gln Lys Ala Ala Phe Ile Ala Ser Leu Gln Asp
               20                  25
<210> 124
   <211> 29
   <212> PRT
   <213> Artificial Sequence
<220>
   <223> Engineered polypeptide
<400> 124
   Glu Arg Tyr Leu Ala Tyr Tyr Glu Ile Trp Gln Leu Pro Asn Leu Asn
   1               5                   10                  15
   Arg Glu Gln Lys Ala Ala Phe Ile Ala Ser Leu Gln Asp
               20                  25
<210> 125
   <211> 29
   <212> PRT
   <213> Artificial Sequence
<220>
   <223> Engineered polypeptide
<400> 125
   Glu Arg Tyr Val Ala Tyr Gly Glu Ile Trp Gln Leu Pro Asn Leu Asn
   1               5                   10                  15
   Arg Tyr Gln Lys Ala Ala Phe Ile Ala Ser Leu Gln Asp
               20                  25
<210> 126
   <211> 29
   <212> PRT
   <213> Artificial Sequence
<220>
   <223> Engineered polypeptide
<400> 126
   Glu Arg Tyr Ile Ala Tyr Tyr Glu Ile Trp Gln Leu Pro Asn Leu Asn
   1               5                   10                  15
   Arg Ala Gln Lys Ala Ala Phe Ile Ala Ser Leu Gln Asp
               20                  25
<210> 127
   <211> 29
   <212> PRT
   <213> Artificial Sequence
<220>
   <223> Engineered polypeptide
<400> 127
   Glu Arg Tyr Gln Ala Tyr Tyr Glu Ile Trp Gln Leu Pro Asn Leu Asn
   1               5                   10                  15
   Arg Ile Gln Lys Ala Ala Phe Ile Lys Ser Leu Gln Asp
               20                  25
<210> 128
   <211> 29
   <212> PRT
   <213> Artificial Sequence
<220>
   <223> Engineered polypeptide
<400> 128
   Glu Arg Tyr Thr Ala Tyr Tyr Glu Ile Trp Gln Leu Pro Asn Leu Asn
   1               5                   10                  15
   Thr Gln Gln Lys Ala Ala Phe Ile Arg Ser Leu Glu Asp
               20                  25
<210> 129
   <211> 29
   <212> PRT
   <213> Artificial Sequence
<220>
   <223> Engineered polypeptide
<400> 129
   Glu Arg Tyr Gln Ala Tyr Tyr Glu Ile Trp Gln Leu Pro Asn Leu Asn
   1               5                   10                  15
   Thr Ile Gln Lys Ala Ala Phe Ile Arg Ser Leu Gln Asp
               20                  25
<210> 130
   <211> 29
   <212> PRT
   <213> Artificial Sequence
<220>
   <223> Engineered polypeptide
<400> 130
   Glu Arg Tyr Ala Ala Tyr Ala Glu Ile Trp Gln Leu Pro Asn Leu Asn
   1               5                   10                  15
   Arg Trp Gln Lys Ala Ala Phe Ile Ser Ser Leu Gln Asp
               20                  25
<210> 131
   <211> 29
   <212> PRT
   <213> Artificial Sequence
<220>
   <223> Engineered polypeptide
<400> 131
   Glu Arg Tyr Arg Ala Tyr Ala Glu Ile Trp Gln Leu Pro Asn Leu Asn
   1               5                   10                  15
   Arg Trp Gln Lys Ala Ala Phe Ile Ala Ser Leu Gln Asp
               20                  25
<210> 132
   <211> 29
   <212> PRT
   <213> Artificial Sequence
<220>
   <223> Engineered polypeptide
<400> 132
   Glu Arg Tyr Arg Ala Tyr Ala Glu Ile Trp Gln Leu Pro Asn Leu Asn
   1               5                   10                  15
   Arg Ile Gln Lys Ala Ala Phe Ile Gly Ser Leu Glu Asp
               20                  25
<210> 133
   <211> 29
   <212> PRT
   <213> Artificial Sequence
<220>
   <223> Engineered polypeptide
<400> 133
   Glu Arg Tyr His Ala Tyr Ala Glu Ile Trp Gln Leu Pro Asn Leu Asn
   1               5                   10                  15
   Arg Ile Gln Lys Ala Ala Phe Ile Gly Ser Leu Gln Asp
               20                  25
<210> 134
   <211> 29
   <212> PRT
   <213> Artificial Sequence
<220>
   <223> Engineered polypeptide
<400> 134
   Glu Arg Tyr Arg Ala Tyr Tyr Glu Ile Trp Gln Leu Pro Asn Leu Asn
   1 5 10 15
   Arg Tyr Gln Lys Ala Ala Phe Ile Ala Ser Leu Ser Asp
   20 25
<210> 135
   <211> 29
   <212> PRT
   <213> Artificial Sequence
<220>
   <223> Engineered polypeptide
<400> 135
   Glu Arg Tyr Thr Ala Tyr Tyr Glu Ile Trp Gln Leu Pro Asn Leu Asn
   1 5 10 15
   Arg Val Gln Lys Ala Ala Phe Ile Ala Ser Leu Ser Asp
   20 25
<210> 136
   <211> 29
   <212> PRT
   <213> Artificial Sequence
<220>
   <223> Engineered polypeptide
<400> 136
   Glu Arg Tyr His Ala Tyr Tyr Glu Ile Trp Gln Leu Pro Asn Leu Asn
   1 5 10 15
   Arg Thr Gln Lys Ala Ala Phe Ile Ala Ser Leu Ser Asp
   20 25
<210> 137
   <211> 29
   <212> PRT
   <213> Artificial Sequence
<220>
   <223> Engineered polypeptide
<400> 137
   Glu Arg Tyr Val Ala Tyr Tyr Glu Ile Trp Gln Leu Pro Asn Leu Asn
   1 5 10 15
   Arg Arg Gln Lys Ala Ala Phe Ile Ser Ser Leu Ser Asp
   20 25
<210> 138
   <211> 29
   <212> PRT
   <213> Artificial Sequence
<220>
   <223> Engineered polypeptide
<400> 138
   Glu Arg Tyr Leu Ala Tyr Phe Glu Ile Trp Gln Leu Pro Asn Leu Asn
   1               5                   10                  15
   Arg His Gln Lys Gly Ala Phe Ile Ala Ser Leu Glu Asp
               20                  25
<210> 139
   <211> 29
   <212> PRT
   <213> Artificial Sequence
<220>
   <223> Engineered polypeptide
<400> 139
   Glu Arg Tyr Thr Ala Tyr Phe Glu Ile Trp Gln Leu Pro Asn Leu Asn
   1               5                   10                  15
   Arg Trp Gln Lys Gly Ala Phe Ile Ala Ser Leu Glu Asp
               20                  25
<210> 140
   <211> 29
   <212> PRT
   <213> Artificial Sequence
<220>
   <223> Engineered polypeptide
<400> 140
   Glu Arg Tyr Ala Ala Tyr Phe Glu Ile Trp Gln Leu Pro Asn Leu Asn
   1               5                   10                  15
   Arg Leu Gln Lys Ala Ala Phe Ile Ser Ser Leu Glu Asp
               20                  25
<210> 141
   <211> 29
   <212> PRT
   <213> Artificial Sequence
<220>
   <223> Engineered polypeptide
<400> 141
   Glu Arg Tyr Val Ala Tyr Phe Glu Ile Trp Gln Leu Pro Asn Leu Asn
   1 5 10 15
   Arg Ser Gln Lys Ala Ala Phe Ile Ser Ser Leu Glu Asp
   20 25
<210> 142
   <211> 29
   <212> PRT
   <213> Artificial Sequence
<220>
   <223> Engineered polypeptide
<400> 142
   Glu Arg Tyr Gly Ala Tyr Phe Glu Ile Trp Gln Leu Pro Asn Leu Asn
   1 5 10 15
   Arg Thr Gln Lys Ala Ala Phe Ile Ala Ser Leu Ala Asp
   20 25
<210> 143
   <211> 29
   <212> PRT
   <213> Artificial Sequence
<220>
   <223> Engineered polypeptide
<400> 143
   Glu Lys Tyr Ile Ala Tyr Trp Glu Ile Trp Gln Leu Pro Asn Leu Asn
   1 5 10 15
   Arg Glu Gln Lys Ala Ala Phe Ile Ser Ser Leu Gln Asp
   20 25
<210> 144
   <211> 29
   <212> PRT
   <213> Artificial Sequence
<220>
   <223> Engineered polypeptide
<400> 144
   Glu Arg Tyr Ile Ala Tyr Ala Glu Ile Trp Gln Leu Pro Asn Leu Asn
   1 5 10 15
   Arg Arg Gln Lys Ala Ala Phe Ile Ala Ser Leu Glu Asp
   20 25
<210> 145
   <211> 29
   <212> PRT
   <213> Artificial Sequence
<220>
   <223> Engineered polypeptide
<400> 145
   Glu Arg Tyr Ile Ala Tyr Trp Glu Ile Trp Gln Leu Pro Asn Leu Asn
   1               5                   10                  15
   Gln Arg Gln Lys Ala Ala Phe Ile Ala Ser Leu Ala Asp
               20                  25
<210> 146
   <211> 29
   <212> PRT
   <213> Artificial Sequence
<220>
   <223> Engineered polypeptide
<400> 146
   Glu Arg Tyr Ile Ala Tyr Trp Glu Ile Trp Gln Leu Pro Asn Leu Asn
   1               5                   10                  15
   Arg Leu Gln Lys Ser Ala Phe Ile Ala Ser Leu Gln Asp
               20                  25
<210> 147
   <211> 29
   <212> PRT
   <213> Artificial Sequence
<220>
   <223> Engineered polypeptide
<400> 147
   Glu Arg Tyr His Ala Tyr Trp Glu Ile Trp Gln Leu Pro Asn Leu Asn
   1               5                   10                  15
   Arg Ala Gln Lys Ala Ala Phe Ile Arg Ser Leu Gln Asp
               20                  25
<210> 148
   <211> 29
   <212> PRT
   <213> Artificial Sequence
<220>
   <223> Engineered polypeptide
<400> 148
   Glu Arg Tyr Asn Ala Tyr Trp Glu Ile Trp Gln Leu Pro Asn Leu Asn
   1               5                   10                  15
   Arg Leu Gln Lys Ala Ala Phe Ile Arg Ser Leu Gln Asp
               20                  25
<210> 149
   <211> 29
   <212> PRT
   <213> Artificial Sequence
<220>
   <223> Engineered polypeptide
<400> 149
   Glu Arg Tyr Thr Ala Tyr Gly Glu Ile Trp Glu Leu Pro Asn Leu Asn
   1               5                   10                  15
   Arg Val Gln Arg Ala Ala Phe Ile Ala Ser Leu Ala Asp
               20                  25
<210> 150
   <211> 29
   <212> PRT
   <213> Artificial Sequence
<220>
   <223> Engineered polypeptide
<400> 150
   Glu Arg Tyr Thr Ala Tyr Gly Glu Ile Trp Glu Leu Pro Asn Leu Asn
   1               5                   10                  15
   Arg Leu Gln Lys Ala Ala Phe Ile Gly Ser Leu Ala Asp
               20                  25
<210> 151
   <211> 29
   <212> PRT
   <213> Artificial Sequence
<220>
   <223> Engineered polypeptide
<400> 151
   Glu Arg Tyr His Ala Tyr Gly Glu Ile Trp Glu Leu Pro Asn Leu Asn
   1               5                   10                  15
   Arg Arg Gln Lys Ala Ala Phe Ile Thr Ser Leu Ala Asp
               20                  25
<210> 152
   <211> 29
   <212> PRT
   <213> Artificial Sequence
<220>
   <223> Engineered polypeptide
<400> 152
   Glu Arg Tyr Lys Ala Tyr Gly Glu Ile Trp Gln Leu Pro Asn Leu Asn
   1               5                   10                  15
   Arg Leu Gln Lys Ala Ala Phe Ile Gly Ser Leu Ala Asp
               20                  25
<210> 153
   <211> 29
   <212> PRT
   <213> Artificial Sequence
<220>
   <223> Engineered polypeptide
<400> 153
   Glu Arg Tyr Gln Ala Tyr Gly Glu Ile Trp Gln Leu Pro Asn Leu Asn
   1               5                   10                  15
   Arg Leu Gln Lys Ala Ala Phe Ile Gly Ser Leu Ala Asp
               20                  25
<210> 154
   <211> 29
   <212> PRT
   <213> Artificial Sequence
<220>
   <223> Engineered polypeptide
<400> 154
   Glu Arg Tyr Val Ala Tyr Gly Glu Ile Trp Gln Leu Pro Asn Leu Asn
   1               5                   10                  15
   Arg Leu Gln Lys Ala Ala Phe Ile Ser Ser Leu Ala Asp
               20                  25
<210> 155
   <211> 29
   <212> PRT
   <213> Artificial Sequence
<220>
   <223> Engineered polypeptide
<400> 155
   Glu Arg Tyr Ile Ala Tyr Gly Glu Ile Trp Glu Leu Pro Asn Leu Asn
   1 5 10 15
   Arg Leu Gln Lys Ala Ala Phe Ile Gly Ser Leu Ser Asp
   20 25
<210> 156
   <211> 29
   <212> PRT
   <213> Artificial Sequence
<220>
   <223> Engineered polypeptide
<400> 156
   Glu Arg Tyr Gln Ala Tyr Gly Glu Ile Trp Gln Leu Pro Asn Leu Asn
   1 5 10 15
   Arg Arg Gln Lys Ala Ala Phe Ile Ser Ser Leu Gln Asp
   20 25
<210> 157
   <211> 29
   <212> PRT
   <213> Artificial Sequence
<220>
   <223> Engineered polypeptide
<400> 157
   Glu Arg Arg Gln Ala Tyr Phe Glu Ile Trp Glu Leu Pro Asn Leu Asn
   1 5 10 15
   Arg Tyr Gln Lys Ala Ala Phe Ile Ser Ser Leu Gln Asp
   20 25
<210> 158
   <211> 29
   <212> PRT
   <213> Artificial Sequence
<220>
   <223> Engineered polypeptide
<400> 158
   Glu Arg Tyr Arg Ala Tyr Val Glu Ile Trp Glu Leu Pro Asn Leu Asn
   1               5                   10                  15
   Arg Ile Gln Lys Val Ala Phe Ile Ala Ser Leu Gln Asp
               20                  25
<210> 159
   <211> 29
   <212> PRT
   <213> Artificial Sequence
   <220>
   <223> Engineered polypeptide
<400> 159
   Glu Lys Tyr Thr Ala Tyr Gly Glu Ile Trp Glu Leu Pro Asn Leu Asn
   1               5                   10                  15
   Arg Ile Gln Lys Val Ala Phe Ile Ala Ser Leu Gln Asp
               20                  25
<210> 160
   <211> 29
   <212> PRT
   <213> Artificial Sequence
<220>
   <223> Engineered polypeptide
<400> 160
   Glu Lys Tyr Ala Ala Tyr Val Glu Ile Trp Glu Leu Pro Asn Leu Asn
   1               5                   10                  15
   Arg Arg Gln Lys Ala Ala Phe Ile Ala Ser Leu Gln Asp
               20                  25
<210> 161
   <211> 29
   <212> PRT
   <213> Artificial Sequence
<220>
   <223> Engineered polypeptide
<400> 161
   Glu Arg Tyr Arg Ala Tyr Val Glu Ile Trp Glu Leu Pro Asn Leu Asn
   1 5 10 15
   Arg Trp Gln Lys Ala Ala Phe Ile Arg Ser Leu Gln Asp
   20 25
<210> 162
   <211> 29
   <212> PRT
   <213> Artificial Sequence
<220>
   <223> Engineered polypeptide
<400> 162
   Glu Arg Tyr Arg Ala Tyr Phe Glu Ile Trp Glu Leu Pro Asn Leu Asn
   1 5 10 15
   Arg Ala Gln Lys Ala Ala Phe Ile Ser Ser Leu Ser Asp
   20 25
<210> 163
   <211> 29
   <212> PRT
   <213> Artificial Sequence
<220>
   <223> Engineered polypeptide
<400> 163
   Glu Arg Tyr Gln Ala Tyr Phe Glu Ile Trp Gln Leu Pro Asn Leu Asn
   1 5 10 15
   Arg Leu Gln Ala Gly Ala Phe Ile Ala Ser Leu Ser Asp
   20 25
<210> 164
   <211> 29
   <212> PRT
   <213> Artificial Sequence
<220>
   <223> Engineered polypeptide
<400> 164
   Glu Lys Tyr Gln Ala Tyr Val Glu Ile Trp Gln Leu Pro Asn Leu Asn
   1 5 10 15
   Arg Leu Gln Lys Gly Ala Phe Ile Ala Ser Leu Ser Asp
   20 25
<210> 165
   <211> 29
   <212> PRT
   <213> Artificial Sequence
<220>
   <223> Engineered polypeptide
<400> 165
   Glu Lys Tyr Ala Ala Tyr Tyr Glu Ile Trp Gln Leu Pro Asn Leu Asn
   1 5 10 15
   Arg Ser Gln Lys Gly Ala Phe Ile Ala Ser Leu Ser Asp
   20 25
<210> 166
   <211> 29
   <212> PRT
   <213> Artificial Sequence
<220>
   <223> Engineered polypeptide
<400> 166
   Glu Lys Tyr Gln Ala Tyr Tyr Glu Ile Trp Glu Leu Pro Asn Leu Asn
   1 5 10 15
   Arg Gly Gln Lys Ala Ala Phe Ile Ala Ser Leu Ser Asp
   20 25
<210> 167
   <211> 29
   <212> PRT
   <213> Artificial Sequence
<220>
   <223> Engineered polypeptide
<400> 167
   Glu Lys Tyr Asn Ala Tyr Phe Glu Ile Trp Gln Leu Pro Asn Leu Asn
   1 5 10 15
   Arg Leu Gln Lys Ala Ala Phe Ile Thr Ser Leu Ser Asp
   20 25
<210> 168
   <211> 29
   <212> PRT
   <213> Artificial Sequence
<220>
   <223> Engineered polypeptide
<400> 168
   Glu Lys Tyr Ser Ala Tyr Phe Glu Ile Trp Gln Leu Pro Asn Leu Asn
   1               5                   10                  15
   Thr Ser Gln Lys Ala Ala Phe Ile Ala Ser Leu Ser Asp
               20                  25
<210> 169
   <211> 29
   <212> PRT
   <213> Artificial Sequence
<220>
   <223> Engineered polypeptide
<400> 169
   Glu Arg Tyr Asn Ala Tyr Phe Glu Ile Trp Glu Leu Pro Asn Leu Asn
   1               5                   10                  15
   Val Arg Gln Lys Ala Ala Phe Ile Arg Ser Leu Ser Asp
               20                  25
<210> 170
   <211> 29
   <212> PRT
   <213> Artificial Sequence
<220>
   <223> Engineered polypeptide
<400> 170
   Glu Lys Tyr Glu Ala Tyr Phe Glu Ile Trp Glu Leu Pro Asn Leu Asn
   1               5                   10                  15
   Ser Arg Gln Lys Ala Ala Phe Ile Gly Ser Leu Ser Asp
               20                  25
<210> 171
   <211> 29
   <212> PRT
   <213> Artificial Sequence
<220>
   <223> Engineered polypeptide
<400> 171
   Glu Arg Tyr Arg Ala Tyr Trp Glu Ile Trp Glu Leu Pro Asn Leu Asn
   1               5                   10                  15
   Gln Gln Gln Lys Val Ala Phe Ile Arg Ser Leu Tyr Asp
               20                  25
<210> 172
   <211> 29
   <212> PRT
   <213> Artificial Sequence
<220>
   <223> Engineered polypeptide
<400> 172
   Glu Arg Tyr Asn Ala Tyr Trp Glu Ile Trp Glu Leu Pro Asn Leu Asn
   1               5                   10                  15
   Gln Gly Gln Lys Val Ala Phe Ile Arg Ser Leu Gln Asp
               20                  25
<210> 173
   <211> 29
   <212> PRT
   <213> Artificial Sequence
<220>
   <223> Engineered polypeptide
<400> 173
   Glu Arg Arg His Ala Tyr Gly Glu Ile Trp Gln Leu Pro Asn Leu Asn
   1               5                   10                  15
   Gln Arg Gln Lys Val Ala Phe Ile Ser Ser Leu Gln Asp
               20                  25
<210> 174
   <211> 29
   <212> PRT
   <213> Artificial Sequence
<220>
   <223> Engineered polypeptide
<400> 174
   Glu Lys Tyr Val Ala Thr Trp Glu Ile Trp Glu Leu Pro Asn Leu Asn
   1               5                   10                  15
   Arg Ala Gln Lys Val Ala Phe Ile Gly Ser Leu Trp Asp
               20                  25
<210> 175
   <211> 29
   <212> PRT
   <213> Artificial Sequence
<220>
   <223> Engineered polypeptide
<400> 175
   Glu Arg Arg Ile Ala Arg Trp Glu Ile Trp Glu Leu Pro Asn Leu Asn
   1               5                   10                  15
   Arg Leu Gln Lys Val Ala Phe Ile Gly Ser Leu Glu Asp
               20                  25
<210> 176
   <211> 29
   <212> PRT
   <213> Artificial Sequence
<220>
   <223> Engineered polypeptide
<400> 176
   Glu Lys Tyr Lys Ala Ala Gly Glu Ile Trp Gln Leu Pro Asn Leu Asn
   1               5                   10                  15
   Arg Gly Gln Lys Val Ala Phe Ile Gly Ser Leu Glu Asp
               20                  25
<210> 177
   <211> 29
   <212> PRT
   <213> Artificial Sequence
   <220>
   <223> Engineered polypeptide
<400> 177
   Glu Lys Tyr Leu Ala Tyr Gly Glu Ile Trp Gln Leu Pro Asn Leu Asn
   1               5                   10                  15
   Arg His Gln Lys Val Ala Phe Ile Gly Ser Leu Ser Asp
               20                  25
<210> 178
   <211> 29
   <212> PRT
   <213> Artificial Sequence
<220>
   <223> Engineered polypeptide
<400> 178
   Glu Lys Tyr Gln Ala Tyr Gly Glu Ile Trp Gln Leu Pro Asn Leu Asn
   1 5 10 15
   Arg Gln Gln Lys Val Ala Phe Ile Ser Ser Leu Gln Asp
   20 25
<210> 179
   <211> 29
   <212> PRT
   <213> Artificial Sequence
<220>
   <223> Engineered polypeptide
<400> 179
   Glu Lys Tyr Lys Ala Tyr Gly Glu Ile Trp Gln Leu Pro Asn Leu Asn
   1 5 10 15
   Arg Gln Gln Lys Val Ala Phe Ile Ile Ser Leu Gln Asp
   20 25
<210> 180
   <211> 29
   <212> PRT
   <213> Artificial Sequence
<220>
   <223> Engineered polypeptide
<400> 180
   Glu Lys Tyr Ala Ala Ala Tyr Glu Ile Trp Gln Leu Pro Asn Leu Asn
   1 5 10 15
   Gln Arg Gln Lys Gly Ala Phe Ile Ala Ser Leu Phe Asp
   20 25
<210> 181
   <211> 29
   <212> PRT
   <213> Artificial Sequence
<220>
   <223> Engineered polypeptide
<400> 181
   Glu Lys Tyr Ala Ala Ser Tyr Glu Ile Trp Glu Leu Pro Asn Leu Asn
   1 5 10 15
   Gln Arg Gln Lys Ala Ala Phe Ile Lys Ser Leu Phe Asp
   20 25
<210> 182
   <211> 29
   <212> PRT
   <213> Artificial Sequence
<220>
   <223> Engineered polypeptide
<400> 182
   Glu Lys Tyr Leu Ala Ser Tyr Glu Ile Trp Gln Leu Pro Asn Leu Asn
   1 5 10 15
   Arg Arg Gln Lys Ala Ala Phe Ile Ala Ser Leu Phe Asp
   20 25
<210> 183
   <211> 29
   <212> PRT
   <213> Artificial Sequence
<220>
   <223> Engineered polypeptide
<400> 183
   Glu Lys Tyr Ser Ala Thr Phe Glu Ile Trp Gln Leu Pro Asn Leu Asn
   1 5 10 15
   Gln Arg Gln Lys Ala Ala Phe Ile Ser Ser Leu Trp Asp
   20 25
<210> 184
   <211> 29
   <212> PRT
   <213> Artificial Sequence
<220>
   <223> Engineered polypeptide
<400> 184
   Glu Lys Tyr Ala Ala Ser Phe Glu Ile Trp Gln Leu Pro Asn Leu Asn
   1 5 10 15
   Thr His Gln Lys Ala Ala Phe Ile Ser Ser Leu Trp Asp
   20 25
<210> 185
   <211> 29
   <212> PRT
   <213> Artificial Sequence
<220>
   <223> Engineered polypeptide
<400> 185
   Glu Lys Tyr Lys Ala Ser Gly Glu Ile Trp Gln Leu Pro Asn Leu Asn
   1 5 10 15
   Arg Leu Gln Arg Ala Ala Phe Ile Lys Ser Leu Phe Asp
   20 25
<210> 186
   <211> 29
   <212> PRT
   <213> Artificial Sequence
<220>
   <223> Engineered polypeptide
<400> 186
   Glu Lys Tyr Ser Ala Ser Gly Glu Ile Trp Gln Leu Pro Asn Leu Asn
   1 5 10 15
   Arg Thr Gln Lys Ala Ala Phe Ile Lys Ser Leu Trp Asp
   20 25
<210> 187
   <211> 29
   <212> PRT
   <213> Artificial Sequence
<220>
   <223> Engineered polypeptide
<400> 187
   Glu Lys Tyr His Ala Ala Gly Glu Ile Trp Glu Leu Pro Asn Leu Asn
   1 5 10 15
   Arg Leu Gln Lys Ala Ala Phe Ile Arg Ser Leu Trp Asp
   20 25
<210> 188
   <211> 29
   <212> PRT
   <213> Artificial Sequence
<220>
   <223> Engineered polypeptide
<400> 188
   Glu Lys Tyr Arg Ala Ala Gly Glu Ile Trp Glu Leu Pro Asn Leu Asn
   1 5 10 15
   Arg Gln Gln Lys Ala Ala Phe Ile Arg Ser Leu Phe Asp
   20 25
<210> 189
   <211> 29
   <212> PRT
   <213> Artificial Sequence
<220>
   <223> Engineered polypeptide
<400> 189
   Glu Lys Tyr Gln Ala Ser Gly Glu Ile Trp Glu Leu Pro Asn Leu Asn
   1 5 10 15
   Arg Leu Gln Lys Ala Ala Phe Ile Arg Ser Leu Phe Asp
   20 25
<210> 190
   <211> 29
   <212> PRT
   <213> Artificial Sequence
<220>
   <223> Engineered polypeptide
<400> 190
   Glu Lys Tyr Lys Ala Thr Gly Glu Ile Trp Glu Leu Pro Asn Leu Asn
   1 5 10 15
   Arg Ile Gln Lys Ala Ala Phe Ile Arg Ser Leu Trp Asp
   20 25
<210> 191
   <211> 29
   <212> PRT
   <213> Artificial Sequence
<220>
   <223> Engineered polypeptide
<400> 191
   Glu Lys Tyr Gln Ala Ser Phe Glu Ile Trp Glu Leu Pro Asn Leu Asn
   1 5 10 15
   Arg Ser Gln Lys Ala Ala Phe Ile Lys Ser Leu Trp Asp
   20 25
<210> 192
   <211> 29
   <212> PRT
   <213> Artificial Sequence
<220>
   <223> Engineered polypeptide
<400> 192
   Glu Arg Lys Val Ala Ala Val Glu Ile Trp Gln Leu Pro Asn Leu Asn
   1               5                   10                  15
   Arg Trp Gln Arg Gly Ala Phe Ile Ser Ser Leu Phe Asp
               20                  25
<210> 193
   <211> 29
   <212> PRT
   <213> Artificial Sequence
<220>
   <223> Engineered polypeptide
<400> 193
   Glu Arg Lys Leu Ala Ala Val Glu Ile Trp Glu Leu Pro Asn Leu Asn
   1               5                   10                  15
   Gln Tyr Gln Arg Gly Ala Phe Ile Ser Ser Leu Trp Asp
               20                  25
<210> 194
   <211> 29
   <212> PRT
   <213> Artificial Sequence
<220>
   <223> Engineered polypeptide
<400> 194
   Glu Arg Lys Lys Ala Ser Val Glu Ile Trp Glu Leu Pro Asn Leu Asn
   1               5                   10                  15
   Arg Tyr Gln Lys Gly Ala Phe Ile Ser Ser Leu Phe Asp
               20                  25
<210> 195
   <211> 29
   <212> PRT
   <213> Artificial Sequence
<220>
   <223> Engineered polypeptide
<400> 195
   Glu Arg Lys His Ala Ala Trp Glu Ile Trp Glu Leu Pro Asn Leu Asn
   1 5 10 15
   Arg His Gln Lys Gly Ala Phe Ile Lys Ser Leu Trp Asp
   20 25
<210> 196
   <211> 29
   <212> PRT
   <213> Artificial Sequence
<220>
   <223> Engineered polypeptide
<400> 196
   Glu Arg Lys His Ala Ala Val Glu Ile Trp Glu Leu Pro Asn Leu Asn
   1 5 10 15
   Arg Trp Gln Lys Gly Ala Phe Ile Gly Ser Leu Trp Asp
   20 25
<210> 197
   <211> 29
   <212> PRT
   <213> Artificial Sequence
<220>
   <223> Engineered polypeptide
<400> 197
   Glu Lys Arg His Ala Ala Val Glu Ile Trp Glu Leu Pro Asn Leu Asn
   1 5 10 15
   Val Phe Gln Arg Gly Ala Phe Ile Ala Ser Leu Tyr Asp
   20 25
<210> 198
   <211> 29
   <212> PRT
   <213> Artificial Sequence
<220>
   <223> Engineered polypeptide
<400> 198
   Glu Arg Lys His Ala Ala Val Glu Ile Trp Glu Leu Pro Asn Leu Asn
   1               5                   10                  15
   Arg Leu Gln Arg Gly Ala Phe Ile Ala Ser Leu Tyr Asp
               20                  25
<210> 199
   <211> 29
   <212> PRT
   <213> Artificial Sequence
<220>
   <223> Engineered polypeptide
<400> 199
   Glu Trp Lys Thr Ala Ala Val Glu Ile Trp Glu Leu Pro Asn Leu Asn
   1               5                   10                  15
   Arg Arg Gln Lys Ala Ala Phe Ile Gly Ser Leu Tyr Asp
               20                  25
<210> 200
   <211> 29
   <212> PRT
   <213> Artificial Sequence
<220>
   <223> Engineered polypeptide
<400> 200
   Glu Arg Lys Val Ala Ala Phe Glu Ile Trp Glu Leu Pro Asn Leu Asn
   1               5                   10                  15
   Arg Gly Gln Lys Ala Ala Phe Ile Gly Ser Leu Tyr Asp
               20                  25
<210> 201
   <211> 29
   <212> PRT
   <213> Artificial Sequence
<220>
   <223> Engineered polypeptide
<400> 201
   Glu Lys Arg Asn Ala Ala Val Glu Ile Trp Glu Leu Pro Asn Leu Asn
   1               5                   10                  15
   Arg Arg Gln Arg Ala Ala Phe Ile Ser Ser Leu Phe Asp
               20                  25
<210> 202
   <211> 29
   <212> PRT
   <213> Artificial Sequence
<220>
   <223> Engineered polypeptide
<400> 202
   Glu Arg Arg Asn Ala Ala Val Glu Ile Trp Glu Leu Pro Asn Leu Asn
   1               5                   10                  15
   Arg His Gln Arg Ala Ala Phe Ile Ser Ser Leu Trp Asp
               20                  25
<210> 203
   <211> 29
   <212> PRT
   <213> Artificial Sequence
<220>
   <223> Engineered polypeptide
<400> 203
   Glu Lys Arg Gln Ala Ser Val Glu Ile Trp Glu Leu Pro Asn Leu Asn
   1               5                   10                  15
   Arg Ile Gln Lys Ala Ala Phe Ile Ser Ser Leu Phe Asp
               20                  25
<210> 204
   <211> 29
   <212> PRT
   <213> Artificial Sequence
<220>
   <223> Engineered polypeptide
<400> 204
   Glu Lys Arg Arg Ala Ala Val Glu Ile Trp Glu Leu Pro Asn Leu Asn
   1               5                   10                  15
   Arg Ile Gln Lys Ser Ala Phe Ile Ala Ser Leu Trp Asp
               20                  25
<210> 205
   <211> 29
   <212> PRT
   <213> Artificial Sequence
<220>
   <223> Engineered polypeptide
<400> 205
   Glu Lys Arg His Ala Ala Trp Glu Ile Trp Glu Leu Pro Asn Leu Asn
   1               5                   10                  15
   Arg Ala Gln Lys Ser Ala Phe Ile Lys Ser Leu Trp Asp
               20                  25
<210> 206
   <211> 29
   <212> PRT
   <213> Artificial Sequence
<220>
   <223> Engineered polypeptide
<400> 206
   Glu Lys Arg Asn Ala Thr Val Glu Ile Trp Glu Leu Pro Asn Leu Asn
   1               5                   10                  15
   Arg Ile Gln Arg Ala Ala Phe Ile Ala Ser Leu Trp Asp
               20                  25
<210> 207
   <211> 29
   <212> PRT
   <213> Artificial Sequence
<220>
   <223> Engineered polypeptide
<400> 207
   Glu Lys Arg Ala Ala Thr Val Glu Ile Trp Glu Leu Pro Asn Leu Asn
   1               5                   10                  15
   Arg Leu Gln Lys Gly Ala Phe Ile Ala Ser Leu Trp Asp
               20                  25
<210> 208
   <211> 29
   <212> PRT
   <213> Artificial Sequence
<220>
   <223> Engineered polypeptide
<400> 208
   Glu Arg Arg Gly Ala Ala Val Glu Ile Trp Glu Leu Pro Asn Leu Asn
   1               5                   10                  15
   Arg Gln Gln Lys Ala Ala Phe Ile Ala Ser Leu Trp Asp
               20                  25
<210> 209
   <211> 29
   <212> PRT
   <213> Artificial Sequence
<220>
   <223> Engineered polypeptide
<400> 209
   Glu Arg Arg Lys Ala Thr Val Glu Ile Trp Glu Leu Pro Asn Leu Asn
   1               5                   10                  15
   Arg Gln Gln Lys Ala Ala Phe Ile Ser Ser Leu Trp Asp
               20                  25
<210> 210
   <211> 29
   <212> PRT
   <213> Artificial Sequence
<220>
   <223> Engineered polypeptide
<400> 210
   Glu Lys Ser Thr Ala Ala Val Glu Ile Trp Glu Leu Pro Asn Leu Asn
   1               5                   10                  15
   Arg Ile Gln Lys Ala Ala Phe Ile Lys Ser Leu Phe Asp
               20                  25
<210> 211
   <211> 29
   <212> PRT
   <213> Artificial Sequence
<220>
   <223> Engineered polypeptide
<400> 211
   Glu Lys Ser Thr Ala Ala Val Glu Ile Trp Glu Leu Pro Asn Leu Asn
   1               5                   10                  15
   Arg Tyr Gln Lys Ala Ala Phe Ile Lys Ser Leu Phe Asp
               20                  25
<210> 212
   <211> 29
   <212> PRT
   <213> Artificial Sequence
<220>
   <223> Engineered polypeptide
<400> 212
   Glu Lys Ser His Ala Ala Val Glu Ile Trp Glu Leu Pro Asn Leu Asn
   1               5                   10                  15
   Arg Trp Gln Lys Ala Ala Phe Ile Lys Ser Leu Tyr Asp
               20                  25
<210> 213
   <211> 29
   <212> PRT
   <213> Artificial Sequence
<220>
   <223> Engineered polypeptide
<400> 213
   Glu Arg Ser Ser Ala Ala Val Glu Ile Trp Glu Leu Pro Asn Leu Asn
   1               5                   10                  15
   Arg Phe Gln Lys Ala Ala Phe Ile Lys Ser Leu Phe Asp
               20                  25
<210> 214
   <211> 29
   <212> PRT
   <213> Artificial Sequence
<220>
   <223> Engineered polypeptide
<400> 214
   Glu Lys Arg Ile Ala Ala Ala Glu Ile Trp Glu Leu Pro Asn Leu Asn
   1               5                   10                  15
   Arg Val Gln Lys Ala Ala Phe Ile Lys Ser Leu Phe Asp
               20                  25
<210> 215
   <211> 29
   <212> PRT
   <213> Artificial Sequence
<220>
   <223> Engineered polypeptide
<400> 215
   Glu Lys Arg His Ala Ala Gly Glu Ile Trp Glu Leu Pro Asn Leu Asn
   1               5                   10                  15
   Arg Ile Gln Lys Ala Ala Phe Ile Lys Ser Leu Phe Asp
               20                  25
<210> 216
   <211> 29
   <212> PRT
   <213> Artificial Sequence
<220>
   <223> Engineered polypeptide
<400> 216
   Glu Lys Arg Ile Ala Ala Val Glu Ile Trp Glu Leu Pro Asn Leu Asn
   1               5                   10                  15
   Arg Val Gln Lys Ala Ala Phe Ile Ala Ser Leu Tyr Asp
               20                  25
<210> 217
   <211> 29
   <212> PRT
   <213> Artificial Sequence
<220>
   <223> Engineered polypeptide
<400> 217
   Glu Lys Arg Ile Ala Ser Tyr Glu Ile Trp Glu Leu Pro Asn Leu Asn
   1               5                   10                  15
   Arg Arg Gln Arg Ala Ala Phe Ile Ala Ser Leu Trp Asp
               20                  25
<210> 218
   <211> 29
   <212> PRT
   <213> Artificial Sequence
<220>
   <223> Engineered polypeptide
<400> 218
   Glu Lys Arg Asn Ala Thr Phe Glu Ile Trp Glu Leu Pro Asn Leu Asn
   1 5 10 15
   Arg Ser Gln Lys Gly Ala Phe Ile Ser Ser Leu Tyr Asp
   20 25
<210> 219
   <211> 29
   <212> PRT
   <213> Artificial Sequence
<220>
   <223> Engineered polypeptide
<400> 219
   Glu Arg Lys Val Ala Thr Phe Glu Ile Trp Glu Leu Pro Asn Leu Asn
   1 5 10 15
   Ser Arg Gln Lys Ala Ala Phe Ile Lys Ser Leu Phe Asp
   20 25
<210> 220
   <211> 29
   <212> PRT
   <213> Artificial Sequence
<220>
   <223> Engineered polypeptide
<400> 220
   Glu Arg Lys Asn Ala Thr Phe Glu Ile Trp Glu Leu Pro Asn Leu Asn
   1 5 10 15
   Gln Arg Gln Lys Ser Ala Phe Ile Ala Ser Leu Trp Asp
   20 25
<210> 221
   <211> 29
   <212> PRT
   <213> Artificial Sequence
<220>
   <223> Engineered polypeptide
<400> 221
   Glu Arg Ser Ala Ala Thr Phe Glu Ile Trp Glu Leu Pro Asn Leu Asn
   1               5                   10                  15
   Gln Arg Gln Lys Ser Ala Phe Ile Ala Ser Leu Trp Asp
               20                  25
<210> 222
   <211> 29
   <212> PRT
   <213> Artificial Sequence
<220>
   <223> Engineered polypeptide
<400> 222
   Glu Arg Lys His Ala Thr Phe Glu Ile Trp Glu Leu Pro Asn Leu Asn
   1               5                   10                  15
   Gln Leu Gln Lys Ser Ala Phe Ile Gly Ser Leu Trp Asp
               20                  25
<210> 223
   <211> 29
   <212> PRT
   <213> Artificial Sequence
<220>
   <223> Engineered polypeptide
<400> 223
   Glu Arg Lys Gln Ala Thr Phe Glu Ile Trp Glu Leu Pro Asn Leu Asn
   1               5                   10                  15
   Gln Gln Gln Lys Gly Ala Phe Ile Ala Ser Leu Trp Asp
               20                  25
<210> 224
   <211> 29
   <212> PRT
   <213> Artificial Sequence
   <220>
   <223> Engineered polypeptide
<400> 224
   Glu Arg Lys Arg Ala Thr Phe Glu Ile Trp Glu Leu Pro Asn Leu Asn
   1               5                   10                  15
   Arg Thr Gln Lys Ala Ala Phe Ile Ser Ser Leu Trp Asp
               20                  25
<210> 225
   <211> 29
   <212> PRT
   <213> Artificial Sequence
<220>
   <223> Engineered polypeptide
<400> 225
   Glu Arg Lys Thr Ala Thr Phe Glu Ile Trp Glu Leu Pro Asn Leu Asn
   1               5                   10                  15
   Arg Val Gln Lys Ala Ala Phe Ile Ser Ser Leu Trp Asp
               20                  25
<210> 226
   <211> 29
   <212> PRT
   <213> Artificial Sequence
<220>
   <223> Engineered polypeptide
<400> 226
   Glu Arg Lys Leu Ala Thr Trp Glu Ile Trp Glu Leu Pro Asn Leu Asn
   1               5                   10                  15
   Arg Ser Gln Lys Ala Ala Phe Ile Ile Ser Leu Trp Asp
               20                  25
<210> 227
   <211> 29
   <212> PRT
   <213> Artificial Sequence
<220>
   <223> Engineered polypeptide
<400> 227
   Glu Arg Lys Val Ala Ala Val Glu Ile Trp Glu Leu Pro Asn Leu Asn
   1               5                   10                  15
   Arg Ile Gln Lys Ala Ala Phe Ile Arg Ser Leu Phe Asp
                       20                  25
<210> 228
   <211> 29
   <212> PRT
   <213> Artificial Sequence
<220>
   <223> Engineered polypeptide
<400> 228
   Glu Arg Lys Glu Ala Ala Val Glu Ile Trp Glu Leu Pro Asn Leu Asn
   1               5                   10                  15
   Arg Leu Gln Lys Ala Ala Phe Ile Arg Ser Leu Phe Asp
               20                  25
<210> 229
   <211> 29
   <212> PRT
   <213> Artificial Sequence
<220>
   <223> Engineered polypeptide
<400> 229
   Glu Arg Lys Arg Ala Ala Val Glu Ile Trp Glu Leu Pro Asn Leu Asn
   1               5                   10                  15
   Arg Gly Gln Lys Ala Ala Phe Ile Lys Ser Leu Phe Asp
               20                  25
<210> 230
   <211> 29
   <212> PRT
   <213> Artificial Sequence
<220>
   <223> Engineered polypeptide
<400> 230
   Glu Arg Lys Leu Ala Ala Val Glu Ile Trp Glu Leu Pro Asn Leu Asn
   1               5                   10                  15
   Arg Thr Gln Lys Ala Ala Phe Ile Lys Ser Leu Phe Asp
               20                  25
<210> 231
   <211> 29
   <212> PRT
   <213> Artificial Sequence
<220>
   <223> Engineered polypeptide
<400> 231
   Glu Arg Lys Val Ala Ala Val Glu Ile Trp Glu Leu Pro Asn Leu Asn
   1 5 10 15
   Arg Val Gln Lys Ala Ala Phe Ile Lys Ser Leu Phe Asp
   20 25
<210> 232
   <211> 29
   <212> PRT
   <213> Artificial Sequence
<220>
   <223> Engineered polypeptide
<400> 232
   Glu Arg Lys Glu Ala Ala Val Glu Ile Trp Glu Leu Pro Asn Leu Asn
   1 5 10 15
   Arg Val Gln Lys Ala Ala Phe Ile Lys Ser Leu Tyr Asp
   20 25
<210> 233
   <211> 29
   <212> PRT
   <213> Artificial Sequence
<220>
   <223> Engineered polypeptide
<400> 233
   Glu Arg Lys Ala Ala Thr Val Glu Ile Trp Glu Leu Pro Asn Leu Asn
   1 5 10 15
   Arg Arg Gln Lys Ala Ala Phe Ile Ala Ser Leu Phe Asp
   20 25
<210> 234
   <211> 29
   <212> PRT
   <213> Artificial Sequence
<220>
   <223> Engineered polypeptide
<400> 234
   Glu Lys Arg His Ala Ser Trp Glu Ile Trp Glu Leu Pro Asn Leu Asn
   1 5 10 15
   Gln Trp Gln Lys Ala Ala Phe Ile Arg Ser Leu Phe Asp
   20 25
<210> 235
   <211> 29
   <212> PRT
   <213> Artificial Sequence
<220>
   <223> Engineered polypeptide
<400> 235
   Glu Arg Lys Ser Ala Thr Trp Glu Ile Trp Glu Leu Pro Asn Leu Asn
   1               5                   10                  15
   Gln Leu Gln Lys Ala Ala Phe Ile Arg Ser Leu Phe Asp
               20                  25
<210> 236
   <211> 29
   <212> PRT
   <213> Artificial Sequence
<220>
   <223> Engineered polypeptide
<400> 236
   Glu Lys Lys Lys Ala Ala Val Glu Ile Trp Glu Leu Pro Asn Leu Asn
   1               5                   10                  15
   Gln Arg Gln Lys Ala Ala Phe Ile Gly Ser Leu Trp Asp
               20                  25
<210> 237
   <211> 29
   <212> PRT
   <213> Artificial Sequence
<220>
   <223> Engineered polypeptide
<400> 237
   Glu Lys Lys Leu Ala Ser Phe Glu Ile Trp Glu Leu Pro Asn Leu Asn
   1               5                   10                  15
   Gln Arg Gln Lys Ala Ala Phe Ile Arg Ser Leu Trp Asp
               20                  25
<210> 238
   <211> 29
   <212> PRT
   <213> Artificial Sequence
<220>
   <223> Engineered polypeptide
<400> 238
   Glu Arg Lys Val Ala Thr Val Glu Ile Trp Glu Leu Pro Asn Leu Asn
   1 5 10 15
   Arg Val Gln Ala Ala Ala Phe Ile Lys Ser Leu Trp Asp
   20 25
<210> 239
   <211> 29
   <212> PRT
   <213> Artificial Sequence
<220>
   <223> Engineered polypeptide
<400> 239
   Glu Arg Lys Thr Ala Thr Val Glu Ile Trp Glu Leu Pro Asn Leu Asn
   1 5 10 15
   Arg Ile Gln Lys Ala Ala Phe Ile Lys Ser Leu Trp Asp
   20 25
<210> 240
   <211> 29
   <212> PRT
   <213> Artificial Sequence
<220>
   <223> Engineered polypeptide
<400> 240
   Glu Arg Lys Ser Ala Thr Val Glu Ile Trp Glu Leu Pro Asn Leu Asn
   1 5 10 15
   Arg Val Gln Lys Ala Ala Phe Ile Ser Ser Leu Trp Asp
   20 25
<210> 241
   <211> 29
   <212> PRT
   <213> Artificial Sequence
<220>
   <223> Engineered polypeptide
<400> 241
   Glu Arg Lys Val Ala Thr Val Glu Ile Trp Glu Leu Pro Asn Leu Asn
   1 5 10 15
   Arg Gly Gln Lys Ala Ala Phe Ile Ser Ser Leu Trp Asp
   20 25
<210> 242
   <211> 29
   <212> PRT
   <213> Artificial Sequence
   <220>
   <223> Engineered polypeptide
<400> 242
   Glu Arg Lys Gln Ala Thr Val Glu Ile Trp Glu Leu Pro Asn Leu Asn
   1               5                   10                  15
   Arg Arg Gln Arg Ala Ala Phe Ile Ser Ser Leu Trp Asp
               20                  25
<210> 243
   <211> 29
   <212> PRT
   <213> Artificial Sequence
<220>
   <223> Engineered polypeptide
<400> 243
   Glu Arg Lys Lys Ala Thr Val Glu Ile Trp Glu Leu Pro Asn Leu Asn
   1               5                   10                  15
   Arg Arg Gln Lys Gly Ala Phe Ile Ala Ser Leu Trp Asp
               20                  25
<210> 244
   <211> 29
   <212> PRT
   <213> Artificial Sequence
<220>
   <223> Engineered polypeptide
<400> 244
   Glu Arg Lys Val Ala Thr Val Glu Ile Trp Glu Leu Pro Asn Leu Asn
   1               5                   10                  15
   Arg Arg Gln Lys Gly Ala Phe Ile Ala Ser Leu Trp Asp
               20                  25
<210> 245
   <211> 29
   <212> PRT
   <213> Artificial Sequence
<220>
   <223> Engineered polypeptide
<400> 245
   Glu Arg Lys Leu Ala Thr Val Glu Ile Trp Glu Leu Pro Asn Leu Asn
   1               5                   10                  15
   Arg Tyr Gln Lys Gly Ala Phe Ile Ala Ser Leu Trp Asp
               20                  25
<210> 246
   <211> 29
   <212> PRT
   <213> Artificial Sequence
<220>
   <223> Engineered polypeptide
<400> 246
   Glu Arg Lys Ala Ala Thr Val Glu Ile Trp Glu Leu Pro Asn Leu Asn
   1               5                   10                  15
   Arg Leu Gln Lys Gly Ala Phe Ile Ala Ser Leu Trp Asp
               20                  25
<210> 247
   <211> 29
   <212> PRT
   <213> Artificial Sequence
<220>
   <223> Engineered polypeptide
<400> 247
   Glu Arg Lys Ile Ala Thr Val Glu Ile Trp Glu Leu Pro Asn Leu Asn
   1               5                   10                  15
   Arg Val Gln Lys Ser Ala Phe Ile Ala Ser Leu Trp Asp
               20                  25
<210> 248
   <211> 29
   <212> PRT
   <213> Artificial Sequence
<220>
   <223> Engineered polypeptide
<400> 248
   Glu Arg Lys Asn Ala Thr Val Glu Ile Trp Glu Leu Pro Asn Leu Asn
   1 5 10 15
   Arg Ala Gln Lys Ala Ala Phe Ile Ala Ser Leu Trp Asp
   20 25
<210> 249
   <211> 29
   <212> PRT
   <213> Artificial Sequence
<220>
   <223> Engineered polypeptide
<400> 249
   Glu Arg Lys Asn Ala Thr Val Glu Ile Trp Glu Leu Pro Asn Leu Asn
   1 5 10 15
   Arg Ser Gln Lys Ala Ala Phe Ile Gly Ser Leu Trp Asp
   20 25
<210> 250
   <211> 29
   <212> PRT
   <213> Artificial Sequence
<220>
   <223> Engineered polypeptide
<400> 250
   Glu Arg Lys Thr Ala Thr Val Glu Ile Trp Glu Leu Pro Asn Leu Asn
   1 5 10 15
   Arg Gly Gln Lys Ala Ala Phe Ile Ala Ser Leu Phe Asp
   20 25
<210> 251
   <211> 29
   <212> PRT
   <213> Artificial Sequence
<220>
   <223> Engineered polypeptide
<400> 251
   Glu Arg Lys His Ala Ala Val Glu Ile Trp Glu Leu Pro Asn Leu Asn
   1 5 10 15
   Arg Ile Gln Arg Ala Ala Phe Ile Ala Ser Leu Phe Asp
   20 25
<210> 252
   <211> 29
   <212> PRT
   <213> Artificial Sequence
<220>
   <223> Engineered polypeptide
<400> 252
   Glu Arg Lys Ile Ala Thr Val Glu Ile Trp Glu Leu Pro Asn Leu Asn
   1               5                   10                  15
   Arg Ile Gln Arg Ala Ala Phe Ile Ala Ser Leu Phe Asp
               20                  25
<210> 253
   <211> 29
   <212> PRT
   <213> Artificial Sequence
<220>
   <223> Engineered polypeptide
<400> 253
   Glu Arg Lys Ile Ala Ala Val Glu Ile Trp Glu Leu Pro Asn Leu Asn
   1               5                   10                  15
   Arg Ile Gln Ala Ala Ala Phe Ile Ala Ser Leu Trp Asp
               20                  25
<210> 254
   <211> 29
   <212> PRT
   <213> Artificial Sequence
<220>
   <223> Engineered polypeptide
<400> 254
   Glu Arg Lys Ser Ala Thr Val Glu Ile Trp Glu Leu Pro Asn Leu Asn
   1               5                   10                  15
   Arg Thr Gln Arg Ala Ala Phe Ile Ala Ser Leu Trp Asp
               20                  25
<210> 255
   <211> 29
   <212> PRT
   <213> Artificial Sequence
<220>
   <223> Engineered polypeptide
<400> 255
   Glu Arg Lys Ser Ala Ala Val Glu Ile Trp Glu Leu Pro Asn Leu Asn
   1               5                   10                  15
   Arg Thr Gln Lys Ala Ala Phe Ile Ala Ser Leu Trp Asp
               20                  25
<210> 256
   <211> 29
   <212> PRT
   <213> Artificial Sequence
<220>
   <223> Engineered polypeptide
<400> 256
   Glu Lys Lys Ser Ala Thr Val Glu Ile Trp Glu Leu Pro Asn Leu Asn
   1               5                   10                  15
   Arg Ile Gln Lys Ala Ala Phe Ile Gly Ser Leu Trp Asp
               20                  25
<210> 257
   <211> 29
   <212> PRT
   <213> Artificial Sequence
<220>
   <223> Engineered polypeptide
<400> 257
   Glu Lys Lys Ser Ala Tyr Val Glu Ile Trp Glu Leu Pro Asn Leu Asn
   1               5                   10                  15
   Arg Tyr Gln Lys Ala Ala Phe Ile Lys Ser Leu Phe Asp
               20                  25
<210> 258
   <211> 29
   <212> PRT
   <213> Artificial Sequence
<220>
   <223> Engineered polypeptide
<400> 258
   Glu Arg Lys Ile Ala Thr Tyr Glu Ile Trp Glu Leu Pro Asn Leu Asn
   1               5                   10                  15
   Gln Arg Gln Lys Ala Ala Phe Ile Ala Ser Leu Trp Asp
               20                  25
<210> 259
   <211> 29
   <212> PRT
   <213> Artificial Sequence
<220>
   <223> Engineered polypeptide
<400> 259
   Glu Arg Ser Val Ala Thr Trp Glu Ile Trp Gln Leu Pro Asn Leu Asn
   1               5                   10                  15
   Gln Arg Gln Lys Ala Ala Phe Ile Ala Ser Leu Trp Asp
               20                  25
<210> 260
   <211> 29
   <212> PRT
   <213> Artificial Sequence
<220>
   <223> Engineered polypeptide
<400> 260
   Glu Lys Arg Lys Ala Thr Gly Glu Ile Trp Gln Leu Pro Asn Leu Asn
   1               5                   10                  15
   Gln Arg Gln Lys Gly Ala Phe Ile Ala Ser Leu Trp Asp
               20                  25
<210> 261
   <211> 29
   <212> PRT
   <213> Artificial Sequence
<220>
   <223> Engineered polypeptide
<400> 261
   Glu Arg Lys Glu Ala Arg Phe Glu Ile Trp Glu Leu Pro Asn Leu Asn
   1               5                   10                  15
   Gln Arg Gln Ala Ala Ala Phe Ile Ser Ser Leu Phe Asp
               20                  25
<210> 262
   <211> 29
   <212> PRT
   <213> Artificial Sequence
<220>
   <223> Engineered polypeptide
<400> 262
   Glu Arg Lys Ala Ala Ser Trp Glu Ile Trp Glu Leu Pro Asn Leu Asn
   1               5                   10                  15
   Gln Arg Gln Lys Ala Ala Phe Ile Ser Ser Leu Tyr Asp
               20                  25
<210> 263
   <211> 29
   <212> PRT
   <213> Artificial Sequence
<220>
   <223> Engineered polypeptide
<400> 263
   Glu Trp Lys His Ala Ala Gly Glu Ile Trp Glu Leu Pro Asn Leu Asn
   1               5                   10                  15
   Arg Ser Gln Lys Val Ala Phe Ile Ala Ser Leu Glu Asp
               20                  25
<210> 264
   <211> 29
   <212> PRT
   <213> Artificial Sequence
<220>
   <223> Engineered polypeptide
<400> 264
   Glu Trp Lys Arg Ala Ala Gly Glu Ile Trp Glu Leu Pro Asn Leu Asn
   1               5                   10                  15
   Arg Ala Gln Lys Val Ala Phe Ile Ala Ser Leu Ala Asp
               20                  25
<210> 265
   <211> 29
   <212> PRT
   <213> Artificial Sequence
<220>
   <223> Engineered polypeptide
<400> 265
   Glu Trp Lys Ile Ala Ala Gly Glu Ile Trp Glu Leu Pro Asn Leu Asn
   1               5                   10                  15
   Gln Arg Gln Lys Val Ala Phe Ile Arg Ser Leu Gln Asp
               20                  25
<210> 266
   <211> 29
   <212> PRT
   <213> Artificial Sequence
<220>
   <223> Engineered polypeptide
<400> 266
   Glu Trp Lys Thr Ala Ala Tyr Glu Ile Trp Glu Leu Pro Asn Leu Asn
   1               5                   10                  15
   Thr Tyr Gln Lys Val Ala Phe Ile Arg Ser Leu Gln Asp
               20                  25
<210> 267
   <211> 29
   <212> PRT
   <213> Artificial Sequence
<220>
   <223> Engineered polypeptide
<400> 267
   Glu Trp Lys Val Ala Ala Gly Glu Ile Trp Glu Leu Pro Asn Leu Asn
   1               5                   10                  15
   Arg Thr Gln Lys Val Ala Phe Ile Arg Ser Leu Ser Asp
               20                  25
<210> 268
   <211> 29
   <212> PRT
   <213> Artificial Sequence
<220>
   <223> Engineered polypeptide
<400> 268
   Glu Trp Lys Val Ala Ala Gly Glu Ile Trp Glu Leu Pro Asn Leu Asn
   1               5                   10                  15
   Arg Trp Gln Lys Val Ala Phe Ile Arg Ser Leu Ser Asp
               20                  25
<210> 269
   <211> 29
   <212> PRT
   <213> Artificial Sequence
<220>
   <223> Engineered polypeptide
<400> 269
   Glu Trp Lys Gln Ala Ala Gly Glu Ile Trp Glu Leu Pro Asn Leu Asn
   1               5                   10                  15
   Arg Leu Gln Lys Val Ala Phe Ile Arg Ser Leu Ser Asp
               20                  25
<210> 270
   <211> 29
   <212> PRT
   <213> Artificial Sequence
<220>
   <223> Engineered polypeptide
<400> 270
   Glu Trp Lys Val Ala Ala Gly Glu Ile Trp Glu Leu Pro Asn Leu Asn
   1               5                   10                  15
   Val Trp Gln Lys Ala Ala Phe Ile Arg Ser Leu Ser Asp
               20                  25
<210> 271
   <211> 29
   <212> PRT
   <213> Artificial Sequence
<220>
   <223> Engineered polypeptide
<400> 271
   Glu Trp Lys Val Ala Ala Trp Glu Ile Trp Glu Leu Pro Asn Leu Asn
   1               5                   10                  15
   Arg Gly Gln Lys Val Ala Phe Ile Arg Ser Leu Gln Asp
               20                  25
<210> 272
   <211> 29
   <212> PRT
   <213> Artificial Sequence
<220>
   <223> Engineered polypeptide
<400> 272
   Glu Trp Lys Arg Ala Ala Phe Glu Ile Trp Glu Leu Pro Asn Leu Asn
   1               5                   10                  15
   Gln Ala Gln Lys Val Ala Phe Ile Thr Ser Leu Ser Asp
               20                  25
<210> 273
   <211> 29
   <212> PRT
   <213> Artificial Sequence
<220>
   <223> Engineered polypeptide
<400> 273
   Glu Trp Lys Lys Ala Ala Gly Glu Ile Trp Leu Leu Pro Asn Leu Asn
   1               5                   10                  15
   Arg Arg Gln Arg Gly Ala Phe Ile Ala Ser Leu Ala Asp
               20                  25
<210> 274
   <211> 29
   <212> PRT
   <213> Artificial Sequence
<220>
   <223> Engineered polypeptide
<400> 274
   Glu Trp Lys Lys Ala Ala Gly Glu Ile Trp Glu Leu Pro Asn Leu Asn
   1               5                   10                  15
   Ser Arg Gln Lys Gly Ala Phe Ile Ala Ser Leu Ala Asp
               20                  25
<210> 275
   <211> 29
   <212> PRT
   <213> Artificial Sequence
<220>
   <223> Engineered polypeptide
<400> 275
   Glu Trp Arg Gly Ala Ala Gly Glu Ile Trp Glu Leu Pro Asn Leu Asn
   1               5                   10                  15
   Arg Arg Gln Arg Ala Ala Phe Ile Ala Ser Leu Ala Asp
               20                  25
<210> 276
   <211> 29
   <212> PRT
   <213> Artificial Sequence
<220>
   <223> Engineered polypeptide
<400> 276
   Glu Trp Lys Val Ala Ala Gly Glu Ile Trp Gln Leu Pro Asn Leu Asn
   1               5                   10                  15
   Arg Arg Gln Arg Ala Ala Phe Ile Ala Ser Leu Ala Asp
               20                  25
<210> 277
   <211> 29
   <212> PRT
   <213> Artificial Sequence
<220>
   <223> Engineered polypeptide
<400> 277
   Glu Trp Lys Ile Ala Ala Val Glu Ile Trp Glu Leu Pro Asn Leu Asn
   1               5                   10                  15
   Thr Arg Gln Lys Gly Ala Phe Ile Ala Ser Leu Ser Asp
               20                  25
<210> 278
   <211> 29
   <212> PRT
   <213> Artificial Sequence
<220>
   <223> Engineered polypeptide
<400> 278
   Glu Trp Lys Ile Ala Ala Phe Glu Ile Trp Glu Leu Pro Asn Leu Asn
   1               5                   10                  15
   Val Arg Gln Arg Ser Ala Phe Ile Ser Ser Leu Ser Asp
               20                  25
<210> 279
   <211> 29
   <212> PRT
   <213> Artificial Sequence
<220>
   <223> Engineered polypeptide
<400> 279
   Glu Trp Lys Thr Ala Ala Tyr Glu Ile Trp Glu Leu Pro Asn Leu Asn
   1 5 10 15
   Gln Arg Gln Arg Gly Ala Phe Ile Ala Ser Leu Gln Asp
   20 25
<210> 280
   <211> 29
   <212> PRT
   <213> Artificial Sequence
<220>
   <223> Engineered polypeptide
<400> 280
   Glu Trp Lys Thr Ala Ala Gly Glu Ile Trp Glu Leu Pro Asn Leu Asn
   1 5 10 15
   Ser Arg Gln Lys Ala Ala Phe Ile Gly Ser Leu Ala Asp
   20 25
<210> 281
   <211> 29
   <212> PRT
   <213> Artificial Sequence
<220>
   <223> Engineered polypeptide
<400> 281
   Glu Trp Lys Thr Ala Ala Gly Glu Ile Trp Glu Leu Pro Asn Leu Asn
   1 5 10 15
   Val Gly Gln Lys Ala Ala Phe Ile Gly Ser Leu Ala Asp
   20 25
<210> 282
   <211> 29
   <212> PRT
   <213> Artificial Sequence
<220>
   <223> Engineered polypeptide
<400> 282
   Glu Trp Lys His Ala Ala Gly Glu Ile Trp Glu Leu Pro Asn Leu Asn
   1 5 10 15
   Arg Thr Gln Lys Ala Ala Phe Ile Arg Ser Leu Ala Asp
   20 25
<210> 283
   <211> 29
   <212> PRT
   <213> Artificial Sequence
<220>
   <223> Engineered polypeptide
<400> 283
   Glu Trp Lys Arg Ala Ala Gly Glu Ile Trp Glu Leu Pro Asn Leu Asn
   1 5 10 15
   Arg Leu Gln Lys Ala Ala Phe Ile Arg Ser Leu Ala Asp
   20 25
<210> 284
   <211> 29
   <212> PRT
   <213> Artificial Sequence
<220>
   <223> Engineered polypeptide
<400> 284
   Glu Trp Lys Gln Ala Ala Gly Glu Ile Trp Glu Leu Pro Asn Leu Asn
   1 5 10 15
   Arg Gly Gln Lys Ala Ala Phe Ile Ser Ser Leu Ala Asp
   20 25
<210> 285
   <211> 29
   <212> PRT
   <213> Artificial Sequence
<220>
   <223> Engineered polypeptide
<400> 285
   Glu Trp Lys Gln Ala Ala Gly Glu Ile Trp Glu Leu Pro Asn Leu Asn
   1               5                   10                  15
   Arg Gly Gln Lys Ala Ala Phe Ile Thr Ser Leu Ala Asp
               20                  25
<210> 286
   <211> 29
   <212> PRT
   <213> Artificial Sequence
<220>
   <223> Engineered polypeptide
<400> 286
   Glu Trp Lys Glu Ala Ala Gly Glu Ile Trp Glu Leu Pro Asn Leu Asn
   1               5                   10                  15
   Arg Gly Gln Lys Ala Ala Phe Ile Lys Ser Leu Ala Asp
               20                  25
<210> 287
   <211> 29
   <212> PRT
   <213> Artificial Sequence
<220>
   <223> Engineered polypeptide
<400> 287
   Glu Trp Lys Thr Ala Ala Gly Glu Ile Trp Glu Leu Pro Asn Leu Asn
   1               5                   10                  15
   Arg Arg Gln Lys Ala Ala Phe Ile Ala Ser Leu Gln Asp
               20                  25
<210> 288
   <211> 29
   <212> PRT
   <213> Artificial Sequence
<220>
   <223> Engineered polypeptide
<400> 288
   Glu Trp Lys Arg Ala Ala Gly Glu Ile Trp Glu Leu Pro Asn Leu Asn
   1               5                   10                  15
   Arg Ala Gln Lys Ala Ala Phe Ile Ser Ser Leu Gln Asp
               20                  25
<210> 289
   <211> 29
   <212> PRT
   <213> Artificial Sequence
<220>
   <223> Engineered polypeptide
<400> 289
   Glu Trp Lys His Ala Ala Phe Glu Ile Trp Glu Leu Pro Asn Leu Asn
   1 5 10 15
   Val Arg Gln Lys Ala Ala Phe Ile Gly Ser Leu Gln Asp
   20 25
<210> 290
   <211> 29
   <212> PRT
   <213> Artificial Sequence
<220>
   <223> Engineered polypeptide
<400> 290
   Glu Trp Lys Asn Ala Ala Phe Glu Ile Trp Glu Leu Pro Asn Leu Asn
   1 5 10 15
   Val Ser Gln Lys Ala Ala Phe Ile Arg Ser Leu Gln Asp
   20 25
<210> 291
   <211> 29
   <212> PRT
   <213> Artificial Sequence
<220>
   <223> Engineered polypeptide
<400> 291
   Glu Trp Lys Ala Ala Ala Tyr Glu Ile Trp Glu Leu Pro Asn Leu Asn
   1 5 10 15
   Val Trp Gln Lys Ala Ala Phe Ile Ser Ser Leu Gln Asp
   20 25
<210> 292
   <211> 29
   <212> PRT
   <213> Artificial Sequence
<220>
   <223> Engineered polypeptide
<400> 292
   Glu Trp Lys Val Ala Ala Gly Glu Ile Trp Glu Leu Pro Asn Leu Asn
   1               5                   10                  15
   Arg Trp Gln Lys Gly Ala Phe Ile Thr Ser Leu Tyr Asp
                       20                  25
<210> 293
   <211> 29
   <212> PRT
   <213> Artificial Sequence
<220>
   <223> Engineered polypeptide
<400> 293
   Glu Trp Lys Val Ala Ala Gly Glu Ile Trp Glu Leu Pro Asn Leu Asn
   1               5                   10                  15
   Arg Trp Gln Lys Ala Ala Phe Ile Ala Ser Leu Tyr Asp
               20                  25
<210> 294
   <211> 29
   <212> PRT
   <213> Artificial Sequence
<220>
   <223> Engineered polypeptide
<400> 294
   Glu Trp Lys Val Ala Ala Gly Glu Ile Trp Glu Leu Pro Asn Leu Asn
   1               5                   10                  15
   Arg Leu Gln Arg Ala Ala Phe Ile Lys Ser Leu Tyr Asp
               20                  25
<210> 295
   <211> 29
   <212> PRT
   <213> Artificial Sequence
<220>
   <223> Engineered polypeptide
<400> 295
   Glu Trp Lys Val Ala Ala Tyr Glu Ile Trp Glu Leu Pro Asn Leu Asn
   1 5 10 15
   Arg Leu Gln Lys Ala Ala Phe Ile Ala Ser Leu Tyr Asp
   20 25
<210> 296
   <211> 29
   <212> PRT
   <213> Artificial Sequence
<220>
   <223> Engineered polypeptide
<400> 296
   Glu Trp Lys Val Ala Ala Phe Glu Ile Trp Glu Leu Pro Asn Leu Asn
   1 5 10 15
   Arg Thr Gln Arg Ala Ala Phe Ile Ile Ser Leu Gln Asp
   20 25
<210> 297
   <211> 29
   <212> PRT
   <213> Artificial Sequence
<220>
   <223> Engineered polypeptide
<400> 297
   Glu Trp Lys Thr Ala Ala Val Glu Ile Trp Glu Leu Pro Asn Leu Asn
   1 5 10 15
   Arg Leu Gln Lys Ala Ala Phe Ile Thr Ser Leu Ser Asp
   20 25
<210> 298
   <211> 29
   <212> PRT
   <213> Artificial Sequence
<220>
   <223> Engineered polypeptide
<400> 298
   Glu Trp Lys Thr Ala Ala Val Glu Ile Trp Glu Leu Pro Asn Leu Asn
   1 5 10 15
   Arg Leu Gln Lys Ala Ala Phe Ile Gly Ser Leu Ser Asp
   20 25
<210> 299
   <211> 29
   <212> PRT
   <213> Artificial Sequence
<220>
   <223> Engineered polypeptide
<400> 299
   Glu Trp Lys Thr Ala Ala Phe Glu Ile Trp Glu Leu Pro Asn Leu Asn
   1 5 10 15
   Arg Gly Gln Lys Ala Ala Phe Ile Arg Ser Leu Ser Asp
   20 25
<210> 300
   <211> 29
   <212> PRT
   <213> Artificial Sequence
<220>
   <223> Engineered polypeptide
<400> 300
   Glu Trp Lys Arg Ala Ala Phe Glu Ile Trp Glu Leu Pro Asn Leu Asn
   1 5 10 15
   Arg Leu Gln Lys Ser Ala Phe Ile Ala Ser Leu Ser Asp
   20 25
<210> 301
   <211> 29
   <212> PRT
   <213> Artificial Sequence
<220>
   <223> Engineered polypeptide
<400> 301
   Glu Trp Lys Gln Ala Ala Phe Glu Ile Trp Glu Leu Pro Asn Leu Asn
   1 5 10 15
   Arg Tyr Gln Ala Ala Ala Phe Ile Gly Ser Leu Gln Asp
   20 25
<210> 302
   <211> 29
   <212> PRT
   <213> Artificial Sequence
<220>
   <223> Engineered polypeptide
<400> 302
   Glu Trp Lys His Ala Ala Phe Glu Ile Trp Glu Leu Pro Asn Leu Asn
   1               5                   10                  15
   Arg Arg Gln Ala Gly Ala Phe Ile Ala Ser Leu Gln Asp
               20                  25
<210> 303
   <211> 29
   <212> PRT
   <213> Artificial Sequence
<220>
   <223> Engineered polypeptide
<400> 303
   Glu Trp Lys Gln Ala Ser Gly Glu Ile Trp Glu Leu Pro Asn Leu Asn
   1               5                   10                  15
   Val Arg Gln Lys Ala Ala Phe Ile Ser Ser Leu Ala Asp
               20                  25
<210> 304
   <211> 29
   <212> PRT
   <213> Artificial Sequence
<220>
   <223> Engineered polypeptide
<400> 304
   Glu Trp Lys Gln Ala Ser Phe Glu Ile Trp Glu Leu Pro Asn Leu Asn
   1               5                   10                  15
   Val Arg Gln Lys Ala Ala Phe Ile Ser Ser Leu Ser Asp
               20                  25
<210> 305
   <211> 29
   <212> PRT
   <213> Artificial Sequence
<220>
   <223> Engineered polypeptide
<400> 305
   Glu Trp Lys Leu Ala Ala Gly Glu Ile Trp Glu Leu Pro Asn Leu Asn
   1               5                   10                  15
   Val Arg Gln Lys Ala Ala Phe Ile Ala Ser Leu Gln Asp
               20                  25
<210> 306
   <211> 29
   <212> PRT
   <213> Artificial Sequence
<220>
   <223> Engineered polypeptide
<400> 306
   Glu Trp Lys Ile Ala Ala Gly Glu Ile Trp Glu Leu Pro Asn Leu Asn
   1               5                   10                  15
   Val Arg Gln Lys Ala Ala Phe Ile Ala Ser Leu Ser Asp
               20                  25
<210> 307
   <211> 29
   <212> PRT
   <213> Artificial Sequence
   <220>
   <223> Engineered polypeptide
<400> 307
   Glu Trp Lys His Ala Ala Gly Glu Ile Trp Gln Leu Pro Asn Leu Asn
   1               5                   10                  15
   Val Thr Gln Lys Ala Ala Phe Ile Ala Ser Leu Ser Asp
               20                  25
<210> 308
   <211> 29
   <212> PRT
   <213> Artificial Sequence
<220>
   <223> Engineered polypeptide
<400> 308
   Glu Trp Lys Ile Ala Ala Gly Glu Ile Trp Glu Leu Pro Asn Leu Asn
   1               5                   10                  15
   Arg Arg Gln Ala Ala Ala Phe Ile Val Ser Leu Ser Asp
               20                  25
<210> 309
   <211> 29
   <212> PRT
   <213> Artificial Sequence
<220>
   <223> Engineered polypeptide
<400> 309
   Glu Trp Lys Ser Ala Ala Gly Glu Ile Trp Glu Leu Pro Asn Leu Asn
   1               5                   10                  15
   Arg Ala Gln Lys Ala Ala Phe Ile Ile Ser Leu Ser Asp
               20                  25
<210> 310
   <211> 29
   <212> PRT
   <213> Artificial Sequence
<220>
   <223> Engineered polypeptide
<400> 310
   Glu Trp Lys His Ala Ala Val Glu Ile Trp Gln Leu Pro Asn Leu Asn
   1               5                   10                  15
   Arg Phe Gln Lys Ala Ala Phe Ile Ala Ser Leu Ser Asp
               20                  25
<210> 311
   <211> 29
   <212> PRT
   <213> Artificial Sequence
<220>
   <223> Engineered polypeptide
<400> 311
   Glu Trp Lys Gln Ala Ala Trp Glu Ile Trp Gln Leu Pro Asn Leu Asn
   1               5                   10                  15
   Thr Gly Gln Lys Ala Ala Phe Ile Arg Ser Leu Ser Asp
               20                  25
<210> 312
   <211> 29
   <212> PRT
   <213> Artificial Sequence
<220>
   <223> Engineered polypeptide
<400> 312
   Glu Trp Lys Gln Ala Ser Trp Glu Ile Trp Glu Leu Pro Asn Leu Asn
   1 5 10 15
   Arg Gly Gln Lys Ala Ala Phe Ile Ala Ser Leu Ser Asp
   20 25
<210> 313
   <211> 29
   <212> PRT
   <213> Artificial Sequence
<220>
   <223> Engineered polypeptide
<400> 313
   Glu Trp Lys Thr Ala Ser Tyr Glu Ile Trp Gln Leu Pro Asn Leu Asn
   1 5 10 15
   Arg Arg Gln Ala Ala Ala Phe Ile Ala Ser Leu Ser Asp
   20 25
<210> 314
   <211> 29
   <212> PRT
   <213> Artificial Sequence
<220>
   <223> Engineered polypeptide
<400> 314
   Glu Trp Lys Asn Ala Ser Phe Glu Ile Trp Leu Leu Pro Asn Leu Asn
   1 5 10 15
   Arg Ile Gln Lys Ala Ala Phe Ile Ala Ser Leu Ser Asp
   20 25
<210> 315
   <211> 29
   <212> PRT
   <213> Artificial Sequence
<220>
   <223> Engineered polypeptide
<400> 315
   Glu Trp Lys Asn Ala Ala Phe Glu Ile Trp Leu Leu Pro Asn Leu Asn
   1 5 10 15
   Gln Arg Gln Lys Ala Ala Phe Ile Ala Ser Leu Ser Asp
   20 25
<210> 316
   <211> 29
   <212> PRT
   <213> Artificial Sequence
<220>
   <223> Engineered polypeptide
<400> 316
   Glu Trp Arg Gly Ala Ala Phe Glu Ile Trp Gln Leu Pro Asn Leu Asn
   1               5                   10                  15
   Gln Arg Gln Lys Ala Ala Phe Ile Ala Ser Leu Tyr Asp
               20                  25
<210> 317
   <211> 29
   <212> PRT
   <213> Artificial Sequence
<220>
   <223> Engineered polypeptide
<400> 317
   Glu Trp Lys Val Ala Ser Trp Glu Ile Trp Gln Leu Pro Asn Leu Asn
   1               5                   10                  15
   Arg Gln Gln Lys Ala Ala Phe Ile Gly Ser Leu Ala Asp
               20                  25
<210> 318
   <211> 29
   <212> PRT
   <213> Artificial Sequence
<220>
   <223> Engineered polypeptide
<400> 318
   Glu Trp Lys Leu Ala Ser Trp Glu Ile Trp Gln Leu Pro Asn Leu Asn
   1               5                   10                  15
   Arg Trp Gln Lys Ala Ala Phe Ile Gly Ser Leu Ser Asp
               20                  25
<210> 319
   <211> 29
   <212> PRT
   <213> Artificial Sequence
<220>
   <223> Engineered polypeptide
<400> 319
   Glu Trp Lys Arg Ala Ser Val Glu Ile Trp Glu Leu Pro Asn Leu Asn
   1               5                   10                  15
   Arg Gln Gln Lys Ala Ala Phe Ile Gly Ser Leu Ser Asp
               20                  25
<210> 320
   <211> 29
   <212> PRT
   <213> Artificial Sequence
<220>
   <223> Engineered polypeptide
<400> 320
   Glu Trp Lys Arg Ala Ser Phe Glu Ile Trp Gln Leu Pro Asn Leu Asn
   1               5                   10                  15
   Arg Leu Gln Lys Ala Ala Phe Ile Ala Ser Leu Ala Asp
               20                  25
<210> 321
   <211> 29
   <212> PRT
   <213> Artificial Sequence
<220>
   <223> Engineered polypeptide
<400> 321
   Glu Trp Lys Gly Ala Ala Gly Glu Ile Trp Gln Leu Pro Asn Leu Asn
   1               5                   10                  15
   Arg Arg Gln Lys Ala Ala Phe Ile Ser Ser Leu Tyr Asp
               20                  25
<210> 322
   <211> 29
   <212> PRT
   <213> Artificial Sequence
<220>
   <223> Engineered polypeptide
<400> 322
   Glu Trp Lys Val Ala Ala Phe Glu Ile Trp Gln Leu Pro Asn Leu Asn
   1               5                   10                  15
   Arg Ala Gln Lys Ala Ala Phe Ile Ser Ser Leu Ala Asp
               20                  25
<210> 323
   <211> 29
   <212> PRT
   <213> Artificial Sequence
<220>
   <223> Engineered polypeptide
<400> 323
   Glu Trp Lys His Ala Ala Gly Glu Ile Trp Gln Leu Pro Asn Leu Asn
   1               5                   10                  15
   Arg Ala Gln Lys Ala Ala Phe Ile Ser Ser Leu Ala Asp
               20                  25
<210> 324
   <211> 29
   <212> PRT
   <213> Artificial Sequence
<220>
   <223> Engineered polypeptide
<400> 324
   Glu Trp Lys Lys Ala Ala Tyr Glu Ile Trp Gln Leu Pro Asn Leu Asn
   1               5                   10                  15
   Arg Ala Gln Lys Ala Ala Phe Ile Ser Ser Leu Tyr Asp
               20                  25
<210> 325
   <211> 29
   <212> PRT
   <213> Artificial Sequence
<220>
   <223> Engineered polypeptide
<400> 325
   Glu Trp Lys Leu Ala Ala Tyr Glu Ile Trp Gln Leu Pro Asn Leu Asn
   1               5                   10                  15
   Arg His Gln Lys Ala Ala Phe Ile Ser Ser Leu Ser Asp
               20                  25
<210> 326
   <211> 29
   <212> PRT
   <213> Artificial Sequence
<220>
   <223> Engineered polypeptide
<400> 326
   Glu Trp Lys Arg Ala Ala Phe Glu Ile Trp Gln Leu Pro Asn Leu Asn
   1               5                   10                  15
   Arg Ser Gln Lys Ala Ala Phe Ile Ser Ser Leu Ala Asp
               20                  25
<210> 327
   <211> 29
   <212> PRT
   <213> Artificial Sequence
<220>
   <223> Engineered polypeptide
<400> 327
   Glu Trp Lys Thr Ala Ala Phe Glu Ile Trp Gln Leu Pro Asn Leu Asn
   1               5                   10                  15
   Arg Ser Gln Lys Ala Ala Phe Ile Ala Ser Leu Ala Asp
               20                  25
<210> 328
   <211> 29
   <212> PRT
   <213> Artificial Sequence
<220>
   <223> Engineered polypeptide
<400> 328
   Glu Trp Lys Ile Ala Ala Phe Glu Ile Trp Gln Leu Pro Asn Leu Asn
   1               5                   10                  15
   Arg Ile Gln Lys Ala Ala Phe Ile Gly Ser Leu Ala Asp
               20                  25
<210> 329
   <211> 29
   <212> PRT
   <213> Artificial Sequence
<220>
   <223> Engineered polypeptide
<400> 329
   Glu Trp Lys Gln Ala Ala Phe Glu Ile Trp Gln Leu Pro Asn Leu Asn
   1 5 10 15
   Arg Ile Gln Lys Ala Ala Phe Ile Thr Ser Leu Ser Asp
   20 25
<210> 330
   <211> 29
   <212> PRT
   <213> Artificial Sequence
<220>
   <223> Engineered polypeptide
<400> 330
   Glu Trp Lys Gln Ala Ala Phe Glu Ile Trp Gln Leu Pro Asn Leu Asn
   1 5 10 15
   Arg Val Gln Lys Ala Ala Phe Ile Val Ser Leu Gln Asp
   20 25
<210> 331
   <211> 29
   <212> PRT
   <213> Artificial Sequence
<220>
   <223> Engineered polypeptide
<400> 331
   Glu Trp Lys Val Ala Ala Gly Glu Ile Trp Gln Leu Pro Asn Leu Asn
   1 5 10 15
   Arg Ile Gln Lys Ala Ala Phe Ile Gly Ser Leu Ser Asp
   20 25
<210> 332
   <211> 29
   <212> PRT
   <213> Artificial Sequence
<220>
   <223> Engineered polypeptide
<400> 332
   Glu Trp Lys Ile Ala Ala Phe Glu Ile Trp Gln Leu Pro Asn Leu Asn
   1 5 10 15
   Arg Trp Gln Lys Ala Ala Phe Ile Ser Ser Leu Gln Asp
   20 25
<210> 333
   <211> 29
   <212> PRT
   <213> Artificial Sequence
<220>
   <223> Engineered polypeptide
<400> 333
   Glu Trp Lys Glu Ala Ala Gly Glu Ile Trp Gln Leu Pro Asn Leu Asn
   1 5 10 15
   Arg Tyr Gln Lys Ala Ala Phe Ile Ala Ser Leu Gln Asp
   20 25
<210> 334
   <211> 29
   <212> PRT
   <213> Artificial Sequence
<220>
   <223> Engineered polypeptide
<400> 334
   Glu Trp Lys Thr Ala Ser Phe Glu Ile Trp Gln Leu Pro Asn Leu Asn
   1 5 10 15
   Arg Tyr Gln Lys Ala Ala Phe Ile Ser Ser Leu Gln Asp
   20 25
<210> 335
   <211> 49
   <212> PRT
   <213> Artificial Sequence
<220>
   <223> Engineered polypeptide
<400> 335
   Lys Glu Lys Val Ala Ala Thr Gly Glu Ile Trp Asp Leu Pro Asn Leu
   1 5 10 15
   Asn Thr Arg Gln Lys Asn Ala Phe Ile Gly Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 336
   <211> 49
   <212> PRT
   <213> Artificial Sequence
<220>
   <223> Engineered polypeptide
<400> 336
   Lys Glu Trp Arg Trp Ala Ala His Glu Ile Trp Asp Leu Pro Asn Leu
   1               5                   10                  15
   Asn Val Tyr Gln Arg Ala Ala Phe Ile Arg Ser Leu Glu Asp Asp Pro
               20                  25                  30
   Ser Gln Ser Ala Asn Leu Leu Ala Glu Ala Lys Lys Leu Asn Asp Ala
           35                  40                  45
   Gln
<210> 337
   <211> 49
   <212> PRT
   <213> Artificial Sequence
<220>
   <223> Engineered polypeptide
<400> 337
   Lys Glu Arg Arg Leu Ala Ser Val Glu Ile Trp Glu Leu Pro Asn Leu
   1               5                   10                  15
   Asn Ala Val Gln Lys Ser Ala Phe Ile Ser Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 338
   <211> 49
   <212> PRT
   <213> Artificial Sequence
<220>
   <223> Engineered polypeptide
<400> 338
   Lys Glu Val Lys Glu Ala Arg Phe Glu Ile Trp Asp Leu Pro Asn Leu
   1               5                   10                  15
   Asn Arg Thr Gln Lys His Ala Phe Ile Val Ser Leu Glu Asp Asp Pro
               20                  25                  30
   Ser Gln Ser Ala Asn Leu Leu Ala Glu Ala Lys Lys Leu Asn Asp Ala
           35                  40                  45
   Gln
<210> 339
   <211> 49
   <212> PRT
   <213> Artificial Sequence
<220>
   <223> Engineered polypeptide
<400> 339
   Lys Glu Trp Lys Gly Ala Ala Gly Glu Ile Trp Asn Leu Pro Asn Leu
   1               5                   10                  15
   Asn Val Ser Gln Arg Val Ala Phe Ile Gly Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 340
   <211> 49
   <212> PRT
   <213> Artificial Sequence
<220>
   <223> Engineered polypeptide
<400> 340
   Lys Glu Lys Tyr Lys Ala Ala His Glu Ile Trp Glu Leu Pro Asn Leu
   1               5                   10                  15
   Asn Arg Asp Gln Arg Ala Ala Phe Ile Thr Ser Leu Thr Asp Asp Pro
               20                  25                  30
   Ser Gln Ser Ala Asn Leu Leu Ala Glu Ala Lys Lys Leu Asn Asp Ala
           35                  40                  45
   Gln
<210> 341
   <211> 49
   <212> PRT
   <213> Artificial Sequence
<220>
   <223> Engineered polypeptide
<400> 341
   Lys Glu Arg Ser Leu Ala Ser Phe Glu Ile Trp Glu Leu Pro Asn Leu
   1 5 10 15
   Asn Pro Lys Gln Lys Ala Ala Phe Ile Val Ser Leu Phe Asp Asp Pro
   20 25 30
   Ser Gln Ser Ala Asn Leu Leu Ala Glu Ala Lys Lys Leu Asn Asp Ala
   35 40 45
   Gln
<210> 342
   <211> 49
   <212> PRT
   <213> Artificial Sequence
<220>
   <223> Engineered polypeptide
<400> 342
   Lys Glu Trp Lys Arg Ala Ser Trp Glu Ile Trp Glu Leu Pro Asn Leu
   1 5 10 15
   Asn Asn Ala Gln Lys Arg Ala Phe Ile Ser Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 343
   <211> 49
   <212> PRT
   <213> Artificial Sequence
<220>
   <223> Engineered polypeptide
<400> 343
   Lys Glu Lys Tyr Lys Ala Met Thr Glu Ile Trp Ile Leu Pro Asn Leu
   1               5                   10                  15
   Asn Gln Arg Gln Lys Val Ala Phe Ile Gly Ser Leu Asp Asp Asp Pro
               20                  25                  30
   Ser Gln Ser Ala Asn Leu Leu Ala Glu Ala Lys Lys Leu Asn Asp Ala
           35                  40                  45
   Gln
<210> 344
   <211> 49
   <212> PRT
   <213> Artificial Sequence
<220>
   <223> Engineered polypeptide
<400> 344
   Lys Glu Trp Arg Gly Ala Ala Gly Glu Ile Trp Ala Leu Pro Asn Leu
   1               5                   10                  15
   Asn Asn Arg Gln Lys Gly Ala Phe Ile Glu Ser Leu Pro Asp Asp Pro
               20                  25                  30
   Ser Gln Ser Ala Asn Leu Leu Ala Glu Ala Lys Lys Leu Asn Asp Ala
           35                  40                  45
   Gln
<210> 345
   <211> 49
   <212> PRT
   <213> Artificial Sequence
<220>
   <223> Engineered polypeptide
<400> 345
   Lys Glu Arg Trp Glu Ala Thr Val Glu Ile Trp Asp Leu Pro Asn Leu
   1 5 10 15
   Asn Arg Asn Gln Lys Ala Ala Phe Ile Ala Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 346
   <211> 49
   <212> PRT
   <213> Artificial Sequence
<220>
   <223> Engineered polypeptide
<400> 346
   Lys Glu Lys Tyr Asn Ala Tyr Ala Glu Ile Trp Leu Leu Pro Asn Leu
   1 5 10 15
   Asn Arg Tyr Gln Lys Ala Ala Phe Ile Gly Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 347
   <211> 49
   <212> PRT
   <213> Artificial Sequence
<220>
   <223> Engineered polypeptide
<400> 347
   Lys Glu Arg Ser Val Ala Gln Lys Glu Ile Trp Glu Leu Pro Asn Leu
   1 5 10 15
   Asn Arg Trp Gln Ala Gly Ala Phe Ile Lys Ser Leu Tyr Asp Asp Pro
   20 25 30
   Ser Gln Ser Ala Asn Leu Leu Ala Glu Ala Lys Lys Leu Asn Asp Ala
   35 40 45
   Gln
<210> 348
   <211> 49
   <212> PRT
   <213> Artificial Sequence
<220>
   <223> Engineered polypeptide
<400> 348
   Lys Glu Lys Met Asp Ala Met Gly Glu Ile Trp Asp Leu Pro Asn Leu
   1 5 10 15
   Asn Arg Gly Gln Ala Ser Ala Phe Ile Ala Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 349
   <211> 49
   <212> PRT
   <213> Artificial Sequence
<220>
   <223> Engineered polypeptide
<400> 349
   Lys Glu Lys Arg Asn Ala Gln Val Glu Ile Trp Thr Leu Pro Asn Leu
   1               5                   10                  15
   Asn Ser Lys Gln Arg Ala Ala Phe Ile Lys Ser Leu Tyr Asp Asp Pro
               20                  25                  30
   Ser Gln Ser Ala Asn Leu Leu Ala Glu Ala Lys Lys Leu Asn Asp Ala
           35                  40                  45
   Gln
<210> 350
   <211> 49
   <212> PRT
   <213> Artificial Sequence
<220>
   <223> Engineered polypeptide
<400> 350
   Lys Glu Arg Arg Asp Ala Arg Phe Glu Ile Trp Glu Leu Pro Asn Leu
   1               5                   10                  15
   Asn Lys Tyr Gln Arg Ala Ala Phe Ile Ser Ser Leu Asp Asp Asp Pro
               20                  25                  30
   Ser Gln Ser Ala Asn Leu Leu Ala Glu Ala Lys Lys Leu Asn Asp Ala
           35                  40                  45
   Gln
<210> 351
   <211> 49
   <212> PRT
   <213> Artificial Sequence
<220>
   <223> Engineered polypeptide
<400> 351
   Lys Glu Arg Ser Met Ala Arg Phe Glu Ile Trp Glu Leu Pro Asn Leu
   1               5                   10                  15
   Asn Arg Gly Gln Lys Ser Ala Phe Ile Ala Ser Leu Glu Asp Asp Pro
               20                  25                  30
   Ser Gln Ser Ala Asn Leu Leu Ala Glu Ala Lys Lys Leu Asn Asp Ala
           35                  40                  45
   Gln
<210> 352
   <211> 49
   <212> PRT
   <213> Artificial Sequence
<220>
   <223> Engineered polypeptide
<400> 352
   Lys Glu Trp His Gly Ala Ala Ser Glu Ile Trp Glu Leu Pro Asn Leu
   1               5                   10                  15
   Asn Lys Ser Gln Lys Ser Ala Phe Ile Lys Ser Leu Pro Asp Asp Pro
               20                  25                  30
   Ser Gln Ser Ala Asn Leu Leu Ala Glu Ala Lys Lys Leu Asn Asp Ala
           35                  40                  45
   Gln
<210> 353
   <211> 49
   <212> PRT
   <213> Artificial Sequence
<220>
   <223> Engineered polypeptide
<400> 353
   Lys Glu Trp Lys Gln Ala Ala Glu Glu Ile Trp Asp Leu Pro Asn Leu
   1 5 10 15
   Asn Arg Arg Gln Ala Gly Ala Phe Ile Thr Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 354
   <211> 49
   <212> PRT
   <213> Artificial Sequence
<220>
   <223> Engineered polypeptide
<400> 354
   Lys Glu Lys Val Gln Ala Ser Glu Glu Ile Trp Asn Leu Pro Asn Leu
   1 5 10 15
   Asn Arg Arg Gln Arg Ala Ala Phe Ile Gly Ser Leu Tyr Asp Asp Pro
   20 25 30
   Ser Gln Ser Ala Asn Leu Leu Ala Glu Ala Lys Lys Leu Asn Asp Ala
   35 40 45
   Gln
<210> 355
   <211> 49
   <212> PRT
   <213> Artificial Sequence
<220>
   <223> Engineered polypeptide
<400> 355
   Lys Glu Arg Tyr Ser Ala Thr Val Glu Ile Trp Asp Leu Pro Asn Leu
   1 5 10 15
   Asn Thr Leu Gln Lys Ser Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 356
   <211> 49
   <212> PRT
   <213> Artificial Sequence
<220>
   <223> Engineered polypeptide
<400> 356
   Lys Glu Arg Tyr Ser Ala Tyr Tyr Glu Ile Trp Gln Leu Pro Asn Leu
   1 5 10 15
   Asn Arg Ile Gln Lys Ala Ala Phe Ile Ser Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 357
   <211> 49
   <212> PRT
   <213> Artificial Sequence
<220>
   <223> Engineered polypeptide
<400> 357
   Lys Glu Arg Tyr Arg Ala Tyr Phe Glu Ile Trp Gln Leu Pro Asn Leu
   1 5 10 15
   Asn Arg Leu Gln Lys Ala Ala Phe Ile Ser Ser Leu Glu Asp Asp Pro
   20 25 30
   Ser Gln Ser Ala Asn Leu Leu Ala Glu Ala Lys Lys Leu Asn Asp Ala
   35 40 45
   Gln
<210> 358
   <211> 49
   <212> PRT
   <213> Artificial Sequence
<220>
   <223> Engineered polypeptide
<400> 358
   Lys Glu Lys Tyr Lys Ala Tyr Gly Glu Ile Trp Gln Leu Pro Asn Leu
   1               5                   10                  15
   Asn Arg Val Gln Lys Ala Ala Phe Ile Ala Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 359
   <211> 49
   <212> PRT
   <213> Artificial Sequence
<220>
   <223> Engineered polypeptide
<400> 359
   Lys Glu Arg Lys Gln Ala Thr Phe Glu Ile Trp Glu Leu Pro Asn Leu
   1               5                   10                  15
   Asn Gln Arg Gln Lys Ala Ala Phe Ile Lys Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 360
   <211> 49
   <212> PRT
   <213> Artificial Sequence
<220>
   <223> Engineered polypeptide
<400> 360
   Lys Glu Trp Lys Ile Ala Ala Gly Glu Ile Trp Gln Leu Pro Asn Leu
   1               5                   10                  15
   Asn Arg His Gln Lys Gly Ala Phe Ile Ser Ser Leu Tyr Asp Asp Pro
               20                  25                  30
   Ser Gln Ser Ala Asn Leu Leu Ala Glu Ala Lys Lys Leu Asn Asp Ala
           35                  40                  45
   Gln
<210> 361
   <211> 49
   <212> PRT
   <213> Artificial Sequence
<220>
   <223> Engineered polypeptide
<400> 361
   Lys Glu Lys Tyr Lys Ala Tyr Val Glu Ile Trp Gln Leu Pro Asn Leu
   1               5                   10                  15
   Asn Arg Tyr Gln Arg Ala Ala Phe Ile Gly Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 362
   <211> 49
   <212> PRT
   <213> Artificial Sequence
<220>
   <223> Engineered polypeptide
<400> 362
   Lys Glu Arg Lys Ala Ala Thr Phe Glu Ile Trp Glu Leu Pro Asn Leu
   1               5                   10                  15
   Asn Arg Ile Gln Lys Ala Ala Phe Ile Ala Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 363
   <211> 49
   <212> PRT
   <213> Artificial Sequence
<220>
   <223> Engineered polypeptide
<400> 363
   Lys Glu Lys Tyr Lys Ala Tyr Gly Glu Ile Trp Gln Leu Pro Asn Leu
   1               5                   10                  15
   Asn Arg Ile Gln Lys Ala Ala Phe Ile Ser Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 364
   <211> 49
   <212> PRT
   <213> Artificial Sequence
<220>
   <223> Engineered polypeptide
<400> 364
   Lys Glu Lys Arg Ile Ala Thr Trp Glu Ile Trp Gln Leu Pro Asn Leu
   1 5 10 15
   Asn Gln His Gln Lys Ala Ala Phe Ile Ser Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 365
   <211> 49
   <212> PRT
   <213> Artificial Sequence
<220>
   <223> Engineered polypeptide
<400> 365
   Lys Glu Lys Tyr Lys Ala Tyr Val Glu Ile Trp Gln Leu Pro Asn Leu
   1 5 10 15
   Asn Arg Gly Gln Lys Ala Ala Phe Ile Ser Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 366
   <211> 49
   <212> PRT
   <213> Artificial Sequence
<220>
   <223> Engineered polypeptide
<400> 366
   Lys Glu Arg Tyr Leu Ala Tyr Tyr Glu Ile Trp Gln Leu Pro Asn Leu
   1 5 10 15
   Asn Arg Thr Gln Lys Ala Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 367
   <211> 49
   <212> PRT
   <213> Artificial Sequence
<220>
   <223> Engineered polypeptide
<400> 367
   Lys Glu Arg Tyr Ile Ala Tyr Trp Glu Ile Trp Gln Leu Pro Asn Leu
   1 5 10 15
   Asn Arg Arg Gln Lys Ala Ala Phe Ile Ser Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 368
   <211> 49
   <212> PRT
   <213> Artificial Sequence
<220>
   <223> Engineered polypeptide
<400> 368
   Lys Glu Lys Tyr Arg Ala Tyr Gly Glu Ile Trp Gln Leu Pro Asn Leu
   1 5 10 15
   Asn Arg Gly Gln Lys Ala Ala Phe Ile Ala Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 369
   <211> 49
   <212> PRT
   <213> Artificial Sequence
<220>
   <223> Engineered polypeptide
<400> 369
   Lys Glu Lys Tyr Thr Ala Tyr Phe Glu Ile Trp Gln Leu Pro Asn Leu
   1 5 10 15
   Asn Val Arg Gln Lys Ala Ala Phe Ile Ser Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 370
   <211> 49
   <212> PRT
   <213> Artificial Sequence
<220>
   <223> Engineered polypeptide
<400> 370
   Lys Glu Arg Tyr Ser Ala Tyr Tyr Glu Ile Trp Gln Leu Pro Asn Leu
   1 5 10 15
   Asn Val Arg Gln Lys Ala Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 371
   <211> 49
   <212> PRT
   <213> Artificial Sequence
<220>
   <223> Engineered polypeptide
<400> 371
   Lys Glu Lys Tyr Ala Ala Tyr Gly Glu Ile Trp Gln Leu Pro Asn Leu
   1               5                   10                  15
   Asn Arg Ser Gln Lys Ala Ala Phe Ile Gly Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 372
   <211> 49
   <212> PRT
   <213> Artificial Sequence
<220>
   <223> Engineered polypeptide
<400> 372
   Lys Glu Arg Lys Arg Ala Thr Val Glu Ile Trp Glu Leu Pro Asn Leu
   1               5                   10                  15
   Asn Arg Leu Gln Arg Gly Ala Phe Ile Ala Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 373
   <211> 49
   <212> PRT
   <213> Artificial Sequence
<220>
   <223> Engineered polypeptide
<400> 373
   Lys Glu Trp Lys Gln Ala Ser Phe Glu Ile Trp Glu Leu Pro Asn Leu
   1               5                   10                  15
   Asn Arg Leu Gln Lys Ala Ala Phe Ile Gly Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 374
   <211> 49
   <212> PRT
   <213> Artificial Sequence
<220>
   <223> Engineered polypeptide
<400> 374
   Lys Glu Arg Lys His Ala Thr Val Glu Ile Trp Glu Leu Pro Asn Leu
   1               5                   10                  15
   Asn Arg Val Gln Lys Ala Ala Phe Ile Ser Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 375
   <211> 49
   <212> PRT
   <213> Artificial Sequence
<220>
   <223> Engineered polypeptide
<400> 375
   Lys Glu Lys Tyr Lys Ala Tyr Val Glu Ile Trp Gln Leu Pro Asn Leu
   1 5 10 15
   Asn Gln Arg Gln Lys Ala Ala Phe Ile Gly Ser Leu Ala Asp Asp Pro
   20 25 30
   Ser Gln Ser Ala Asn Leu Leu Ala Glu Ala Lys Lys Leu Asn Asp Ala
   35 40 45
   Gln
<210> 376
   <211> 49
   <212> PRT
   <213> Artificial Sequence
<220>
   <223> Engineered polypeptide
<400> 376
   Lys Glu Lys Tyr Val Ala Tyr Gly Glu Ile Trp Gln Leu Pro Asn Leu
   1 5 10 15
   Asn Arg Thr Gln Lys Ala Ala Phe Ile Ser Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 377
   <211> 49
   <212> PRT
   <213> Artificial Sequence
<220>
   <223> Engineered polypeptide
<400> 377
   Lys Glu Arg Tyr Ile Ala Tyr Tyr Glu Ile Trp Gln Leu Pro Asn Leu
   1               5                   10                  15
   Asn Arg Tyr Gln Lys Ala Ala Phe Ile Ala Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 378
   <211> 49
   <212> PRT
   <213> Artificial Sequence
<220>
   <223> Engineered polypeptide
<400> 378
   Lys Glu Lys Tyr Asn Ala Tyr Gly Glu Ile Trp Gln Leu Pro Asn Leu
   1               5                   10                  15
   Asn Val Arg Gln Lys Ala Ala Phe Ile Ser Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 379
   <211> 49
   <212> PRT
   <213> Artificial Sequence
<220>
   <223> Engineered polypeptide
<400> 379
   Lys Glu Arg Lys Ala Ala Thr Val Glu Ile Trp Glu Leu Pro Asn Leu
   1               5                   10                  15
   Asn Arg Val Gln Lys Ala Ala Phe Ile Lys Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 380
   <211> 49
   <212> PRT
   <213> Artificial Sequence
<220>
   <223> Engineered polypeptide
<400> 380
   Lys Glu Arg Tyr Val Ala Tyr Tyr Glu Ile Trp Glu Leu Pro Asn Leu
   1               5                   10                  15
   Asn Gln Arg Gln Lys Ala Ala Phe Ile Gly Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 381
   <211> 49
   <212> PRT
   <213> Artificial Sequence
<220>
   <223> Engineered polypeptide
<400> 381
   Lys Glu Arg Tyr Arg Ala Tyr Tyr Glu Ile Trp Gln Leu Pro Asn Leu
   1 5 10 15
   Asn Gln Arg Gln Lys Ala Ala Phe Ile Gly Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 382
   <211> 49
   <212> PRT
   <213> Artificial Sequence
<220>
   <223> Engineered polypeptide
<400> 382
   Lys Glu Trp Lys Ser Ala Ala Phe Glu Ile Trp Glu Leu Pro Asn Leu
   1 5 10 15
   Asn Arg Leu Gln Lys Ala Ala Phe Ile Arg Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 383
   <211> 49
   <212> PRT
   <213> Artificial Sequence
<220>
   <223> Engineered polypeptide
<400> 383
   Lys Glu Arg Lys Gln Ala Thr Phe Glu Ile Trp Glu Leu Pro Asn Leu
   1               5                   10                  15
   Asn Arg His Gln Lys Gly Ala Phe Ile Ala Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 384
   <211> 49
   <212> PRT
   <213> Artificial Sequence
<220>
   <223> Engineered polypeptide
<400> 384
   Lys Glu Trp Lys Ile Ala Ala Gly Glu Ile Trp Gln Leu Pro Asn Leu
   1               5                   10                  15
   Asn Arg Arg Gln Lys Ala Ala Phe Ile Ala Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 385
   <211> 49
   <212> PRT
   <213> Artificial Sequence
<220>
   <223> Engineered polypeptide
<400> 385
   Lys Glu Arg Tyr Leu Ala Tyr Tyr Glu Ile Trp Gln Leu Pro Asn Leu
   1               5                   10                  15
   Asn Arg Arg Gln Lys Ala Ala Phe Ile Gly Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 386
   <211> 49
   <212> PRT
   <213> Artificial Sequence
<220>
   <223> Engineered polypeptide
<400> 386
   Lys Glu Arg Tyr His Ala Tyr Tyr Glu Ile Trp Glu Leu Pro Asn Leu
   1               5                   10                  15
   Asn Arg Leu Gln Lys Ala Ala Phe Ile Ser Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 387
   <211> 49
   <212> PRT
   <213> Artificial Sequence
<220>
   <223> Engineered polypeptide
<400> 387
   Lys Glu Lys Tyr Lys Ala Tyr Ala Glu Ile Trp Gln Leu Pro Asn Leu
   1               5                   10                  15
   Asn Arg Thr Gln Lys Ala Ala Phe Ile Ser Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 388
   <211> 49
   <212> PRT
   <213> Artificial Sequence
<220>
   <223> Engineered polypeptide
<400> 388
   Lys Glu Arg Tyr Lys Ala Tyr Tyr Glu Ile Trp Gln Leu Pro Asn Leu
   1               5                   10                  15
   Asn Arg Gln Gln Lys Gly Ala Phe Ile Ser Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 389
   <211> 49
   <212> PRT
   <213> Artificial Sequence
<220>
   <223> Engineered polypeptide
<400> 389
   Lys Glu Arg Lys Leu Ala Thr Phe Glu Ile Trp Glu Leu Pro Asn Leu
   1               5                   10                  15
   Asn Thr Ser Gln Lys Gly Ala Phe Ile Ser Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 390
   <211> 49
   <212> PRT
   <213> Artificial Sequence
<220>
   <223> Engineered polypeptide
<400> 390
   Lys Glu Arg Tyr Val Ala Tyr Tyr Glu Ile Trp Gln Leu Pro Asn Leu
   1               5                   10                  15
   Asn Val Gln Gln Lys Ala Ala Phe Ile Ala Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 391
   <211> 49
   <212> PRT
   <213> Artificial Sequence
<220>
   <223> Engineered polypeptide
<400> 391
   Lys Glu Arg Ser Ile Ala Ser Val Glu Ile Trp Glu Leu Pro Asn Leu
   1               5                   10                  15
   Asn Gln Arg Gln Lys Gly Ala Phe Ile Ala Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 392
   <211> 49
   <212> PRT
   <213> Artificial Sequence
<220>
   <223> Engineered polypeptide
<400> 392
   Lys Glu Arg Lys Gln Ala Ala Val Glu Ile Trp Glu Leu Pro Asn Leu
   1               5                   10                  15
   Asn Val Arg Gln Lys Ala Ala Phe Ile Arg Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 393
   <211> 49
   <212> PRT
   <213> Artificial Sequence
<220>
   <223> Engineered polypeptide
<400> 393
   Lys Glu Lys Lys Ile Ala Ala Phe Glu Ile Trp Glu Leu Pro Asn Leu
   1               5                   10                  15
   Asn Gln Trp Gln Lys Ala Ala Phe Ile Ala Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 394
   <211> 49
   <212> PRT
   <213> Artificial Sequence
<220>
   <223> Engineered polypeptide
<400> 394
   Lys Glu Arg Lys Ile Ala Thr Val Glu Ile Trp Glu Leu Pro Asn Leu
   1               5                   10                  15
   Asn Arg Ile Gln Lys Gly Ala Phe Ile Ala Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 395
   <211> 49
   <212> PRT
   <213> Artificial Sequence
<220>
   <223> Engineered polypeptide
<400> 395
   Lys Glu Lys Tyr Arg Ala Ala Gly Glu Ile Trp Glu Leu Pro Asn Leu
   1               5                   10                  15
   Asn Val Leu Gln Lys Ala Ala Phe Ile Lys Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 396
   <211> 49
   <212> PRT
   <213> Artificial Sequence
<220>
   <223> Engineered polypeptide
<400> 396
   Lys Glu Arg Lys Ile Ala Ala Val Glu Ile Trp Glu Leu Pro Asn Leu
   1 5 10 15
   Asn Arg Tyr Gln Lys Ala Ala Phe Ile Lys Ser Leu Phe Asp Asp Pro
   20 25 30
   Ser Gln Ser Ala Asn Leu Leu Ala Glu Ala Lys Lys Leu Asn Asp Ala
   35 40 45
   Gln
<210> 397
   <211> 49
   <212> PRT
   <213> Artificial Sequence
<220>
   <223> Engineered polypeptide
<400> 397
   Lys Glu Lys Arg Thr Ala Thr Trp Glu Ile Trp Gln Leu Pro Asn Leu
   1 5 10 15
   Asn Gln Arg Gln Arg Ala Ala Phe Ile Gly Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 398
   <211> 49
   <212> PRT
   <213> Artificial Sequence
<220>
   <223> Engineered polypeptide
<400> 398
   Lys Glu Arg Lys Thr Ala Thr Val Glu Ile Trp Glu Leu Pro Asn Leu
   1               5                   10                  15
   Asn Arg Arg Gln Lys Ala Ala Phe Ile Ala Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 399
   <211> 49
   <212> PRT
   <213> Artificial Sequence
<220>
   <223> Engineered polypeptide
<400> 399
   Lys Glu Arg Lys Gln Ala Ala Val Glu Ile Trp Glu Leu Pro Asn Leu
   1               5                   10                  15
   Asn Arg Ile Gln Lys Gly Ala Phe Ile Lys Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 400
   <211> 49
   <212> PRT
   <213> Artificial Sequence
<220>
   <223> Engineered polypeptide
<400> 400
   Lys Glu Arg Tyr Ile Ala Tyr Gly Glu Ile Trp Gln Leu Pro Asn Leu
   1               5                   10                  15
   Asn Arg Arg Gln Lys Ala Ala Phe Ile Gly Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 401
   <211> 49
   <212> PRT
   <213> Artificial Sequence
<220>
   <223> Engineered polypeptide
<400> 401
   Lys Glu Arg Tyr Arg Ala Tyr Tyr Glu Ile Trp Gln Leu Pro Asn Leu
   1               5                   10                  15
   Asn Arg Thr Gln Lys Val Ala Phe Ile Gly Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 402
   <211> 49
   <212> PRT
   <213> Artificial Sequence
<220>
   <223> Engineered polypeptide
<400> 402
   Lys Glu Arg Arg Leu Ala Tyr Trp Glu Ile Trp Gln Leu Pro Asn Leu
   1               5                   10                  15
   Asn Thr Thr Gln Lys Ala Ala Phe Ile Gly Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 403
   <211> 49
   <212> PRT
   <213> Artificial Sequence
<220>
   <223> Engineered polypeptide
<400> 403
   Lys Glu Arg Arg Arg Ala Tyr Tyr Glu Ile Trp Gln Leu Pro Asn Leu
   1 5 10 15
   Asn Arg Ile Gln Lys Ala Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 404
   <211> 49
   <212> PRT
   <213> Artificial Sequence
<220>
   <223> Engineered polypeptide
<400> 404
   Lys Glu Arg Arg Leu Ala Tyr Tyr Glu Ile Trp Glu Leu Pro Asn Leu
   1 5 10 15
   Asn Val Ala Gln Lys Ala Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 405
   <211> 49
   <212> PRT
   <213> Artificial Sequence
<220>
   <223> Engineered polypeptide
<400> 405
   Lys Glu Arg Tyr Leu Ala Tyr Tyr Glu Ile Trp Glu Leu Pro Asn Leu
   1               5                   10                  15
   Asn Val Arg Gln Lys Ala Ala Phe Ile Gly Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 406
   <211> 49
   <212> PRT
   <213> Artificial Sequence
<220>
   <223> Engineered polypeptide
<400> 406
   Lys Glu Arg Tyr Ala Ala Tyr Tyr Glu Ile Trp Gln Leu Pro Asn Leu
   1               5                   10                  15
   Asn Ser Arg Gln Lys Ala Ala Phe Ile Gly Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 407
   <211> 49
   <212> PRT
   <213> Artificial Sequence
<220>
   <223> Engineered polypeptide
<400> 407
   Lys Glu Arg Tyr Leu Ala Tyr Tyr Glu Ile Trp Gln Leu Pro Asn Leu
   1               5                   10                  15
   Asn Gln Leu Gln Lys Ala Ala Phe Ile Gly Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 408
   <211> 49
   <212> PRT
   <213> Artificial Sequence
<220>
   <223> Engineered polypeptide
<400> 408
   Lys Glu Arg Tyr His Ala Tyr Tyr Glu Ile Trp Gln Leu Pro Asn Leu
   1               5                   10                  15
   Asn Ser Trp Gln Lys Ala Ala Phe Ile Gly Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 409
   <211> 49
   <212> PRT
   <213> Artificial Sequence
<220>
   <223> Engineered polypeptide
<400> 409
   Lys Glu Arg Tyr His Ala Tyr Tyr Glu Ile Trp Gln Leu Pro Asn Leu
   1 5 10 15
   Asn Ser Ala Gln Lys Ala Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 410
   <211> 49
   <212> PRT
   <213> Artificial Sequence
<220>
   <223> Engineered polypeptide
<400> 410
   Lys Glu Arg Tyr Glu Ala Tyr Tyr Glu Ile Trp Gln Leu Pro Asn Leu
   1 5 10 15
   Asn Ser Val Gln Lys Ala Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 411
   <211> 49
   <212> PRT
   <213> Artificial Sequence
<220>
   <223> Engineered polypeptide
<400> 411
   Lys Glu Arg Tyr His Ala Tyr Tyr Glu Ile Trp Gln Leu Pro Asn Leu
   1 5 10 15
   Asn Arg Arg Gln Lys Ala Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 412
   <211> 49
   <212> PRT
   <213> Artificial Sequence
<220>
   <223> Engineered polypeptide
<400> 412
   Lys Glu Arg Tyr Thr Ala Tyr Tyr Glu Ile Trp Gln Leu Pro Asn Leu
   1 5 10 15
   Asn Arg Ala Gln Lys Ala Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 413
   <211> 49
   <212> PRT
   <213> Artificial Sequence
<220>
   <223> Engineered polypeptide
<400> 413
   Lys Glu Arg Tyr Ser Ala Tyr Tyr Glu Ile Trp Gln Leu Pro Asn Leu
   1 5 10 15
   Asn Arg Ala Gln Lys Ala Ala Phe Ile Gly Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 414
   <211> 49
   <212> PRT
   <213> Artificial Sequence
<220>
   <223> Engineered polypeptide
<400> 414
   Lys Glu Arg Tyr Ser Ala Tyr Tyr Glu Ile Trp Glu Leu Pro Asn Leu
   1 5 10 15
   Asn Arg Ala Gln Lys Ala Ala Phe Ile Gly Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 415
   <211> 49
   <212> PRT
   <213> Artificial Sequence
<220>
   <223> Engineered polypeptide
<400> 415
   Lys Glu Arg Tyr His Ala Tyr Tyr Glu Ile Trp Gln Leu Pro Asn Leu
   1 5 10 15
   Asn Arg Ala Gln Lys Ala Ala Phe Ile Gly Ser Leu Tyr Asp Asp Pro
   20 25 30
   Ser Gln Ser Ala Asn Leu Leu Ala Glu Ala Lys Lys Leu Asn Asp Ala
   35 40 45
   Gln
<210> 416
   <211> 49
   <212> PRT
   <213> Artificial Sequence
<220>
   <223> Engineered polypeptide
<400> 416
   Lys Glu Arg Tyr Ser Ala Tyr Val Glu Ile Trp Gln Leu Pro Asn Leu
   1 5 10 15
   Asn Arg Gly Gln Lys Ala Ala Phe Ile Gly Ser Leu Tyr Asp Asp Pro
   20 25 30
   Ser Gln Ser Ala Asn Leu Leu Ala Glu Ala Lys Lys Leu Asn Asp Ala
   35 40 45
   Gln
<210> 417
   <211> 49
   <212> PRT
   <213> Artificial Sequence
<220>
   <223> Engineered polypeptide
<400> 417
   Lys Glu Arg Tyr Ser Ala Tyr Phe Glu Ile Trp Gln Leu Pro Asn Leu
   1 5 10 15
   Asn Arg Leu Gln Lys Ala Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 418
   <211> 49
   <212> PRT
   <213> Artificial Sequence
<220>
   <223> Engineered polypeptide
<400> 418
   Lys Glu Arg Tyr Asn Ala Tyr Phe Glu Ile Trp Gln Leu Pro Asn Leu
   1               5                   10                  15
   Asn Arg Tyr Gln Lys Ser Ala Phe Ile Gly Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 419
   <211> 49
   <212> PRT
   <213> Artificial Sequence
<220>
   <223> Engineered polypeptide
<400> 419
   Lys Glu Arg Tyr Ala Ala Tyr Phe Glu Ile Trp Gln Leu Pro Asn Leu
   1               5                   10                  15
   Asn Val Ala Gln Lys Ala Ala Phe Ile Gly Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 420
   <211> 49
   <212> PRT
   <213> Artificial Sequence
<220>
   <223> Engineered polypeptide
<400> 420
   Lys Glu Arg Tyr Gln Ala Tyr Phe Glu Ile Trp Gln Leu Pro Asn Leu
   1               5                   10                  15
   Asn Gln Trp Gln Lys Ala Ala Phe Ile Gly Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 421
   <211> 49
   <212> PRT
   <213> Artificial Sequence
<220>
   <223> Engineered polypeptide
<400> 421
   Lys Glu Arg Tyr Lys Ala Tyr Trp Glu Ile Trp Gln Leu Pro Asn Leu
   1               5                   10                  15
   Asn Gln Gly Gln Lys Ala Ala Phe Ile Gly Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 422
   <211> 49
   <212> PRT
   <213> Artificial Sequence
<220>
   <223> Engineered polypeptide
<400> 422
   Lys Glu Arg Tyr Ile Ala Tyr Gly Glu Ile Trp Gln Leu Pro Asn Leu
   1 5 10 15
   Asn Val Gly Gln Lys Ala Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 423
   <211> 49
   <212> PRT
   <213> Artificial Sequence
<220>
   <223> Engineered polypeptide
<400> 423
   Lys Glu Arg Tyr Arg Ala Tyr Val Glu Ile Trp Gln Leu Pro Asn Leu
   1 5 10 15
   Asn Arg Val Gln Lys Ala Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 424
   <211> 49
   <212> PRT
   <213> Artificial Sequence
<220>
   <223> Engineered polypeptide
<400> 424
   Lys Glu Arg Tyr Lys Ala Tyr Val Glu Ile Trp Gln Leu Pro Asn Leu
   1 5 10 15
   Asn Arg Thr Gln Lys Ala Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 425
   <211> 49
   <212> PRT
   <213> Artificial Sequence
<220>
   <223> Engineered polypeptide
<400> 425
   Lys Glu Lys Tyr Glu Ala Tyr Gly Glu Ile Trp Glu Leu Pro Asn Leu
   1 5 10 15
   Asn Arg Ser Gln Lys Ala Ala Phe Ile Gly Ser Leu Glu Asp Asp Pro
   20 25 30
   Ser Gln Ser Ala Asn Leu Leu Ala Glu Ala Lys Lys Leu Asn Asp Ala
   35 40 45
   Gln
<210> 426
   <211> 49
   <212> PRT
   <213> Artificial Sequence
<220>
   <223> Engineered polypeptide
<400> 426
   Lys Glu Lys Tyr Gln Ala Tyr Gly Glu Ile Trp Gln Leu Pro Asn Leu
   1               5                   10                  15
   Asn Arg Ser Gln Lys Ala Ala Phe Ile Thr Ser Leu Glu Asp Asp Pro
               20                  25                  30
   Ser Gln Ser Ala Asn Leu Leu Ala Glu Ala Lys Lys Leu Asn Asp Ala
           35                  40                  45
   Gln
<210> 427
   <211> 49
   <212> PRT
   <213> Artificial Sequence
<220>
   <223> Engineered polypeptide
<400> 427
   Lys Glu Lys Tyr Ala Ala Tyr Gly Glu Ile Trp Leu Leu Pro Asn Leu
   1               5                   10                  15
   Asn Arg Ile Gln Lys Ala Ala Phe Ile Gly Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 428
   <211> 49
   <212> PRT
   <213> Artificial Sequence
<220>
   <223> Engineered polypeptide
<400> 428
   Lys Glu Lys Tyr Lys Ala Tyr Gly Glu Ile Trp Glu Leu Pro Asn Leu
   1 5 10 15
   Asn Arg Arg Gln Lys Val Ala Phe Ile Ser Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 429
   <211> 49
   <212> PRT
   <213> Artificial Sequence
<220>
   <223> Engineered polypeptide
<400> 429
   Lys Glu Lys Tyr Lys Ala Tyr Gly Glu Ile Trp Glu Leu Pro Asn Leu
   1 5 10 15
   Asn Arg Ser Gln Lys Ala Ala Phe Ile Ala Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 430
   <211> 49
   <212> PRT
   <213> Artificial Sequence
<220>
   <223> Engineered polypeptide
<400> 430
   Lys Glu Lys Tyr Lys Ala Tyr Gly Glu Ile Trp Gln Leu Pro Asn Leu
   1 5 10 15
   Asn Gln Arg Gln Lys Ala Ala Phe Ile Ala Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 431
   <211> 49
   <212> PRT
   <213> Artificial Sequence
<220>
   <223> Engineered polypeptide
<400> 431
   Lys Glu Lys Tyr Lys Ala Tyr Gly Glu Ile Trp Gln Leu Pro Asn Leu
   1               5                   10                  15
   Asn Arg Ser Gln Lys Ala Ala Phe Ile Ser Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 432
   <211> 49
   <212> PRT
   <213> Artificial Sequence
<220>
   <223> Engineered polypeptide
<400> 432
   Lys Glu Lys Tyr Lys Ala Tyr Gly Glu Ile Trp Gln Leu Pro Asn Leu
   1               5                   10                  15
   Asn Arg Arg Gln Lys Ala Ala Phe Ile Ser Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 433
   <211> 49
   <212> PRT
   <213> Artificial Sequence
<220>
   <223> Engineered polypeptide
<400> 433
   Lys Glu Lys Tyr Asn Ala Tyr Gly Glu Ile Trp Gln Leu Pro Asn Leu
   1 5 10 15
   Asn Arg Gly Gln Lys Ala Ala Phe Ile Ser Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 434
   <211> 49
   <212> PRT
   <213> Artificial Sequence
<220>
   <223> Engineered polypeptide
<400> 434
   Lys Glu Lys Tyr Val Ala Tyr Ala Glu Ile Trp Gln Leu Pro Asn Leu
   1 5 10 15
   Asn Arg Val Gln Lys Ala Ala Phe Ile Gly Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 435
   <211> 49
   <212> PRT
   <213> Artificial Sequence
<220>
   <223> Engineered polypeptide
<400> 435
   Lys Glu Lys Tyr Gln Ala Tyr Gly Glu Ile Trp Gln Leu Pro Asn Leu
   1 5 10 15
   Asn Arg Val Gln Lys Ala Ala Phe Ile Gly Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 436
   <211> 49
   <212> PRT
   <213> Artificial Sequence
<220>
   <223> Engineered polypeptide
<400> 436
   Lys Glu Lys Tyr His Ala Tyr Gly Glu Ile Trp Gln Leu Pro Asn Leu
   1 5 10 15
   Asn Arg Ile Gln Lys Ala Ala Phe Ile Ala Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 437
   <211> 49
   <212> PRT
   <213> Artificial Sequence
<220>
   <223> Engineered polypeptide
<400> 437
   Lys Glu Lys Tyr Thr Ala Tyr Val Glu Ile Trp Gln Leu Pro Asn Leu
   1 5 10 15
   Asn Arg Tyr Gln Arg Ala Ala Phe Ile Ala Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 438
   <211> 49
   <212> PRT
   <213> Artificial Sequence
<220>
   <223> Engineered polypeptide
<400> 438
   Lys Glu Lys Tyr Lys Ala Tyr Val Glu Ile Trp Gln Leu Pro Asn Leu
   1 5 10 15
   Asn Arg Gly Gln Lys Ala Ala Phe Ile Gly Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 439
   <211> 49
   <212> PRT
   <213> Artificial Sequence
<220>
   <223> Engineered polypeptide
<400> 439
   Lys Glu Lys Tyr Val Ala Tyr Val Glu Ile Trp Gln Leu Pro Asn Leu
   1 5 10 15
   Asn Arg Thr Gln Lys Ala Ala Phe Ile Ala Ser Leu Glu Asp Asp Pro
   20 25 30
   Ser Gln Ser Ala Asn Leu Leu Ala Glu Ala Lys Lys Leu Asn Asp Ala
   35 40 45
   Gln
<210> 440
   <211> 49
   <212> PRT
   <213> Artificial Sequence
<220>
   <223> Engineered polypeptide
<400> 440
   Lys Glu Lys Tyr Ile Ala Tyr Val Glu Ile Trp Gln Leu Pro Asn Leu
   1 5 10 15
   Asn Arg Thr Gln Lys Ala Ala Phe Ile Ala Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 441
   <211> 49
   <212> PRT
   <213> Artificial Sequence
<220>
   <223> Engineered polypeptide
<400> 441
   Lys Glu Lys Tyr Lys Ala Tyr Ala Glu Ile Trp Gln Leu Pro Asn Leu
   1               5                   10                  15
   Asn Arg Ala Gln Lys Ala Ala Phe Ile Gly Ser Leu Tyr Asp Asp Pro
               20                  25                  30
   Ser Gln Ser Ala Asn Leu Leu Ala Glu Ala Lys Lys Leu Asn Asp Ala
           35                  40                  45
   Gln
<210> 442
   <211> 49
   <212> PRT
   <213> Artificial Sequence
<220>
   <223> Engineered polypeptide
<400> 442
   Lys Glu Lys Tyr Thr Ala Tyr Gly Glu Ile Trp Gln Leu Pro Asn Leu
   1               5                   10                  15
   Asn Val Arg Gln Lys Ala Ala Phe Ile Ser Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 443
   <211> 49
   <212> PRT
   <213> Artificial Sequence
<220>
   <223> Engineered polypeptide
<400> 443
   Lys Glu Lys Tyr Thr Ala Tyr Gly Glu Ile Trp Gln Leu Pro Asn Leu
   1 5 10 15
   Asn Gln Arg Gln Lys Ala Ala Phe Ile Ser Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 444
   <211> 49
   <212> PRT
   <213> Artificial Sequence
<220>
   <223> Engineered polypeptide
<400> 444
   Lys Glu Lys Tyr Thr Ala Tyr Phe Glu Ile Trp Gln Leu Pro Asn Leu
   1 5 10 15
   Asn Gln Gly Gln Lys Ala Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 445
   <211> 49
   <212> PRT
   <213> Artificial Sequence
<220>
   <223> Engineered polypeptide
<400> 445
   Lys Glu Lys Tyr Gln Ala Tyr Val Glu Ile Trp Gln Leu Pro Asn Leu
   1 5 10 15
   Asn Gln Arg Gln Lys Ala Ala Phe Ile Gly Ser Leu Ala Asp Asp Pro
   20 25 30
   Ser Gln Ser Ala Asn Leu Leu Ala Glu Ala Lys Lys Leu Asn Asp Ala
   35 40 45
   Gln
<210> 446
   <211> 49
   <212> PRT
   <213> Artificial Sequence
<220>
   <223> Engineered polypeptide
<400> 446
   Lys Glu Lys Tyr Ala Ala Tyr Val Glu Ile Trp Gln Leu Pro Asn Leu
   1               5                   10                  15
   Asn Gln Arg Gln Lys Ala Ala Phe Ile Gly Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 447
   <211> 49
   <212> PRT
   <213> Artificial Sequence
<220>
   <223> Engineered polypeptide
<400> 447
   Lys Glu Lys Tyr Val Ala Tyr Val Glu Ile Trp Gln Leu Pro Asn Leu
   1               5                   10                  15
   Asn Thr Ser Gln Lys Ala Ala Phe Ile Gly Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 448
   <211> 49
   <212> PRT
   <213> Artificial Sequence
<220>
   <223> Engineered polypeptide
<400> 448
   Lys Glu Lys Tyr Gln Ala Tyr Ala Glu Ile Trp Gln Leu Pro Asn Leu
   1 5 10 15
   Asn Val Arg Gln Lys Ser Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 449
   <211> 49
   <212> PRT
   <213> Artificial Sequence
<220>
   <223> Engineered polypeptide
<400> 449
   Lys Glu Lys Tyr Gln Ala Tyr Tyr Glu Ile Trp Glu Leu Pro Asn Leu
   1 5 10 15
   Asn Val Gln Gln Lys Ser Ala Phe Ile Thr Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 450
   <211> 49
   <212> PRT
   <213> Artificial Sequence
<220>
   <223> Engineered polypeptide
<400> 450
   Lys Glu Lys Tyr His Ala Tyr Tyr Glu Ile Trp Gln Leu Pro Asn Leu
   1 5 10 15
   Asn Val His Gln Lys Ala Ala Phe Ile Arg Ser Leu Ala Asp Asp Pro
   20 25 30
   Ser Gln Ser Ala Asn Leu Leu Ala Glu Ala Lys Lys Leu Asn Asp Ala
   35 40 45
   Gln
<210> 451
   <211> 49
   <212> PRT
   <213> Artificial Sequence
<220>
   <223> Engineered polypeptide
<400> 451
   Lys Glu Lys Tyr Gln Ala Tyr Tyr Glu Ile Trp Gln Leu Pro Asn Leu
   1 5 10 15
   Asn Val Ala Gln Lys Ala Ala Phe Ile Arg Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 452
   <211> 49
   <212> PRT
   <213> Artificial Sequence
<220>
   <223> Engineered polypeptide
<400> 452
   Lys Glu Lys Tyr Thr Ala Tyr Tyr Glu Ile Trp Gln Leu Pro Asn Leu
   1               5                   10                  15
   Asn Arg Thr Gln Lys Ala Ala Phe Ile Gly Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 453
   <211> 49
   <212> PRT
   <213> Artificial Sequence
<220>
   <223> Engineered polypeptide
<400> 453
   Lys Glu Arg Tyr Arg Ala Tyr Tyr Glu Ile Trp Gln Leu Pro Asn Leu
   1               5                   10                  15
   Asn Arg Gln Gln Lys Gly Ala Phe Ile Ala Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 454
   <211> 49
   <212> PRT
   <213> Artificial Sequence
<220>
   <223> Engineered polypeptide
<400> 454
   Lys Glu Lys Tyr Lys Ala Tyr Tyr Glu Ile Trp Gln Leu Pro Asn Leu
   1 5 10 15
   Asn Arg Ser Gln Lys Gly Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 455
   <211> 49
   <212> PRT
   <213> Artificial Sequence
<220>
   <223> Engineered polypeptide
<400> 455
   Lys Glu Lys Tyr Ile Ala Tyr Tyr Glu Ile Trp Gln Leu Pro Asn Leu
   1 5 10 15
   Asn Arg Ser Gln Lys Gly Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 456
   <211> 49
   <212> PRT
   <213> Artificial Sequence
<220>
   <223> Engineered polypeptide
<400> 456
   Lys Glu Arg Tyr Lys Ala Tyr Tyr Glu Ile Trp Gln Leu Pro Asn Leu
   1 5 10 15
   Asn Arg Gln Gln Lys Ala Ala Phe Ile Gly Ser Leu Ala Asp Asp Pro
   20 25 30
   Ser Gln Ser Ala Asn Leu Leu Ala Glu Ala Lys Lys Leu Asn Asp Ala
   35 40 45
   Gln
<210> 457
   <211> 49
   <212> PRT
   <213> Artificial Sequence
<220>
   <223> Engineered polypeptide
<400> 457
   Lys Glu Arg Tyr Leu Ala Tyr Tyr Glu Ile Trp Gln Leu Pro Asn Leu
   1 5 10 15
   Asn Gln Phe Gln Lys Ala Ala Phe Ile Ala Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 458
   <211> 49
   <212> PRT
   <213> Artificial Sequence
<220>
   <223> Engineered polypeptide
<400> 458
   Lys Glu Arg Tyr Leu Ala Tyr Tyr Glu Ile Trp Gln Leu Pro Asn Leu
   1               5                   10                  15
   Asn Arg Glu Gln Lys Ala Ala Phe Ile Ala Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 459
   <211> 49
   <212> PRT
   <213> Artificial Sequence
<220>
   <223> Engineered polypeptide
<400> 459
   Lys Glu Arg Tyr Val Ala Tyr Gly Glu Ile Trp Gln Leu Pro Asn Leu
   1               5                   10                  15
   Asn Arg Tyr Gln Lys Ala Ala Phe Ile Ala Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 460
   <211> 49
   <212> PRT
   <213> Artificial Sequence
<220>
   <223> Engineered polypeptide
<400> 460
   Lys Glu Arg Tyr Ile Ala Tyr Tyr Glu Ile Trp Gln Leu Pro Asn Leu
   1               5                   10                  15
   Asn Arg Ala Gln Lys Ala Ala Phe Ile Ala Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 461
   <211> 49
   <212> PRT
   <213> Artificial Sequence
<220>
   <223> Engineered polypeptide
<400> 461
   Lys Glu Arg Tyr Gln Ala Tyr Tyr Glu Ile Trp Gln Leu Pro Asn Leu
   1               5                   10                  15
   Asn Arg Ile Gln Lys Ala Ala Phe Ile Lys Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 462
   <211> 49
   <212> PRT
   <213> Artificial Sequence
<220>
   <223> Engineered polypeptide
<400> 462
   Lys Glu Arg Tyr Thr Ala Tyr Tyr Glu Ile Trp Gln Leu Pro Asn Leu
   1               5                   10                  15
   Asn Thr Gln Gln Lys Ala Ala Phe Ile Arg Ser Leu Glu Asp Asp Pro
               20                  25                  30
   Ser Gln Ser Ala Asn Leu Leu Ala Glu Ala Lys Lys Leu Asn Asp Ala
           35                  40                  45
   Gln
<210> 463
   <211> 49
   <212> PRT
   <213> Artificial Sequence
<220>
   <223> Engineered polypeptide
<400> 463
   Lys Glu Arg Tyr Gln Ala Tyr Tyr Glu Ile Trp Gln Leu Pro Asn Leu
   1               5                   10                  15
   Asn Thr Ile Gln Lys Ala Ala Phe Ile Arg Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 464
   <211> 49
   <212> PRT
   <213> Artificial Sequence
<220>
   <223> Engineered polypeptide
<400> 464
   Lys Glu Arg Tyr Ala Ala Tyr Ala Glu Ile Trp Gln Leu Pro Asn Leu
   1               5                   10                  15
   Asn Arg Trp Gln Lys Ala Ala Phe Ile Ser Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 465
   <211> 49
   <212> PRT
   <213> Artificial Sequence
<220>
   <223> Engineered polypeptide
<400> 465
   Lys Glu Arg Tyr Arg Ala Tyr Ala Glu Ile Trp Gln Leu Pro Asn Leu
   1               5                   10                  15
   Asn Arg Trp Gln Lys Ala Ala Phe Ile Ala Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 466
   <211> 49
   <212> PRT
   <213> Artificial Sequence
<220>
   <223> Engineered polypeptide
<400> 466
   Lys Glu Arg Tyr Arg Ala Tyr Ala Glu Ile Trp Gln Leu Pro Asn Leu
   1               5                   10                  15
   Asn Arg Ile Gln Lys Ala Ala Phe Ile Gly Ser Leu Glu Asp Asp Pro
               20                  25                  30
   Ser Gln Ser Ala Asn Leu Leu Ala Glu Ala Lys Lys Leu Asn Asp Ala
           35                  40                  45
   Gln
<210> 467
   <211> 49
   <212> PRT
   <213> Artificial Sequence
<220>
   <223> Engineered polypeptide
<400> 467
   Lys Glu Arg Tyr His Ala Tyr Ala Glu Ile Trp Gln Leu Pro Asn Leu
   1               5                   10                  15
   Asn Arg Ile Gln Lys Ala Ala Phe Ile Gly Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 468
   <211> 49
   <212> PRT
   <213> Artificial Sequence
<220>
   <223> Engineered polypeptide
<400> 468
   Lys Glu Arg Tyr Arg Ala Tyr Tyr Glu Ile Trp Gln Leu Pro Asn Leu
   1               5                   10                  15
   Asn Arg Tyr Gln Lys Ala Ala Phe Ile Ala Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 469
   <211> 49
   <212> PRT
   <213> Artificial Sequence
<220>
   <223> Engineered polypeptide
<400> 469
   Lys Glu Arg Tyr Thr Ala Tyr Tyr Glu Ile Trp Gln Leu Pro Asn Leu
   1               5                   10                  15
   Asn Arg Val Gln Lys Ala Ala Phe Ile Ala Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 470
   <211> 49
   <212> PRT
   <213> Artificial Sequence
<220>
   <223> Engineered polypeptide
<400> 470
   Lys Glu Arg Tyr His Ala Tyr Tyr Glu Ile Trp Gln Leu Pro Asn Leu
   1               5                   10                  15
   Asn Arg Thr Gln Lys Ala Ala Phe Ile Ala Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 471
   <211> 49
   <212> PRT
   <213> Artificial Sequence
<220>
   <223> Engineered polypeptide
<400> 471
   Lys Glu Arg Tyr Val Ala Tyr Tyr Glu Ile Trp Gln Leu Pro Asn Leu
   1               5                   10                  15
   Asn Arg Arg Gln Lys Ala Ala Phe Ile Ser Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 472
   <211> 49
   <212> PRT
   <213> Artificial Sequence
<220>
   <223> Engineered polypeptide
<400> 472
   Lys Glu Arg Tyr Leu Ala Tyr Phe Glu Ile Trp Gln Leu Pro Asn Leu
   1               5                   10                  15
   Asn Arg His Gln Lys Gly Ala Phe Ile Ala Ser Leu Glu Asp Asp Pro
               20                  25                  30
   Ser Gln Ser Ala Asn Leu Leu Ala Glu Ala Lys Lys Leu Asn Asp Ala
           35                  40                  45
   Gln
<210> 473
   <211> 49
   <212> PRT
   <213> Artificial Sequence
<220>
   <223> Engineered polypeptide
<400> 473
   Lys Glu Arg Tyr Thr Ala Tyr Phe Glu Ile Trp Gln Leu Pro Asn Leu
   1               5                   10                  15
   Asn Arg Trp Gln Lys Gly Ala Phe Ile Ala Ser Leu Glu Asp Asp Pro
               20                  25                  30
   Ser Gln Ser Ala Asn Leu Leu Ala Glu Ala Lys Lys Leu Asn Asp Ala
           35                  40                  45
   Gln
<210> 474
   <211> 49
   <212> PRT
   <213> Artificial Sequence
<220>
   <223> Engineered polypeptide
<400> 474
   Lys Glu Arg Tyr Ala Ala Tyr Phe Glu Ile Trp Gln Leu Pro Asn Leu
   1               5                   10                  15
   Asn Arg Leu Gln Lys Ala Ala Phe Ile Ser Ser Leu Glu Asp Asp Pro
               20                  25                  30
   Ser Gln Ser Ala Asn Leu Leu Ala Glu Ala Lys Lys Leu Asn Asp Ala
           35                  40                  45
   Gln
<210> 475
   <211> 49
   <212> PRT
   <213> Artificial Sequence
<220>
   <223> Engineered polypeptide
<400> 475
   Lys Glu Arg Tyr Val Ala Tyr Phe Glu Ile Trp Gln Leu Pro Asn Leu
   1               5                   10                  15
   Asn Arg Ser Gln Lys Ala Ala Phe Ile Ser Ser Leu Glu Asp Asp Pro
               20                  25                  30
   Ser Gln Ser Ala Asn Leu Leu Ala Glu Ala Lys Lys Leu Asn Asp Ala
           35                  40                  45
   Gln
<210> 476
   <211> 49
   <212> PRT
   <213> Artificial Sequence
<220>
   <223> Engineered polypeptide
<400> 476
   Lys Glu Arg Tyr Gly Ala Tyr Phe Glu Ile Trp Gln Leu Pro Asn Leu
   1               5                   10                  15
   Asn Arg Thr Gln Lys Ala Ala Phe Ile Ala Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 477
   <211> 49
   <212> PRT
   <213> Artificial Sequence
<220>
   <223> Engineered polypeptide
<400> 477
   Lys Glu Lys Tyr Ile Ala Tyr Trp Glu Ile Trp Gln Leu Pro Asn Leu
   1               5                   10                  15
   Asn Arg Glu Gln Lys Ala Ala Phe Ile Ser Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 478
   <211> 49
   <212> PRT
   <213> Artificial Sequence
<220>
   <223> Engineered polypeptide
<400> 478
   Lys Glu Arg Tyr Ile Ala Tyr Ala Glu Ile Trp Gln Leu Pro Asn Leu
   1               5                   10                  15
   Asn Arg Arg Gln Lys Ala Ala Phe Ile Ala Ser Leu Glu Asp Asp Pro
               20                  25                  30
   Ser Gln Ser Ala Asn Leu Leu Ala Glu Ala Lys Lys Leu Asn Asp Ala
           35                  40                  45
   Gln
<210> 479
   <211> 49
   <212> PRT
   <213> Artificial Sequence
<220>
   <223> Engineered polypeptide
<400> 479
   Lys Glu Arg Tyr Ile Ala Tyr Trp Glu Ile Trp Gln Leu Pro Asn Leu
   1               5                   10                  15
   Asn Gln Arg Gln Lys Ala Ala Phe Ile Ala Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 480
   <211> 49
   <212> PRT
   <213> Artificial Sequence
<220>
   <223> Engineered polypeptide
<400> 480
   Lys Glu Arg Tyr Ile Ala Tyr Trp Glu Ile Trp Gln Leu Pro Asn Leu
   1 5 10 15
   Asn Arg Leu Gln Lys Ser Ala Phe Ile Ala Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 481
   <211> 49
   <212> PRT
   <213> Artificial Sequence
<220>
   <223> Engineered polypeptide
<400> 481
   Lys Glu Arg Tyr His Ala Tyr Trp Glu Ile Trp Gln Leu Pro Asn Leu
   1 5 10 15
   Asn Arg Ala Gln Lys Ala Ala Phe Ile Arg Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 482
   <211> 49
   <212> PRT
   <213> Artificial Sequence
<220>
   <223> Engineered polypeptide
<400> 482
   Lys Glu Arg Tyr Asn Ala Tyr Trp Glu Ile Trp Gln Leu Pro Asn Leu
   1 5 10 15
   Asn Arg Leu Gln Lys Ala Ala Phe Ile Arg Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 483
   <211> 49
   <212> PRT
   <213> Artificial Sequence
<220>
   <223> Engineered polypeptide
<400> 483
   Lys Glu Arg Tyr Thr Ala Tyr Gly Glu Ile Trp Glu Leu Pro Asn Leu
   1 5 10 15
   Asn Arg Val Gln Arg Ala Ala Phe Ile Ala Ser Leu Ala Asp Asp Pro
   20 25 30
   Ser Gln Ser Ala Asn Leu Leu Ala Glu Ala Lys Lys Leu Asn Asp Ala
   35 40 45
   Gln
<210> 484
   <211> 49
   <212> PRT
   <213> Artificial Sequence
<220>
   <223> Engineered polypeptide
<400> 484
   Lys Glu Arg Tyr Thr Ala Tyr Gly Glu Ile Trp Glu Leu Pro Asn Leu
   1               5                   10                  15
   Asn Arg Leu Gln Lys Ala Ala Phe Ile Gly Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 485
   <211> 49
   <212> PRT
   <213> Artificial Sequence
<220>
   <223> Engineered polypeptide
<400> 485
   Lys Glu Arg Tyr His Ala Tyr Gly Glu Ile Trp Glu Leu Pro Asn Leu
   1               5                   10                  15
   Asn Arg Arg Gln Lys Ala Ala Phe Ile Thr Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 486
   <211> 49
   <212> PRT
   <213> Artificial Sequence
<220>
   <223> Engineered polypeptide
<400> 486
   Lys Glu Arg Tyr Lys Ala Tyr Gly Glu Ile Trp Gln Leu Pro Asn Leu
   1 5 10 15
   Asn Arg Leu Gln Lys Ala Ala Phe Ile Gly Ser Leu Ala Asp Asp Pro
   20 25 30
   Ser Gln Ser Ala Asn Leu Leu Ala Glu Ala Lys Lys Leu Asn Asp Ala
   35 40 45
   Gln
<210> 487
   <211> 49
   <212> PRT
   <213> Artificial Sequence
<220>
   <223> Engineered polypeptide
<400> 487
   Lys Glu Arg Tyr Gln Ala Tyr Gly Glu Ile Trp Gln Leu Pro Asn Leu
   1 5 10 15
   Asn Arg Leu Gln Lys Ala Ala Phe Ile Gly Ser Leu Ala Asp Asp Pro
   20 25 30
   Ser Gln Ser Ala Asn Leu Leu Ala Glu Ala Lys Lys Leu Asn Asp Ala
   35 40 45
   Gln
<210> 488
   <211> 49
   <212> PRT
   <213> Artificial Sequence
<220>
   <223> Engineered polypeptide
<400> 488
   Lys Glu Arg Tyr Val Ala Tyr Gly Glu Ile Trp Gln Leu Pro Asn Leu
   1               5                   10                  15
   Asn Arg Leu Gln Lys Ala Ala Phe Ile Ser Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 489
   <211> 49
   <212> PRT
   <213> Artificial Sequence
<220>
   <223> Engineered polypeptide
<400> 489
   Lys Glu Arg Tyr Ile Ala Tyr Gly Glu Ile Trp Glu Leu Pro Asn Leu
   1               5                   10                  15
   Asn Arg Leu Gln Lys Ala Ala Phe Ile Gly Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 490
   <211> 49
   <212> PRT
   <213> Artificial Sequence
<220>
   <223> Engineered polypeptide
<400> 490
   Lys Glu Arg Tyr Gln Ala Tyr Gly Glu Ile Trp Gln Leu Pro Asn Leu
   1               5                   10                  15
   Asn Arg Arg Gln Lys Ala Ala Phe Ile Ser Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 491
   <211> 49
   <212> PRT
   <213> Artificial Sequence
<220>
   <223> Engineered polypeptide
<400> 491
   Lys Glu Arg Arg Gln Ala Tyr Phe Glu Ile Trp Glu Leu Pro Asn Leu
   1               5                   10                  15
   Asn Arg Tyr Gln Lys Ala Ala Phe Ile Ser Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 492
   <211> 49
   <212> PRT
   <213> Artificial Sequence
<220>
   <223> Engineered polypeptide
<400> 492
   Lys Glu Arg Tyr Arg Ala Tyr Val Glu Ile Trp Glu Leu Pro Asn Leu
   1               5                   10                  15
   Asn Arg Ile Gln Lys Val Ala Phe Ile Ala Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 493
   <211> 49
   <212> PRT
   <213> Artificial Sequence
<220>
   <223> Engineered polypeptide
<400> 493
   Lys Glu Lys Tyr Thr Ala Tyr Gly Glu Ile Trp Glu Leu Pro Asn Leu
   1               5                   10                  15
   Asn Arg Ile Gln Lys Val Ala Phe Ile Ala Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 494
   <211> 49
   <212> PRT
   <213> Artificial Sequence
<220>
   <223> Engineered polypeptide
<400> 494
   Lys Glu Lys Tyr Ala Ala Tyr Val Glu Ile Trp Glu Leu Pro Asn Leu
   1               5                   10                  15
   Asn Arg Arg Gln Lys Ala Ala Phe Ile Ala Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 495
   <211> 49
   <212> PRT
   <213> Artificial Sequence
<220>
   <223> Engineered polypeptide
<400> 495
   Lys Glu Arg Tyr Arg Ala Tyr Val Glu Ile Trp Glu Leu Pro Asn Leu
   1               5                   10                  15
   Asn Arg Trp Gln Lys Ala Ala Phe Ile Arg Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 496
   <211> 49
   <212> PRT
   <213> Artificial Sequence
<220>
   <223> Engineered polypeptide
<400> 496
   Lys Glu Arg Tyr Arg Ala Tyr Phe Glu Ile Trp Glu Leu Pro Asn Leu
   1               5                   10                  15
   Asn Arg Ala Gln Lys Ala Ala Phe Ile Ser Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 497
   <211> 49
   <212> PRT
   <213> Artificial Sequence
<220>
   <223> Engineered polypeptide
<400> 497
   Lys Glu Arg Tyr Gln Ala Tyr Phe Glu Ile Trp Gln Leu Pro Asn Leu
   1               5                   10                  15
   Asn Arg Leu Gln Ala Gly Ala Phe Ile Ala Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 498
   <211> 49
   <212> PRT
   <213> Artificial Sequence
<220>
   <223> Engineered polypeptide
<400> 498
   Lys Glu Lys Tyr Gln Ala Tyr Val Glu Ile Trp Gln Leu Pro Asn Leu
   1               5                   10                  15
   Asn Arg Leu Gln Lys Gly Ala Phe Ile Ala Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 499
   <211> 49
   <212> PRT
   <213> Artificial Sequence
<220>
   <223> Engineered polypeptide
<400> 499
   Lys Glu Lys Tyr Ala Ala Tyr Tyr Glu Ile Trp Gln Leu Pro Asn Leu
   1 5 10 15
   Asn Arg Ser Gln Lys Gly Ala Phe Ile Ala Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 500
   <211> 49
   <212> PRT
   <213> Artificial Sequence
<220>
   <223> Engineered polypeptide
<400> 500
   Lys Glu Lys Tyr Gln Ala Tyr Tyr Glu Ile Trp Glu Leu Pro Asn Leu
   1 5 10 15
   Asn Arg Gly Gln Lys Ala Ala Phe Ile Ala Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 501
   <211> 49
   <212> PRT
   <213> Artificial Sequence
<220>
   <223> Engineered polypeptide
<400> 501
   Lys Glu Lys Tyr Asn Ala Tyr Phe Glu Ile Trp Gln Leu Pro Asn Leu
   1               5                   10                  15
   Asn Arg Leu Gln Lys Ala Ala Phe Ile Thr Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 502
   <211> 49
   <212> PRT
   <213> Artificial Sequence
<220>
   <223> Engineered polypeptide
<400> 502
   Lys Glu Lys Tyr Ser Ala Tyr Phe Glu Ile Trp Gln Leu Pro Asn Leu
   1               5                   10                  15
   Asn Thr Ser Gln Lys Ala Ala Phe Ile Ala Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 503
   <211> 49
   <212> PRT
   <213> Artificial Sequence
<220>
   <223> Engineered polypeptide
<400> 503
   Lys Glu Arg Tyr Asn Ala Tyr Phe Glu Ile Trp Glu Leu Pro Asn Leu
   1 5 10 15
   Asn Val Arg Gln Lys Ala Ala Phe Ile Arg Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 504
   <211> 49
   <212> PRT
   <213> Artificial Sequence
<220>
   <223> Engineered polypeptide
<400> 504
   Lys Glu Lys Tyr Glu Ala Tyr Phe Glu Ile Trp Glu Leu Pro Asn Leu
   1 5 10 15
   Asn Ser Arg Gln Lys Ala Ala Phe Ile Gly Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 505
   <211> 49
   <212> PRT
   <213> Artificial Sequence
<220>
   <223> Engineered polypeptide
<400> 505
   Lys Glu Arg Tyr Arg Ala Tyr Trp Glu Ile Trp Glu Leu Pro Asn Leu
   1 5 10 15
   Asn Gln Gln Gln Lys Val Ala Phe Ile Arg Ser Leu Tyr Asp Asp Pro
   20 25 30
   Ser Gln Ser Ala Asn Leu Leu Ala Glu Ala Lys Lys Leu Asn Asp Ala
   35 40 45
   Gln
<210> 506
   <211> 49
   <212> PRT
   <213> Artificial Sequence
<220>
   <223> Engineered polypeptide
<400> 506
   Lys Glu Arg Tyr Asn Ala Tyr Trp Glu Ile Trp Glu Leu Pro Asn Leu
   1 5 10 15
   Asn Gln Gly Gln Lys Val Ala Phe Ile Arg Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 507
   <211> 49
   <212> PRT
   <213> Artificial Sequence
<220>
   <223> Engineered polypeptide
<400> 507
   Lys Glu Arg Arg His Ala Tyr Gly Glu Ile Trp Gln Leu Pro Asn Leu
   1 5 10 15
   Asn Gln Arg Gln Lys Val Ala Phe Ile Ser Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 508
   <211> 49
   <212> PRT
   <213> Artificial Sequence
<220>
   <223> Engineered polypeptide
<400> 508
   Lys Glu Lys Tyr Val Ala Thr Trp Glu Ile Trp Glu Leu Pro Asn Leu
   1               5                   10                  15
   Asn Arg Ala Gln Lys Val Ala Phe Ile Gly Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 509
   <211> 49
   <212> PRT
   <213> Artificial Sequence
<220>
   <223> Engineered polypeptide
<400> 509
   Lys Glu Arg Arg Ile Ala Arg Trp Glu Ile Trp Glu Leu Pro Asn Leu
   1               5                   10                  15
   Asn Arg Leu Gln Lys Val Ala Phe Ile Gly Ser Leu Glu Asp Asp Pro
               20                  25                  30
   Ser Gln Ser Ala Asn Leu Leu Ala Glu Ala Lys Lys Leu Asn Asp Ala
           35                  40                  45
   Gln
<210> 510
   <211> 49
   <212> PRT
   <213> Artificial Sequence
<220>
   <223> Engineered polypeptide
<400> 510
   Lys Glu Lys Tyr Lys Ala Ala Gly Glu Ile Trp Gln Leu Pro Asn Leu
   1               5                   10                  15
   Asn Arg Gly Gln Lys Val Ala Phe Ile Gly Ser Leu Glu Asp Asp Pro
               20                  25                  30
   Ser Gln Ser Ala Asn Leu Leu Ala Glu Ala Lys Lys Leu Asn Asp Ala
           35                  40                  45
   Gln
<210> 511
   <211> 49
   <212> PRT
   <213> Artificial Sequence
<220>
   <223> Engineered polypeptide
<400> 511
   Lys Glu Lys Tyr Leu Ala Tyr Gly Glu Ile Trp Gln Leu Pro Asn Leu
   1               5                   10                  15
   Asn Arg His Gln Lys Val Ala Phe Ile Gly Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 512
   <211> 49
   <212> PRT
   <213> Artificial Sequence
<220>
   <223> Engineered polypeptide
<400> 512
   Lys Glu Lys Tyr Gln Ala Tyr Gly Glu Ile Trp Gln Leu Pro Asn Leu
   1 5 10 15
   Asn Arg Gln Gln Lys Val Ala Phe Ile Ser Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 513
   <211> 49
   <212> PRT
   <213> Artificial Sequence
<220>
   <223> Engineered polypeptide
<400> 513
   Lys Glu Lys Tyr Lys Ala Tyr Gly Glu Ile Trp Gln Leu Pro Asn Leu
   1 5 10 15
   Asn Arg Gln Gln Lys Val Ala Phe Ile Ile Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 514
   <211> 49
   <212> PRT
   <213> Artificial Sequence
<220>
   <223> Engineered polypeptide
<400> 514
   Lys Glu Lys Tyr Ala Ala Ala Tyr Glu Ile Trp Gln Leu Pro Asn Leu
   1               5                   10                  15
   Asn Gln Arg Gln Lys Gly Ala Phe Ile Ala Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 515
   <211> 49
   <212> PRT
   <213> Artificial Sequence
<220>
   <223> Engineered polypeptide
<400> 515
   Lys Glu Lys Tyr Ala Ala Ser Tyr Glu Ile Trp Glu Leu Pro Asn Leu
   1               5                   10                  15
   Asn Gln Arg Gln Lys Ala Ala Phe Ile Lys Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 516
   <211> 49
   <212> PRT
   <213> Artificial Sequence
<220>
   <223> Engineered polypeptide
<400> 516
   Lys Glu Lys Tyr Leu Ala Ser Tyr Glu Ile Trp Gln Leu Pro Asn Leu
   1 5 10 15
   Asn Arg Arg Gln Lys Ala Ala Phe Ile Ala Ser Leu Phe Asp Asp Pro
   20 25 30
   Ser Gln Ser Ala Asn Leu Leu Ala Glu Ala Lys Lys Leu Asn Asp Ala
   35 40 45
   Gln
<210> 517
   <211> 49
   <212> PRT
   <213> Artificial Sequence
<220>
   <223> Engineered polypeptide
<400> 517
   Lys Glu Lys Tyr Ser Ala Thr Phe Glu Ile Trp Gln Leu Pro Asn Leu
   1 5 10 15
   Asn Gln Arg Gln Lys Ala Ala Phe Ile Ser Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 518
   <211> 49
   <212> PRT
   <213> Artificial Sequence
<220>
   <223> Engineered polypeptide
<400> 518
   Lys Glu Lys Tyr Ala Ala Ser Phe Glu Ile Trp Gln Leu Pro Asn Leu
   1 5 10 15
   Asn Thr His Gln Lys Ala Ala Phe Ile Ser Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 519
   <211> 49
   <212> PRT
   <213> Artificial Sequence
<220>
   <223> Engineered polypeptide
<400> 519
   Lys Glu Lys Tyr Lys Ala Ser Gly Glu Ile Trp Gln Leu Pro Asn Leu
   1 5 10 15
   Asn Arg Leu Gln Arg Ala Ala Phe Ile Lys Ser Leu Phe Asp Asp Pro
   20 25 30
   Ser Gln Ser Ala Asn Leu Leu Ala Glu Ala Lys Lys Leu Asn Asp Ala
   35 40 45
   Gln
<210> 520
   <211> 49
   <212> PRT
   <213> Artificial Sequence
<220>
   <223> Engineered polypeptide
<400> 520
   Lys Glu Lys Tyr Ser Ala Ser Gly Glu Ile Trp Gln Leu Pro Asn Leu
   1 5 10 15
   Asn Arg Thr Gln Lys Ala Ala Phe Ile Lys Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 521
   <211> 49
   <212> PRT
   <213> Artificial Sequence
<220>
   <223> Engineered polypeptide
<400> 521
   Lys Glu Lys Tyr His Ala Ala Gly Glu Ile Trp Glu Leu Pro Asn Leu
   1               5                   10                  15
   Asn Arg Leu Gln Lys Ala Ala Phe Ile Arg Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 522
   <211> 49
   <212> PRT
   <213> Artificial Sequence
<220>
   <223> Engineered polypeptide
<400> 522
   Lys Glu Lys Tyr Arg Ala Ala Gly Glu Ile Trp Glu Leu Pro Asn Leu
   1               5                   10                  15
   Asn Arg Gln Gln Lys Ala Ala Phe Ile Arg Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 523
   <211> 49
   <212> PRT
   <213> Artificial Sequence
<220>
   <223> Engineered polypeptide
<400> 523
   Lys Glu Lys Tyr Gln Ala Ser Gly Glu Ile Trp Glu Leu Pro Asn Leu
   1 5 10 15
   Asn Arg Leu Gln Lys Ala Ala Phe Ile Arg Ser Leu Phe Asp Asp Pro
   20 25 30
   Ser Gln Ser Ala Asn Leu Leu Ala Glu Ala Lys Lys Leu Asn Asp Ala
   35 40 45
   Gln
<210> 524
   <211> 49
   <212> PRT
   <213> Artificial Sequence
<220>
   <223> Engineered polypeptide
<400> 524
   Lys Glu Lys Tyr Lys Ala Thr Gly Glu Ile Trp Glu Leu Pro Asn Leu
   1 5 10 15
   Asn Arg Ile Gln Lys Ala Ala Phe Ile Arg Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 525
   <211> 49
   <212> PRT
   <213> Artificial Sequence
<220>
   <223> Engineered polypeptide
<400> 525
   Lys Glu Lys Tyr Gln Ala Ser Phe Glu Ile Trp Glu Leu Pro Asn Leu
   1               5                   10                  15
   Asn Arg Ser Gln Lys Ala Ala Phe Ile Lys Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 526
   <211> 49
   <212> PRT
   <213> Artificial Sequence
<220>
   <223> Engineered polypeptide
<400> 526
   Lys Glu Arg Lys Val Ala Ala Val Glu Ile Trp Gln Leu Pro Asn Leu
   1               5                   10                  15
   Asn Arg Trp Gln Arg Gly Ala Phe Ile Ser Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 527
   <211> 49
   <212> PRT
   <213> Artificial Sequence
<220>
   <223> Engineered polypeptide
<400> 527
   Lys Glu Arg Lys Leu Ala Ala Val Glu Ile Trp Glu Leu Pro Asn Leu
   1               5                   10                  15
   Asn Gln Tyr Gln Arg Gly Ala Phe Ile Ser Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 528
   <211> 49
   <212> PRT
   <213> Artificial Sequence
<220>
   <223> Engineered polypeptide
<400> 528
   Lys Glu Arg Lys Lys Ala Ser Val Glu Ile Trp Glu Leu Pro Asn Leu
   1               5                   10                  15
   Asn Arg Tyr Gln Lys Gly Ala Phe Ile Ser Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 529
   <211> 49
   <212> PRT
   <213> Artificial Sequence
<220>
   <223> Engineered polypeptide
<400> 529
   Lys Glu Arg Lys His Ala Ala Trp Glu Ile Trp Glu Leu Pro Asn Leu
   1               5                   10                  15
   Asn Arg His Gln Lys Gly Ala Phe Ile Lys Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 530
   <211> 49
   <212> PRT
   <213> Artificial Sequence
<220>
   <223> Engineered polypeptide
<400> 530
   Lys Glu Arg Lys His Ala Ala Val Glu Ile Trp Glu Leu Pro Asn Leu
   1               5                   10                  15
   Asn Arg Trp Gln Lys Gly Ala Phe Ile Gly Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 531
   <211> 49
   <212> PRT
   <213> Artificial Sequence
<220>
   <223> Engineered polypeptide
<400> 531
   Lys Glu Lys Arg His Ala Ala Val Glu Ile Trp Glu Leu Pro Asn Leu
   1 5 10 15
   Asn Val Phe Gln Arg Gly Ala Phe Ile Ala Ser Leu Tyr Asp Asp Pro
   20 25 30
   Ser Gln Ser Ala Asn Leu Leu Ala Glu Ala Lys Lys Leu Asn Asp Ala
   35 40 45
   Gln
<210> 532
   <211> 49
   <212> PRT
   <213> Artificial Sequence
<220>
   <223> Engineered polypeptide
<400> 532
   Lys Glu Arg Lys His Ala Ala Val Glu Ile Trp Glu Leu Pro Asn Leu
   1 5 10 15
   Asn Arg Leu Gln Arg Gly Ala Phe Ile Ala Ser Leu Tyr Asp Asp Pro
   20 25 30
   Ser Gln Ser Ala Asn Leu Leu Ala Glu Ala Lys Lys Leu Asn Asp Ala
   35 40 45
   Gln
<210> 533
   <211> 49
   <212> PRT
   <213> Artificial Sequence
<220>
   <223> Engineered polypeptide
<400> 533
   Lys Glu Trp Lys Thr Ala Ala Val Glu Ile Trp Glu Leu Pro Asn Leu
   1               5                   10                  15
   Asn Arg Arg Gln Lys Ala Ala Phe Ile Gly Ser Leu Tyr Asp Asp Pro
               20                  25                  30
   Ser Gln Ser Ala Asn Leu Leu Ala Glu Ala Lys Lys Leu Asn Asp Ala
           35                  40                  45
   Gln
<210> 534
   <211> 49
   <212> PRT
   <213> Artificial Sequence
<220>
   <223> Engineered polypeptide
<400> 534
   Lys Glu Arg Lys Val Ala Ala Phe Glu Ile Trp Glu Leu Pro Asn Leu
   1               5                   10                  15
   Asn Arg Gly Gln Lys Ala Ala Phe Ile Gly Ser Leu Tyr Asp Asp Pro
               20                  25                  30
   Ser Gln Ser Ala Asn Leu Leu Ala Glu Ala Lys Lys Leu Asn Asp Ala
           35                  40                  45
   Gln
<210> 535
   <211> 49
   <212> PRT
   <213> Artificial Sequence
<220>
   <223> Engineered polypeptide
<400> 535
   Lys Glu Lys Arg Asn Ala Ala Val Glu Ile Trp Glu Leu Pro Asn Leu
   1               5                   10                  15
   Asn Arg Arg Gln Arg Ala Ala Phe Ile Ser Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 536
   <211> 49
   <212> PRT
   <213> Artificial Sequence
<220>
   <223> Engineered polypeptide
<400> 536
   Lys Glu Arg Arg Asn Ala Ala Val Glu Ile Trp Glu Leu Pro Asn Leu
   1               5                   10                  15
   Asn Arg His Gln Arg Ala Ala Phe Ile Ser Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 537
   <211> 49
   <212> PRT
   <213> Artificial Sequence
<220>
   <223> Engineered polypeptide
<400> 537
   Lys Glu Lys Arg Gln Ala Ser Val Glu Ile Trp Glu Leu Pro Asn Leu
   1               5                   10                  15
   Asn Arg Ile Gln Lys Ala Ala Phe Ile Ser Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 538
   <211> 49
   <212> PRT
   <213> Artificial Sequence
<220>
   <223> Engineered polypeptide
<400> 538
   Lys Glu Lys Arg Arg Ala Ala Val Glu Ile Trp Glu Leu Pro Asn Leu
   1               5                   10                  15
   Asn Arg Ile Gln Lys Ser Ala Phe Ile Ala Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 539
   <211> 49
   <212> PRT
   <213> Artificial Sequence
<220>
   <223> Engineered polypeptide
<400> 539
   Lys Glu Lys Arg His Ala Ala Trp Glu Ile Trp Glu Leu Pro Asn Leu
   1               5                   10                  15
   Asn Arg Ala Gln Lys Ser Ala Phe Ile Lys Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 540
   <211> 49
   <212> PRT
   <213> Artificial Sequence
<220>
   <223> Engineered polypeptide
<400> 540
   Lys Glu Lys Arg Asn Ala Thr Val Glu Ile Trp Glu Leu Pro Asn Leu
   1 5 10 15
   Asn Arg Ile Gln Arg Ala Ala Phe Ile Ala Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 541
   <211> 49
   <212> PRT
   <213> Artificial Sequence
<220>
   <223> Engineered polypeptide
<400> 541
   Lys Glu Lys Arg Ala Ala Thr Val Glu Ile Trp Glu Leu Pro Asn Leu
   1 5 10 15
   Asn Arg Leu Gln Lys Gly Ala Phe Ile Ala Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 542
   <211> 49
   <212> PRT
   <213> Artificial Sequence
<220>
   <223> Engineered polypeptide
<400> 542
   Lys Glu Arg Arg Gly Ala Ala Val Glu Ile Trp Glu Leu Pro Asn Leu
   1               5                   10                  15
   Asn Arg Gln Gln Lys Ala Ala Phe Ile Ala Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 543
   <211> 49
   <212> PRT
   <213> Artificial Sequence
<220>
   <223> Engineered polypeptide
<400> 543
   Lys Glu Arg Arg Lys Ala Thr Val Glu Ile Trp Glu Leu Pro Asn Leu
   1               5                   10                  15
   Asn Arg Gln Gln Lys Ala Ala Phe Ile Ser Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 544
   <211> 49
   <212> PRT
   <213> Artificial Sequence
<220>
   <223> Engineered polypeptide
<400> 544
   Lys Glu Lys Ser Thr Ala Ala Val Glu Ile Trp Glu Leu Pro Asn Leu
   1 5 10 15
   Asn Arg Ile Gln Lys Ala Ala Phe Ile Lys Ser Leu Phe Asp Asp Pro
   20 25 30
   Ser Gln Ser Ala Asn Leu Leu Ala Glu Ala Lys Lys Leu Asn Asp Ala
   35 40 45
   Gln
<210> 545
   <211> 49
   <212> PRT
   <213> Artificial Sequence
<220>
   <223> Engineered polypeptide
<400> 545
   Lys Glu Lys Ser Thr Ala Ala Val Glu Ile Trp Glu Leu Pro Asn Leu
   1 5 10 15
   Asn Arg Tyr Gln Lys Ala Ala Phe Ile Lys Ser Leu Phe Asp Asp Pro
   20 25 30
   Ser Gln Ser Ala Asn Leu Leu Ala Glu Ala Lys Lys Leu Asn Asp Ala
   35 40 45
   Gln
<210> 546
   <211> 49
   <212> PRT
   <213> Artificial Sequence
<220>
   <223> Engineered polypeptide
<400> 546
   Lys Glu Lys Ser His Ala Ala Val Glu Ile Trp Glu Leu Pro Asn Leu
   1               5                   10                  15
   Asn Arg Trp Gln Lys Ala Ala Phe Ile Lys Ser Leu Tyr Asp Asp Pro
               20                  25                  30
   Ser Gln Ser Ala Asn Leu Leu Ala Glu Ala Lys Lys Leu Asn Asp Ala
           35                  40                  45
   Gln
<210> 547
   <211> 49
   <212> PRT
   <213> Artificial Sequence
<220>
   <223> Engineered polypeptide
<400> 547
   Lys Glu Arg Ser Ser Ala Ala Val Glu Ile Trp Glu Leu Pro Asn Leu
   1               5                   10                  15
   Asn Arg Phe Gln Lys Ala Ala Phe Ile Lys Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 548
   <211> 49
   <212> PRT
   <213> Artificial Sequence
<220>
   <223> Engineered polypeptide
<400> 548
   Lys Glu Lys Arg Ile Ala Ala Ala Glu Ile Trp Glu Leu Pro Asn Leu
   1               5                   10                  15
   Asn Arg Val Gln Lys Ala Ala Phe Ile Lys Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 549
   <211> 49
   <212> PRT
   <213> Artificial Sequence
<220>
   <223> Engineered polypeptide
<400> 549
   Lys Glu Lys Arg His Ala Ala Gly Glu Ile Trp Glu Leu Pro Asn Leu
   1               5                   10                  15
   Asn Arg Ile Gln Lys Ala Ala Phe Ile Lys Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 550
   <211> 49
   <212> PRT
   <213> Artificial Sequence
<220>
   <223> Engineered polypeptide
<400> 550
   Lys Glu Lys Arg Ile Ala Ala Val Glu Ile Trp Glu Leu Pro Asn Leu
   1               5                   10                  15
   Asn Arg Val Gln Lys Ala Ala Phe Ile Ala Ser Leu Tyr Asp Asp Pro
               20                  25                  30
   Ser Gln Ser Ala Asn Leu Leu Ala Glu Ala Lys Lys Leu Asn Asp Ala
           35                  40                  45
   Gln
<210> 551
   <211> 49
   <212> PRT
   <213> Artificial Sequence
<220>
   <223> Engineered polypeptide
<400> 551
   Lys Glu Lys Arg Ile Ala Ser Tyr Glu Ile Trp Glu Leu Pro Asn Leu
   1 5 10 15
   Asn Arg Arg Gln Arg Ala Ala Phe Ile Ala Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 552
   <211> 49
   <212> PRT
   <213> Artificial Sequence
<220>
   <223> Engineered polypeptide
<400> 552
   Lys Glu Lys Arg Asn Ala Thr Phe Glu Ile Trp Glu Leu Pro Asn Leu
   1 5 10 15
   Asn Arg Ser Gln Lys Gly Ala Phe Ile Ser Ser Leu Tyr Asp Asp Pro
   20 25 30
   Ser Gln Ser Ala Asn Leu Leu Ala Glu Ala Lys Lys Leu Asn Asp Ala
   35 40 45
   Gln
<210> 553
   <211> 49
   <212> PRT
   <213> Artificial Sequence
<220>
   <223> Engineered polypeptide
<400> 553
   Lys Glu Arg Lys Val Ala Thr Phe Glu Ile Trp Glu Leu Pro Asn Leu
   1               5                   10                  15
   Asn Ser Arg Gln Lys Ala Ala Phe Ile Lys Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 554
   <211> 49
   <212> PRT
   <213> Artificial Sequence
<220>
   <223> Engineered polypeptide
<400> 554
   Lys Glu Arg Lys Asn Ala Thr Phe Glu Ile Trp Glu Leu Pro Asn Leu
   1               5                   10                  15
   Asn Gln Arg Gln Lys Ser Ala Phe Ile Ala Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 555
   <211> 49
   <212> PRT
   <213> Artificial Sequence
<220>
   <223> Engineered polypeptide
<400> 555
   Lys Glu Arg Ser Ala Ala Thr Phe Glu Ile Trp Glu Leu Pro Asn Leu
   1               5                   10                  15
   Asn Gln Arg Gln Lys Ser Ala Phe Ile Ala Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 556
   <211> 49
   <212> PRT
   <213> Artificial Sequence
<220>
   <223> Engineered polypeptide
<400> 556
   Lys Glu Arg Lys His Ala Thr Phe Glu Ile Trp Glu Leu Pro Asn Leu
   1               5                   10                  15
   Asn Gln Leu Gln Lys Ser Ala Phe Ile Gly Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 557
   <211> 49
   <212> PRT
   <213> Artificial Sequence
<220>
   <223> Engineered polypeptide
<400> 557
   Lys Glu Arg Lys Gln Ala Thr Phe Glu Ile Trp Glu Leu Pro Asn Leu
   1 5 10 15
   Asn Gln Gln Gln Lys Gly Ala Phe Ile Ala Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 558
   <211> 49
   <212> PRT
   <213> Artificial Sequence
<220>
   <223> Engineered polypeptide
<400> 558
   Lys Glu Arg Lys Arg Ala Thr Phe Glu Ile Trp Glu Leu Pro Asn Leu
   1 5 10 15
   Asn Arg Thr Gln Lys Ala Ala Phe Ile Ser Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 559
   <211> 49
   <212> PRT
   <213> Artificial Sequence
<220>
   <223> Engineered polypeptide
<400> 559
   Lys Glu Arg Lys Thr Ala Thr Phe Glu Ile Trp Glu Leu Pro Asn Leu
   1               5                   10                  15
   Asn Arg Val Gln Lys Ala Ala Phe Ile Ser Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 560
   <211> 49
   <212> PRT
   <213> Artificial Sequence
<220>
   <223> Engineered polypeptide
<400> 560
   Lys Glu Arg Lys Leu Ala Thr Trp Glu Ile Trp Glu Leu Pro Asn Leu
   1               5                   10                  15
   Asn Arg Ser Gln Lys Ala Ala Phe Ile Ile Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 561
   <211> 49
   <212> PRT
   <213> Artificial Sequence
<220>
   <223> Engineered polypeptide
<400> 561
   Lys Glu Arg Lys Val Ala Ala Val Glu Ile Trp Glu Leu Pro Asn Leu
   1 5 10 15
   Asn Arg Ile Gln Lys Ala Ala Phe Ile Arg Ser Leu Phe Asp Asp Pro
   20 25 30
   Ser Gln Ser Ala Asn Leu Leu Ala Glu Ala Lys Lys Leu Asn Asp Ala
   35 40 45
   Gln
<210> 562
   <211> 49
   <212> PRT
   <213> Artificial Sequence
<220>
   <223> Engineered polypeptide
<400> 562
   Lys Glu Arg Lys Glu Ala Ala Val Glu Ile Trp Glu Leu Pro Asn Leu
   1 5 10 15
   Asn Arg Leu Gln Lys Ala Ala Phe Ile Arg Ser Leu Phe Asp Asp Pro
   20 25 30
   Ser Gln Ser Ala Asn Leu Leu Ala Glu Ala Lys Lys Leu Asn Asp Ala
   35 40 45
   Gln
<210> 563
   <211> 49
   <212> PRT
   <213> Artificial Sequence
<220>
   <223> Engineered polypeptide
<400> 563
   Lys Glu Arg Lys Arg Ala Ala Val Glu Ile Trp Glu Leu Pro Asn Leu
   1               5                   10                  15
   Asn Arg Gly Gln Lys Ala Ala Phe Ile Lys Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 564
   <211> 49
   <212> PRT
   <213> Artificial Sequence
<220>
   <223> Engineered polypeptide
<400> 564
   Lys Glu Arg Lys Leu Ala Ala Val Glu Ile Trp Glu Leu Pro Asn Leu
   1               5                   10                  15
   Asn Arg Thr Gln Lys Ala Ala Phe Ile Lys Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 565
   <211> 49
   <212> PRT
   <213> Artificial Sequence
<220>
   <223> Engineered polypeptide
<400> 565
   Lys Glu Arg Lys Val Ala Ala Val Glu Ile Trp Glu Leu Pro Asn Leu
   1               5                   10                  15
   Asn Arg Val Gln Lys Ala Ala Phe Ile Lys Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 566
   <211> 49
   <212> PRT
   <213> Artificial Sequence
<220>
   <223> Engineered polypeptide
<400> 566
   Lys Glu Arg Lys Glu Ala Ala Val Glu Ile Trp Glu Leu Pro Asn Leu
   1 5 10 15
   Asn Arg Val Gln Lys Ala Ala Phe Ile Lys Ser Leu Tyr Asp Asp Pro
   20 25 30
   Ser Gln Ser Ala Asn Leu Leu Ala Glu Ala Lys Lys Leu Asn Asp Ala
   35 40 45
   Gln
<210> 567
   <211> 49
   <212> PRT
   <213> Artificial Sequence
<220>
   <223> Engineered polypeptide
<400> 567
   Lys Glu Arg Lys Ala Ala Thr Val Glu Ile Trp Glu Leu Pro Asn Leu
   1 5 10 15
   Asn Arg Arg Gln Lys Ala Ala Phe Ile Ala Ser Leu Phe Asp Asp Pro
   20 25 30
   Ser Gln Ser Ala Asn Leu Leu Ala Glu Ala Lys Lys Leu Asn Asp Ala
   35 40 45
   Gln
<210> 568
   <211> 49
   <212> PRT
   <213> Artificial Sequence
<220>
   <223> Engineered polypeptide
<400> 568
   Lys Glu Lys Arg His Ala Ser Trp Glu Ile Trp Glu Leu Pro Asn Leu
   1               5                   10                  15
   Asn Gln Trp Gln Lys Ala Ala Phe Ile Arg Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 569
   <211> 49
   <212> PRT
   <213> Artificial Sequence
<220>
   <223> Engineered polypeptide
<400> 569
   Lys Glu Arg Lys Ser Ala Thr Trp Glu Ile Trp Glu Leu Pro Asn Leu
   1               5                   10                  15
   Asn Gln Leu Gln Lys Ala Ala Phe Ile Arg Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 570
   <211> 49
   <212> PRT
   <213> Artificial Sequence
<220>
   <223> Engineered polypeptide
<400> 570
   Lys Glu Lys Lys Lys Ala Ala Val Glu Ile Trp Glu Leu Pro Asn Leu
   1               5                   10                  15
   Asn Gln Arg Gln Lys Ala Ala Phe Ile Gly Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 571
   <211> 49
   <212> PRT
   <213> Artificial Sequence
<220>
   <223> Engineered polypeptide
<400> 571
   Lys Glu Lys Lys Leu Ala Ser Phe Glu Ile Trp Glu Leu Pro Asn Leu
   1               5                   10                  15
   Asn Gln Arg Gln Lys Ala Ala Phe Ile Arg Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 572
   <211> 49
   <212> PRT
   <213> Artificial Sequence
<220>
   <223> Engineered polypeptide
<400> 572
   Lys Glu Arg Lys Val Ala Thr Val Glu Ile Trp Glu Leu Pro Asn Leu
   1 5 10 15
   Asn Arg Val Gln Ala Ala Ala Phe Ile Lys Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 573
   <211> 49
   <212> PRT
   <213> Artificial Sequence
<220>
   <223> Engineered polypeptide
<400> 573
   Lys Glu Arg Lys Thr Ala Thr Val Glu Ile Trp Glu Leu Pro Asn Leu
   1 5 10 15
   Asn Arg Ile Gln Lys Ala Ala Phe Ile Lys Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 574
   <211> 49
   <212> PRT
   <213> Artificial Sequence
<220>
   <223> Engineered polypeptide
<400> 574
   Lys Glu Arg Lys Ser Ala Thr Val Glu Ile Trp Glu Leu Pro Asn Leu
   1 5 10 15
   Asn Arg Val Gln Lys Ala Ala Phe Ile Ser Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 575
   <211> 49
   <212> PRT
   <213> Artificial Sequence
<220>
   <223> Engineered polypeptide
<400> 575
   Lys Glu Arg Lys Val Ala Thr Val Glu Ile Trp Glu Leu Pro Asn Leu
   1 5 10 15
   Asn Arg Gly Gln Lys Ala Ala Phe Ile Ser Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 576
   <211> 49
   <212> PRT
   <213> Artificial Sequence
<220>
   <223> Engineered polypeptide
<400> 576
   Lys Glu Arg Lys Gln Ala Thr Val Glu Ile Trp Glu Leu Pro Asn Leu
   1 5 10 15
   Asn Arg Arg Gln Arg Ala Ala Phe Ile Ser Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 577
   <211> 49
   <212> PRT
   <213> Artificial Sequence
<220>
   <223> Engineered polypeptide
<400> 577
   Lys Glu Arg Lys Lys Ala Thr Val Glu Ile Trp Glu Leu Pro Asn Leu
   1 5 10 15
   Asn Arg Arg Gln Lys Gly Ala Phe Ile Ala Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 578
   <211> 49
   <212> PRT
   <213> Artificial Sequence
<220>
   <223> Engineered polypeptide
<400> 578
   Lys Glu Arg Lys Val Ala Thr Val Glu Ile Trp Glu Leu Pro Asn Leu
   1               5                   10                  15
   Asn Arg Arg Gln Lys Gly Ala Phe Ile Ala Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 579
   <211> 49
   <212> PRT
   <213> Artificial Sequence
<220>
   <223> Engineered polypeptide
<400> 579
   Lys Glu Arg Lys Leu Ala Thr Val Glu Ile Trp Glu Leu Pro Asn Leu
   1               5                   10                  15
   Asn Arg Tyr Gln Lys Gly Ala Phe Ile Ala Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 580
   <211> 49
   <212> PRT
   <213> Artificial Sequence
<220>
   <223> Engineered polypeptide
<400> 580
   Lys Glu Arg Lys Ala Ala Thr Val Glu Ile Trp Glu Leu Pro Asn Leu
   1               5                   10                  15
   Asn Arg Leu Gln Lys Gly Ala Phe Ile Ala Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 581
   <211> 49
   <212> PRT
   <213> Artificial Sequence
<220>
   <223> Engineered polypeptide
<400> 581
   Lys Glu Arg Lys Ile Ala Thr Val Glu Ile Trp Glu Leu Pro Asn Leu
   1 5 10 15
   Asn Arg Val Gln Lys Ser Ala Phe Ile Ala Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 582
   <211> 49
   <212> PRT
   <213> Artificial Sequence
<220>
   <223> Engineered polypeptide
<400> 582
   Lys Glu Arg Lys Asn Ala Thr Val Glu Ile Trp Glu Leu Pro Asn Leu
   1 5 10 15
   Asn Arg Ala Gln Lys Ala Ala Phe Ile Ala Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 583
   <211> 49
   <212> PRT
   <213> Artificial Sequence
<220>
   <223> Engineered polypeptide
<400> 583
   Lys Glu Arg Lys Asn Ala Thr Val Glu Ile Trp Glu Leu Pro Asn Leu
   1               5                   10                  15
   Asn Arg Ser Gln Lys Ala Ala Phe Ile Gly Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 584
   <211> 49
   <212> PRT
   <213> Artificial Sequence
<220>
   <223> Engineered polypeptide
<400> 584
   Lys Glu Arg Lys Thr Ala Thr Val Glu Ile Trp Glu Leu Pro Asn Leu
   1               5                   10                  15
   Asn Arg Gly Gln Lys Ala Ala Phe Ile Ala Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 585
   <211> 49
   <212> PRT
   <213> Artificial Sequence
<220>
   <223> Engineered polypeptide
<400> 585
   Lys Glu Arg Lys His Ala Ala Val Glu Ile Trp Glu Leu Pro Asn Leu
   1               5                   10                  15
   Asn Arg Ile Gln Arg Ala Ala Phe Ile Ala Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 586
   <211> 49
   <212> PRT
   <213> Artificial Sequence
<220>
   <223> Engineered polypeptide
<400> 586
   Lys Glu Arg Lys Ile Ala Thr Val Glu Ile Trp Glu Leu Pro Asn Leu
   1               5                   10                  15
   Asn Arg Ile Gln Arg Ala Ala Phe Ile Ala Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 587
   <211> 49
   <212> PRT
   <213> Artificial Sequence
<220>
   <223> Engineered polypeptide
<400> 587
   Lys Glu Arg Lys Ile Ala Ala Val Glu Ile Trp Glu Leu Pro Asn Leu
   1 5 10 15
   Asn Arg Ile Gln Ala Ala Ala Phe Ile Ala Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 588
   <211> 49
   <212> PRT
   <213> Artificial Sequence
<220>
   <223> Engineered polypeptide
<400> 588
   Lys Glu Arg Lys Ser Ala Thr Val Glu Ile Trp Glu Leu Pro Asn Leu
   1 5 10 15
   Asn Arg Thr Gln Arg Ala Ala Phe Ile Ala Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 589
   <211> 49
   <212> PRT
   <213> Artificial Sequence
<220>
   <223> Engineered polypeptide
<400> 589
   Lys Glu Arg Lys Ser Ala Ala Val Glu Ile Trp Glu Leu Pro Asn Leu
   1 5 10 15
   Asn Arg Thr Gln Lys Ala Ala Phe Ile Ala Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 590
   <211> 49
   <212> PRT
   <213> Artificial Sequence
<220>
   <223> Engineered polypeptide
<400> 590
   Lys Glu Lys Lys Ser Ala Thr Val Glu Ile Trp Glu Leu Pro Asn Leu
   1 5 10 15
   Asn Arg Ile Gln Lys Ala Ala Phe Ile Gly Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 591
   <211> 49
   <212> PRT
   <213> Artificial Sequence
<220>
   <223> Engineered polypeptide
<400> 591
   Lys Glu Lys Lys Ser Ala Tyr Val Glu Ile Trp Glu Leu Pro Asn Leu
   1 5 10 15
   Asn Arg Tyr Gln Lys Ala Ala Phe Ile Lys Ser Leu Phe Asp Asp Pro
   20 25 30
   Ser Gln Ser Ala Asn Leu Leu Ala Glu Ala Lys Lys Leu Asn Asp Ala
   35 40 45
   Gln
<210> 592
   <211> 49
   <212> PRT
   <213> Artificial Sequence
<220>
   <223> Engineered polypeptide
<400> 592
   Lys Glu Arg Lys Ile Ala Thr Tyr Glu Ile Trp Glu Leu Pro Asn Leu
   1 5 10 15
   Asn Gln Arg Gln Lys Ala Ala Phe Ile Ala Ser Leu Trp Asp Asp Pro
   20 25 30
   Ser Gln Ser Ala Asn Leu Leu Ala Glu Ala Lys Lys Leu Asn Asp Ala
   35 40 45
   Gln
<210> 593
   <211> 49
   <212> PRT
   <213> Artificial Sequence
<220>
   <223> Engineered polypeptide
<400> 593
   Lys Glu Arg Ser Val Ala Thr Trp Glu Ile Trp Gln Leu Pro Asn Leu
   1               5                   10                  15
   Asn Gln Arg Gln Lys Ala Ala Phe Ile Ala Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 594
   <211> 49
   <212> PRT
   <213> Artificial Sequence
<220>
   <223> Engineered polypeptide
<400> 594
   Lys Glu Lys Arg Lys Ala Thr Gly Glu Ile Trp Gln Leu Pro Asn Leu
   1               5                   10                  15
   Asn Gln Arg Gln Lys Gly Ala Phe Ile Ala Ser Leu Trp Asp Asp Pro
               20                  25                  30
   Ser Gln Ser Ala Asn Leu Leu Ala Glu Ala Lys Lys Leu Asn Asp Ala
           35                  40                  45
   Gln
<210> 595
   <211> 49
   <212> PRT
   <213> Artificial Sequence
<220>
   <223> Engineered polypeptide
<400> 595
   Lys Glu Arg Lys Glu Ala Arg Phe Glu Ile Trp Glu Leu Pro Asn Leu
   1               5                   10                  15
   Asn Gln Arg Gln Ala Ala Ala Phe Ile Ser Ser Leu Phe Asp Asp Pro
               20                  25                  30
   Ser Gln Ser Ala Asn Leu Leu Ala Glu Ala Lys Lys Leu Asn Asp Ala
           35                  40                  45
   Gln
<210> 596
   <211> 49
   <212> PRT
   <213> Artificial Sequence
<220>
   <223> Engineered polypeptide
<400> 596
   Lys Glu Arg Lys Ala Ala Ser Trp Glu Ile Trp Glu Leu Pro Asn Leu
   1               5                   10                  15
   Asn Gln Arg Gln Lys Ala Ala Phe Ile Ser Ser Leu Tyr Asp Asp Pro
               20                  25                  30
   Ser Gln Ser Ala Asn Leu Leu Ala Glu Ala Lys Lys Leu Asn Asp Ala
           35                  40                  45
   Gln
<210> 597
   <211> 49
   <212> PRT
   <213> Artificial Sequence
<220>
   <223> Engineered polypeptide
<400> 597
   Lys Glu Trp Lys His Ala Ala Gly Glu Ile Trp Glu Leu Pro Asn Leu
   1               5                   10                  15
   Asn Arg Ser Gln Lys Val Ala Phe Ile Ala Ser Leu Glu Asp Asp Pro
               20                  25                  30
   Ser Gln Ser Ala Asn Leu Leu Ala Glu Ala Lys Lys Leu Asn Asp Ala
           35                  40                  45
   Gln
<210> 598
   <211> 49
   <212> PRT
   <213> Artificial Sequence
<220>
   <223> Engineered polypeptide
<400> 598
   Lys Glu Trp Lys Arg Ala Ala Gly Glu Ile Trp Glu Leu Pro Asn Leu
   1               5                   10                  15
   Asn Arg Ala Gln Lys Val Ala Phe Ile Ala Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 599
   <211> 49
   <212> PRT
   <213> Artificial Sequence
<220>
   <223> Engineered polypeptide
<400> 599
   Lys Glu Trp Lys Ile Ala Ala Gly Glu Ile Trp Glu Leu Pro Asn Leu
   1               5                   10                  15
   Asn Gln Arg Gln Lys Val Ala Phe Ile Arg Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 600
   <211> 49
   <212> PRT
   <213> Artificial Sequence
<220>
   <223> Engineered polypeptide
<400> 600
   Lys Glu Trp Lys Thr Ala Ala Tyr Glu Ile Trp Glu Leu Pro Asn Leu
   1               5                   10                  15
   Asn Thr Tyr Gln Lys Val Ala Phe Ile Arg Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 601
   <211> 49
   <212> PRT
   <213> Artificial Sequence
<220>
   <223> Engineered polypeptide
<400> 601
   Lys Glu Trp Lys Val Ala Ala Gly Glu Ile Trp Glu Leu Pro Asn Leu
   1               5                   10                  15
   Asn Arg Thr Gln Lys Val Ala Phe Ile Arg Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 602
   <211> 49
   <212> PRT
   <213> Artificial Sequence
<220>
   <223> Engineered polypeptide
<400> 602
   Lys Glu Trp Lys Val Ala Ala Gly Glu Ile Trp Glu Leu Pro Asn Leu
   1               5                   10                  15
   Asn Arg Trp Gln Lys Val Ala Phe Ile Arg Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 603
   <211> 49
   <212> PRT
   <213> Artificial Sequence
<220>
   <223> Engineered polypeptide
<400> 603
   Lys Glu Trp Lys Gln Ala Ala Gly Glu Ile Trp Glu Leu Pro Asn Leu
   1               5                   10                  15
   Asn Arg Leu Gln Lys Val Ala Phe Ile Arg Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 604
   <211> 49
   <212> PRT
   <213> Artificial Sequence
<220>
   <223> Engineered polypeptide
<400> 604
   Lys Glu Trp Lys Val Ala Ala Gly Glu Ile Trp Glu Leu Pro Asn Leu
   1 5 10 15
   Asn Val Trp Gln Lys Ala Ala Phe Ile Arg Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 605
   <211> 49
   <212> PRT
   <213> Artificial Sequence
<220>
   <223> Engineered polypeptide
<400> 605
   Lys Glu Trp Lys Val Ala Ala Trp Glu Ile Trp Glu Leu Pro Asn Leu
   1 5 10 15
   Asn Arg Gly Gln Lys Val Ala Phe Ile Arg Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 606
   <211> 49
   <212> PRT
   <213> Artificial Sequence
<220>
   <223> Engineered polypeptide
<400> 606
   Lys Glu Trp Lys Arg Ala Ala Phe Glu Ile Trp Glu Leu Pro Asn Leu
   1               5                   10                  15
   Asn Gln Ala Gln Lys Val Ala Phe Ile Thr Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 607
   <211> 49
   <212> PRT
   <213> Artificial Sequence
<220>
   <223> Engineered polypeptide
<400> 607
   Lys Glu Trp Lys Lys Ala Ala Gly Glu Ile Trp Leu Leu Pro Asn Leu
   1               5                   10                  15
   Asn Arg Arg Gln Arg Gly Ala Phe Ile Ala Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 608
   <211> 49
   <212> PRT
   <213> Artificial Sequence
<220>
   <223> Engineered polypeptide
<400> 608
   Lys Glu Trp Lys Lys Ala Ala Gly Glu Ile Trp Glu Leu Pro Asn Leu
   1 5 10 15
   Asn Ser Arg Gln Lys Gly Ala Phe Ile Ala Ser Leu Ala Asp Asp Pro
   20 25 30
   Ser Gln Ser Ala Asn Leu Leu Ala Glu Ala Lys Lys Leu Asn Asp Ala
   35 40 45
   Gln
<210> 609
   <211> 49
   <212> PRT
   <213> Artificial Sequence
<220>
   <223> Engineered polypeptide
<400> 609
   Lys Glu Trp Arg Gly Ala Ala Gly Glu Ile Trp Glu Leu Pro Asn Leu
   1 5 10 15
   Asn Arg Arg Gln Arg Ala Ala Phe Ile Ala Ser Leu Ala Asp Asp Pro
   20 25 30
   Ser Gln Ser Ala Asn Leu Leu Ala Glu Ala Lys Lys Leu Asn Asp Ala
   35 40 45
   Gln
<210> 610
   <211> 49
   <212> PRT
   <213> Artificial Sequence
<220>
   <223> Engineered polypeptide
<400> 610
   Lys Glu Trp Lys Val Ala Ala Gly Glu Ile Trp Gln Leu Pro Asn Leu
   1 5 10 15
   Asn Arg Arg Gln Arg Ala Ala Phe Ile Ala Ser Leu Ala Asp Asp Pro
   20 25 30
   Ser Gln Ser Ala Asn Leu Leu Ala Glu Ala Lys Lys Leu Asn Asp Ala
   35 40 45
   Gln
<210> 611
   <211> 49
   <212> PRT
   <213> Artificial Sequence
<220>
   <223> Engineered polypeptide
<400> 611
   Lys Glu Trp Lys Ile Ala Ala Val Glu Ile Trp Glu Leu Pro Asn Leu
   1 5 10 15
   Asn Thr Arg Gln Lys Gly Ala Phe Ile Ala Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 612
   <211> 49
   <212> PRT
   <213> Artificial Sequence
<220>
   <223> Engineered polypeptide
<400> 612
   Lys Glu Trp Lys Ile Ala Ala Phe Glu Ile Trp Glu Leu Pro Asn Leu
   1 5 10 15
   Asn Val Arg Gln Arg Ser Ala Phe Ile Ser Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 613
   <211> 49
   <212> PRT
   <213> Artificial Sequence
<220>
   <223> Engineered polypeptide
<400> 613
   Lys Glu Trp Lys Thr Ala Ala Tyr Glu Ile Trp Glu Leu Pro Asn Leu
   1               5                   10                  15
   Asn Gln Arg Gln Arg Gly Ala Phe Ile Ala Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 614
   <211> 49
   <212> PRT
   <213> Artificial Sequence
<220>
   <223> Engineered polypeptide
<400> 614
   Lys Glu Trp Lys Thr Ala Ala Gly Glu Ile Trp Glu Leu Pro Asn Leu
   1               5                   10                  15
   Asn Ser Arg Gln Lys Ala Ala Phe Ile Gly Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 615
   <211> 49
   <212> PRT
   <213> Artificial Sequence
<220>
   <223> Engineered polypeptide
<400> 615
   Lys Glu Trp Lys Thr Ala Ala Gly Glu Ile Trp Glu Leu Pro Asn Leu
   1               5                   10                  15
   Asn Val Gly Gln Lys Ala Ala Phe Ile Gly Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 616
   <211> 49
   <212> PRT
   <213> Artificial Sequence
<220>
   <223> Engineered polypeptide
<400> 616
   Lys Glu Trp Lys His Ala Ala Gly Glu Ile Trp Glu Leu Pro Asn Leu
   1               5                   10                  15
   Asn Arg Thr Gln Lys Ala Ala Phe Ile Arg Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 617
   <211> 49
   <212> PRT
   <213> Artificial Sequence
<220>
   <223> Engineered polypeptide
<400> 617
   Lys Glu Trp Lys Arg Ala Ala Gly Glu Ile Trp Glu Leu Pro Asn Leu
   1               5                   10                  15
   Asn Arg Leu Gln Lys Ala Ala Phe Ile Arg Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 618
   <211> 49
   <212> PRT
   <213> Artificial Sequence
<220>
   <223> Engineered polypeptide
<400> 618
   Lys Glu Trp Lys Gln Ala Ala Gly Glu Ile Trp Glu Leu Pro Asn Leu
   1               5                   10                  15
   Asn Arg Gly Gln Lys Ala Ala Phe Ile Ser Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 619
   <211> 49
   <212> PRT
   <213> Artificial Sequence
<220>
   <223> Engineered polypeptide
<400> 619
   Lys Glu Trp Lys Gln Ala Ala Gly Glu Ile Trp Glu Leu Pro Asn Leu
   1 5 10 15
   Asn Arg Gly Gln Lys Ala Ala Phe Ile Thr Ser Leu Ala Asp Asp Pro
   20 25 30
   Ser Gln Ser Ala Asn Leu Leu Ala Glu Ala Lys Lys Leu Asn Asp Ala
   35 40 45
   Gln
<210> 620
   <211> 49
   <212> PRT
   <213> Artificial Sequence
<220>
   <223> Engineered polypeptide
<400> 620
   Lys Glu Trp Lys Glu Ala Ala Gly Glu Ile Trp Glu Leu Pro Asn Leu
   1 5 10 15
   Asn Arg Gly Gln Lys Ala Ala Phe Ile Lys Ser Leu Ala Asp Asp Pro
   20 25 30
   Ser Gln Ser Ala Asn Leu Leu Ala Glu Ala Lys Lys Leu Asn Asp Ala
   35 40 45
   Gln
<210> 621
   <211> 49
   <212> PRT
   <213> Artificial Sequence
<220>
   <223> Engineered polypeptide
<400> 621
   Lys Glu Trp Lys Thr Ala Ala Gly Glu Ile Trp Glu Leu Pro Asn Leu
   1 5 10 15
   Asn Arg Arg Gln Lys Ala Ala Phe Ile Ala Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 622
   <211> 49
   <212> PRT
   <213> Artificial Sequence
<220>
   <223> Engineered polypeptide
<400> 622
   Lys Glu Trp Lys Arg Ala Ala Gly Glu Ile Trp Glu Leu Pro Asn Leu
   1 5 10 15
   Asn Arg Ala Gln Lys Ala Ala Phe Ile Ser Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 623
   <211> 49
   <212> PRT
   <213> Artificial Sequence
<220>
   <223> Engineered polypeptide
<400> 623
   Lys Glu Trp Lys His Ala Ala Phe Glu Ile Trp Glu Leu Pro Asn Leu
   1 5 10 15
   Asn Val Arg Gln Lys Ala Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 624
   <211> 49
   <212> PRT
   <213> Artificial Sequence
<220>
   <223> Engineered polypeptide
<400> 624
   Lys Glu Trp Lys Asn Ala Ala Phe Glu Ile Trp Glu Leu Pro Asn Leu
   1 5 10 15
   Asn Val Ser Gln Lys Ala Ala Phe Ile Arg Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 625
   <211> 49
   <212> PRT
   <213> Artificial Sequence
<220>
   <223> Engineered polypeptide
<400> 625
   Lys Glu Trp Lys Ala Ala Ala Tyr Glu Ile Trp Glu Leu Pro Asn Leu
   1 5 10 15
   Asn Val Trp Gln Lys Ala Ala Phe Ile Ser Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 626
   <211> 49
   <212> PRT
   <213> Artificial Sequence
<220>
   <223> Engineered polypeptide
<400> 626
   Lys Glu Trp Lys Val Ala Ala Gly Glu Ile Trp Glu Leu Pro Asn Leu
   1 5 10 15
   Asn Arg Trp Gln Lys Gly Ala Phe Ile Thr Ser Leu Tyr Asp Asp Pro
   20 25 30
   Ser Gln Ser Ala Asn Leu Leu Ala Glu Ala Lys Lys Leu Asn Asp Ala
   35 40 45
   Gln
<210> 627
   <211> 49
   <212> PRT
   <213> Artificial Sequence
<220>
   <223> Engineered polypeptide
<400> 627
   Lys Glu Trp Lys Val Ala Ala Gly Glu Ile Trp Glu Leu Pro Asn Leu
   1 5 10 15
   Asn Arg Trp Gln Lys Ala Ala Phe Ile Ala Ser Leu Tyr Asp Asp Pro
   20 25 30
   Ser Gln Ser Ala Asn Leu Leu Ala Glu Ala Lys Lys Leu Asn Asp Ala
   35 40 45
   Gln
<210> 628
   <211> 49
   <212> PRT
   <213> Artificial Sequence
<220>
   <223> Engineered polypeptide
<400> 628
   Lys Glu Trp Lys Val Ala Ala Gly Glu Ile Trp Glu Leu Pro Asn Leu
   1               5                   10                  15
   Asn Arg Leu Gln Arg Ala Ala Phe Ile Lys Ser Leu Tyr Asp Asp Pro
               20                  25                  30
   Ser Gln Ser Ala Asn Leu Leu Ala Glu Ala Lys Lys Leu Asn Asp Ala
           35                  40                  45
   Gln
<210> 629
   <211> 49
   <212> PRT
   <213> Artificial Sequence
<220>
   <223> Engineered polypeptide
<400> 629
   Lys Glu Trp Lys Val Ala Ala Tyr Glu Ile Trp Glu Leu Pro Asn Leu
   1               5                   10                  15
   Asn Arg Leu Gln Lys Ala Ala Phe Ile Ala Ser Leu Tyr Asp Asp Pro
               20                  25                  30
   Ser Gln Ser Ala Asn Leu Leu Ala Glu Ala Lys Lys Leu Asn Asp Ala
           35                  40                  45
   Gln
<210> 630
   <211> 49
   <212> PRT
   <213> Artificial Sequence
<220>
   <223> Engineered polypeptide
<400> 630
   Lys Glu Trp Lys Val Ala Ala Phe Glu Ile Trp Glu Leu Pro Asn Leu
   1 5 10 15
   Asn Arg Thr Gln Arg Ala Ala Phe Ile Ile Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 631
   <211> 49
   <212> PRT
   <213> Artificial Sequence
<220>
   <223> Engineered polypeptide
<400> 631
   Lys Glu Trp Lys Thr Ala Ala Val Glu Ile Trp Glu Leu Pro Asn Leu
   1 5 10 15
   Asn Arg Leu Gln Lys Ala Ala Phe Ile Thr Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 632
   <211> 49
   <212> PRT
   <213> Artificial Sequence
<220>
   <223> Engineered polypeptide
<400> 632
   Lys Glu Trp Lys Thr Ala Ala Val Glu Ile Trp Glu Leu Pro Asn Leu
   1               5                   10                  15
   Asn Arg Leu Gln Lys Ala Ala Phe Ile Gly Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 633
   <211> 49
   <212> PRT
   <213> Artificial Sequence
<220>
   <223> Engineered polypeptide
<400> 633
   Lys Glu Trp Lys Thr Ala Ala Phe Glu Ile Trp Glu Leu Pro Asn Leu
   1               5                   10                  15
   Asn Arg Gly Gln Lys Ala Ala Phe Ile Arg Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 634
   <211> 49
   <212> PRT
   <213> Artificial Sequence
<220>
   <223> Engineered polypeptide
<400> 634
   Lys Glu Trp Lys Arg Ala Ala Phe Glu Ile Trp Glu Leu Pro Asn Leu
   1 5 10 15
   Asn Arg Leu Gln Lys Ser Ala Phe Ile Ala Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 635
   <211> 49
   <212> PRT
   <213> Artificial Sequence
<220>
   <223> Engineered polypeptide
<400> 635
   Lys Glu Trp Lys Gln Ala Ala Phe Glu Ile Trp Glu Leu Pro Asn Leu
   1 5 10 15
   Asn Arg Tyr Gln Ala Ala Ala Phe Ile Gly Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 636
   <211> 49
   <212> PRT
   <213> Artificial Sequence
<220>
   <223> Engineered polypeptide
<400> 636
   Lys Glu Trp Lys His Ala Ala Phe Glu Ile Trp Glu Leu Pro Asn Leu
   1               5                   10                  15
   Asn Arg Arg Gln Ala Gly Ala Phe Ile Ala Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 637
   <211> 49
   <212> PRT
   <213> Artificial Sequence
<220>
   <223> Engineered polypeptide
<400> 637
   Lys Glu Trp Lys Gln Ala Ser Gly Glu Ile Trp Glu Leu Pro Asn Leu
   1               5                   10                  15
   Asn Val Arg Gln Lys Ala Ala Phe Ile Ser Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 638
   <211> 49
   <212> PRT
   <213> Artificial Sequence
<220>
   <223> Engineered polypeptide
<400> 638
   Lys Glu Trp Lys Gln Ala Ser Phe Glu Ile Trp Glu Leu Pro Asn Leu
   1               5                   10                  15
   Asn Val Arg Gln Lys Ala Ala Phe Ile Ser Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 639
   <211> 49
   <212> PRT
   <213> Artificial Sequence
<220>
   <223> Engineered polypeptide
<400> 639
   Lys Glu Trp Lys Leu Ala Ala Gly Glu Ile Trp Glu Leu Pro Asn Leu
   1               5                   10                  15
   Asn Val Arg Gln Lys Ala Ala Phe Ile Ala Ser Leu Gln Asp Asp Pro
               20                  25                  30
   Ser Gln Ser Ala Asn Leu Leu Ala Glu Ala Lys Lys Leu Asn Asp Ala
           35                  40                  45
   Gln
<210> 640
   <211> 49
   <212> PRT
   <213> Artificial Sequence
<220>
   <223> Engineered polypeptide
<400> 640
   Lys Glu Trp Lys Ile Ala Ala Gly Glu Ile Trp Glu Leu Pro Asn Leu
   1               5                   10                  15
   Asn Val Arg Gln Lys Ala Ala Phe Ile Ala Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 641
   <211> 49
   <212> PRT
   <213> Artificial Sequence
<220>
   <223> Engineered polypeptide
<400> 641
   Lys Glu Trp Lys His Ala Ala Gly Glu Ile Trp Gln Leu Pro Asn Leu
   1               5                   10                  15
   Asn Val Thr Gln Lys Ala Ala Phe Ile Ala Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 642
   <211> 49
   <212> PRT
   <213> Artificial Sequence
<220>
   <223> Engineered polypeptide
<400> 642
   Lys Glu Trp Lys Ile Ala Ala Gly Glu Ile Trp Glu Leu Pro Asn Leu
   1               5                   10                  15
   Asn Arg Arg Gln Ala Ala Ala Phe Ile Val Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 643
   <211> 49
   <212> PRT
   <213> Artificial Sequence
<220>
   <223> Engineered polypeptide
<400> 643
   Lys Glu Trp Lys Ser Ala Ala Gly Glu Ile Trp Glu Leu Pro Asn Leu
   1               5                   10                  15
   Asn Arg Ala Gln Lys Ala Ala Phe Ile Ile Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 644
   <211> 49
   <212> PRT
   <213> Artificial Sequence
<220>
   <223> Engineered polypeptide
<400> 644
   Lys Glu Trp Lys His Ala Ala Val Glu Ile Trp Gln Leu Pro Asn Leu
   1               5                   10                  15
   Asn Arg Phe Gln Lys Ala Ala Phe Ile Ala Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 645
   <211> 49
   <212> PRT
   <213> Artificial Sequence
<220>
   <223> Engineered polypeptide
<400> 645
   Lys Glu Trp Lys Gln Ala Ala Trp Glu Ile Trp Gln Leu Pro Asn Leu
   1 5 10 15
   Asn Thr Gly Gln Lys Ala Ala Phe Ile Arg Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 646
   <211> 49
   <212> PRT
   <213> Artificial Sequence
<220>
   <223> Engineered polypeptide
<400> 646
   Lys Glu Trp Lys Gln Ala Ser Trp Glu Ile Trp Glu Leu Pro Asn Leu
   1 5 10 15
   Asn Arg Gly Gln Lys Ala Ala Phe Ile Ala Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 647
   <211> 49
   <212> PRT
   <213> Artificial Sequence
<220>
   <223> Engineered polypeptide
<400> 647
   Lys Glu Trp Lys Thr Ala Ser Tyr Glu Ile Trp Gln Leu Pro Asn Leu
   1               5                   10                  15
   Asn Arg Arg Gln Ala Ala Ala Phe Ile Ala Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 648
   <211> 49
   <212> PRT
   <213> Artificial Sequence
<220>
   <223> Engineered polypeptide
<400> 648
   Lys Glu Trp Lys Asn Ala Ser Phe Glu Ile Trp Leu Leu Pro Asn Leu
   1               5                   10                  15
   Asn Arg Ile Gln Lys Ala Ala Phe Ile Ala Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 649
   <211> 49
   <212> PRT
   <213> Artificial Sequence
<220>
   <223> Engineered polypeptide
<400> 649
   Lys Glu Trp Lys Asn Ala Ala Phe Glu Ile Trp Leu Leu Pro Asn Leu
   1 5 10 15
   Asn Gln Arg Gln Lys Ala Ala Phe Ile Ala Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 650
   <211> 49
   <212> PRT
   <213> Artificial Sequence
<220>
   <223> Engineered polypeptide
<400> 650
   Lys Glu Trp Arg Gly Ala Ala Phe Glu Ile Trp Gln Leu Pro Asn Leu
   1 5 10 15
   Asn Gln Arg Gln Lys Ala Ala Phe Ile Ala Ser Leu Tyr Asp Asp Pro
   20 25 30
   Ser Gln Ser Ala Asn Leu Leu Ala Glu Ala Lys Lys Leu Asn Asp Ala
   35 40 45
   Gln
<210> 651
   <211> 49
   <212> PRT
   <213> Artificial Sequence
<220>
   <223> Engineered polypeptide
<400> 651
   Lys Glu Trp Lys Val Ala Ser Trp Glu Ile Trp Gln Leu Pro Asn Leu
   1 5 10 15
   Asn Arg Gln Gln Lys Ala Ala Phe Ile Gly Ser Leu Ala Asp Asp Pro
   20 25 30
   Ser Gln Ser Ala Asn Leu Leu Ala Glu Ala Lys Lys Leu Asn Asp Ala
   35 40 45
   Gln
<210> 652
   <211> 49
   <212> PRT
   <213> Artificial Sequence
<220>
   <223> Engineered polypeptide
<400> 652
   Lys Glu Trp Lys Leu Ala Ser Trp Glu Ile Trp Gln Leu Pro Asn Leu
   1 5 10 15
   Asn Arg Trp Gln Lys Ala Ala Phe Ile Gly Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 653
   <211> 49
   <212> PRT
   <213> Artificial Sequence
<220>
   <223> Engineered polypeptide
<400> 653
   Lys Glu Trp Lys Arg Ala Ser Val Glu Ile Trp Glu Leu Pro Asn Leu
   1 5 10 15
   Asn Arg Gln Gln Lys Ala Ala Phe Ile Gly Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 654
   <211> 49
   <212> PRT
   <213> Artificial Sequence
<220>
   <223> Engineered polypeptide
<400> 654
   Lys Glu Trp Lys Arg Ala Ser Phe Glu Ile Trp Gln Leu Pro Asn Leu
   1 5 10 15
   Asn Arg Leu Gln Lys Ala Ala Phe Ile Ala Ser Leu Ala Asp Asp Pro
   20 25 30
   Ser Gln Ser Ala Asn Leu Leu Ala Glu Ala Lys Lys Leu Asn Asp Ala
   35 40 45
   Gln
<210> 655
   <211> 49
   <212> PRT
   <213> Artificial Sequence
<220>
   <223> Engineered polypeptide
<400> 655
   Lys Glu Trp Lys Gly Ala Ala Gly Glu Ile Trp Gln Leu Pro Asn Leu
   1 5 10 15
   Asn Arg Arg Gln Lys Ala Ala Phe Ile Ser Ser Leu Tyr Asp Asp Pro
   20 25 30
   Ser Gln Ser Ala Asn Leu Leu Ala Glu Ala Lys Lys Leu Asn Asp Ala
   35 40 45
   Gln
<210> 656
   <211> 49
   <212> PRT
   <213> Artificial Sequence
<220>
   <223> Engineered polypeptide
<400> 656
   Lys Glu Trp Lys Val Ala Ala Phe Glu Ile Trp Gln Leu Pro Asn Leu
   1 5 10 15
   Asn Arg Ala Gln Lys Ala Ala Phe Ile Ser Ser Leu Ala Asp Asp Pro
   20 25 30
   Ser Gln Ser Ala Asn Leu Leu Ala Glu Ala Lys Lys Leu Asn Asp Ala
   35 40 45
   Gln
<210> 657
   <211> 49
   <212> PRT
   <213> Artificial Sequence
<220>
   <223> Engineered polypeptide
<400> 657
   Lys Glu Trp Lys His Ala Ala Gly Glu Ile Trp Gln Leu Pro Asn Leu
   1 5 10 15
   Asn Arg Ala Gln Lys Ala Ala Phe Ile Ser Ser Leu Ala Asp Asp Pro
   20 25 30
   Ser Gln Ser Ala Asn Leu Leu Ala Glu Ala Lys Lys Leu Asn Asp Ala
   35 40 45
   Gln
<210> 658
   <211> 49
   <212> PRT
   <213> Artificial Sequence
<220>
   <223> Engineered polypeptide
<400> 658
   Lys Glu Trp Lys Lys Ala Ala Tyr Glu Ile Trp Gln Leu Pro Asn Leu
   1               5                   10                  15
   Asn Arg Ala Gln Lys Ala Ala Phe Ile Ser Ser Leu Tyr Asp Asp Pro
               20                  25                  30
   Ser Gln Ser Ala Asn Leu Leu Ala Glu Ala Lys Lys Leu Asn Asp Ala
           35                  40                  45
   Gln
<210> 659
   <211> 49
   <212> PRT
   <213> Artificial Sequence
<220>
   <223> Engineered polypeptide
<400> 659
   Lys Glu Trp Lys Leu Ala Ala Tyr Glu Ile Trp Gln Leu Pro Asn Leu
   1               5                   10                  15
   Asn Arg His Gln Lys Ala Ala Phe Ile Ser Ser Leu Ser Asp Asp Pro
               20                  25                  30
   Ser Gln Ser Ala Asn Leu Leu Ala Glu Ala Lys Lys Leu Asn Asp Ala
           35                  40                  45
   Gln
<210> 660
   <211> 49
   <212> PRT
   <213> Artificial Sequence
<220>
   <223> Engineered polypeptide
<400> 660
   Lys Glu Trp Lys Arg Ala Ala Phe Glu Ile Trp Gln Leu Pro Asn Leu
   1               5                   10                  15
   Asn Arg Ser Gln Lys Ala Ala Phe Ile Ser Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 661
   <211> 49
   <212> PRT
   <213> Artificial Sequence
<220>
   <223> Engineered polypeptide
<400> 661
   Lys Glu Trp Lys Thr Ala Ala Phe Glu Ile Trp Gln Leu Pro Asn Leu
   1               5                   10                  15
   Asn Arg Ser Gln Lys Ala Ala Phe Ile Ala Ser Leu Ala Asp Asp Pro
               20                  25                  30
   Ser Gln Ser Ala Asn Leu Leu Ala Glu Ala Lys Lys Leu Asn Asp Ala
           35                  40                  45
   Gln
<210> 662
   <211> 49
   <212> PRT
   <213> Artificial Sequence
<220>
   <223> Engineered polypeptide
<400> 662
   Lys Glu Trp Lys Ile Ala Ala Phe Glu Ile Trp Gln Leu Pro Asn Leu
   1 5 10 15
   Asn Arg Ile Gln Lys Ala Ala Phe Ile Gly Ser Leu Ala Asp Asp Pro
   20 25 30
   Ser Gln Ser Ala Asn Leu Leu Ala Glu Ala Lys Lys Leu Asn Asp Ala
   35 40 45
   Gln
<210> 663
   <211> 49
   <212> PRT
   <213> Artificial Sequence
<220>
   <223> Engineered polypeptide
<400> 663
   Lys Glu Trp Lys Gln Ala Ala Phe Glu Ile Trp Gln Leu Pro Asn Leu
   1 5 10 15
   Asn Arg Ile Gln Lys Ala Ala Phe Ile Thr Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 664
   <211> 49
   <212> PRT
   <213> Artificial Sequence
<220>
   <223> Engineered polypeptide
<400> 664
   Lys Glu Trp Lys Gln Ala Ala Phe Glu Ile Trp Gln Leu Pro Asn Leu
   1 5 10 15
   Asn Arg Val Gln Lys Ala Ala Phe Ile Val Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 665
   <211> 49
   <212> PRT
   <213> Artificial Sequence
<220>
   <223> Engineered polypeptide
<400> 665
   Lys Glu Trp Lys Val Ala Ala Gly Glu Ile Trp Gln Leu Pro Asn Leu
   1 5 10 15
   Asn Arg Ile Gln Lys Ala Ala Phe Ile Gly Ser Leu Ser Asp Asp Pro
   20 25 30
   Ser Gln Ser Ala Asn Leu Leu Ala Glu Ala Lys Lys Leu Asn Asp Ala
   35 40 45
   Gln
<210> 666
   <211> 49
   <212> PRT
   <213> Artificial Sequence
<220>
   <223> Engineered polypeptide
<400> 666
   Lys Glu Trp Lys Ile Ala Ala Phe Glu Ile Trp Gln Leu Pro Asn Leu
   1 5 10 15
   Asn Arg Trp Gln Lys Ala Ala Phe Ile Ser Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 667
   <211> 49
   <212> PRT
   <213> Artificial Sequence
<220>
   <223> Engineered polypeptide
<400> 667
   Lys Glu Trp Lys Glu Ala Ala Gly Glu Ile Trp Gln Leu Pro Asn Leu
   1 5 10 15
   Asn Arg Tyr Gln Lys Ala Ala Phe Ile Ala Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 668
   <211> 49
   <212> PRT
   <213> Artificial Sequence
<220>
   <223> Engineered polypeptide
<400> 668
   Lys Glu Trp Lys Thr Ala Ser Phe Glu Ile Trp Gln Leu Pro Asn Leu
   1 5 10 15
   Asn Arg Tyr Gln Lys Ala Ala Phe Ile Ser Ser Leu Gln Asp Asp Pro
   20 25 30
   Ser Gln Ser Ala Asn Leu Leu Ala Glu Ala Lys Lys Leu Asn Asp Ala
   35 40 45
   Gln
<210> 669
   <211> 58
   <212> PRT
   <213> Artificial Sequence
<220>
   <223> Engineered polypeptide
<400> 669
   Val Asp Asn Lys Phe Asn Lys Glu Lys Val Ala Ala Thr Gly Glu Ile
   1 5 10 15
   Trp Asp Leu Pro Asn Leu Asn Thr Arg Gln Lys Asn Ala Phe Ile Gly
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 670
   <211> 58
   <212> PRT
   <213> Artificial Sequence
<220>
   <223> Engineered polypeptide
<400> 670
   Val Asp Asn Lys Phe Asn Lys Glu Trp Arg Trp Ala Ala His Glu Ile
   1 5 10 15
   Trp Asp Leu Pro Asn Leu Asn Val Tyr Gln Arg Ala Ala Phe Ile Arg
   20 25 30
   Ser Leu Glu Asp Asp Pro Ser Gln Ser Ala Asn Leu Leu Ala Glu Ala
   35 40 45
   Lys Lys Leu Asn Asp Ala Gln Ala Pro Lys
   50 55
<210> 671
   <211> 58
   <212> PRT
   <213> Artificial Sequence
<220>
   <223> Engineered polypeptide
<400> 671
   Val Asp Asn Lys Phe Asn Lys Glu Arg Arg Leu Ala Ser Val Glu Ile
   1 5 10 15
   Trp Glu Leu Pro Asn Leu Asn Ala Val Gln Lys Ser Ala Phe Ile Ser
   20 25 30
   Ser Leu Phe Asp Asp Pro Ser Gln Ser Ala Asn Leu Leu Ala Glu Ala
   35 40 45
   Lys Lys Leu Asn Asp Ala Gln Ala Pro Lys
   50 55
<210> 672
   <211> 58
   <212> PRT
   <213> Artificial Sequence
<220>
   <223> Engineered polypeptide
<400> 672
   Val Asp Asn Lys Phe Asn Lys Glu Val Lys Glu Ala Arg Phe Glu Ile
   1               5                   10                  15
   Trp Asp Leu Pro Asn Leu Asn Arg Thr Gln Lys His Ala Phe Ile Val
               20                  25                  30
   Ser Leu Glu Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 673
   <211> 58
   <212> PRT
   <213> Artificial Sequence
<220>
   <223> Engineered polypeptide
<400> 673
   Val Asp Asn Lys Phe Asn Lys Glu Trp Lys Gly Ala Ala Gly Glu Ile
   1               5                   10                  15
   Trp Asn Leu Pro Asn Leu Asn Val Ser Gln Arg Val Ala Phe Ile Gly
               20                  25                  30
   Ser Leu Ala Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 674
   <211> 58
   <212> PRT
   <213> Artificial Sequence
<220>
   <223> Engineered polypeptide
<400> 674
   Val Asp Asn Lys Phe Asn Lys Glu Lys Tyr Lys Ala Ala His Glu Ile
   1 5 10 15
   Trp Glu Leu Pro Asn Leu Asn Arg Asp Gln Arg Ala Ala Phe Ile Thr
   20 25 30
   Ser Leu Thr Asp Asp Pro Ser Gln Ser Ala Asn Leu Leu Ala Glu Ala
   35 40 45
   Lys Lys Leu Asn Asp Ala Gln Ala Pro Lys
   50 55
<210> 675
   <211> 58
   <212> PRT
   <213> Artificial Sequence
<220>
   <223> Engineered polypeptide
<400> 675
   Val Asp Asn Lys Phe Asn Lys Glu Arg Ser Leu Ala Ser Phe Glu Ile
   1 5 10 15
   Trp Glu Leu Pro Asn Leu Asn Pro Lys Gln Lys Ala Ala Phe Ile Val
   20 25 30
   Ser Leu Phe Asp Asp Pro Ser Gln Ser Ala Asn Leu Leu Ala Glu Ala
   35 40 45
   Lys Lys Leu Asn Asp Ala Gln Ala Pro Lys
   50 55
<210> 676
   <211> 58
   <212> PRT
   <213> Artificial Sequence
<220>
   <223> Engineered polypeptide
<400> 676
   Val Asp Asn Lys Phe Asn Lys Glu Trp Lys Arg Ala Ser Trp Glu Ile
   1 5 10 15
   Trp Glu Leu Pro Asn Leu Asn Asn Ala Gln Lys Arg Ala Phe Ile Ser
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 677
   <211> 58
   <212> PRT
   <213> Artificial Sequence
<220>
   <223> Engineered polypeptide
<400> 677
   Val Asp Asn Lys Phe Asn Lys Glu Lys Tyr Lys Ala Met Thr Glu Ile
   1 5 10 15
   Trp Ile Leu Pro Asn Leu Asn Gln Arg Gln Lys Val Ala Phe Ile Gly
   20 25 30
   Ser Leu Asp Asp Asp Pro Ser Gln Ser Ala Asn Leu Leu Ala Glu Ala
   35 40 45
   Lys Lys Leu Asn Asp Ala Gln Ala Pro Lys
   50 55
<210> 678
   <211> 58
   <212> PRT
   <213> Artificial Sequence
<220>
   <223> Engineered polypeptide
<400> 678
   Val Asp Asn Lys Phe Asn Lys Glu Trp Arg Gly Ala Ala Gly Glu Ile
   1               5                   10                  15
   Trp Ala Leu Pro Asn Leu Asn Asn Arg Gln Lys Gly Ala Phe Ile Glu
               20                  25                  30
   Ser Leu Pro Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 679
   <211> 58
   <212> PRT
   <213> Artificial Sequence
<220>
   <223> Engineered polypeptide
<400> 679
   Val Asp Asn Lys Phe Asn Lys Glu Arg Trp Glu Ala Thr Val Glu Ile
   1               5                   10                  15
   Trp Asp Leu Pro Asn Leu Asn Arg Asn Gln Lys Ala Ala Phe Ile Ala
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 680
   <211> 58
   <212> PRT
   <213> Artificial Sequence
<220>
   <223> Engineered polypeptide
<400> 680
   Val Asp Asn Lys Phe Asn Lys Glu Lys Tyr Asn Ala Tyr Ala Glu Ile
   1               5                   10                  15
   Trp Leu Leu Pro Asn Leu Asn Arg Tyr Gln Lys Ala Ala Phe Ile Gly
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 681
   <211> 58
   <212> PRT
   <213> Artificial Sequence
<220>
   <223> Engineered polypeptide
<400> 681
   Val Asp Asn Lys Phe Asn Lys Glu Arg Ser Val Ala Gln Lys Glu Ile
   1               5                   10                  15
   Trp Glu Leu Pro Asn Leu Asn Arg Trp Gln Ala Gly Ala Phe Ile Lys
               20                  25                  30
   Ser Leu Tyr Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 682
   <211> 58
   <212> PRT
   <213> Artificial Sequence
<220>
   <223> Engineered polypeptide
<400> 682
   Val Asp Asn Lys Phe Asn Lys Glu Lys Met Asp Ala Met Gly Glu Ile
   1 5 10 15
   Trp Asp Leu Pro Asn Leu Asn Arg Gly Gln Ala Ser Ala Phe Ile Ala
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 683
   <211> 58
   <212> PRT
   <213> Artificial Sequence
<220>
   <223> Engineered polypeptide
<400> 683
   Val Asp Asn Lys Phe Asn Lys Glu Lys Arg Asn Ala Gln Val Glu Ile
   1 5 10 15
   Trp Thr Leu Pro Asn Leu Asn Ser Lys Gln Arg Ala Ala Phe Ile Lys
   20 25 30
   Ser Leu Tyr Asp Asp Pro Ser Gln Ser Ala Asn Leu Leu Ala Glu Ala
   35 40 45
   Lys Lys Leu Asn Asp Ala Gln Ala Pro Lys
   50 55
<210> 684
   <211> 58
   <212> PRT
   <213> Artificial Sequence
<220>
   <223> Engineered polypeptide
<400> 684
   Val Asp Asn Lys Phe Asn Lys Glu Arg Arg Asp Ala Arg Phe Glu Ile
   1               5                   10                  15
   Trp Glu Leu Pro Asn Leu Asn Lys Tyr Gln Arg Ala Ala Phe Ile Ser
               20                  25                  30
   Ser Leu Asp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 685
   <211> 58
   <212> PRT
   <213> Artificial Sequence
<220>
   <223> Engineered polypeptide
<400> 685
   Val Asp Asn Lys Phe Asn Lys Glu Arg Ser Met Ala Arg Phe Glu Ile
   1               5                   10                  15
   Trp Glu Leu Pro Asn Leu Asn Arg Gly Gln Lys Ser Ala Phe Ile Ala
               20                  25                  30
   Ser Leu Glu Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 686
   <211> 58
   <212> PRT
   <213> Artificial Sequence
<220>
   <223> Engineered polypeptide
<400> 686
   Val Asp Asn Lys Phe Asn Lys Glu Trp His Gly Ala Ala Ser Glu Ile
   1               5                   10                  15
   Trp Glu Leu Pro Asn Leu Asn Lys Ser Gln Lys Ser Ala Phe Ile Lys
               20                  25                  30
   Ser Leu Pro Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 687
   <211> 58
   <212> PRT
   <213> Artificial Sequence
<220>
   <223> Engineered polypeptide
<400> 687
   Val Asp Asn Lys Phe Asn Lys Glu Trp Lys Gln Ala Ala Glu Glu Ile
   1               5                   10                  15
   Trp Asp Leu Pro Asn Leu Asn Arg Arg Gln Ala Gly Ala Phe Ile Thr
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 688
   <211> 58
   <212> PRT
   <213> Artificial Sequence
<220>
   <223> Engineered polypeptide
<400> 688
   Val Asp Asn Lys Phe Asn Lys Glu Lys Val Gln Ala Ser Glu Glu Ile
   1               5                   10                  15
   Trp Asn Leu Pro Asn Leu Asn Arg Arg Gln Arg Ala Ala Phe Ile Gly
               20                  25                  30
   Ser Leu Tyr Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 689
   <211> 58
   <212> PRT
   <213> Artificial Sequence
<220>
   <223> Engineered polypeptide
<400> 689
   Val Asp Asn Lys Phe Asn Lys Glu Arg Tyr Ser Ala Thr Val Glu Ile
   1               5                   10                  15
   Trp Asp Leu Pro Asn Leu Asn Thr Leu Gln Lys Ser Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 690
   <211> 58
   <212> PRT
   <213> Artificial Sequence
<220>
   <223> Engineered polypeptide
<400> 690
   Val Asp Asn Lys Phe Asn Lys Glu Arg Tyr Ser Ala Tyr Tyr Glu Ile
   1               5                   10                  15
   Trp Gln Leu Pro Asn Leu Asn Arg Ile Gln Lys Ala Ala Phe Ile Ser
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 691
   <211> 58
   <212> PRT
   <213> Artificial Sequence
<220>
   <223> Engineered polypeptide
<400> 691
   Val Asp Asn Lys Phe Asn Lys Glu Arg Tyr Arg Ala Tyr Phe Glu Ile
   1               5                   10                  15
   Trp Gln Leu Pro Asn Leu Asn Arg Leu Gln Lys Ala Ala Phe Ile Ser
               20                  25                  30
   Ser Leu Glu Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 692
   <211> 58
   <212> PRT
   <213> Artificial Sequence
<220>
   <223> Engineered polypeptide
<400> 692
   Val Asp Asn Lys Phe Asn Lys Glu Lys Tyr Lys Ala Tyr Gly Glu Ile
   1 5 10 15
   Trp Gln Leu Pro Asn Leu Asn Arg Val Gln Lys Ala Ala Phe Ile Ala
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 693
   <211> 58
   <212> PRT
   <213> Artificial Sequence
<220>
   <223> Engineered polypeptide
<400> 693
   Val Asp Asn Lys Phe Asn Lys Glu Arg Lys Gln Ala Thr Phe Glu Ile
   1 5 10 15
   Trp Glu Leu Pro Asn Leu Asn Gln Arg Gln Lys Ala Ala Phe Ile Lys
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 694
   <211> 58
   <212> PRT
   <213> Artificial Sequence
<220>
   <223> Engineered polypeptide
<400> 694
   Val Asp Asn Lys Phe Asn Lys Glu Trp Lys Ile Ala Ala Gly Glu Ile
   1               5                   10                  15
   Trp Gln Leu Pro Asn Leu Asn Arg His Gln Lys Gly Ala Phe Ile Ser
               20                  25                  30
   Ser Leu Tyr Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 695
   <211> 58
   <212> PRT
   <213> Artificial Sequence
<220>
   <223> Engineered polypeptide
<400> 695
   Val Asp Asn Lys Phe Asn Lys Glu Lys Tyr Lys Ala Tyr Val Glu Ile
   1               5                   10                  15
   Trp Gln Leu Pro Asn Leu Asn Arg Tyr Gln Arg Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 696
   <211> 58
   <212> PRT
   <213> Artificial Sequence
<220>
   <223> Engineered polypeptide
<400> 696
   Val Asp Asn Lys Phe Asn Lys Glu Arg Lys Ala Ala Thr Phe Glu Ile
   1               5                   10                  15
   Trp Glu Leu Pro Asn Leu Asn Arg Ile Gln Lys Ala Ala Phe Ile Ala
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 697
   <211> 58
   <212> PRT
   <213> Artificial Sequence
<220>
   <223> Engineered polypeptide
<400> 697
   Val Asp Asn Lys Phe Asn Lys Glu Lys Tyr Lys Ala Tyr Gly Glu Ile
   1               5                   10                  15
   Trp Gln Leu Pro Asn Leu Asn Arg Ile Gln Lys Ala Ala Phe Ile Ser
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 698
   <211> 58
   <212> PRT
   <213> Artificial Sequence
<220>
   <223> Engineered polypeptide
<400> 698
   Val Asp Asn Lys Phe Asn Lys Glu Lys Arg Ile Ala Thr Trp Glu Ile
   1 5 10 15
   Trp Gln Leu Pro Asn Leu Asn Gln His Gln Lys Ala Ala Phe Ile Ser
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 699
   <211> 58
   <212> PRT
   <213> Artificial Sequence
<220>
   <223> Engineered polypeptide
<400> 699
   Val Asp Asn Lys Phe Asn Lys Glu Lys Tyr Lys Ala Tyr Val Glu Ile
   1 5 10 15
   Trp Gln Leu Pro Asn Leu Asn Arg Gly Gln Lys Ala Ala Phe Ile Ser
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 700
   <211> 58
   <212> PRT
   <213> Artificial Sequence
<220>
   <223> Engineered polypeptide
<400> 700
   Val Asp Asn Lys Phe Asn Lys Glu Arg Tyr Leu Ala Tyr Tyr Glu Ile
   1               5                   10                  15
   Trp Gln Leu Pro Asn Leu Asn Arg Thr Gln Lys Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 701
   <211> 58
   <212> PRT
   <213> Artificial Sequence
<220>
   <223> Engineered polypeptide
<400> 701
   Val Asp Asn Lys Phe Asn Lys Glu Arg Tyr Ile Ala Tyr Trp Glu Ile
   1               5                   10                  15
   Trp Gln Leu Pro Asn Leu Asn Arg Arg Gln Lys Ala Ala Phe Ile Ser
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 702
   <211> 58
   <212> PRT
   <213> Artificial Sequence
<220>
   <223> Engineered polypeptide
<400> 702
   Val Asp Asn Lys Phe Asn Lys Glu Lys Tyr Arg Ala Tyr Gly Glu Ile
   1 5 10 15
   Trp Gln Leu Pro Asn Leu Asn Arg Gly Gln Lys Ala Ala Phe Ile Ala
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 703
   <211> 58
   <212> PRT
   <213> Artificial Sequence
<220>
   <223> Engineered polypeptide
<400> 703
   Val Asp Asn Lys Phe Asn Lys Glu Lys Tyr Thr Ala Tyr Phe Glu Ile
   1 5 10 15
   Trp Gln Leu Pro Asn Leu Asn Val Arg Gln Lys Ala Ala Phe Ile Ser
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 704
   <211> 58
   <212> PRT
   <213> Artificial Sequence
<220>
   <223> Engineered polypeptide
<400> 704
   Val Asp Asn Lys Phe Asn Lys Glu Arg Tyr Ser Ala Tyr Tyr Glu Ile
   1 5 10 15
   Trp Gln Leu Pro Asn Leu Asn Val Arg Gln Lys Ala Ala Phe Ile Gly
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 705
   <211> 58
   <212> PRT
   <213> Artificial Sequence
<220>
   <223> Engineered polypeptide
<400> 705
   Val Asp Asn Lys Phe Asn Lys Glu Lys Tyr Ala Ala Tyr Gly Glu Ile
   1 5 10 15
   Trp Gln Leu Pro Asn Leu Asn Arg Ser Gln Lys Ala Ala Phe Ile Gly
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 706
   <211> 58
   <212> PRT
   <213> Artificial Sequence
<220>
   <223> Engineered polypeptide
<400> 706
   Val Asp Asn Lys Phe Asn Lys Glu Arg Lys Arg Ala Thr Val Glu Ile
   1 5 10 15
   Trp Glu Leu Pro Asn Leu Asn Arg Leu Gln Arg Gly Ala Phe Ile Ala
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 707
   <211> 58
   <212> PRT
   <213> Artificial Sequence
<220>
   <223> Engineered polypeptide
<400> 707
   Val Asp Asn Lys Phe Asn Lys Glu Trp Lys Gln Ala Ser Phe Glu Ile
   1 5 10 15
   Trp Glu Leu Pro Asn Leu Asn Arg Leu Gln Lys Ala Ala Phe Ile Gly
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 708
   <211> 58
   <212> PRT
   <213> Artificial Sequence
<220>
   <223> Engineered polypeptide
<400> 708
   Val Asp Asn Lys Phe Asn Lys Glu Arg Lys His Ala Thr Val Glu Ile
   1               5                   10                  15
   Trp Glu Leu Pro Asn Leu Asn Arg Val Gln Lys Ala Ala Phe Ile Ser
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 709
   <211> 58
   <212> PRT
   <213> Artificial Sequence
<220>
   <223> Engineered polypeptide
<400> 709
   Val Asp Asn Lys Phe Asn Lys Glu Lys Tyr Lys Ala Tyr Val Glu Ile
   1               5                   10                  15
   Trp Gln Leu Pro Asn Leu Asn Gln Arg Gln Lys Ala Ala Phe Ile Gly
               20                  25                  30
   Ser Leu Ala Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 710
   <211> 58
   <212> PRT
   <213> Artificial Sequence
<220>
   <223> Engineered polypeptide
<400> 710
   Val Asp Asn Lys Phe Asn Lys Glu Lys Tyr Val Ala Tyr Gly Glu Ile
   1 5 10 15
   Trp Gln Leu Pro Asn Leu Asn Arg Thr Gln Lys Ala Ala Phe Ile Ser
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 711
   <211> 58
   <212> PRT
   <213> Artificial Sequence
<220>
   <223> Engineered polypeptide
<400> 711
   Val Asp Asn Lys Phe Asn Lys Glu Arg Tyr Ile Ala Tyr Tyr Glu Ile
   1 5 10 15
   Trp Gln Leu Pro Asn Leu Asn Arg Tyr Gln Lys Ala Ala Phe Ile Ala
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 712
   <211> 58
   <212> PRT
   <213> Artificial Sequence
<220>
   <223> Engineered polypeptide
<400> 712
   Val Asp Asn Lys Phe Asn Lys Glu Lys Tyr Asn Ala Tyr Gly Glu Ile
   1 5 10 15
   Trp Gln Leu Pro Asn Leu Asn Val Arg Gln Lys Ala Ala Phe Ile Ser
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 713
   <211> 58
   <212> PRT
   <213> Artificial Sequence
<220>
   <223> Engineered polypeptide
<400> 713
   Val Asp Asn Lys Phe Asn Lys Glu Arg Lys Ala Ala Thr Val Glu Ile
   1 5 10 15
   Trp Glu Leu Pro Asn Leu Asn Arg Val Gln Lys Ala Ala Phe Ile Lys
   20 25 30
   Ser Leu Phe Asp Asp Pro Ser Gln Ser Ala Asn Leu Leu Ala Glu Ala
   35 40 45
   Lys Lys Leu Asn Asp Ala Gln Ala Pro Lys
   50 55
<210> 714
   <211> 58
   <212> PRT
   <213> Artificial Sequence
<220>
   <223> Engineered polypeptide
<400> 714
   Val Asp Asn Lys Phe Asn Lys Glu Arg Tyr Val Ala Tyr Tyr Glu Ile
   1               5                   10                  15
   Trp Glu Leu Pro Asn Leu Asn Gln Arg Gln Lys Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 715
   <211> 58
   <212> PRT
   <213> Artificial Sequence
<220>
   <223> Engineered polypeptide
<400> 715
   Val Asp Asn Lys Phe Asn Lys Glu Arg Tyr Arg Ala Tyr Tyr Glu Ile
   1               5                   10                  15
   Trp Gln Leu Pro Asn Leu Asn Gln Arg Gln Lys Ala Ala Phe Ile Gly
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 716
   <211> 58
   <212> PRT
   <213> Artificial Sequence
<220>
   <223> Engineered polypeptide
<400> 716
   Val Asp Asn Lys Phe Asn Lys Glu Trp Lys Ser Ala Ala Phe Glu Ile
   1               5                   10                  15
   Trp Glu Leu Pro Asn Leu Asn Arg Leu Gln Lys Ala Ala Phe Ile Arg
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 717
   <211> 58
   <212> PRT
   <213> Artificial Sequence
<220>
   <223> Engineered polypeptide
<400> 717
   Val Asp Asn Lys Phe Asn Lys Glu Arg Lys Gln Ala Thr Phe Glu Ile
   1               5                   10                  15
   Trp Glu Leu Pro Asn Leu Asn Arg His Gln Lys Gly Ala Phe Ile Ala
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 718
   <211> 58
   <212> PRT
   <213> Artificial Sequence
<220>
   <223> Engineered polypeptide
<400> 718
   Val Asp Asn Lys Phe Asn Lys Glu Trp Lys Ile Ala Ala Gly Glu Ile
   1 5 10 15
   Trp Gln Leu Pro Asn Leu Asn Arg Arg Gln Lys Ala Ala Phe Ile Ala
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 719
   <211> 58
   <212> PRT
   <213> Artificial Sequence
<220>
   <223> Engineered polypeptide
<400> 719
   Val Asp Asn Lys Phe Asn Lys Glu Arg Tyr Leu Ala Tyr Tyr Glu Ile
   1 5 10 15
   Trp Gln Leu Pro Asn Leu Asn Arg Arg Gln Lys Ala Ala Phe Ile Gly
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 720
   <211> 58
   <212> PRT
   <213> Artificial Sequence
<220>
   <223> Engineered polypeptide
<400> 720
   Val Asp Asn Lys Phe Asn Lys Glu Arg Tyr His Ala Tyr Tyr Glu Ile
   1 5 10 15
   Trp Glu Leu Pro Asn Leu Asn Arg Leu Gln Lys Ala Ala Phe Ile Ser
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 721
   <211> 58
   <212> PRT
   <213> Artificial Sequence
<220>
   <223> Engineered polypeptide
<400> 721
   Val Asp Asn Lys Phe Asn Lys Glu Lys Tyr Lys Ala Tyr Ala Glu Ile
   1 5 10 15
   Trp Gln Leu Pro Asn Leu Asn Arg Thr Gln Lys Ala Ala Phe Ile Ser
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 722
   <211> 58
   <212> PRT
   <213> Artificial Sequence
<220>
   <223> Engineered polypeptide
<400> 722
   Val Asp Asn Lys Phe Asn Lys Glu Arg Tyr Lys Ala Tyr Tyr Glu Ile
   1 5 10 15
   Trp Gln Leu Pro Asn Leu Asn Arg Gln Gln Lys Gly Ala Phe Ile Ser
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 723
   <211> 58
   <212> PRT
   <213> Artificial Sequence
<220>
   <223> Engineered polypeptide
<400> 723
   Val Asp Asn Lys Phe Asn Lys Glu Arg Lys Leu Ala Thr Phe Glu Ile
   1 5 10 15
   Trp Glu Leu Pro Asn Leu Asn Thr Ser Gln Lys Gly Ala Phe Ile Ser
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 724
   <211> 58
   <212> PRT
   <213> Artificial Sequence
<220>
   <223> Engineered polypeptide
<400> 724
   Val Asp Asn Lys Phe Asn Lys Glu Arg Tyr Val Ala Tyr Tyr Glu Ile
   1 5 10 15
   Trp Gln Leu Pro Asn Leu Asn Val Gln Gln Lys Ala Ala Phe Ile Ala
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 725
   <211> 58
   <212> PRT
   <213> Artificial Sequence
<220>
   <223> Engineered polypeptide
<400> 725
   Val Asp Asn Lys Phe Asn Lys Glu Arg Ser Ile Ala Ser Val Glu Ile
   1 5 10 15
   Trp Glu Leu Pro Asn Leu Asn Gln Arg Gln Lys Gly Ala Phe Ile Ala
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 726
   <211> 58
   <212> PRT
   <213> Artificial Sequence
<220>
   <223> Engineered polypeptide
<400> 726
   Val Asp Asn Lys Phe Asn Lys Glu Arg Lys Gln Ala Ala Val Glu Ile
   1               5                   10                  15
   Trp Glu Leu Pro Asn Leu Asn Val Arg Gln Lys Ala Ala Phe Ile Arg
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 727
   <211> 58
   <212> PRT
   <213> Artificial Sequence
<220>
   <223> Engineered polypeptide
<400> 727
   Val Asp Asn Lys Phe Asn Lys Glu Lys Lys Ile Ala Ala Phe Glu Ile
   1               5                   10                  15
   Trp Glu Leu Pro Asn Leu Asn Gln Trp Gln Lys Ala Ala Phe Ile Ala
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 728
   <211> 58
   <212> PRT
   <213> Artificial Sequence
<220>
   <223> Engineered polypeptide
<400> 728
   Val Asp Asn Lys Phe Asn Lys Glu Arg Lys Ile Ala Thr Val Glu Ile
   1 5 10 15
   Trp Glu Leu Pro Asn Leu Asn Arg Ile Gln Lys Gly Ala Phe Ile Ala
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 729
   <211> 58
   <212> PRT
   <213> Artificial Sequence
<220>
   <223> Engineered polypeptide
<400> 729
   Val Asp Asn Lys Phe Asn Lys Glu Lys Tyr Arg Ala Ala Gly Glu Ile
   1 5 10 15
   Trp Glu Leu Pro Asn Leu Asn Val Leu Gln Lys Ala Ala Phe Ile Lys
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 730
   <211> 58
   <212> PRT
   <213> Artificial Sequence
<220>
   <223> Engineered polypeptide
<400> 730
   Val Asp Asn Lys Phe Asn Lys Glu Arg Lys Ile Ala Ala Val Glu Ile
   1               5                   10                  15
   Trp Glu Leu Pro Asn Leu Asn Arg Tyr Gln Lys Ala Ala Phe Ile Lys
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 731
   <211> 58
   <212> PRT
   <213> Artificial Sequence
<220>
   <223> Engineered polypeptide
<400> 731
   Val Asp Asn Lys Phe Asn Lys Glu Lys Arg Thr Ala Thr Trp Glu Ile
   1               5                   10                  15
   Trp Gln Leu Pro Asn Leu Asn Gln Arg Gln Arg Ala Ala Phe Ile Gly
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 732
   <211> 58
   <212> PRT
   <213> Artificial Sequence
<220>
   <223> Engineered polypeptide
<400> 732
   Val Asp Asn Lys Phe Asn Lys Glu Arg Lys Thr Ala Thr Val Glu Ile
   1               5                   10                  15
   Trp Glu Leu Pro Asn Leu Asn Arg Arg Gln Lys Ala Ala Phe Ile Ala
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 733
   <211> 58
   <212> PRT
   <213> Artificial Sequence
<220>
   <223> Engineered polypeptide
<400> 733
   Val Asp Asn Lys Phe Asn Lys Glu Arg Lys Gln Ala Ala Val Glu Ile
   1               5                   10                  15
   Trp Glu Leu Pro Asn Leu Asn Arg Ile Gln Lys Gly Ala Phe Ile Lys
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 734
   <211> 58
   <212> PRT
   <213> Artificial Sequence
<220>
   <223> Engineered polypeptide
<400> 734
   Val Asp Asn Lys Phe Asn Lys Glu Arg Tyr Ile Ala Tyr Gly Glu Ile
   1 5 10 15
   Trp Gln Leu Pro Asn Leu Asn Arg Arg Gln Lys Ala Ala Phe Ile Gly
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 735
   <211> 58
   <212> PRT
   <213> Artificial Sequence
<220>
   <223> Engineered polypeptide
<400> 735
   Val Asp Asn Lys Phe Asn Lys Glu Arg Tyr Arg Ala Tyr Tyr Glu Ile
   1 5 10 15
   Trp Gln Leu Pro Asn Leu Asn Arg Thr Gln Lys Val Ala Phe Ile Gly
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 736
   <211> 58
   <212> PRT
   <213> Artificial Sequence
<220>
   <223> Engineered polypeptide
<400> 736
   Val Asp Asn Lys Phe Asn Lys Glu Arg Arg Leu Ala Tyr Trp Glu Ile
   1 5 10 15
   Trp Gln Leu Pro Asn Leu Asn Thr Thr Gln Lys Ala Ala Phe Ile Gly
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 737
   <211> 58
   <212> PRT
   <213> Artificial Sequence
<220>
   <223> Engineered polypeptide
<400> 737
   Val Asp Asn Lys Phe Asn Lys Glu Arg Arg Arg Ala Tyr Tyr Glu Ile
   1 5 10 15
   Trp Gln Leu Pro Asn Leu Asn Arg Ile Gln Lys Ala Ala Phe Ile Gly
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 738
   <211> 58
   <212> PRT
   <213> Artificial Sequence
<220>
   <223> Engineered polypeptide
<400> 738
   Val Asp Asn Lys Phe Asn Lys Glu Arg Arg Leu Ala Tyr Tyr Glu Ile
   1               5                   10                  15
   Trp Glu Leu Pro Asn Leu Asn Val Ala Gln Lys Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 739
   <211> 58
   <212> PRT
   <213> Artificial Sequence
<220>
   <223> Engineered polypeptide
<400> 739
   Val Asp Asn Lys Phe Asn Lys Glu Arg Tyr Leu Ala Tyr Tyr Glu Ile
   1               5                   10                  15
   Trp Glu Leu Pro Asn Leu Asn Val Arg Gln Lys Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 740
   <211> 58
   <212> PRT
   <213> Artificial Sequence
<220>
   <223> Engineered polypeptide
<400> 740
   Val Asp Asn Lys Phe Asn Lys Glu Arg Tyr Ala Ala Tyr Tyr Glu Ile
   1               5                   10                  15
   Trp Gln Leu Pro Asn Leu Asn Ser Arg Gln Lys Ala Ala Phe Ile Gly
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 741
   <211> 58
   <212> PRT
   <213> Artificial Sequence
<220>
   <223> Engineered polypeptide
<400> 741
   Val Asp Asn Lys Phe Asn Lys Glu Arg Tyr Leu Ala Tyr Tyr Glu Ile
   1               5                   10                  15
   Trp Gln Leu Pro Asn Leu Asn Gln Leu Gln Lys Ala Ala Phe Ile Gly
               20                  25                  30
   Ser Leu Ala Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 742
   <211> 58
   <212> PRT
   <213> Artificial Sequence
<220>
   <223> Engineered polypeptide
<400> 742
   Val Asp Asn Lys Phe Asn Lys Glu Arg Tyr His Ala Tyr Tyr Glu Ile
   1               5                   10                  15
   Trp Gln Leu Pro Asn Leu Asn Ser Trp Gln Lys Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 743
   <211> 58
   <212> PRT
   <213> Artificial Sequence
<220>
   <223> Engineered polypeptide
<400> 743
   Val Asp Asn Lys Phe Asn Lys Glu Arg Tyr His Ala Tyr Tyr Glu Ile
   1               5                   10                  15
   Trp Gln Leu Pro Asn Leu Asn Ser Ala Gln Lys Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 744
   <211> 58
   <212> PRT
   <213> Artificial Sequence
<220>
   <223> Engineered polypeptide
<400> 744
   Val Asp Asn Lys Phe Asn Lys Glu Arg Tyr Glu Ala Tyr Tyr Glu Ile
   1 5 10 15
   Trp Gln Leu Pro Asn Leu Asn Ser Val Gln Lys Ala Ala Phe Ile Gly
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 745
   <211> 58
   <212> PRT
   <213> Artificial Sequence
<220>
   <223> Engineered polypeptide
<400> 745
   Val Asp Asn Lys Phe Asn Lys Glu Arg Tyr His Ala Tyr Tyr Glu Ile
   1 5 10 15
   Trp Gln Leu Pro Asn Leu Asn Arg Arg Gln Lys Ala Ala Phe Ile Gly
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 746
   <211> 58
   <212> PRT
   <213> Artificial Sequence
<220>
   <223> Engineered polypeptide
<400> 746
   Val Asp Asn Lys Phe Asn Lys Glu Arg Tyr Thr Ala Tyr Tyr Glu Ile
   1               5                   10                  15
   Trp Gln Leu Pro Asn Leu Asn Arg Ala Gln Lys Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 747
   <211> 58
   <212> PRT
   <213> Artificial Sequence
<220>
   <223> Engineered polypeptide
<400> 747
   Val Asp Asn Lys Phe Asn Lys Glu Arg Tyr Ser Ala Tyr Tyr Glu Ile
   1               5                   10                  15
   Trp Gln Leu Pro Asn Leu Asn Arg Ala Gln Lys Ala Ala Phe Ile Gly
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 748
   <211> 58
   <212> PRT
   <213> Artificial Sequence
<220>
   <223> Engineered polypeptide
<400> 748
   Val Asp Asn Lys Phe Asn Lys Glu Arg Tyr Ser Ala Tyr Tyr Glu Ile
   1               5                   10                  15
   Trp Glu Leu Pro Asn Leu Asn Arg Ala Gln Lys Ala Ala Phe Ile Gly
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 749
   <211> 58
   <212> PRT
   <213> Artificial Sequence
<220>
   <223> Engineered polypeptide
<400> 749
   Val Asp Asn Lys Phe Asn Lys Glu Arg Tyr His Ala Tyr Tyr Glu Ile
   1               5                   10                  15
   Trp Gln Leu Pro Asn Leu Asn Arg Ala Gln Lys Ala Ala Phe Ile Gly
               20                  25                  30
   Ser Leu Tyr Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 750
   <211> 58
   <212> PRT
   <213> Artificial Sequence
<220>
   <223> Engineered polypeptide
<400> 750
   Val Asp Asn Lys Phe Asn Lys Glu Arg Tyr Ser Ala Tyr Val Glu Ile
   1               5                   10                  15
   Trp Gln Leu Pro Asn Leu Asn Arg Gly Gln Lys Ala Ala Phe Ile Gly
               20                  25                  30
   Ser Leu Tyr Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 751
   <211> 58
   <212> PRT
   <213> Artificial Sequence
<220>
   <223> Engineered polypeptide
<400> 751
   Val Asp Asn Lys Phe Asn Lys Glu Arg Tyr Ser Ala Tyr Phe Glu Ile
   1               5                   10                  15
   Trp Gln Leu Pro Asn Leu Asn Arg Leu Gln Lys Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 752
   <211> 58
   <212> PRT
   <213> Artificial Sequence
<220>
   <223> Engineered polypeptide
<400> 752
   Val Asp Asn Lys Phe Asn Lys Glu Arg Tyr Asn Ala Tyr Phe Glu Ile
   1               5                   10                  15
   Trp Gln Leu Pro Asn Leu Asn Arg Tyr Gln Lys Ser Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 753
   <211> 58
   <212> PRT
   <213> Artificial Sequence
<220>
   <223> Engineered polypeptide
<400> 753
   Val Asp Asn Lys Phe Asn Lys Glu Arg Tyr Ala Ala Tyr Phe Glu Ile
   1               5                   10                  15
   Trp Gln Leu Pro Asn Leu Asn Val Ala Gln Lys Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 754
   <211> 58
   <212> PRT
   <213> Artificial Sequence
<220>
   <223> Engineered polypeptide
<400> 754
   Val Asp Asn Lys Phe Asn Lys Glu Arg Tyr Gln Ala Tyr Phe Glu Ile
   1               5                   10                  15
   Trp Gln Leu Pro Asn Leu Asn Gln Trp Gln Lys Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 755
   <211> 58
   <212> PRT
   <213> Artificial Sequence
<220>
   <223> Engineered polypeptide
<400> 755
   Val Asp Asn Lys Phe Asn Lys Glu Arg Tyr Lys Ala Tyr Trp Glu Ile
   1               5                   10                  15
   Trp Gln Leu Pro Asn Leu Asn Gln Gly Gln Lys Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 756
   <211> 58
   <212> PRT
   <213> Artificial Sequence
<220>
   <223> Engineered polypeptide
<400> 756
   Val Asp Asn Lys Phe Asn Lys Glu Arg Tyr Ile Ala Tyr Gly Glu Ile
   1 5 10 15
   Trp Gln Leu Pro Asn Leu Asn Val Gly Gln Lys Ala Ala Phe Ile Gly
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 757
   <211> 58
   <212> PRT
   <213> Artificial Sequence
<220>
   <223> Engineered polypeptide
<400> 757
   Val Asp Asn Lys Phe Asn Lys Glu Arg Tyr Arg Ala Tyr Val Glu Ile
   1 5 10 15
   Trp Gln Leu Pro Asn Leu Asn Arg Val Gln Lys Ala Ala Phe Ile Gly
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 758
   <211> 58
   <212> PRT
   <213> Artificial Sequence
<220>
   <223> Engineered polypeptide
<400> 758
   Val Asp Asn Lys Phe Asn Lys Glu Arg Tyr Lys Ala Tyr Val Glu Ile
   1 5 10 15
   Trp Gln Leu Pro Asn Leu Asn Arg Thr Gln Lys Ala Ala Phe Ile Gly
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 759
   <211> 58
   <212> PRT
   <213> Artificial Sequence
<220>
   <223> Engineered polypeptide
<400> 759
   Val Asp Asn Lys Phe Asn Lys Glu Lys Tyr Glu Ala Tyr Gly Glu Ile
   1 5 10 15
   Trp Glu Leu Pro Asn Leu Asn Arg Ser Gln Lys Ala Ala Phe Ile Gly
   20 25 30
   Ser Leu Glu Asp Asp Pro Ser Gln Ser Ala Asn Leu Leu Ala Glu Ala
   35 40 45
   Lys Lys Leu Asn Asp Ala Gln Ala Pro Lys
   50 55
<210> 760
   <211> 58
   <212> PRT
   <213> Artificial Sequence
<220>
   <223> Engineered polypeptide
<400> 760
   Val Asp Asn Lys Phe Asn Lys Glu Lys Tyr Gln Ala Tyr Gly Glu Ile
   1               5                   10                  15
   Trp Gln Leu Pro Asn Leu Asn Arg Ser Gln Lys Ala Ala Phe Ile Thr
               20                  25                  30
   Ser Leu Glu Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 761
   <211> 58
   <212> PRT
   <213> Artificial Sequence
<220>
   <223> Engineered polypeptide
<400> 761
   Val Asp Asn Lys Phe Asn Lys Glu Lys Tyr Ala Ala Tyr Gly Glu Ile
   1               5                   10                  15
   Trp Leu Leu Pro Asn Leu Asn Arg Ile Gln Lys Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 762
   <211> 58
   <212> PRT
   <213> Artificial Sequence
<220>
   <223> Engineered polypeptide
<400> 762
   Val Asp Asn Lys Phe Asn Lys Glu Lys Tyr Lys Ala Tyr Gly Glu Ile
   1               5                   10                  15
   Trp Glu Leu Pro Asn Leu Asn Arg Arg Gln Lys Val Ala Phe Ile Ser
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 763
   <211> 58
   <212> PRT
   <213> Artificial Sequence
<220>
   <223> Engineered polypeptide
<400> 763
   Val Asp Asn Lys Phe Asn Lys Glu Lys Tyr Lys Ala Tyr Gly Glu Ile
   1               5                   10                  15
   Trp Glu Leu Pro Asn Leu Asn Arg Ser Gln Lys Ala Ala Phe Ile Ala
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 764
   <211> 58
   <212> PRT
   <213> Artificial Sequence
<220>
   <223> Engineered polypeptide
<400> 764
   Val Asp Asn Lys Phe Asn Lys Glu Lys Tyr Lys Ala Tyr Gly Glu Ile
   1 5 10 15
   Trp Gln Leu Pro Asn Leu Asn Gln Arg Gln Lys Ala Ala Phe Ile Ala
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 765
   <211> 58
   <212> PRT
   <213> Artificial Sequence
<220>
   <223> Engineered polypeptide
<400> 765
   Val Asp Asn Lys Phe Asn Lys Glu Lys Tyr Lys Ala Tyr Gly Glu Ile
   1 5 10 15
   Trp Gln Leu Pro Asn Leu Asn Arg Ser Gln Lys Ala Ala Phe Ile Ser
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 766
   <211> 58
   <212> PRT
   <213> Artificial Sequence
<220>
   <223> Engineered polypeptide
<400> 766
   Val Asp Asn Lys Phe Asn Lys Glu Lys Tyr Lys Ala Tyr Gly Glu Ile
   1 5 10 15
   Trp Gln Leu Pro Asn Leu Asn Arg Arg Gln Lys Ala Ala Phe Ile Ser
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 767
   <211> 58
   <212> PRT
   <213> Artificial Sequence
<220>
   <223> Engineered polypeptide
<400> 767
   Val Asp Asn Lys Phe Asn Lys Glu Lys Tyr Asn Ala Tyr Gly Glu Ile
   1 5 10 15
   Trp Gln Leu Pro Asn Leu Asn Arg Gly Gln Lys Ala Ala Phe Ile Ser
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 768
   <211> 58
   <212> PRT
   <213> Artificial Sequence
<220>
   <223> Engineered polypeptide
<400> 768
   Val Asp Asn Lys Phe Asn Lys Glu Lys Tyr Val Ala Tyr Ala Glu Ile
   1               5                   10                  15
   Trp Gln Leu Pro Asn Leu Asn Arg Val Gln Lys Ala Ala Phe Ile Gly
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 769
   <211> 58
   <212> PRT
   <213> Artificial Sequence
<220>
   <223> Engineered polypeptide
<400> 769
   Val Asp Asn Lys Phe Asn Lys Glu Lys Tyr Gln Ala Tyr Gly Glu Ile
   1               5                   10                  15
   Trp Gln Leu Pro Asn Leu Asn Arg Val Gln Lys Ala Ala Phe Ile Gly
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 770
   <211> 58
   <212> PRT
   <213> Artificial Sequence
<220>
   <223> Engineered polypeptide
<400> 770
   Val Asp Asn Lys Phe Asn Lys Glu Lys Tyr His Ala Tyr Gly Glu Ile
   1 5 10 15
   Trp Gln Leu Pro Asn Leu Asn Arg Ile Gln Lys Ala Ala Phe Ile Ala
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 771
   <211> 58
   <212> PRT
   <213> Artificial Sequence
<220>
   <223> Engineered polypeptide
<400> 771
   Val Asp Asn Lys Phe Asn Lys Glu Lys Tyr Thr Ala Tyr Val Glu Ile
   1 5 10 15
   Trp Gln Leu Pro Asn Leu Asn Arg Tyr Gln Arg Ala Ala Phe Ile Ala
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 772
   <211> 58
   <212> PRT
   <213> Artificial Sequence
<220>
   <223> Engineered polypeptide
<400> 772
   Val Asp Asn Lys Phe Asn Lys Glu Lys Tyr Lys Ala Tyr Val Glu Ile
   1 5 10 15
   Trp Gln Leu Pro Asn Leu Asn Arg Gly Gln Lys Ala Ala Phe Ile Gly
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 773
   <211> 58
   <212> PRT
   <213> Artificial Sequence
<220>
   <223> Engineered polypeptide
<400> 773
   Val Asp Asn Lys Phe Asn Lys Glu Lys Tyr Val Ala Tyr Val Glu Ile
   1 5 10 15
   Trp Gln Leu Pro Asn Leu Asn Arg Thr Gln Lys Ala Ala Phe Ile Ala
   20 25 30
   Ser Leu Glu Asp Asp Pro Ser Gln Ser Ala Asn Leu Leu Ala Glu Ala
   35 40 45
   Lys Lys Leu Asn Asp Ala Gln Ala Pro Lys
   50 55
<210> 774
   <211> 58
   <212> PRT
   <213> Artificial Sequence
<220>
   <223> Engineered polypeptide
<400> 774
   Val Asp Asn Lys Phe Asn Lys Glu Lys Tyr Ile Ala Tyr Val Glu Ile
   1 5 10 15
   Trp Gln Leu Pro Asn Leu Asn Arg Thr Gln Lys Ala Ala Phe Ile Ala
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 775
   <211> 58
   <212> PRT
   <213> Artificial Sequence
<220>
   <223> Engineered polypeptide
<400> 775
   Val Asp Asn Lys Phe Asn Lys Glu Lys Tyr Lys Ala Tyr Ala Glu Ile
   1 5 10 15
   Trp Gln Leu Pro Asn Leu Asn Arg Ala Gln Lys Ala Ala Phe Ile Gly
   20 25 30
   Ser Leu Tyr Asp Asp Pro Ser Gln Ser Ala Asn Leu Leu Ala Glu Ala
   35 40 45
   Lys Lys Leu Asn Asp Ala Gln Ala Pro Lys
   50 55
<210> 776
   <211> 58
   <212> PRT
   <213> Artificial Sequence
<220>
   <223> Engineered polypeptide
<400> 776
   Val Asp Asn Lys Phe Asn Lys Glu Lys Tyr Thr Ala Tyr Gly Glu Ile
   1               5                   10                  15
   Trp Gln Leu Pro Asn Leu Asn Val Arg Gln Lys Ala Ala Phe Ile Ser
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 777
   <211> 58
   <212> PRT
   <213> Artificial Sequence
<220>
   <223> Engineered polypeptide
<400> 777
   Val Asp Asn Lys Phe Asn Lys Glu Lys Tyr Thr Ala Tyr Gly Glu Ile
   1               5                   10                  15
   Trp Gln Leu Pro Asn Leu Asn Gln Arg Gln Lys Ala Ala Phe Ile Ser
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 778
   <211> 58
   <212> PRT
   <213> Artificial Sequence
<220>
   <223> Engineered polypeptide
<400> 778
   Val Asp Asn Lys Phe Asn Lys Glu Lys Tyr Thr Ala Tyr Phe Glu Ile
   1               5                   10                  15
   Trp Gln Leu Pro Asn Leu Asn Gln Gly Gln Lys Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 779
   <211> 58
   <212> PRT
   <213> Artificial Sequence
<220>
   <223> Engineered polypeptide
<400> 779
   Val Asp Asn Lys Phe Asn Lys Glu Lys Tyr Gln Ala Tyr Val Glu Ile
   1               5                   10                  15
   Trp Gln Leu Pro Asn Leu Asn Gln Arg Gln Lys Ala Ala Phe Ile Gly
               20                  25                  30
   Ser Leu Ala Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 780
   <211> 58
   <212> PRT
   <213> Artificial Sequence
<220>
   <223> Engineered polypeptide
<400> 780
   Val Asp Asn Lys Phe Asn Lys Glu Lys Tyr Ala Ala Tyr Val Glu Ile
   1               5                   10                  15
   Trp Gln Leu Pro Asn Leu Asn Gln Arg Gln Lys Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 781
   <211> 58
   <212> PRT
   <213> Artificial Sequence
<220>
   <223> Engineered polypeptide
<400> 781
   Val Asp Asn Lys Phe Asn Lys Glu Lys Tyr Val Ala Tyr Val Glu Ile
   1               5                   10                  15
   Trp Gln Leu Pro Asn Leu Asn Thr Ser Gln Lys Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 782
   <211> 58
   <212> PRT
   <213> Artificial Sequence
<220>
   <223> Engineered polypeptide
<400> 782
   Val Asp Asn Lys Phe Asn Lys Glu Lys Tyr Gln Ala Tyr Ala Glu Ile
   1 5 10 15
   Trp Gln Leu Pro Asn Leu Asn Val Arg Gln Lys Ser Ala Phe Ile Gly
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 783
   <211> 58
   <212> PRT
   <213> Artificial Sequence
<220>
   <223> Engineered polypeptide
<400> 783
   Val Asp Asn Lys Phe Asn Lys Glu Lys Tyr Gln Ala Tyr Tyr Glu Ile
   1 5 10 15
   Trp Glu Leu Pro Asn Leu Asn Val Gln Gln Lys Ser Ala Phe Ile Thr
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 784
   <211> 58
   <212> PRT
   <213> Artificial Sequence
<220>
   <223> Engineered polypeptide
<400> 784
   Val Asp Asn Lys Phe Asn Lys Glu Lys Tyr His Ala Tyr Tyr Glu Ile
   1               5                   10                  15
   Trp Gln Leu Pro Asn Leu Asn Val His Gln Lys Ala Ala Phe Ile Arg
               20                  25                  30
   Ser Leu Ala Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 785
   <211> 58
   <212> PRT
   <213> Artificial Sequence
<220>
   <223> Engineered polypeptide
<400> 785
   Val Asp Asn Lys Phe Asn Lys Glu Lys Tyr Gln Ala Tyr Tyr Glu Ile
   1               5                   10                  15
   Trp Gln Leu Pro Asn Leu Asn Val Ala Gln Lys Ala Ala Phe Ile Arg
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 786
   <211> 58
   <212> PRT
   <213> Artificial Sequence
<220>
   <223> Engineered polypeptide
<400> 786
   Val Asp Asn Lys Phe Asn Lys Glu Lys Tyr Thr Ala Tyr Tyr Glu Ile
   1 5 10 15
   Trp Gln Leu Pro Asn Leu Asn Arg Thr Gln Lys Ala Ala Phe Ile Gly
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 787
   <211> 58
   <212> PRT
   <213> Artificial Sequence
<220>
   <223> Engineered polypeptide
<400> 787
   Val Asp Asn Lys Phe Asn Lys Glu Arg Tyr Arg Ala Tyr Tyr Glu Ile
   1 5 10 15
   Trp Gln Leu Pro Asn Leu Asn Arg Gln Gln Lys Gly Ala Phe Ile Ala
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 788
   <211> 58
   <212> PRT
   <213> Artificial Sequence
<220>
   <223> Engineered polypeptide
<400> 788
   Val Asp Asn Lys Phe Asn Lys Glu Lys Tyr Lys Ala Tyr Tyr Glu Ile
   1               5                   10                  15
   Trp Gln Leu Pro Asn Leu Asn Arg Ser Gln Lys Gly Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 789
   <211> 58
   <212> PRT
   <213> Artificial Sequence
<220>
   <223> Engineered polypeptide
<400> 789
   Val Asp Asn Lys Phe Asn Lys Glu Lys Tyr Ile Ala Tyr Tyr Glu Ile
   1               5                   10                  15
   Trp Gln Leu Pro Asn Leu Asn Arg Ser Gln Lys Gly Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 790
   <211> 58
   <212> PRT
   <213> Artificial Sequence
<220>
   <223> Engineered polypeptide
<400> 790
   Val Asp Asn Lys Phe Asn Lys Glu Arg Tyr Lys Ala Tyr Tyr Glu Ile
   1 5 10 15
   Trp Gln Leu Pro Asn Leu Asn Arg Gln Gln Lys Ala Ala Phe Ile Gly
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 791
   <211> 58
   <212> PRT
   <213> Artificial Sequence
<220>
   <223> Engineered polypeptide
<400> 791
   Val Asp Asn Lys Phe Asn Lys Glu Arg Tyr Leu Ala Tyr Tyr Glu Ile
   1 5 10 15
   Trp Gln Leu Pro Asn Leu Asn Gln Phe Gln Lys Ala Ala Phe Ile Ala
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 792
   <211> 58
   <212> PRT
   <213> Artificial Sequence
<220>
   <223> Engineered polypeptide
<400> 792
   Val Asp Asn Lys Phe Asn Lys Glu Arg Tyr Leu Ala Tyr Tyr Glu Ile
   1 5 10 15
   Trp Gln Leu Pro Asn Leu Asn Arg Glu Gln Lys Ala Ala Phe Ile Ala
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 793
   <211> 58
   <212> PRT
   <213> Artificial Sequence
<220>
   <223> Engineered polypeptide
<400> 793
   Val Asp Asn Lys Phe Asn Lys Glu Arg Tyr Val Ala Tyr Gly Glu Ile
   1 5 10 15
   Trp Gln Leu Pro Asn Leu Asn Arg Tyr Gln Lys Ala Ala Phe Ile Ala
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 794
   <211> 58
   <212> PRT
   <213> Artificial Sequence
<220>
   <223> Engineered polypeptide
<400> 794
   Val Asp Asn Lys Phe Asn Lys Glu Arg Tyr Ile Ala Tyr Tyr Glu Ile
   1 5 10 15
   Trp Gln Leu Pro Asn Leu Asn Arg Ala Gln Lys Ala Ala Phe Ile Ala
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 795
   <211> 58
   <212> PRT
   <213> Artificial Sequence
<220>
   <223> Engineered polypeptide
<400> 795
   Val Asp Asn Lys Phe Asn Lys Glu Arg Tyr Gln Ala Tyr Tyr Glu Ile
   1 5 10 15
   Trp Gln Leu Pro Asn Leu Asn Arg Ile Gln Lys Ala Ala Phe Ile Lys
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 796
   <211> 58
   <212> PRT
   <213> Artificial Sequence
<220>
   <223> Engineered polypeptide
<400> 796
   Val Asp Asn Lys Phe Asn Lys Glu Arg Tyr Thr Ala Tyr Tyr Glu Ile
   1 5 10 15
   Trp Gln Leu Pro Asn Leu Asn Thr Gln Gln Lys Ala Ala Phe Ile Arg
   20 25 30
   Ser Leu Glu Asp Asp Pro Ser Gln Ser Ala Asn Leu Leu Ala Glu Ala
   35 40 45
   Lys Lys Leu Asn Asp Ala Gln Ala Pro Lys
   50 55
<210> 797
   <211> 58
   <212> PRT
   <213> Artificial Sequence
<220>
   <223> Engineered polypeptide
<400> 797
   Val Asp Asn Lys Phe Asn Lys Glu Arg Tyr Gln Ala Tyr Tyr Glu Ile
   1 5 10 15
   Trp Gln Leu Pro Asn Leu Asn Thr Ile Gln Lys Ala Ala Phe Ile Arg
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 798
   <211> 58
   <212> PRT
   <213> Artificial Sequence
<220>
   <223> Engineered polypeptide
<400> 798
   Val Asp Asn Lys Phe Asn Lys Glu Arg Tyr Ala Ala Tyr Ala Glu Ile
   1               5                   10                  15
   Trp Gln Leu Pro Asn Leu Asn Arg Trp Gln Lys Ala Ala Phe Ile Ser
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 799
   <211> 58
   <212> PRT
   <213> Artificial Sequence
<220>
   <223> Engineered polypeptide
<400> 799
   Val Asp Asn Lys Phe Asn Lys Glu Arg Tyr Arg Ala Tyr Ala Glu Ile
   1               5                   10                  15
   Trp Gln Leu Pro Asn Leu Asn Arg Trp Gln Lys Ala Ala Phe Ile Ala
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 800
   <211> 58
   <212> PRT
   <213> Artificial Sequence
<220>
   <223> Engineered polypeptide
<400> 800
   Val Asp Asn Lys Phe Asn Lys Glu Arg Tyr Arg Ala Tyr Ala Glu Ile
   1               5                   10                  15
   Trp Gln Leu Pro Asn Leu Asn Arg Ile Gln Lys Ala Ala Phe Ile Gly
               20                  25                  30
   Ser Leu Glu Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 801
   <211> 58
   <212> PRT
   <213> Artificial Sequence
<220>
   <223> Engineered polypeptide
<400> 801
   Val Asp Asn Lys Phe Asn Lys Glu Arg Tyr His Ala Tyr Ala Glu Ile
   1               5                   10                  15
   Trp Gln Leu Pro Asn Leu Asn Arg Ile Gln Lys Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 802
   <211> 58
   <212> PRT
   <213> Artificial Sequence
<220>
   <223> Engineered polypeptide
<400> 802
   Val Asp Asn Lys Phe Asn Lys Glu Arg Tyr Arg Ala Tyr Tyr Glu Ile
   1               5                   10                  15
   Trp Gln Leu Pro Asn Leu Asn Arg Tyr Gln Lys Ala Ala Phe Ile Ala
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 803
   <211> 58
   <212> PRT
   <213> Artificial Sequence
<220>
   <223> Engineered polypeptide
<400> 803
   Val Asp Asn Lys Phe Asn Lys Glu Arg Tyr Thr Ala Tyr Tyr Glu Ile
   1               5                   10                  15
   Trp Gln Leu Pro Asn Leu Asn Arg Val Gln Lys Ala Ala Phe Ile Ala
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 804
   <211> 58
   <212> PRT
   <213> Artificial Sequence
<220>
   <223> Engineered polypeptide
<400> 804
   Val Asp Asn Lys Phe Asn Lys Glu Arg Tyr His Ala Tyr Tyr Glu Ile
   1               5                   10                  15
   Trp Gln Leu Pro Asn Leu Asn Arg Thr Gln Lys Ala Ala Phe Ile Ala
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 805
   <211> 58
   <212> PRT
   <213> Artificial Sequence
<220>
   <223> Engineered polypeptide
<400> 805
   Val Asp Asn Lys Phe Asn Lys Glu Arg Tyr Val Ala Tyr Tyr Glu Ile
   1               5                   10                  15
   Trp Gln Leu Pro Asn Leu Asn Arg Arg Gln Lys Ala Ala Phe Ile Ser
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 806
   <211> 58
   <212> PRT
   <213> Artificial Sequence
<220>
   <223> Engineered polypeptide
<400> 806
   Val Asp Asn Lys Phe Asn Lys Glu Arg Tyr Leu Ala Tyr Phe Glu Ile
   1               5                   10                  15
   Trp Gln Leu Pro Asn Leu Asn Arg His Gln Lys Gly Ala Phe Ile Ala
               20                  25                  30
   Ser Leu Glu Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 807
   <211> 58
   <212> PRT
   <213> Artificial Sequence
<220>
   <223> Engineered polypeptide
<400> 807
   Val Asp Asn Lys Phe Asn Lys Glu Arg Tyr Thr Ala Tyr Phe Glu Ile
   1               5                   10                  15
   Trp Gln Leu Pro Asn Leu Asn Arg Trp Gln Lys Gly Ala Phe Ile Ala
               20                  25                  30
   Ser Leu Glu Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 808
   <211> 58
   <212> PRT
   <213> Artificial Sequence
<220>
   <223> Engineered polypeptide
<400> 808
   Val Asp Asn Lys Phe Asn Lys Glu Arg Tyr Ala Ala Tyr Phe Glu Ile
   1               5                   10                  15
   Trp Gln Leu Pro Asn Leu Asn Arg Leu Gln Lys Ala Ala Phe Ile Ser
               20                  25                  30
   Ser Leu Glu Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 809
   <211> 58
   <212> PRT
   <213> Artificial Sequence
<220>
   <223> Engineered polypeptide
<400> 809
   Val Asp Asn Lys Phe Asn Lys Glu Arg Tyr Val Ala Tyr Phe Glu Ile
   1               5                   10                  15
   Trp Gln Leu Pro Asn Leu Asn Arg Ser Gln Lys Ala Ala Phe Ile Ser
               20                  25                  30
   Ser Leu Glu Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 810
   <211> 58
   <212> PRT
   <213> Artificial Sequence
<220>
   <223> Engineered polypeptide
<400> 810
   Val Asp Asn Lys Phe Asn Lys Glu Arg Tyr Gly Ala Tyr Phe Glu Ile
   1 5 10 15
   Trp Gln Leu Pro Asn Leu Asn Arg Thr Gln Lys Ala Ala Phe Ile Ala
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 811
   <211> 58
   <212> PRT
   <213> Artificial Sequence
<220>
   <223> Engineered polypeptide
<400> 811
   Val Asp Asn Lys Phe Asn Lys Glu Lys Tyr Ile Ala Tyr Trp Glu Ile
   1 5 10 15
   Trp Gln Leu Pro Asn Leu Asn Arg Glu Gln Lys Ala Ala Phe Ile Ser
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 812
   <211> 58
   <212> PRT
   <213> Artificial Sequence
<220>
   <223> Engineered polypeptide
<400> 812
   Val Asp Asn Lys Phe Asn Lys Glu Arg Tyr Ile Ala Tyr Ala Glu Ile
   1               5                   10                  15
   Trp Gln Leu Pro Asn Leu Asn Arg Arg Gln Lys Ala Ala Phe Ile Ala
               20                  25                  30
   Ser Leu Glu Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 813
   <211> 58
   <212> PRT
   <213> Artificial Sequence
<220>
   <223> Engineered polypeptide
<400> 813
   Val Asp Asn Lys Phe Asn Lys Glu Arg Tyr Ile Ala Tyr Trp Glu Ile
   1               5                   10                  15
   Trp Gln Leu Pro Asn Leu Asn Gln Arg Gln Lys Ala Ala Phe Ile Ala
               20                  25                  30
   Ser Leu Ala Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 814
   <211> 58
   <212> PRT
   <213> Artificial Sequence
<220>
   <223> Engineered polypeptide
<400> 814
   Val Asp Asn Lys Phe Asn Lys Glu Arg Tyr Ile Ala Tyr Trp Glu Ile
   1               5                   10                  15
   Trp Gln Leu Pro Asn Leu Asn Arg Leu Gln Lys Ser Ala Phe Ile Ala
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 815
   <211> 58
   <212> PRT
   <213> Artificial Sequence
<220>
   <223> Engineered polypeptide
<400> 815
   Val Asp Asn Lys Phe Asn Lys Glu Arg Tyr His Ala Tyr Trp Glu Ile
   1               5                   10                  15
   Trp Gln Leu Pro Asn Leu Asn Arg Ala Gln Lys Ala Ala Phe Ile Arg
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 816
   <211> 58
   <212> PRT
   <213> Artificial Sequence
<220>
   <223> Engineered polypeptide
<400> 816
   Val Asp Asn Lys Phe Asn Lys Glu Arg Tyr Asn Ala Tyr Trp Glu Ile
   1 5 10 15
   Trp Gln Leu Pro Asn Leu Asn Arg Leu Gln Lys Ala Ala Phe Ile Arg
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 817
   <211> 58
   <212> PRT
   <213> Artificial Sequence
<220>
   <223> Engineered polypeptide
<400> 817
   Val Asp Asn Lys Phe Asn Lys Glu Arg Tyr Thr Ala Tyr Gly Glu Ile
   1 5 10 15
   Trp Glu Leu Pro Asn Leu Asn Arg Val Gln Arg Ala Ala Phe Ile Ala
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 818
   <211> 58
   <212> PRT
   <213> Artificial Sequence
<220>
   <223> Engineered polypeptide
<400> 818
   Val Asp Asn Lys Phe Asn Lys Glu Arg Tyr Thr Ala Tyr Gly Glu Ile
   1 5 10 15
   Trp Glu Leu Pro Asn Leu Asn Arg Leu Gln Lys Ala Ala Phe Ile Gly
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 819
   <211> 58
   <212> PRT
   <213> Artificial Sequence
<220>
   <223> Engineered polypeptide
<400> 819
   Val Asp Asn Lys Phe Asn Lys Glu Arg Tyr His Ala Tyr Gly Glu Ile
   1 5 10 15
   Trp Glu Leu Pro Asn Leu Asn Arg Arg Gln Lys Ala Ala Phe Ile Thr
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 820
   <211> 58
   <212> PRT
   <213> Artificial Sequence
<220>
   <223> Engineered polypeptide
<400> 820
   Val Asp Asn Lys Phe Asn Lys Glu Arg Tyr Lys Ala Tyr Gly Glu Ile
   1               5                   10                  15
   Trp Gln Leu Pro Asn Leu Asn Arg Leu Gln Lys Ala Ala Phe Ile Gly
               20                  25                  30
   Ser Leu Ala Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 821
   <211> 58
   <212> PRT
   <213> Artificial Sequence
<220>
   <223> Engineered polypeptide
<400> 821
   Val Asp Asn Lys Phe Asn Lys Glu Arg Tyr Gln Ala Tyr Gly Glu Ile
   1               5                   10                  15
   Trp Gln Leu Pro Asn Leu Asn Arg Leu Gln Lys Ala Ala Phe Ile Gly
               20                  25                  30
   Ser Leu Ala Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 822
   <211> 58
   <212> PRT
   <213> Artificial Sequence
<220>
   <223> Engineered polypeptide
<400> 822
   Val Asp Asn Lys Phe Asn Lys Glu Arg Tyr Val Ala Tyr Gly Glu Ile
   1               5                   10                  15
   Trp Gln Leu Pro Asn Leu Asn Arg Leu Gln Lys Ala Ala Phe Ile Ser
               20                  25                  30
   Ser Leu Ala Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 823
   <211> 58
   <212> PRT
   <213> Artificial Sequence
<220>
   <223> Engineered polypeptide
<400> 823
   Val Asp Asn Lys Phe Asn Lys Glu Arg Tyr Ile Ala Tyr Gly Glu Ile
   1               5                   10                  15
   Trp Glu Leu Pro Asn Leu Asn Arg Leu Gln Lys Ala Ala Phe Ile Gly
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 824
   <211> 58
   <212> PRT
   <213> Artificial Sequence
<220>
   <223> Engineered polypeptide
<400> 824
   Val Asp Asn Lys Phe Asn Lys Glu Arg Tyr Gln Ala Tyr Gly Glu Ile
   1               5                   10                  15
   Trp Gln Leu Pro Asn Leu Asn Arg Arg Gln Lys Ala Ala Phe Ile Ser
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 825
   <211> 58
   <212> PRT
   <213> Artificial Sequence
<220>
   <223> Engineered polypeptide
<400> 825
   Val Asp Asn Lys Phe Asn Lys Glu Arg Arg Gln Ala Tyr Phe Glu Ile
   1               5                   10                  15
   Trp Glu Leu Pro Asn Leu Asn Arg Tyr Gln Lys Ala Ala Phe Ile Ser
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 826
   <211> 58
   <212> PRT
   <213> Artificial Sequence
<220>
   <223> Engineered polypeptide
<400> 826
   Val Asp Asn Lys Phe Asn Lys Glu Arg Tyr Arg Ala Tyr Val Glu Ile
   1 5 10 15
   Trp Glu Leu Pro Asn Leu Asn Arg Ile Gln Lys Val Ala Phe Ile Ala
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 827
   <211> 58
   <212> PRT
   <213> Artificial Sequence
<220>
   <223> Engineered polypeptide
<400> 827
   Val Asp Asn Lys Phe Asn Lys Glu Lys Tyr Thr Ala Tyr Gly Glu Ile
   1 5 10 15
   Trp Glu Leu Pro Asn Leu Asn Arg Ile Gln Lys Val Ala Phe Ile Ala
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 828
   <211> 58
   <212> PRT
   <213> Artificial Sequence
<220>
   <223> Engineered polypeptide
<400> 828
   Val Asp Asn Lys Phe Asn Lys Glu Lys Tyr Ala Ala Tyr Val Glu Ile
   1 5 10 15
   Trp Glu Leu Pro Asn Leu Asn Arg Arg Gln Lys Ala Ala Phe Ile Ala
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 829
   <211> 58
   <212> PRT
   <213> Artificial Sequence
<220>
   <223> Engineered polypeptide
<400> 829
   Val Asp Asn Lys Phe Asn Lys Glu Arg Tyr Arg Ala Tyr Val Glu Ile
   1 5 10 15
   Trp Glu Leu Pro Asn Leu Asn Arg Trp Gln Lys Ala Ala Phe Ile Arg
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 830
   <211> 58
   <212> PRT
   <213> Artificial Sequence
<220>
   <223> Engineered polypeptide
<400> 830
   Val Asp Asn Lys Phe Asn Lys Glu Arg Tyr Arg Ala Tyr Phe Glu Ile
   1 5 10 15
   Trp Glu Leu Pro Asn Leu Asn Arg Ala Gln Lys Ala Ala Phe Ile Ser
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 831
   <211> 58
   <212> PRT
   <213> Artificial Sequence
<220>
   <223> Engineered polypeptide
<400> 831
   Val Asp Asn Lys Phe Asn Lys Glu Arg Tyr Gln Ala Tyr Phe Glu Ile
   1 5 10 15
   Trp Gln Leu Pro Asn Leu Asn Arg Leu Gln Ala Gly Ala Phe Ile Ala
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 832
   <211> 58
   <212> PRT
   <213> Artificial Sequence
<220>
   <223> Engineered polypeptide
<400> 832
   Val Asp Asn Lys Phe Asn Lys Glu Lys Tyr Gln Ala Tyr Val Glu Ile
   1 5 10 15
   Trp Gln Leu Pro Asn Leu Asn Arg Leu Gln Lys Gly Ala Phe Ile Ala
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 833
   <211> 58
   <212> PRT
   <213> Artificial Sequence
<220>
   <223> Engineered polypeptide
<400> 833
   Val Asp Asn Lys Phe Asn Lys Glu Lys Tyr Ala Ala Tyr Tyr Glu Ile
   1 5 10 15
   Trp Gln Leu Pro Asn Leu Asn Arg Ser Gln Lys Gly Ala Phe Ile Ala
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 834
   <211> 58
   <212> PRT
   <213> Artificial Sequence
<220>
   <223> Engineered polypeptide
<400> 834
   Val Asp Asn Lys Phe Asn Lys Glu Lys Tyr Gln Ala Tyr Tyr Glu Ile
   1 5 10 15
   Trp Glu Leu Pro Asn Leu Asn Arg Gly Gln Lys Ala Ala Phe Ile Ala
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 835
   <211> 58
   <212> PRT
   <213> Artificial Sequence
<220>
   <223> Engineered polypeptide
<400> 835
   Val Asp Asn Lys Phe Asn Lys Glu Lys Tyr Asn Ala Tyr Phe Glu Ile
   1 5 10 15
   Trp Gln Leu Pro Asn Leu Asn Arg Leu Gln Lys Ala Ala Phe Ile Thr
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 836
   <211> 58
   <212> PRT
   <213> Artificial Sequence
<220>
   <223> Engineered polypeptide
<400> 836
   Val Asp Asn Lys Phe Asn Lys Glu Lys Tyr Ser Ala Tyr Phe Glu Ile
   1               5                   10                  15
   Trp Gln Leu Pro Asn Leu Asn Thr Ser Gln Lys Ala Ala Phe Ile Ala
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 837
   <211> 58
   <212> PRT
   <213> Artificial Sequence
<220>
   <223> Engineered polypeptide
<400> 837
   Val Asp Asn Lys Phe Asn Lys Glu Arg Tyr Asn Ala Tyr Phe Glu Ile
   1               5                   10                  15
   Trp Glu Leu Pro Asn Leu Asn Val Arg Gln Lys Ala Ala Phe Ile Arg
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 838
   <211> 58
   <212> PRT
   <213> Artificial Sequence
<220>
   <223> Engineered polypeptide
<400> 838
   Val Asp Asn Lys Phe Asn Lys Glu Lys Tyr Glu Ala Tyr Phe Glu Ile
   1 5 10 15
   Trp Glu Leu Pro Asn Leu Asn Ser Arg Gln Lys Ala Ala Phe Ile Gly
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 839
   <211> 58
   <212> PRT
   <213> Artificial Sequence
<220>
   <223> Engineered polypeptide
<400> 839
   Val Asp Asn Lys Phe Asn Lys Glu Arg Tyr Arg Ala Tyr Trp Glu Ile
   1 5 10 15
   Trp Glu Leu Pro Asn Leu Asn Gln Gln Gln Lys Val Ala Phe Ile Arg
   20 25 30
   Ser Leu Tyr Asp Asp Pro Ser Gln Ser Ala Asn Leu Leu Ala Glu Ala
   35 40 45
   Lys Lys Leu Asn Asp Ala Gln Ala Pro Lys
   50 55
<210> 840
   <211> 58
   <212> PRT
   <213> Artificial Sequence
<220>
   <223> Engineered polypeptide
<400> 840
   Val Asp Asn Lys Phe Asn Lys Glu Arg Tyr Asn Ala Tyr Trp Glu Ile
   1               5                   10                  15
   Trp Glu Leu Pro Asn Leu Asn Gln Gly Gln Lys Val Ala Phe Ile Arg
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 841
   <211> 58
   <212> PRT
   <213> Artificial Sequence
<220>
   <223> Engineered polypeptide
<400> 841
   Val Asp Asn Lys Phe Asn Lys Glu Arg Arg His Ala Tyr Gly Glu Ile
   1               5                   10                  15
   Trp Gln Leu Pro Asn Leu Asn Gln Arg Gln Lys Val Ala Phe Ile Ser
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 842
   <211> 58
   <212> PRT
   <213> Artificial Sequence
<220>
   <223> Engineered polypeptide
<400> 842
   Val Asp Asn Lys Phe Asn Lys Glu Lys Tyr Val Ala Thr Trp Glu Ile
   1 5 10 15
   Trp Glu Leu Pro Asn Leu Asn Arg Ala Gln Lys Val Ala Phe Ile Gly
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 843
   <211> 58
   <212> PRT
   <213> Artificial Sequence
<220>
   <223> Engineered polypeptide
<400> 843
   Val Asp Asn Lys Phe Asn Lys Glu Arg Arg Ile Ala Arg Trp Glu Ile
   1 5 10 15
   Trp Glu Leu Pro Asn Leu Asn Arg Leu Gln Lys Val Ala Phe Ile Gly
   20 25 30
   Ser Leu Glu Asp Asp Pro Ser Gln Ser Ala Asn Leu Leu Ala Glu Ala
   35 40 45
   Lys Lys Leu Asn Asp Ala Gln Ala Pro Lys
   50 55
<210> 844
   <211> 58
   <212> PRT
   <213> Artificial Sequence
<220>
   <223> Engineered polypeptide
<400> 844
   Val Asp Asn Lys Phe Asn Lys Glu Lys Tyr Lys Ala Ala Gly Glu Ile
   1               5                   10                  15
   Trp Gln Leu Pro Asn Leu Asn Arg Gly Gln Lys Val Ala Phe Ile Gly
               20                  25                  30
   Ser Leu Glu Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 845
   <211> 58
   <212> PRT
   <213> Artificial Sequence
<220>
   <223> Engineered polypeptide
<400> 845
   Val Asp Asn Lys Phe Asn Lys Glu Lys Tyr Leu Ala Tyr Gly Glu Ile
   1               5                   10                  15
   Trp Gln Leu Pro Asn Leu Asn Arg His Gln Lys Val Ala Phe Ile Gly
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 846
   <211> 58
   <212> PRT
   <213> Artificial Sequence
<220>
   <223> Engineered polypeptide
<400> 846
   Val Asp Asn Lys Phe Asn Lys Glu Lys Tyr Gln Ala Tyr Gly Glu Ile
   1               5                   10                  15
   Trp Gln Leu Pro Asn Leu Asn Arg Gln Gln Lys Val Ala Phe Ile Ser
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 847
   <211> 58
   <212> PRT
   <213> Artificial Sequence
<220>
   <223> Engineered polypeptide
<400> 847
   Val Asp Asn Lys Phe Asn Lys Glu Lys Tyr Lys Ala Tyr Gly Glu Ile
   1               5                   10                  15
   Trp Gln Leu Pro Asn Leu Asn Arg Gln Gln Lys Val Ala Phe Ile Ile
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 848
   <211> 58
   <212> PRT
   <213> Artificial Sequence
<220>
   <223> Engineered polypeptide
<400> 848
   Val Asp Asn Lys Phe Asn Lys Glu Lys Tyr Ala Ala Ala Tyr Glu Ile
   1 5 10 15
   Trp Gln Leu Pro Asn Leu Asn Gln Arg Gln Lys Gly Ala Phe Ile Ala
   20 25 30
   Ser Leu Phe Asp Asp Pro Ser Gln Ser Ala Asn Leu Leu Ala Glu Ala
   35 40 45
   Lys Lys Leu Asn Asp Ala Gln Ala Pro Lys
   50 55
<210> 849
   <211> 58
   <212> PRT
   <213> Artificial Sequence
<220>
   <223> Engineered polypeptide
<400> 849
   Val Asp Asn Lys Phe Asn Lys Glu Lys Tyr Ala Ala Ser Tyr Glu Ile
   1 5 10 15
   Trp Glu Leu Pro Asn Leu Asn Gln Arg Gln Lys Ala Ala Phe Ile Lys
   20 25 30
   Ser Leu Phe Asp Asp Pro Ser Gln Ser Ala Asn Leu Leu Ala Glu Ala
   35 40 45
   Lys Lys Leu Asn Asp Ala Gln Ala Pro Lys
   50 55
<210> 850
   <211> 58
   <212> PRT
   <213> Artificial Sequence
<220>
   <223> Engineered polypeptide
<400> 850
   Val Asp Asn Lys Phe Asn Lys Glu Lys Tyr Leu Ala Ser Tyr Glu Ile
   1               5                   10                  15
   Trp Gln Leu Pro Asn Leu Asn Arg Arg Gln Lys Ala Ala Phe Ile Ala
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 851
   <211> 58
   <212> PRT
   <213> Artificial Sequence
<220>
   <223> Engineered polypeptide
<400> 851
   Val Asp Asn Lys Phe Asn Lys Glu Lys Tyr Ser Ala Thr Phe Glu Ile
   1               5                   10                  15
   Trp Gln Leu Pro Asn Leu Asn Gln Arg Gln Lys Ala Ala Phe Ile Ser
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 852
   <211> 58
   <212> PRT
   <213> Artificial Sequence
<220>
   <223> Engineered polypeptide
<400> 852
   Val Asp Asn Lys Phe Asn Lys Glu Lys Tyr Ala Ala Ser Phe Glu Ile
   1               5                   10                  15
   Trp Gln Leu Pro Asn Leu Asn Thr His Gln Lys Ala Ala Phe Ile Ser
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 853
   <211> 58
   <212> PRT
   <213> Artificial Sequence
<220>
   <223> Engineered polypeptide
<400> 853
   Val Asp Asn Lys Phe Asn Lys Glu Lys Tyr Lys Ala Ser Gly Glu Ile
   1               5                   10                  15
   Trp Gln Leu Pro Asn Leu Asn Arg Leu Gln Arg Ala Ala Phe Ile Lys
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 854
   <211> 58
   <212> PRT
   <213> Artificial Sequence
<220>
   <223> Engineered polypeptide
<400> 854
   Val Asp Asn Lys Phe Asn Lys Glu Lys Tyr Ser Ala Ser Gly Glu Ile
   1               5                   10                  15
   Trp Gln Leu Pro Asn Leu Asn Arg Thr Gln Lys Ala Ala Phe Ile Lys
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 855
   <211> 58
   <212> PRT
   <213> Artificial Sequence
<220>
   <223> Engineered polypeptide
<400> 855
   Val Asp Asn Lys Phe Asn Lys Glu Lys Tyr His Ala Ala Gly Glu Ile
   1               5                   10                  15
   Trp Glu Leu Pro Asn Leu Asn Arg Leu Gln Lys Ala Ala Phe Ile Arg
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 856
   <211> 58
   <212> PRT
   <213> Artificial Sequence
<220>
   <223> Engineered polypeptide
<400> 856
   Val Asp Asn Lys Phe Asn Lys Glu Lys Tyr Arg Ala Ala Gly Glu Ile
   1               5                   10                  15
   Trp Glu Leu Pro Asn Leu Asn Arg Gln Gln Lys Ala Ala Phe Ile Arg
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 857
   <211> 58
   <212> PRT
   <213> Artificial Sequence
<220>
   <223> Engineered polypeptide
<400> 857
   Val Asp Asn Lys Phe Asn Lys Glu Lys Tyr Gln Ala Ser Gly Glu Ile
   1               5                   10                  15
   Trp Glu Leu Pro Asn Leu Asn Arg Leu Gln Lys Ala Ala Phe Ile Arg
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 858
   <211> 58
   <212> PRT
   <213> Artificial Sequence
<220>
   <223> Engineered polypeptide
<400> 858
   Val Asp Asn Lys Phe Asn Lys Glu Lys Tyr Lys Ala Thr Gly Glu Ile
   1 5 10 15
   Trp Glu Leu Pro Asn Leu Asn Arg Ile Gln Lys Ala Ala Phe Ile Arg
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 859
   <211> 58
   <212> PRT
   <213> Artificial Sequence
<220>
   <223> Engineered polypeptide
<400> 859
   Val Asp Asn Lys Phe Asn Lys Glu Lys Tyr Gln Ala Ser Phe Glu Ile
   1 5 10 15
   Trp Glu Leu Pro Asn Leu Asn Arg Ser Gln Lys Ala Ala Phe Ile Lys
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 860
   <211> 58
   <212> PRT
   <213> Artificial Sequence
<220>
   <223> Engineered polypeptide
<400> 860
   Val Asp Asn Lys Phe Asn Lys Glu Arg Lys Val Ala Ala Val Glu Ile
   1 5 10 15
   Trp Gln Leu Pro Asn Leu Asn Arg Trp Gln Arg Gly Ala Phe Ile Ser
   20 25 30
   Ser Leu Phe Asp Asp Pro Ser Gln Ser Ala Asn Leu Leu Ala Glu Ala
   35 40 45
   Lys Lys Leu Asn Asp Ala Gln Ala Pro Lys
   50 55
<210> 861
   <211> 58
   <212> PRT
   <213> Artificial Sequence
<220>
   <223> Engineered polypeptide
<400> 861
   Val Asp Asn Lys Phe Asn Lys Glu Arg Lys Leu Ala Ala Val Glu Ile
   1 5 10 15
   Trp Glu Leu Pro Asn Leu Asn Gln Tyr Gln Arg Gly Ala Phe Ile Ser
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 862
   <211> 58
   <212> PRT
   <213> Artificial Sequence
<220>
   <223> Engineered polypeptide
<400> 862
   Val Asp Asn Lys Phe Asn Lys Glu Arg Lys Lys Ala Ser Val Glu Ile
   1               5                   10                  15
   Trp Glu Leu Pro Asn Leu Asn Arg Tyr Gln Lys Gly Ala Phe Ile Ser
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 863
   <211> 58
   <212> PRT
   <213> Artificial Sequence
<220>
   <223> Engineered polypeptide
<400> 863
   Val Asp Asn Lys Phe Asn Lys Glu Arg Lys His Ala Ala Trp Glu Ile
   1               5                   10                  15
   Trp Glu Leu Pro Asn Leu Asn Arg His Gln Lys Gly Ala Phe Ile Lys
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 864
   <211> 58
   <212> PRT
   <213> Artificial Sequence
<220>
   <223> Engineered polypeptide
<400> 864
   Val Asp Asn Lys Phe Asn Lys Glu Arg Lys His Ala Ala Val Glu Ile
   1               5                   10                  15
   Trp Glu Leu Pro Asn Leu Asn Arg Trp Gln Lys Gly Ala Phe Ile Gly
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 865
   <211> 58
   <212> PRT
   <213> Artificial Sequence
<220>
   <223> Engineered polypeptide
<400> 865
   Val Asp Asn Lys Phe Asn Lys Glu Lys Arg His Ala Ala Val Glu Ile
   1               5                   10                  15
   Trp Glu Leu Pro Asn Leu Asn Val Phe Gln Arg Gly Ala Phe Ile Ala
               20                  25                  30
   Ser Leu Tyr Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 866
   <211> 58
   <212> PRT
   <213> Artificial Sequence
<220>
   <223> Engineered polypeptide
<400> 866
   Val Asp Asn Lys Phe Asn Lys Glu Arg Lys His Ala Ala Val Glu Ile
   1               5                   10                  15
   Trp Glu Leu Pro Asn Leu Asn Arg Leu Gln Arg Gly Ala Phe Ile Ala
               20                  25                  30
   Ser Leu Tyr Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 867
   <211> 58
   <212> PRT
   <213> Artificial Sequence
<220>
   <223> Engineered polypeptide
<400> 867
   Val Asp Asn Lys Phe Asn Lys Glu Trp Lys Thr Ala Ala Val Glu Ile
   1               5                   10                  15
   Trp Glu Leu Pro Asn Leu Asn Arg Arg Gln Lys Ala Ala Phe Ile Gly
               20                  25                  30
   Ser Leu Tyr Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 868
   <211> 58
   <212> PRT
   <213> Artificial Sequence
<220>
   <223> Engineered polypeptide
<400> 868
   Val Asp Asn Lys Phe Asn Lys Glu Arg Lys Val Ala Ala Phe Glu Ile
   1               5                   10                  15
   Trp Glu Leu Pro Asn Leu Asn Arg Gly Gln Lys Ala Ala Phe Ile Gly
               20                  25                  30
   Ser Leu Tyr Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 869
   <211> 58
   <212> PRT
   <213> Artificial Sequence
<220>
   <223> Engineered polypeptide
<400> 869
   Val Asp Asn Lys Phe Asn Lys Glu Lys Arg Asn Ala Ala Val Glu Ile
   1               5                   10                  15
   Trp Glu Leu Pro Asn Leu Asn Arg Arg Gln Arg Ala Ala Phe Ile Ser
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 870
   <211> 58
   <212> PRT
   <213> Artificial Sequence
<220>
   <223> Engineered polypeptide
<400> 870
   Val Asp Asn Lys Phe Asn Lys Glu Arg Arg Asn Ala Ala Val Glu Ile
   1               5                   10                  15
   Trp Glu Leu Pro Asn Leu Asn Arg His Gln Arg Ala Ala Phe Ile Ser
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 871
   <211> 58
   <212> PRT
   <213> Artificial Sequence
<220>
   <223> Engineered polypeptide
<400> 871
   Val Asp Asn Lys Phe Asn Lys Glu Lys Arg Gln Ala Ser Val Glu Ile
   1               5                   10                  15
   Trp Glu Leu Pro Asn Leu Asn Arg Ile Gln Lys Ala Ala Phe Ile Ser
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 872
   <211> 58
   <212> PRT
   <213> Artificial Sequence
<220>
   <223> Engineered polypeptide
<400> 872
   Val Asp Asn Lys Phe Asn Lys Glu Lys Arg Arg Ala Ala Val Glu Ile
   1               5                   10                  15
   Trp Glu Leu Pro Asn Leu Asn Arg Ile Gln Lys Ser Ala Phe Ile Ala
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 873
   <211> 58
   <212> PRT
   <213> Artificial Sequence
<220>
   <223> Engineered polypeptide
<400> 873
   Val Asp Asn Lys Phe Asn Lys Glu Lys Arg His Ala Ala Trp Glu Ile
   1               5                   10                  15
   Trp Glu Leu Pro Asn Leu Asn Arg Ala Gln Lys Ser Ala Phe Ile Lys
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 874
   <211> 58
   <212> PRT
   <213> Artificial Sequence
<220>
   <223> Engineered polypeptide
<400> 874
   Val Asp Asn Lys Phe Asn Lys Glu Lys Arg Asn Ala Thr Val Glu Ile
   1 5 10 15
   Trp Glu Leu Pro Asn Leu Asn Arg Ile Gln Arg Ala Ala Phe Ile Ala
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 875
   <211> 58
   <212> PRT
   <213> Artificial Sequence
<220>
   <223> Engineered polypeptide
<400> 875
   Val Asp Asn Lys Phe Asn Lys Glu Lys Arg Ala Ala Thr Val Glu Ile
   1 5 10 15
   Trp Glu Leu Pro Asn Leu Asn Arg Leu Gln Lys Gly Ala Phe Ile Ala
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 876
   <211> 58
   <212> PRT
   <213> Artificial Sequence
<220>
   <223> Engineered polypeptide
<400> 876
   Val Asp Asn Lys Phe Asn Lys Glu Arg Arg Gly Ala Ala Val Glu Ile
   1 5 10 15
   Trp Glu Leu Pro Asn Leu Asn Arg Gln Gln Lys Ala Ala Phe Ile Ala
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 877
   <211> 58
   <212> PRT
   <213> Artificial Sequence
<220>
   <223> Engineered polypeptide
<400> 877
   Val Asp Asn Lys Phe Asn Lys Glu Arg Arg Lys Ala Thr Val Glu Ile
   1 5 10 15
   Trp Glu Leu Pro Asn Leu Asn Arg Gln Gln Lys Ala Ala Phe Ile Ser
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 878
   <211> 58
   <212> PRT
   <213> Artificial Sequence
<220>
   <223> Engineered polypeptide
<400> 878
   Val Asp Asn Lys Phe Asn Lys Glu Lys Ser Thr Ala Ala Val Glu Ile
   1 5 10 15
   Trp Glu Leu Pro Asn Leu Asn Arg Ile Gln Lys Ala Ala Phe Ile Lys
   20 25 30
   Ser Leu Phe Asp Asp Pro Ser Gln Ser Ala Asn Leu Leu Ala Glu Ala
   35 40 45
   Lys Lys Leu Asn Asp Ala Gln Ala Pro Lys
   50 55
<210> 879
   <211> 58
   <212> PRT
   <213> Artificial Sequence
<220>
   <223> Engineered polypeptide
<400> 879
   Val Asp Asn Lys Phe Asn Lys Glu Lys Ser Thr Ala Ala Val Glu Ile
   1 5 10 15
   Trp Glu Leu Pro Asn Leu Asn Arg Tyr Gln Lys Ala Ala Phe Ile Lys
   20 25 30
   Ser Leu Phe Asp Asp Pro Ser Gln Ser Ala Asn Leu Leu Ala Glu Ala
   35 40 45
   Lys Lys Leu Asn Asp Ala Gln Ala Pro Lys
   50 55
<210> 880
   <211> 58
   <212> PRT
   <213> Artificial Sequence
<220>
   <223> Engineered polypeptide
<400> 880
   Val Asp Asn Lys Phe Asn Lys Glu Lys Ser His Ala Ala Val Glu Ile
   1 5 10 15
   Trp Glu Leu Pro Asn Leu Asn Arg Trp Gln Lys Ala Ala Phe Ile Lys
   20 25 30
   Ser Leu Tyr Asp Asp Pro Ser Gln Ser Ala Asn Leu Leu Ala Glu Ala
   35 40 45
   Lys Lys Leu Asn Asp Ala Gln Ala Pro Lys
   50 55
<210> 881
   <211> 58
   <212> PRT
   <213> Artificial Sequence
<220>
   <223> Engineered polypeptide
<400> 881
   Val Asp Asn Lys Phe Asn Lys Glu Arg Ser Ser Ala Ala Val Glu Ile
   1 5 10 15
   Trp Glu Leu Pro Asn Leu Asn Arg Phe Gln Lys Ala Ala Phe Ile Lys
   20 25 30
   Ser Leu Phe Asp Asp Pro Ser Gln Ser Ala Asn Leu Leu Ala Glu Ala
   35 40 45
   Lys Lys Leu Asn Asp Ala Gln Ala Pro Lys
   50 55
<210> 882
   <211> 58
   <212> PRT
   <213> Artificial Sequence
<220>
   <223> Engineered polypeptide
<400> 882
   Val Asp Asn Lys Phe Asn Lys Glu Lys Arg Ile Ala Ala Ala Glu Ile
   1               5                   10                  15
   Trp Glu Leu Pro Asn Leu Asn Arg Val Gln Lys Ala Ala Phe Ile Lys
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 883
   <211> 58
   <212> PRT
   <213> Artificial Sequence
<220>
   <223> Engineered polypeptide
<400> 883
   Val Asp Asn Lys Phe Asn Lys Glu Lys Arg His Ala Ala Gly Glu Ile
   1               5                   10                  15
   Trp Glu Leu Pro Asn Leu Asn Arg Ile Gln Lys Ala Ala Phe Ile Lys
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 884
   <211> 58
   <212> PRT
   <213> Artificial Sequence
<220>
   <223> Engineered polypeptide
<400> 884
   Val Asp Asn Lys Phe Asn Lys Glu Lys Arg Ile Ala Ala Val Glu Ile
   1               5                   10                  15
   Trp Glu Leu Pro Asn Leu Asn Arg Val Gln Lys Ala Ala Phe Ile Ala
               20                  25                  30
   Ser Leu Tyr Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 885
   <211> 58
   <212> PRT
   <213> Artificial Sequence
<220>
   <223> Engineered polypeptide
<400> 885
   Val Asp Asn Lys Phe Asn Lys Glu Lys Arg Ile Ala Ser Tyr Glu Ile
   1               5                   10                  15
   Trp Glu Leu Pro Asn Leu Asn Arg Arg Gln Arg Ala Ala Phe Ile Ala
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 886
   <211> 58
   <212> PRT
   <213> Artificial Sequence
<220>
   <223> Engineered polypeptide
<400> 886
   Val Asp Asn Lys Phe Asn Lys Glu Lys Arg Asn Ala Thr Phe Glu Ile
   1               5                   10                  15
   Trp Glu Leu Pro Asn Leu Asn Arg Ser Gln Lys Gly Ala Phe Ile Ser
               20                  25                  30
   Ser Leu Tyr Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 887
   <211> 58
   <212> PRT
   <213> Artificial Sequence
<220>
   <223> Engineered polypeptide
<400> 887
   Val Asp Asn Lys Phe Asn Lys Glu Arg Lys Val Ala Thr Phe Glu Ile
   1               5                   10                  15
   Trp Glu Leu Pro Asn Leu Asn Ser Arg Gln Lys Ala Ala Phe Ile Lys
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 888
   <211> 58
   <212> PRT
   <213> Artificial Sequence
<220>
   <223> Engineered polypeptide
<400> 888
   Val Asp Asn Lys Phe Asn Lys Glu Arg Lys Asn Ala Thr Phe Glu Ile
   1 5 10 15
   Trp Glu Leu Pro Asn Leu Asn Gln Arg Gln Lys Ser Ala Phe Ile Ala
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 889
   <211> 58
   <212> PRT
   <213> Artificial Sequence
<220>
   <223> Engineered polypeptide
<400> 889
   Val Asp Asn Lys Phe Asn Lys Glu Arg Ser Ala Ala Thr Phe Glu Ile
   1 5 10 15
   Trp Glu Leu Pro Asn Leu Asn Gln Arg Gln Lys Ser Ala Phe Ile Ala
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 890
   <211> 58
   <212> PRT
   <213> Artificial Sequence
<220>
   <223> Engineered polypeptide
<400> 890
   Val Asp Asn Lys Phe Asn Lys Glu Arg Lys His Ala Thr Phe Glu Ile
   1               5                   10                  15
   Trp Glu Leu Pro Asn Leu Asn Gln Leu Gln Lys Ser Ala Phe Ile Gly
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 891
   <211> 58
   <212> PRT
   <213> Artificial Sequence
<220>
   <223> Engineered polypeptide
<400> 891
   Val Asp Asn Lys Phe Asn Lys Glu Arg Lys Gln Ala Thr Phe Glu Ile
   1               5                   10                  15
   Trp Glu Leu Pro Asn Leu Asn Gln Gln Gln Lys Gly Ala Phe Ile Ala
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 892
   <211> 58
   <212> PRT
   <213> Artificial Sequence
<220>
   <223> Engineered polypeptide
<400> 892
   Val Asp Asn Lys Phe Asn Lys Glu Arg Lys Arg Ala Thr Phe Glu Ile
   1 5 10 15
   Trp Glu Leu Pro Asn Leu Asn Arg Thr Gln Lys Ala Ala Phe Ile Ser
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 893
   <211> 58
   <212> PRT
   <213> Artificial Sequence
<220>
   <223> Engineered polypeptide
<400> 893
   Val Asp Asn Lys Phe Asn Lys Glu Arg Lys Thr Ala Thr Phe Glu Ile
   1 5 10 15
   Trp Glu Leu Pro Asn Leu Asn Arg Val Gln Lys Ala Ala Phe Ile Ser
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 894
   <211> 58
   <212> PRT
   <213> Artificial Sequence
<220>
   <223> Engineered polypeptide
<400> 894
   Val Asp Asn Lys Phe Asn Lys Glu Arg Lys Leu Ala Thr Trp Glu Ile
   1               5                   10                  15
   Trp Glu Leu Pro Asn Leu Asn Arg Ser Gln Lys Ala Ala Phe Ile Ile
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 895
   <211> 58
   <212> PRT
   <213> Artificial Sequence
<220>
   <223> Engineered polypeptide
<400> 895
   Val Asp Asn Lys Phe Asn Lys Glu Arg Lys Val Ala Ala Val Glu Ile
   1               5                   10                  15
   Trp Glu Leu Pro Asn Leu Asn Arg Ile Gln Lys Ala Ala Phe Ile Arg
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 896
   <211> 58
   <212> PRT
   <213> Artificial Sequence
<220>
   <223> Engineered polypeptide
<400> 896
   Val Asp Asn Lys Phe Asn Lys Glu Arg Lys Glu Ala Ala Val Glu Ile
   1 5 10 15
   Trp Glu Leu Pro Asn Leu Asn Arg Leu Gln Lys Ala Ala Phe Ile Arg
   20 25 30
   Ser Leu Phe Asp Asp Pro Ser Gln Ser Ala Asn Leu Leu Ala Glu Ala
   35 40 45
   Lys Lys Leu Asn Asp Ala Gln Ala Pro Lys
   50 55
<210> 897
   <211> 58
   <212> PRT
   <213> Artificial Sequence
<220>
   <223> Engineered polypeptide
<400> 897
   Val Asp Asn Lys Phe Asn Lys Glu Arg Lys Arg Ala Ala Val Glu Ile
   1 5 10 15
   Trp Glu Leu Pro Asn Leu Asn Arg Gly Gln Lys Ala Ala Phe Ile Lys
   20 25 30
   Ser Leu Phe Asp Asp Pro Ser Gln Ser Ala Asn Leu Leu Ala Glu Ala
   35 40 45
   Lys Lys Leu Asn Asp Ala Gln Ala Pro Lys
   50 55
<210> 898
   <211> 58
   <212> PRT
   <213> Artificial Sequence
<220>
   <223> Engineered polypeptide
<400> 898
   Val Asp Asn Lys Phe Asn Lys Glu Arg Lys Leu Ala Ala Val Glu Ile
   1               5                   10                  15
   Trp Glu Leu Pro Asn Leu Asn Arg Thr Gln Lys Ala Ala Phe Ile Lys
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 899
   <211> 58
   <212> PRT
   <213> Artificial Sequence
<220>
   <223> Engineered polypeptide
<400> 899
   Val Asp Asn Lys Phe Asn Lys Glu Arg Lys Val Ala Ala Val Glu Ile
   1               5                   10                  15
   Trp Glu Leu Pro Asn Leu Asn Arg Val Gln Lys Ala Ala Phe Ile Lys
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 900
   <211> 58
   <212> PRT
   <213> Artificial Sequence
<220>
   <223> Engineered polypeptide
<400> 900
   Val Asp Asn Lys Phe Asn Lys Glu Arg Lys Glu Ala Ala Val Glu Ile
   1 5 10 15
   Trp Glu Leu Pro Asn Leu Asn Arg Val Gln Lys Ala Ala Phe Ile Lys
   20 25 30
   Ser Leu Tyr Asp Asp Pro Ser Gln Ser Ala Asn Leu Leu Ala Glu Ala
   35 40 45
   Lys Lys Leu Asn Asp Ala Gln Ala Pro Lys
   50 55
<210> 901
   <211> 58
   <212> PRT
   <213> Artificial Sequence
<220>
   <223> Engineered polypeptide
<400> 901
   Val Asp Asn Lys Phe Asn Lys Glu Arg Lys Ala Ala Thr Val Glu Ile
   1 5 10 15
   Trp Glu Leu Pro Asn Leu Asn Arg Arg Gln Lys Ala Ala Phe Ile Ala
   20 25 30
   Ser Leu Phe Asp Asp Pro Ser Gln Ser Ala Asn Leu Leu Ala Glu Ala
   35 40 45
   Lys Lys Leu Asn Asp Ala Gln Ala Pro Lys
   50 55
<210> 902
   <211> 58
   <212> PRT
   <213> Artificial Sequence
<220>
   <223> Engineered polypeptide
<400> 902
   Val Asp Asn Lys Phe Asn Lys Glu Lys Arg His Ala Ser Trp Glu Ile
   1               5                   10                  15
   Trp Glu Leu Pro Asn Leu Asn Gln Trp Gln Lys Ala Ala Phe Ile Arg
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 903
   <211> 58
   <212> PRT
   <213> Artificial Sequence
<220>
   <223> Engineered polypeptide
<400> 903
   Val Asp Asn Lys Phe Asn Lys Glu Arg Lys Ser Ala Thr Trp Glu Ile
   1               5                   10                  15
   Trp Glu Leu Pro Asn Leu Asn Gln Leu Gln Lys Ala Ala Phe Ile Arg
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 904
   <211> 58
   <212> PRT
   <213> Artificial Sequence
<220>
   <223> Engineered polypeptide
<400> 904
   Val Asp Asn Lys Phe Asn Lys Glu Lys Lys Lys Ala Ala Val Glu Ile
   1 5 10 15
   Trp Glu Leu Pro Asn Leu Asn Gln Arg Gln Lys Ala Ala Phe Ile Gly
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 905
   <211> 58
   <212> PRT
   <213> Artificial Sequence
<220>
   <223> Engineered polypeptide
<400> 905
   Val Asp Asn Lys Phe Asn Lys Glu Lys Lys Leu Ala Ser Phe Glu Ile
   1 5 10 15
   Trp Glu Leu Pro Asn Leu Asn Gln Arg Gln Lys Ala Ala Phe Ile Arg
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 906
   <211> 58
   <212> PRT
   <213> Artificial Sequence
<220>
   <223> Engineered polypeptide
<400> 906
   Val Asp Asn Lys Phe Asn Lys Glu Arg Lys Val Ala Thr Val Glu Ile
   1               5                   10                  15
   Trp Glu Leu Pro Asn Leu Asn Arg Val Gln Ala Ala Ala Phe Ile Lys
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 907
   <211> 58
   <212> PRT
   <213> Artificial Sequence
<220>
   <223> Engineered polypeptide
<400> 907
   Val Asp Asn Lys Phe Asn Lys Glu Arg Lys Thr Ala Thr Val Glu Ile
   1               5                   10                  15
   Trp Glu Leu Pro Asn Leu Asn Arg Ile Gln Lys Ala Ala Phe Ile Lys
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 908
   <211> 58
   <212> PRT
   <213> Artificial Sequence
<220>
   <223> Engineered polypeptide
<400> 908
   Val Asp Asn Lys Phe Asn Lys Glu Arg Lys Ser Ala Thr Val Glu Ile
   1 5 10 15
   Trp Glu Leu Pro Asn Leu Asn Arg Val Gln Lys Ala Ala Phe Ile Ser
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 909
   <211> 58
   <212> PRT
   <213> Artificial Sequence
<220>
   <223> Engineered polypeptide
<400> 909
   Val Asp Asn Lys Phe Asn Lys Glu Arg Lys Val Ala Thr Val Glu Ile
   1 5 10 15
   Trp Glu Leu Pro Asn Leu Asn Arg Gly Gln Lys Ala Ala Phe Ile Ser
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 910
   <211> 58
   <212> PRT
   <213> Artificial Sequence
<220>
   <223> Engineered polypeptide
<400> 910
   Val Asp Asn Lys Phe Asn Lys Glu Arg Lys Gln Ala Thr Val Glu Ile
   1               5                   10                  15
   Trp Glu Leu Pro Asn Leu Asn Arg Arg Gln Arg Ala Ala Phe Ile Ser
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 911
   <211> 58
   <212> PRT
   <213> Artificial Sequence
<220>
   <223> Engineered polypeptide
<400> 911
   Val Asp Asn Lys Phe Asn Lys Glu Arg Lys Lys Ala Thr Val Glu Ile
   1               5                   10                  15
   Trp Glu Leu Pro Asn Leu Asn Arg Arg Gln Lys Gly Ala Phe Ile Ala
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 912
   <211> 58
   <212> PRT
   <213> Artificial Sequence
<220>
   <223> Engineered polypeptide
<400> 912
   Val Asp Asn Lys Phe Asn Lys Glu Arg Lys Val Ala Thr Val Glu Ile
   1               5                   10                  15
   Trp Glu Leu Pro Asn Leu Asn Arg Arg Gln Lys Gly Ala Phe Ile Ala
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 913
   <211> 58
   <212> PRT
   <213> Artificial Sequence
<220>
   <223> Engineered polypeptide
<400> 913
   Val Asp Asn Lys Phe Asn Lys Glu Arg Lys Leu Ala Thr Val Glu Ile
   1               5                   10                  15
   Trp Glu Leu Pro Asn Leu Asn Arg Tyr Gln Lys Gly Ala Phe Ile Ala
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 914
   <211> 58
   <212> PRT
   <213> Artificial Sequence
<220>
   <223> Engineered polypeptide
<400> 914
   Val Asp Asn Lys Phe Asn Lys Glu Arg Lys Ala Ala Thr Val Glu Ile
   1 5 10 15
   Trp Glu Leu Pro Asn Leu Asn Arg Leu Gln Lys Gly Ala Phe Ile Ala
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 915
   <211> 58
   <212> PRT
   <213> Artificial Sequence
<220>
   <223> Engineered polypeptide
<400> 915
   Val Asp Asn Lys Phe Asn Lys Glu Arg Lys Ile Ala Thr Val Glu Ile
   1 5 10 15
   Trp Glu Leu Pro Asn Leu Asn Arg Val Gln Lys Ser Ala Phe Ile Ala
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 916
   <211> 58
   <212> PRT
   <213> Artificial Sequence
<220>
   <223> Engineered polypeptide
<400> 916
   Val Asp Asn Lys Phe Asn Lys Glu Arg Lys Asn Ala Thr Val Glu Ile
   1 5 10 15
   Trp Glu Leu Pro Asn Leu Asn Arg Ala Gln Lys Ala Ala Phe Ile Ala
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 917
   <211> 58
   <212> PRT
   <213> Artificial Sequence
<220>
   <223> Engineered polypeptide
<400> 917
   Val Asp Asn Lys Phe Asn Lys Glu Arg Lys Asn Ala Thr Val Glu Ile
   1 5 10 15
   Trp Glu Leu Pro Asn Leu Asn Arg Ser Gln Lys Ala Ala Phe Ile Gly
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 918
   <211> 58
   <212> PRT
   <213> Artificial Sequence
<220>
   <223> Engineered polypeptide
<400> 918
   Val Asp Asn Lys Phe Asn Lys Glu Arg Lys Thr Ala Thr Val Glu Ile
   1               5                   10                  15
   Trp Glu Leu Pro Asn Leu Asn Arg Gly Gln Lys Ala Ala Phe Ile Ala
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 919
   <211> 58
   <212> PRT
   <213> Artificial Sequence
<220>
   <223> Engineered polypeptide
<400> 919
   Val Asp Asn Lys Phe Asn Lys Glu Arg Lys His Ala Ala Val Glu Ile
   1               5                   10                  15
   Trp Glu Leu Pro Asn Leu Asn Arg Ile Gln Arg Ala Ala Phe Ile Ala
               20                  25                  30
   Ser Leu Phe Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 920
   <211> 58
   <212> PRT
   <213> Artificial Sequence
<220>
   <223> Engineered polypeptide
<400> 920
   Val Asp Asn Lys Phe Asn Lys Glu Arg Lys Ile Ala Thr Val Glu Ile
   1 5 10 15
   Trp Glu Leu Pro Asn Leu Asn Arg Ile Gln Arg Ala Ala Phe Ile Ala
   20 25 30
   Ser Leu Phe Asp Asp Pro Ser Gln Ser Ala Asn Leu Leu Ala Glu Ala
   35 40 45
   Lys Lys Leu Asn Asp Ala Gln Ala Pro Lys
   50 55
<210> 921
   <211> 58
   <212> PRT
   <213> Artificial Sequence
<220>
   <223> Engineered polypeptide
<400> 921
   Val Asp Asn Lys Phe Asn Lys Glu Arg Lys Ile Ala Ala Val Glu Ile
   1 5 10 15
   Trp Glu Leu Pro Asn Leu Asn Arg Ile Gln Ala Ala Ala Phe Ile Ala
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 922
   <211> 58
   <212> PRT
   <213> Artificial Sequence
<220>
   <223> Engineered polypeptide
<400> 922
   Val Asp Asn Lys Phe Asn Lys Glu Arg Lys Ser Ala Thr Val Glu Ile
   1               5                   10                  15
   Trp Glu Leu Pro Asn Leu Asn Arg Thr Gln Arg Ala Ala Phe Ile Ala
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 923
   <211> 58
   <212> PRT
   <213> Artificial Sequence
<220>
   <223> Engineered polypeptide
<400> 923
   Val Asp Asn Lys Phe Asn Lys Glu Arg Lys Ser Ala Ala Val Glu Ile
   1               5                   10                  15
   Trp Glu Leu Pro Asn Leu Asn Arg Thr Gln Lys Ala Ala Phe Ile Ala
               20                  25                  30
   Ser Leu Trp Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 924
   <211> 58
   <212> PRT
   <213> Artificial Sequence
<220>
   <223> Engineered polypeptide
<400> 924
   Val Asp Asn Lys Phe Asn Lys Glu Lys Lys Ser Ala Thr Val Glu Ile
   1 5 10 15
   Trp Glu Leu Pro Asn Leu Asn Arg Ile Gln Lys Ala Ala Phe Ile Gly
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 925
   <211> 58
   <212> PRT
   <213> Artificial Sequence
<220>
   <223> Engineered polypeptide
<400> 925
   Val Asp Asn Lys Phe Asn Lys Glu Lys Lys Ser Ala Tyr Val Glu Ile
   1 5 10 15
   Trp Glu Leu Pro Asn Leu Asn Arg Tyr Gln Lys Ala Ala Phe Ile Lys
   20 25 30
   Ser Leu Phe Asp Asp Pro Ser Gln Ser Ala Asn Leu Leu Ala Glu Ala
   35 40 45
   Lys Lys Leu Asn Asp Ala Gln Ala Pro Lys
   50 55
<210> 926
   <211> 58
   <212> PRT
   <213> Artificial Sequence
<220>
   <223> Engineered polypeptide
<400> 926
   Val Asp Asn Lys Phe Asn Lys Glu Arg Lys Ile Ala Thr Tyr Glu Ile
   1 5 10 15
   Trp Glu Leu Pro Asn Leu Asn Gln Arg Gln Lys Ala Ala Phe Ile Ala
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 927
   <211> 58
   <212> PRT
   <213> Artificial Sequence
<220>
   <223> Engineered polypeptide
<400> 927
   Val Asp Asn Lys Phe Asn Lys Glu Arg Ser Val Ala Thr Trp Glu Ile
   1 5 10 15
   Trp Gln Leu Pro Asn Leu Asn Gln Arg Gln Lys Ala Ala Phe Ile Ala
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 928
   <211> 58
   <212> PRT
   <213> Artificial Sequence
<220>
   <223> Engineered polypeptide
<400> 928
   Val Asp Asn Lys Phe Asn Lys Glu Lys Arg Lys Ala Thr Gly Glu Ile
   1 5 10 15
   Trp Gln Leu Pro Asn Leu Asn Gln Arg Gln Lys Gly Ala Phe Ile Ala
   20 25 30
   Ser Leu Trp Asp Asp Pro Ser Gln Ser Ala Asn Leu Leu Ala Glu Ala
   35 40 45
   Lys Lys Leu Asn Asp Ala Gln Ala Pro Lys
   50 55
<210> 929
   <211> 58
   <212> PRT
   <213> Artificial Sequence
<220>
   <223> Engineered polypeptide
<400> 929
   Val Asp Asn Lys Phe Asn Lys Glu Arg Lys Glu Ala Arg Phe Glu Ile
   1 5 10 15
   Trp Glu Leu Pro Asn Leu Asn Gln Arg Gln Ala Ala Ala Phe Ile Ser
   20 25 30
   Ser Leu Phe Asp Asp Pro Ser Gln Ser Ala Asn Leu Leu Ala Glu Ala
   35 40 45
   Lys Lys Leu Asn Asp Ala Gln Ala Pro Lys
   50 55
<210> 930
   <211> 58
   <212> PRT
   <213> Artificial Sequence
<220>
   <223> Engineered polypeptide
<400> 930
   Val Asp Asn Lys Phe Asn Lys Glu Arg Lys Ala Ala Ser Trp Glu Ile
   1 5 10 15
   Trp Glu Leu Pro Asn Leu Asn Gln Arg Gln Lys Ala Ala Phe Ile Ser
   20 25 30
   Ser Leu Tyr Asp Asp Pro Ser Gln Ser Ala Asn Leu Leu Ala Glu Ala
   35 40 45
   Lys Lys Leu Asn Asp Ala Gln Ala Pro Lys
   50 55
<210> 931
   <211> 58
   <212> PRT
   <213> Artificial Sequence
<220>
   <223> Engineered polypeptide
<400> 931
   Val Asp Asn Lys Phe Asn Lys Glu Trp Lys His Ala Ala Gly Glu Ile
   1 5 10 15
   Trp Glu Leu Pro Asn Leu Asn Arg Ser Gln Lys Val Ala Phe Ile Ala
   20 25 30
   Ser Leu Glu Asp Asp Pro Ser Gln Ser Ala Asn Leu Leu Ala Glu Ala
   35 40 45
   Lys Lys Leu Asn Asp Ala Gln Ala Pro Lys
   50 55
<210> 932
   <211> 58
   <212> PRT
   <213> Artificial Sequence
<220>
   <223> Engineered polypeptide
<400> 932
   Val Asp Asn Lys Phe Asn Lys Glu Trp Lys Arg Ala Ala Gly Glu Ile
   1 5 10 15
   Trp Glu Leu Pro Asn Leu Asn Arg Ala Gln Lys Val Ala Phe Ile Ala
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 933
   <211> 58
   <212> PRT
   <213> Artificial Sequence
<220>
   <223> Engineered polypeptide
<400> 933
   Val Asp Asn Lys Phe Asn Lys Glu Trp Lys Ile Ala Ala Gly Glu Ile
   1 5 10 15
   Trp Glu Leu Pro Asn Leu Asn Gln Arg Gln Lys Val Ala Phe Ile Arg
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 934
   <211> 58
   <212> PRT
   <213> Artificial Sequence
<220>
   <223> Engineered polypeptide
<400> 934
   Val Asp Asn Lys Phe Asn Lys Glu Trp Lys Thr Ala Ala Tyr Glu Ile
   1 5 10 15
   Trp Glu Leu Pro Asn Leu Asn Thr Tyr Gln Lys Val Ala Phe Ile Arg
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 935
   <211> 58
   <212> PRT
   <213> Artificial Sequence
<220>
   <223> Engineered polypeptide
<400> 935
   Val Asp Asn Lys Phe Asn Lys Glu Trp Lys Val Ala Ala Gly Glu Ile
   1 5 10 15
   Trp Glu Leu Pro Asn Leu Asn Arg Thr Gln Lys Val Ala Phe Ile Arg
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 936
   <211> 58
   <212> PRT
   <213> Artificial Sequence
<220>
   <223> Engineered polypeptide
<400> 936
   Val Asp Asn Lys Phe Asn Lys Glu Trp Lys Val Ala Ala Gly Glu Ile
   1 5 10 15
   Trp Glu Leu Pro Asn Leu Asn Arg Trp Gln Lys Val Ala Phe Ile Arg
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 937
   <211> 58
   <212> PRT
   <213> Artificial Sequence
<220>
   <223> Engineered polypeptide
<400> 937
   Val Asp Asn Lys Phe Asn Lys Glu Trp Lys Gln Ala Ala Gly Glu Ile
   1 5 10 15
   Trp Glu Leu Pro Asn Leu Asn Arg Leu Gln Lys Val Ala Phe Ile Arg
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 938
   <211> 58
   <212> PRT
   <213> Artificial Sequence
<220>
   <223> Engineered polypeptide
<400> 938
   Val Asp Asn Lys Phe Asn Lys Glu Trp Lys Val Ala Ala Gly Glu Ile
   1 5 10 15
   Trp Glu Leu Pro Asn Leu Asn Val Trp Gln Lys Ala Ala Phe Ile Arg
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 939
   <211> 58
   <212> PRT
   <213> Artificial Sequence
<220>
   <223> Engineered polypeptide
<400> 939
   Val Asp Asn Lys Phe Asn Lys Glu Trp Lys Val Ala Ala Trp Glu Ile
   1 5 10 15
   Trp Glu Leu Pro Asn Leu Asn Arg Gly Gln Lys Val Ala Phe Ile Arg
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 940
   <211> 58
   <212> PRT
   <213> Artificial Sequence
<220>
   <223> Engineered polypeptide
<400> 940
   Val Asp Asn Lys Phe Asn Lys Glu Trp Lys Arg Ala Ala Phe Glu Ile
   1 5 10 15
   Trp Glu Leu Pro Asn Leu Asn Gln Ala Gln Lys Val Ala Phe Ile Thr
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 941
   <211> 58
   <212> PRT
   <213> Artificial Sequence
<220>
   <223> Engineered polypeptide
<400> 941
   Val Asp Asn Lys Phe Asn Lys Glu Trp Lys Lys Ala Ala Gly Glu Ile
   1 5 10 15
   Trp Leu Leu Pro Asn Leu Asn Arg Arg Gln Arg Gly Ala Phe Ile Ala
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 942
   <211> 58
   <212> PRT
   <213> Artificial Sequence
<220>
   <223> Engineered polypeptide
<400> 942
   Val Asp Asn Lys Phe Asn Lys Glu Trp Lys Lys Ala Ala Gly Glu Ile
   1               5                   10                  15
   Trp Glu Leu Pro Asn Leu Asn Ser Arg Gln Lys Gly Ala Phe Ile Ala
               20                  25                  30
   Ser Leu Ala Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 943
   <211> 58
   <212> PRT
   <213> Artificial Sequence
<220>
   <223> Engineered polypeptide
<400> 943
   Val Asp Asn Lys Phe Asn Lys Glu Trp Arg Gly Ala Ala Gly Glu Ile
   1               5                   10                  15
   Trp Glu Leu Pro Asn Leu Asn Arg Arg Gln Arg Ala Ala Phe Ile Ala
               20                  25                  30
   Ser Leu Ala Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 944
   <211> 58
   <212> PRT
   <213> Artificial Sequence
<220>
   <223> Engineered polypeptide
<400> 944
   Val Asp Asn Lys Phe Asn Lys Glu Trp Lys Val Ala Ala Gly Glu Ile
   1               5                   10                  15
   Trp Gln Leu Pro Asn Leu Asn Arg Arg Gln Arg Ala Ala Phe Ile Ala
               20                  25                  30
   Ser Leu Ala Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 945
   <211> 58
   <212> PRT
   <213> Artificial Sequence
<220>
   <223> Engineered polypeptide
<400> 945
   Val Asp Asn Lys Phe Asn Lys Glu Trp Lys Ile Ala Ala Val Glu Ile
   1               5                   10                  15
   Trp Glu Leu Pro Asn Leu Asn Thr Arg Gln Lys Gly Ala Phe Ile Ala
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 946
   <211> 58
   <212> PRT
   <213> Artificial Sequence
<220>
   <223> Engineered polypeptide
<400> 946
   Val Asp Asn Lys Phe Asn Lys Glu Trp Lys Ile Ala Ala Phe Glu Ile
   1 5 10 15
   Trp Glu Leu Pro Asn Leu Asn Val Arg Gln Arg Ser Ala Phe Ile Ser
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 947
   <211> 58
   <212> PRT
   <213> Artificial Sequence
<220>
   <223> Engineered polypeptide
<400> 947
   Val Asp Asn Lys Phe Asn Lys Glu Trp Lys Thr Ala Ala Tyr Glu Ile
   1 5 10 15
   Trp Glu Leu Pro Asn Leu Asn Gln Arg Gln Arg Gly Ala Phe Ile Ala
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 948
   <211> 58
   <212> PRT
   <213> Artificial Sequence
<220>
   <223> Engineered polypeptide
<400> 948
   Val Asp Asn Lys Phe Asn Lys Glu Trp Lys Thr Ala Ala Gly Glu Ile
   1               5                   10                  15
   Trp Glu Leu Pro Asn Leu Asn Ser Arg Gln Lys Ala Ala Phe Ile Gly
               20                  25                  30
   Ser Leu Ala Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 949
   <211> 58
   <212> PRT
   <213> Artificial Sequence
<220>
   <223> Engineered polypeptide
<400> 949
   Val Asp Asn Lys Phe Asn Lys Glu Trp Lys Thr Ala Ala Gly Glu Ile
   1               5                   10                  15
   Trp Glu Leu Pro Asn Leu Asn Val Gly Gln Lys Ala Ala Phe Ile Gly
               20                  25                  30
   Ser Leu Ala Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 950
   <211> 58
   <212> PRT
   <213> Artificial Sequence
<220>
   <223> Engineered polypeptide
<400> 950
   Val Asp Asn Lys Phe Asn Lys Glu Trp Lys His Ala Ala Gly Glu Ile
   1 5 10 15
   Trp Glu Leu Pro Asn Leu Asn Arg Thr Gln Lys Ala Ala Phe Ile Arg
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 951
   <211> 58
   <212> PRT
   <213> Artificial Sequence
<220>
   <223> Engineered polypeptide
<400> 951
   Val Asp Asn Lys Phe Asn Lys Glu Trp Lys Arg Ala Ala Gly Glu Ile
   1 5 10 15
   Trp Glu Leu Pro Asn Leu Asn Arg Leu Gln Lys Ala Ala Phe Ile Arg
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 952
   <211> 58
   <212> PRT
   <213> Artificial Sequence
<220>
   <223> Engineered polypeptide
<400> 952
   Val Asp Asn Lys Phe Asn Lys Glu Trp Lys Gln Ala Ala Gly Glu Ile
   1 5 10 15
   Trp Glu Leu Pro Asn Leu Asn Arg Gly Gln Lys Ala Ala Phe Ile Ser
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 953
   <211> 58
   <212> PRT
   <213> Artificial Sequence
<220>
   <223> Engineered polypeptide
<400> 953
   Val Asp Asn Lys Phe Asn Lys Glu Trp Lys Gln Ala Ala Gly Glu Ile
   1 5 10 15
   Trp Glu Leu Pro Asn Leu Asn Arg Gly Gln Lys Ala Ala Phe Ile Thr
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 954
   <211> 58
   <212> PRT
   <213> Artificial Sequence
<220>
   <223> Engineered polypeptide
<400> 954
   Val Asp Asn Lys Phe Asn Lys Glu Trp Lys Glu Ala Ala Gly Glu Ile
   1 5 10 15
   Trp Glu Leu Pro Asn Leu Asn Arg Gly Gln Lys Ala Ala Phe Ile Lys
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 955
   <211> 58
   <212> PRT
   <213> Artificial Sequence
<220>
   <223> Engineered polypeptide
<400> 955
   Val Asp Asn Lys Phe Asn Lys Glu Trp Lys Thr Ala Ala Gly Glu Ile
   1 5 10 15
   Trp Glu Leu Pro Asn Leu Asn Arg Arg Gln Lys Ala Ala Phe Ile Ala
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 956
   <211> 58
   <212> PRT
   <213> Artificial Sequence
<220>
   <223> Engineered polypeptide
<400> 956
   Val Asp Asn Lys Phe Asn Lys Glu Trp Lys Arg Ala Ala Gly Glu Ile
   1 5 10 15
   Trp Glu Leu Pro Asn Leu Asn Arg Ala Gln Lys Ala Ala Phe Ile Ser
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 957
   <211> 58
   <212> PRT
   <213> Artificial Sequence
<220>
   <223> Engineered polypeptide
<400> 957
   Val Asp Asn Lys Phe Asn Lys Glu Trp Lys His Ala Ala Phe Glu Ile
   1 5 10 15
   Trp Glu Leu Pro Asn Leu Asn Val Arg Gln Lys Ala Ala Phe Ile Gly
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 958
   <211> 58
   <212> PRT
   <213> Artificial Sequence
<220>
   <223> Engineered polypeptide
<400> 958
   Val Asp Asn Lys Phe Asn Lys Glu Trp Lys Asn Ala Ala Phe Glu Ile
   1 5 10 15
   Trp Glu Leu Pro Asn Leu Asn Val Ser Gln Lys Ala Ala Phe Ile Arg
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 959
   <211> 58
   <212> PRT
   <213> Artificial Sequence
<220>
   <223> Engineered polypeptide
<400> 959
   Val Asp Asn Lys Phe Asn Lys Glu Trp Lys Ala Ala Ala Tyr Glu Ile
   1 5 10 15
   Trp Glu Leu Pro Asn Leu Asn Val Trp Gln Lys Ala Ala Phe Ile Ser
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 960
   <211> 58
   <212> PRT
   <213> Artificial Sequence
<220>
   <223> Engineered polypeptide
<400> 960
   Val Asp Asn Lys Phe Asn Lys Glu Trp Lys Val Ala Ala Gly Glu Ile
   1               5                   10                  15
   Trp Glu Leu Pro Asn Leu Asn Arg Trp Gln Lys Gly Ala Phe Ile Thr
               20                  25                  30
   Ser Leu Tyr Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 961
   <211> 58
   <212> PRT
   <213> Artificial Sequence
<220>
   <223> Engineered polypeptide
<400> 961
   Val Asp Asn Lys Phe Asn Lys Glu Trp Lys Val Ala Ala Gly Glu Ile
   1               5                   10                  15
   Trp Glu Leu Pro Asn Leu Asn Arg Trp Gln Lys Ala Ala Phe Ile Ala
               20                  25                  30
   Ser Leu Tyr Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 962
   <211> 58
   <212> PRT
   <213> Artificial Sequence
<220>
   <223> Engineered polypeptide
<400> 962
   Val Asp Asn Lys Phe Asn Lys Glu Trp Lys Val Ala Ala Gly Glu Ile
   1 5 10 15
   Trp Glu Leu Pro Asn Leu Asn Arg Leu Gln Arg Ala Ala Phe Ile Lys
   20 25 30
   Ser Leu Tyr Asp Asp Pro Ser Gln Ser Ala Asn Leu Leu Ala Glu Ala
   35 40 45
   Lys Lys Leu Asn Asp Ala Gln Ala Pro Lys
   50 55
<210> 963
   <211> 58
   <212> PRT
   <213> Artificial Sequence
<220>
   <223> Engineered polypeptide
<400> 963
   Val Asp Asn Lys Phe Asn Lys Glu Trp Lys Val Ala Ala Tyr Glu Ile
   1 5 10 15
   Trp Glu Leu Pro Asn Leu Asn Arg Leu Gln Lys Ala Ala Phe Ile Ala
   20 25 30
   Ser Leu Tyr Asp Asp Pro Ser Gln Ser Ala Asn Leu Leu Ala Glu Ala
   35 40 45
   Lys Lys Leu Asn Asp Ala Gln Ala Pro Lys
   50 55
<210> 964
   <211> 58
   <212> PRT
   <213> Artificial Sequence
<220>
   <223> Engineered polypeptide
<400> 964
   Val Asp Asn Lys Phe Asn Lys Glu Trp Lys Val Ala Ala Phe Glu Ile
   1               5                   10                  15
   Trp Glu Leu Pro Asn Leu Asn Arg Thr Gln Arg Ala Ala Phe Ile Ile
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 965
   <211> 58
   <212> PRT
   <213> Artificial Sequence
<220>
   <223> Engineered polypeptide
<400> 965
   Val Asp Asn Lys Phe Asn Lys Glu Trp Lys Thr Ala Ala Val Glu Ile
   1               5                   10                  15
   Trp Glu Leu Pro Asn Leu Asn Arg Leu Gln Lys Ala Ala Phe Ile Thr
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 966
   <211> 58
   <212> PRT
   <213> Artificial Sequence
<220>
   <223> Engineered polypeptide
<400> 966
   Val Asp Asn Lys Phe Asn Lys Glu Trp Lys Thr Ala Ala Val Glu Ile
   1 5 10 15
   Trp Glu Leu Pro Asn Leu Asn Arg Leu Gln Lys Ala Ala Phe Ile Gly
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 967
   <211> 58
   <212> PRT
   <213> Artificial Sequence
<220>
   <223> Engineered polypeptide
<400> 967
   Val Asp Asn Lys Phe Asn Lys Glu Trp Lys Thr Ala Ala Phe Glu Ile
   1 5 10 15
   Trp Glu Leu Pro Asn Leu Asn Arg Gly Gln Lys Ala Ala Phe Ile Arg
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 968
   <211> 58
   <212> PRT
   <213> Artificial Sequence
<220>
   <223> Engineered polypeptide
<400> 968
   Val Asp Asn Lys Phe Asn Lys Glu Trp Lys Arg Ala Ala Phe Glu Ile
   1               5                   10                  15
   Trp Glu Leu Pro Asn Leu Asn Arg Leu Gln Lys Ser Ala Phe Ile Ala
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 969
   <211> 58
   <212> PRT
   <213> Artificial Sequence
<220>
   <223> Engineered polypeptide
<400> 969
   Val Asp Asn Lys Phe Asn Lys Glu Trp Lys Gln Ala Ala Phe Glu Ile
   1               5                   10                  15
   Trp Glu Leu Pro Asn Leu Asn Arg Tyr Gln Ala Ala Ala Phe Ile Gly
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 970
   <211> 58
   <212> PRT
   <213> Artificial Sequence
<220>
   <223> Engineered polypeptide
<400> 970
   Val Asp Asn Lys Phe Asn Lys Glu Trp Lys His Ala Ala Phe Glu Ile
   1 5 10 15
   Trp Glu Leu Pro Asn Leu Asn Arg Arg Gln Ala Gly Ala Phe Ile Ala
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 971
   <211> 58
   <212> PRT
   <213> Artificial Sequence
<220>
   <223> Engineered polypeptide
<400> 971
   Val Asp Asn Lys Phe Asn Lys Glu Trp Lys Gln Ala Ser Gly Glu Ile
   1 5 10 15
   Trp Glu Leu Pro Asn Leu Asn Val Arg Gln Lys Ala Ala Phe Ile Ser
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 972
   <211> 58
   <212> PRT
   <213> Artificial Sequence
<220>
   <223> Engineered polypeptide
<400> 972
   Val Asp Asn Lys Phe Asn Lys Glu Trp Lys Gln Ala Ser Phe Glu Ile
   1               5                   10                  15
   Trp Glu Leu Pro Asn Leu Asn Val Arg Gln Lys Ala Ala Phe Ile Ser
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 973
   <211> 58
   <212> PRT
   <213> Artificial Sequence
<220>
   <223> Engineered polypeptide
<400> 973
   Val Asp Asn Lys Phe Asn Lys Glu Trp Lys Leu Ala Ala Gly Glu Ile
   1               5                   10                  15
   Trp Glu Leu Pro Asn Leu Asn Val Arg Gln Lys Ala Ala Phe Ile Ala
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 974
   <211> 58
   <212> PRT
   <213> Artificial Sequence
<220>
   <223> Engineered polypeptide
<400> 974
   Val Asp Asn Lys Phe Asn Lys Glu Trp Lys Ile Ala Ala Gly Glu Ile
   1 5 10 15
   Trp Glu Leu Pro Asn Leu Asn Val Arg Gln Lys Ala Ala Phe Ile Ala
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 975
   <211> 58
   <212> PRT
   <213> Artificial Sequence
<220>
   <223> Engineered polypeptide
<400> 975
   Val Asp Asn Lys Phe Asn Lys Glu Trp Lys His Ala Ala Gly Glu Ile
   1 5 10 15
   Trp Gln Leu Pro Asn Leu Asn Val Thr Gln Lys Ala Ala Phe Ile Ala
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 976
   <211> 58
   <212> PRT
   <213> Artificial Sequence
<220>
   <223> Engineered polypeptide
<400> 976
   Val Asp Asn Lys Phe Asn Lys Glu Trp Lys Ile Ala Ala Gly Glu Ile
   1               5                   10                  15
   Trp Glu Leu Pro Asn Leu Asn Arg Arg Gln Ala Ala Ala Phe Ile Val
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 977
   <211> 58
   <212> PRT
   <213> Artificial Sequence
<220>
   <223> Engineered polypeptide
<400> 977
   Val Asp Asn Lys Phe Asn Lys Glu Trp Lys Ser Ala Ala Gly Glu Ile
   1               5                   10                  15
   Trp Glu Leu Pro Asn Leu Asn Arg Ala Gln Lys Ala Ala Phe Ile Ile
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 978
   <211> 58
   <212> PRT
   <213> Artificial Sequence
<220>
   <223> Engineered polypeptide
<400> 978
   Val Asp Asn Lys Phe Asn Lys Glu Trp Lys His Ala Ala Val Glu Ile
   1               5                   10                  15
   Trp Gln Leu Pro Asn Leu Asn Arg Phe Gln Lys Ala Ala Phe Ile Ala
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 979
   <211> 58
   <212> PRT
   <213> Artificial Sequence
<220>
   <223> Engineered polypeptide
<400> 979
   Val Asp Asn Lys Phe Asn Lys Glu Trp Lys Gln Ala Ala Trp Glu Ile
   1               5                   10                  15
   Trp Gln Leu Pro Asn Leu Asn Thr Gly Gln Lys Ala Ala Phe Ile Arg
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 980
   <211> 58
   <212> PRT
   <213> Artificial Sequence
<220>
   <223> Engineered polypeptide
<400> 980
   Val Asp Asn Lys Phe Asn Lys Glu Trp Lys Gln Ala Ser Trp Glu Ile
   1 5 10 15
   Trp Glu Leu Pro Asn Leu Asn Arg Gly Gln Lys Ala Ala Phe Ile Ala
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 981
   <211> 58
   <212> PRT
   <213> Artificial Sequence
<220>
   <223> Engineered polypeptide
<400> 981
   Val Asp Asn Lys Phe Asn Lys Glu Trp Lys Thr Ala Ser Tyr Glu Ile
   1 5 10 15
   Trp Gln Leu Pro Asn Leu Asn Arg Arg Gln Ala Ala Ala Phe Ile Ala
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 982
   <211> 58
   <212> PRT
   <213> Artificial Sequence
<220>
   <223> Engineered polypeptide
<400> 982
   Val Asp Asn Lys Phe Asn Lys Glu Trp Lys Asn Ala Ser Phe Glu Ile
   1 5 10 15
   Trp Leu Leu Pro Asn Leu Asn Arg Ile Gln Lys Ala Ala Phe Ile Ala
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 983
   <211> 58
   <212> PRT
   <213> Artificial Sequence
<220>
   <223> Engineered polypeptide
<400> 983
   Val Asp Asn Lys Phe Asn Lys Glu Trp Lys Asn Ala Ala Phe Glu Ile
   1 5 10 15
   Trp Leu Leu Pro Asn Leu Asn Gln Arg Gln Lys Ala Ala Phe Ile Ala
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 984
   <211> 58
   <212> PRT
   <213> Artificial Sequence
<220>
   <223> Engineered polypeptide
<400> 984
   Val Asp Asn Lys Phe Asn Lys Glu Trp Arg Gly Ala Ala Phe Glu Ile
   1 5 10 15
   Trp Gln Leu Pro Asn Leu Asn Gln Arg Gln Lys Ala Ala Phe Ile Ala
   20 25 30
   Ser Leu Tyr Asp Asp Pro Ser Gln Ser Ala Asn Leu Leu Ala Glu Ala
   35 40 45
   Lys Lys Leu Asn Asp Ala Gln Ala Pro Lys
   50 55
<210> 985
   <211> 58
   <212> PRT
   <213> Artificial Sequence
<220>
   <223> Engineered polypeptide
<400> 985
   Val Asp Asn Lys Phe Asn Lys Glu Trp Lys Val Ala Ser Trp Glu Ile
   1 5 10 15
   Trp Gln Leu Pro Asn Leu Asn Arg Gln Gln Lys Ala Ala Phe Ile Gly
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 986
   <211> 58
   <212> PRT
   <213> Artificial Sequence
<220>
   <223> Engineered polypeptide
<400> 986
   Val Asp Asn Lys Phe Asn Lys Glu Trp Lys Leu Ala Ser Trp Glu Ile
   1               5                   10                  15
   Trp Gln Leu Pro Asn Leu Asn Arg Trp Gln Lys Ala Ala Phe Ile Gly
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 987
   <211> 58
   <212> PRT
   <213> Artificial Sequence
<220>
   <223> Engineered polypeptide
<400> 987
   Val Asp Asn Lys Phe Asn Lys Glu Trp Lys Arg Ala Ser Val Glu Ile
   1               5                   10                  15
   Trp Glu Leu Pro Asn Leu Asn Arg Gln Gln Lys Ala Ala Phe Ile Gly
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 988
   <211> 58
   <212> PRT
   <213> Artificial Sequence
<220>
   <223> Engineered polypeptide
<400> 988
   Val Asp Asn Lys Phe Asn Lys Glu Trp Lys Arg Ala Ser Phe Glu Ile
   1 5 10 15
   Trp Gln Leu Pro Asn Leu Asn Arg Leu Gln Lys Ala Ala Phe Ile Ala
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 989
   <211> 58
   <212> PRT
   <213> Artificial Sequence
<220>
   <223> Engineered polypeptide
<400> 989
   Val Asp Asn Lys Phe Asn Lys Glu Trp Lys Gly Ala Ala Gly Glu Ile
   1 5 10 15
   Trp Gln Leu Pro Asn Leu Asn Arg Arg Gln Lys Ala Ala Phe Ile Ser
   20 25 30
   Ser Leu Tyr Asp Asp Pro Ser Gln Ser Ala Asn Leu Leu Ala Glu Ala
   35 40 45
   Lys Lys Leu Asn Asp Ala Gln Ala Pro Lys
   50 55
<210> 990
   <211> 58
   <212> PRT
   <213> Artificial Sequence
<220>
   <223> Engineered polypeptide
<400> 990
   Val Asp Asn Lys Phe Asn Lys Glu Trp Lys Val Ala Ala Phe Glu Ile
   1 5 10 15
   Trp Gln Leu Pro Asn Leu Asn Arg Ala Gln Lys Ala Ala Phe Ile Ser
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 991
   <211> 58
   <212> PRT
   <213> Artificial Sequence
<220>
   <223> Engineered polypeptide
<400> 991
   Val Asp Asn Lys Phe Asn Lys Glu Trp Lys His Ala Ala Gly Glu Ile
   1 5 10 15
   Trp Gln Leu Pro Asn Leu Asn Arg Ala Gln Lys Ala Ala Phe Ile Ser
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 992
   <211> 58
   <212> PRT
   <213> Artificial Sequence
<220>
   <223> Engineered polypeptide
<400> 992
   Val Asp Asn Lys Phe Asn Lys Glu Trp Lys Lys Ala Ala Tyr Glu Ile
   1 5 10 15
   Trp Gln Leu Pro Asn Leu Asn Arg Ala Gln Lys Ala Ala Phe Ile Ser
   20 25 30
   Ser Leu Tyr Asp Asp Pro Ser Gln Ser Ala Asn Leu Leu Ala Glu Ala
   35 40 45
   Lys Lys Leu Asn Asp Ala Gln Ala Pro Lys
   50 55
<210> 993
   <211> 58
   <212> PRT
   <213> Artificial Sequence
<220>
   <223> Engineered polypeptide
<400> 993
   Val Asp Asn Lys Phe Asn Lys Glu Trp Lys Leu Ala Ala Tyr Glu Ile
   1 5 10 15
   Trp Gln Leu Pro Asn Leu Asn Arg His Gln Lys Ala Ala Phe Ile Ser
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 994
   <211> 58
   <212> PRT
   <213> Artificial Sequence
<220>
   <223> Engineered polypeptide
<400> 994
   Val Asp Asn Lys Phe Asn Lys Glu Trp Lys Arg Ala Ala Phe Glu Ile
   1 5 10 15
   Trp Gln Leu Pro Asn Leu Asn Arg Ser Gln Lys Ala Ala Phe Ile Ser
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 995
   <211> 58
   <212> PRT
   <213> Artificial Sequence
<220>
   <223> Engineered polypeptide
<400> 995
   Val Asp Asn Lys Phe Asn Lys Glu Trp Lys Thr Ala Ala Phe Glu Ile
   1 5 10 15
   Trp Gln Leu Pro Asn Leu Asn Arg Ser Gln Lys Ala Ala Phe Ile Ala
   20 25 30
   Ser Leu Ala Asp Asp Pro Ser Gln Ser Ala Asn Leu Leu Ala Glu Ala
   35 40 45
   Lys Lys Leu Asn Asp Ala Gln Ala Pro Lys
   50 55
<210> 996
   <211> 58
   <212> PRT
   <213> Artificial Sequence
<220>
   <223> Engineered polypeptide
<400> 996
   Val Asp Asn Lys Phe Asn Lys Glu Trp Lys Ile Ala Ala Phe Glu Ile
   1               5                   10                  15
   Trp Gln Leu Pro Asn Leu Asn Arg Ile Gln Lys Ala Ala Phe Ile Gly
               20                  25                  30
   Ser Leu Ala Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 997
   <211> 58
   <212> PRT
   <213> Artificial Sequence
<220>
   <223> Engineered polypeptide
<400> 997
   Val Asp Asn Lys Phe Asn Lys Glu Trp Lys Gln Ala Ala Phe Glu Ile
   1               5                   10                  15
   Trp Gln Leu Pro Asn Leu Asn Arg Ile Gln Lys Ala Ala Phe Ile Thr
               20                  25                  30
   Ser Leu Ser Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 998
   <211> 58
   <212> PRT
   <213> Artificial Sequence
<220>
   <223> Engineered polypeptide
<400> 998
   Val Asp Asn Lys Phe Asn Lys Glu Trp Lys Gln Ala Ala Phe Glu Ile
   1 5 10 15
   Trp Gln Leu Pro Asn Leu Asn Arg Val Gln Lys Ala Ala Phe Ile Val
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 999
   <211> 58
   <212> PRT
   <213> Artificial Sequence
<220>
   <223> Engineered polypeptide
<400> 999
   Val Asp Asn Lys Phe Asn Lys Glu Trp Lys Val Ala Ala Gly Glu Ile
   1 5 10 15
   Trp Gln Leu Pro Asn Leu Asn Arg Ile Gln Lys Ala Ala Phe Ile Gly
   20 25 30
   Ser Leu Ser Asp Asp Pro Ser Gln Ser Ala Asn Leu Leu Ala Glu Ala
   35 40 45
   Lys Lys Leu Asn Asp Ala Gln Ala Pro Lys
   50 55
<210> 1000
   <211> 58
   <212> PRT
   <213> Artificial Sequence
<220>
   <223> Engineered polypeptide
<400> 1000
   Val Asp Asn Lys Phe Asn Lys Glu Trp Lys Ile Ala Ala Phe Glu Ile
   1               5                   10                  15
   Trp Gln Leu Pro Asn Leu Asn Arg Trp Gln Lys Ala Ala Phe Ile Ser
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 1001
   <211> 58
   <212> PRT
   <213> Artificial Sequence
<220>
   <223> Engineered polypeptide
<400> 1001
   Val Asp Asn Lys Phe Asn Lys Glu Trp Lys Glu Ala Ala Gly Glu Ile
   1               5                   10                  15
   Trp Gln Leu Pro Asn Leu Asn Arg Tyr Gln Lys Ala Ala Phe Ile Ala
               20                  25                  30
   Ser Leu Gln Asp Asp Pro Ser Gln Ser Ala Asn Leu Leu Ala Glu Ala
           35                  40                  45
   Lys Lys Leu Asn Asp Ala Gln Ala Pro Lys
       50                  55
<210> 1002
   <211> 58
   <212> PRT
   <213> Artificial Sequence
<220>
   <223> Engineered polypeptide
<400> 1002
   Val Asp Asn Lys Phe Asn Lys Glu Trp Lys Thr Ala Ser Phe Glu Ile
   1 5 10 15
   Trp Gln Leu Pro Asn Leu Asn Arg Tyr Gln Lys Ala Ala Phe Ile Ser
   20 25 30
   Ser Leu Gln Asp Asp Pro Ser Gln Ser Ala Asn Leu Leu Ala Glu Ala
   35 40 45
   Lys Lys Leu Asn Asp Ala Gln Ala Pro Lys
   50 55
<210> 1003
   <211> 51
   <212> PRT
   <213> Artificial Sequence
<220>
   <223> Engineered polypeptide
<400> 1003
   Tyr Ala Lys Glu Met Arg Asn Ala Tyr Trp Glu Ile Ala Leu Leu Pro
   1 5 10 15
   Asn Leu Thr Asn Gln Gln Lys Arg Ala Phe Ile Arg Lys Leu Tyr Asp
   20 25 30
   Asp Pro Ser Gln Ser Ser Glu Leu Leu Ser Glu Ala Lys Lys Leu Asn
   35 40 45
   Asp Ser Gln
   50
<210> 1004
   <211> 51
   <212> PRT
   <213> Artificial Sequence
<220>
   <223> Engineered polypeptide
<400> 1004
   Tyr Ala Lys Glu Met Trp Ile Ala Trp Glu Glu Ile Arg Asn Leu Pro
   1               5                   10                  15
   Asn Leu Asn Gly Trp Gln Met Thr Ala Phe Ile Ala Lys Leu Leu Asp
               20                  25                  30
   Asp Pro Ser Gln Ser Ser Glu Leu Leu Ser Glu Ala Lys Lys Leu Asn
           35                  40                  45
   Asp Ser Gln
       50
<210> 1005
   <211> 46
   <212> PRT
   <213> Artificial Sequence
<220>
   <223> Engineered polypeptide
<400> 1005
   Leu Ala Glu Ala Lys Val Leu Ala Asn Arg Glu Leu Asp Lys Tyr Gly
   1               5                   10                  15
   Val Ser Asp Phe Tyr Lys Arg Leu Ile Asn Lys Ala Lys Thr Val Glu
               20                  25                  30
   Gly Val Glu Ala Leu Lys Leu His Ile Leu Ala Ala Leu Pro
           35                  40                  45
<210> 1006
   <211> 58
   <212> PRT
   <213> Artificial Sequence
<220>
   <223> Engineered polypeptide
<400> 1006
<210> 1007
   <211> 643
   <212> PRT
   <213> Homo sapiens
<400> 1007
   Ser Glu Val Gly Asn Ser Gln Ala Val Cys Pro Gly Thr Leu Asn Gly
   1               5                   10                  15
   Leu Ser Val Thr Gly Asp Ala Glu Asn Gln Tyr Gln Thr Leu Tyr Lys
               20                  25                  30
   Leu Tyr Glu Arg Cys Glu Val Val Met Gly Asn Leu Glu Ile Val Leu
           35                  40                  45
   Thr Gly His Asn Ala Asp Leu Ser Phe Leu Gln Trp Ile Arg Glu Val
       50                  55                  60
   Thr Gly Tyr Val Leu Val Ala Met Asn Glu Phe Ser Thr Leu Pro Leu
   65                  70                  75                  80
   Pro Asn Leu Arg Val Val Arg Gly Thr Gln Val Tyr Asp Gly Lys Phe
                   85                  90                  95
   Ala Ile Phe Val Met Leu Asn Tyr Asn Thr Asn Ser Ser His Ala Leu
               100                 105                 110
   Arg Gln Leu Arg Leu Thr Gln Leu Thr Glu Ile Leu Ser Gly Gly Val
           115                 120                 125
   Tyr Ile Glu Lys Asn Asp Lys Leu Cys His Met Asp Thr Ile Asp Trp
       130                 135                 140
   Arg Asp Ile Val Arg Asp Arg Asp Ala Glu Ile Val Val Lys Asp Asn
   145                 150                 155                 160
   Gly Arg Ser Cys Pro Pro Cys His Glu Val Cys Lys Gly Arg Cys Trp
                   165                 170                 175
   Gly Pro Gly Ser Glu Asp Cys Gln Thr Leu Thr Lys Thr Ile Cys Ala
               180                 185                 190
   Pro Gln Cys Asn Gly His Cys Phe Gly Pro Asn Pro Asn Gln Cys Cys
           195                 200                 205
   His Asp Glu Cys Ala Gly Gly Cys Ser Gly Pro Gln Asp Thr Asp Cys
       210                 215                 220
   Phe Ala Cys Arg His Phe Asn Asp Ser Gly Ala Cys Val Pro Arg Cys
   225                 230                 235                 240
   Pro Gln Pro Leu Val Tyr Asn Lys Leu Thr Phe Gln Leu Glu Pro Asn
                   245                 250                 255
   Pro His Thr Lys Tyr Gln Tyr Gly Gly Val Cys Val Ala Ser Cys Pro
               260                 265                 270
   His Asn Phe Val Val Asp Gln Thr Ser Cys Val Arg Ala Cys Pro Pro
           275                 280                 285
   Asp Lys Met Glu Val Asp Lys Asn Gly Leu Lys Met Cys Glu Pro Cys
       290                 295                 300
   Gly Gly Leu Cys Pro Lys Ala Cys Glu Gly Thr Gly Ser Gly Ser Arg
   305                 310                 315                 320
   Phe Gln Thr Val Asp Ser Ser Asn Ile Asp Gly Phe Val Asn Cys Thr
                   325                 330                 335
   Lys Ile Leu Gly Asn Leu Asp Phe Leu Ile Thr Gly Leu Asn Gly Asp
               340                 345                 350
   Pro Trp His Lys Ile Pro Ala Leu Asp Pro Glu Lys Leu Asn Val Phe
           355                 360                 365
   Arg Thr Val Arg Glu Ile Thr Gly Tyr Leu Asn Ile Gln Ser Trp Pro
       370                 375                 380
   Pro His Met His Asn Phe Ser Val Phe Ser Asn Leu Thr Thr Ile Gly
   385                 390                 395                 400
   Gly Arg Ser Leu Tyr Asn Arg Gly Phe Ser Leu Leu Ile Met Lys Asn
                   405                 410                 415
   Leu Asn Val Thr Ser Leu Gly Phe Arg Ser Leu Lys Glu Ile Ser Ala
               420                 425                 430
   Gly Arg Ile Tyr Ile Ser Ala Asn Arg Gln Leu Cys Tyr His His Ser
           435                 440                 445
   Leu Asn Trp Thr Lys Val Leu Arg Gly Pro Thr Glu Glu Arg Leu Asp
       450                 455                 460
   Ile Lys His Asn Arg Pro Arg Arg Asp Cys Val Ala Glu Gly Lys Val
   465                 470                 475                 480
   Cys Asp Pro Leu Cys Ser Ser Gly Gly Cys Trp Gly Pro Gly Pro Gly
                   485                 490                 495
   Gln Cys Leu Ser Cys Arg Asn Tyr Ser Arg Gly Gly Val Cys Val Thr
               500                 505                 510
   His Cys Asn Phe Leu Asn Gly Glu Pro Arg Glu Phe Ala His Glu Ala
           515                 520                 525
   Glu Cys Phe Ser Cys His Pro Glu Cys Gln Pro Met Gly Gly Thr Ala
       530                 535                 540
   Thr Cys Asn Gly Ser Gly Ser Asp Thr Cys Ala Gln Cys Ala His Phe
   545                 550                 555                 560
   Arg Asp Gly Pro His Cys Val Ser Ser Cys Pro His Gly Val Leu Gly
                   565                 570                 575
   Ala Lys Gly Pro Ile Tyr Lys Tyr Pro Asp Val Gln Asn Glu Cys Arg
               580                 585                 590
   Pro Cys His Glu Asn Cys Thr Gln Gly Cys Lys Gly Pro Glu Leu Gln
           595                 600                 605
   Asp Cys Leu Gly Gln Thr Leu Val Leu Ile Gly Lys Thr His Leu Thr
       610                 615                 620
   Met Ala Leu Thr Val Ile Ala Gly Leu Val Val Ile Phe Met Met Leu
   625                 630                 635                 640
   Gly Gly Thr
<210> 1008
   <211> 631
   <212> PRT
   <213> Homo sapiens
<400> 1008
   Ser Thr Gln Val Cys Thr Gly Thr Asp Met Lys Leu Arg Leu Pro Ala
   1               5                   10                  15
   Ser Pro Glu Thr His Leu Asp Met Leu Arg His Leu Tyr Gln Gly Cys
               20                  25                  30
   Gln Val Val Gln Gly Asn Leu Glu Leu Thr Tyr Leu Pro Thr Asn Ala
           35                  40                  45
   Ser Leu Ser Phe Leu Gln Asp Ile Gln Glu Val Gln Gly Tyr Val Leu
       50                  55                  60
   Ile Ala His Asn Gln Val Arg Gln Val Pro Leu Gln Arg Leu Arg Ile
   65                  70                  75                  80
   Val Arg Gly Thr Gln Leu Phe Glu Asp Asn Tyr Ala Leu Ala Val Leu
                   85                  90                  95
   Asp Asn Gly Asp Pro Leu Asn Asn Thr Thr Pro Val Thr Gly Ala Ser
               100                 105                 110
   Pro Gly Gly Leu Arg Glu Leu Gln Leu Arg Ser Leu Thr Glu Ile Leu
           115                 120                 125
   Lys Gly Gly Val Leu Ile Gln Arg Asn Pro Gln Leu Cys Tyr Gln Asp
       130                 135                 140
   Thr Ile Leu Trp Lys Asp Ile Phe His Lys Asn Asn Gln Leu Ala Leu
   145                 150                 155                 160
   Thr Leu Ile Asp Thr Asn Arg Ser Arg Ala Cys His Pro Cys Ser Pro
                   165                 170                 175
   Met Cys Lys Gly Ser Arg Cys Trp Gly Glu Ser Ser Glu Asp Cys Gln
               180                 185                 190
   Ser Leu Thr Arg Thr Val Cys Ala Gly Gly Cys Ala Arg Cys Lys Gly
           195                 200                 205
   Pro Leu Pro Thr Asp Cys Cys His Glu Gln Cys Ala Ala Gly Cys Thr
       210                 215                 220
   Gly Pro Lys His Ser Asp Cys Leu Ala Cys Leu His Phe Asn His Ser
   225 230 235 240
   Gly Ile Cys Glu Leu His Cys Pro Ala Leu Val Thr Tyr Asn Thr Asp
   245 250 255
   Thr Phe Glu Ser Met Pro Asn Pro Glu Gly Arg Tyr Thr Phe Gly Ala
   260 265 270
   Ser Cys Val Thr Ala Cys Pro Tyr Asn Tyr Leu Ser Thr Asp Val Gly
   275 280 285
   Ser Cys Thr Leu Val Cys Pro Leu His Asn Gln Glu Val Thr Ala Glu
   290 295 300
   Asp Gly Thr Gln Arg Cys Glu Lys Cys Ser Lys Pro Cys Ala Arg Val
   305 310 315 320
   Cys Tyr Gly Leu Gly Met Glu His Leu Arg Glu Val Arg Ala Val Thr
   325 330 335
   Ser Ala Asn Ile Gln Glu Phe Ala Gly Cys Lys Lys Ile Phe Gly Ser
   340 345 350
   Leu Ala Phe Leu Pro Glu Ser Phe Asp Gly Asp Pro Ala Ser Asn Thr
   355 360 365
   Ala Pro Leu Gln Pro Glu Gln Leu Gln Val Phe Glu Thr Leu Glu Glu
   370 375 380
   Ile Thr Gly Tyr Leu Tyr Ile Ser Ala Trp Pro Asp Ser Leu Pro Asp
   385 390 395 400
   Leu Ser Val Phe Gln Asn Leu Gln Val Ile Arg Gly Arg Ile Leu His
   405 410 415
   Asn Gly Ala Tyr Ser Leu Thr Leu Gln Gly Leu Gly Ile Ser Trp Leu
   420 425 430
   Gly Leu Arg Ser Leu Arg Glu Leu Gly Ser Gly Leu Ala Leu Ile His
   435 440 445
   His Asn Thr His Leu Cys Phe Val His Thr Val Pro Trp Asp Gln Leu
   450 455 460
   Phe Arg Asn Pro His Gln Ala Leu Leu His Thr Ala Asn Arg Pro Glu
   465 470 475 480
   Asp Glu Cys Val Gly Glu Gly Leu Ala Cys His Gln Leu Cys Ala Arg
   485 490 495
   Gly His Cys Trp Gly Pro Gly Pro Thr Gln Cys Val Asn Cys Ser Gln
   500 505 510
   Phe Leu Arg Gly Gln Glu Cys Val Glu Glu Cys Arg Val Leu Gln Gly
   515 520 525
   Leu Pro Arg Glu Tyr Val Asn Ala Arg His Cys Leu Pro Cys His Pro
   530 535 540
   Glu Cys Gln Pro Gln Asn Gly Ser Val Thr Cys Phe Gly Pro Glu Ala
   545 550 555 560
   Asp Gln Cys Val Ala Cys Ala His Tyr Lys Asp Pro Pro Phe Cys Val
   565 570 575
   Ala Arg Cys Pro Ser Gly Val Lys Pro Asp Leu Ser Tyr Met Pro Ile
   580 585 590
   Trp Lys Phe Pro Asp Glu Glu Gly Ala Cys Gln Pro Cys Pro Ile Asn
   595 600 605
   Cys Thr His Ser Cys Val Asp Leu Asp Asp Lys Gly Cys Pro Ala Glu
   610 615 620
   Gln Arg Ala Ser Pro Leu Thr
   625 630
<210> 1009
   <211> 626
   <212> PRT
   <213> Homo sapiens
<400> 1009
   Gln Ser Val Cys Ala Gly Thr Glu Asn Lys Leu Ser Ser Leu Ser Asp
   1               5                   10                  15
   Leu Glu Gln Gln Tyr Arg Ala Leu Arg Lys Tyr Tyr Glu Asn Cys Glu
               20                  25                  30
   Val Val Met Gly Asn Leu Glu Ile Thr Ser Ile Glu His Asn Arg Asp
           35                  40                  45
   Leu Ser Phe Leu Arg Ser Val Arg Glu Val Thr Gly Tyr Val Leu Val
       50                  55                  60
   Ala Leu Asn Gln Phe Arg Tyr Leu Pro Leu Glu Asn Leu Arg Ile Ile
   65                  70                  75                  80
   Arg Gly Thr Lys Leu Tyr Glu Asp Arg Tyr Ala Leu Ala Ile Phe Leu
                   85                  90                  95
   Asn Tyr Arg Lys Asp Gly Asn Phe Gly Leu Gln Glu Leu Gly Leu Lys
               100                 105                 110
   Asn Leu Thr Glu Ile Leu Asn Gly Gly Val Tyr Val Asp Gln Asn Lys
           115                 120                 125
   Phe Leu Cys Tyr Ala Asp Thr Ile His Trp Gln Asp Ile Val Arg Asn
       130                 135                 140
   Pro Trp Pro Ser Asn Leu Thr Leu Val Ser Thr Asn Gly Ser Ser Gly
   145                 150                 155                 160
   Cys Gly Arg Cys His Lys Ser Cys Thr Gly Arg Cys Trp Gly Pro Thr
                   165                 170                 175
   Glu Asn His Cys Gln Thr Leu Thr Arg Thr Val Cys Ala Glu Gln Cys
               180                 185                 190
   Asp Gly Arg Cys Tyr Gly Pro Tyr Val Ser Asp Cys Cys His Arg Glu
           195                 200                 205
   Cys Ala Gly Gly Cys Ser Gly Pro Lys Asp Thr Asp Cys Phe Ala Cys
       210                 215                 220
   Met Asn Phe Asn Asp Ser Gly Ala Cys Val Thr Gln Cys Pro Gln Thr
   225                 230                 235                 240
   Phe Val Tyr Asn Pro Thr Thr Phe Gln Leu Glu His Asn Phe Asn Ala
                   245                 250                 255
   Lys Tyr Thr Tyr Gly Ala Phe Cys Val Lys Lys Cys Pro His Asn Phe
               260                 265                 270
   Val Val Asp Ser Ser Ser Cys Val Arg Ala Cys Pro Ser Ser Lys Met
           275                 280                 285
   Glu Val Glu Glu Asn Gly Ile Lys Met Cys Lys Pro Cys Thr Asp Ile
       290                 295                 300
   Cys Pro Lys Ala Cys Asp Gly Ile Gly Thr Gly Ser Leu Met Ser Ala
   305                 310                 315                 320
   Gln Thr Val Asp Ser Ser Asn Ile Asp Lys Phe Ile Asn Cys Thr Lys
                   325                 330                 335
   Ile Asn Gly Asn Leu Ile Phe Leu Val Thr Gly Ile His Gly Asp Pro
   340 345 350
   Tyr Asn Ala Ile Glu Ala Ile Asp Pro Glu Lys Leu Asn Val Phe Arg
   355 360 365
   Thr Val Arg Glu Ile Thr Gly Phe Leu Asn Ile Gln Ser Trp Pro Pro
   370 375 380
   Asn Met Thr Asp Phe Ser Val Phe Ser Asn Leu Val Thr Ile Gly Gly
   385 390 395 400
   Arg Val Leu Tyr Ser Gly Leu Ser Leu Leu Ile Leu Lys Gln Gln Gly
   405 410 415
   Ile Thr Ser Leu Gln Phe Gln Ser Leu Lys Glu Ile Ser Ala Gly Asn
   420 425 430
   Ile Tyr Ile Thr Asp Asn Ser Asn Leu Cys Tyr Tyr His Thr Ile Asn
   435 440 445
   Trp Thr Thr Leu Phe Ser Thr Ile Asn Gln Arg Ile Val Ile Arg Asp
   450 455 460
   Asn Arg Lys Ala Glu Asn Cys Thr Ala Glu Gly Met Val Cys Asn His
   465 470 475 480
   Leu Cys Ser Ser Asp Gly Cys Trp Gly Pro Gly Pro Asp Gln Cys Leu
   485 490 495
   Ser Cys Arg Arg Phe Ser Arg Gly Arg Ile Cys Ile Glu Ser Cys Asn
   500 505 510
   Leu Tyr Asp Gly Glu Phe Arg Glu Phe Glu Asn Gly Ser Ile Cys Val
   515 520 525
   Glu Cys Asp Pro Gln Cys Glu Lys Met Glu Asp Gly Leu Leu Thr Cys
   530 535 540
   His Gly Pro Gly Pro Asp Asn Cys Thr Lys Cys Ser His Phe Lys Asp
   545 550 555 560
   Gly Pro Asn Cys Val Glu Lys Cys Pro Asp Gly Leu Gln Gly Ala Asn
   565 570 575
   Ser Phe Ile Phe Lys Tyr Ala Asp Pro Asp Arg Glu Cys His Pro Cys
   580 585 590
   His Pro Asn Cys Thr Gln Gly Cys Asn Gly Pro Thr Ser His Asp Cys
           595                 600                 605
   Ile Tyr Tyr Pro Trp Thr Gly His Ser Thr Leu Pro Gln His Ala Arg
       610                 615                 620
   Thr Pro
   625

## Description

### Field of the invention

This invention relates to polypeptides which bind to Human Epidermal Growth Factor Receptor 3 (herein referred to as HER3) and to use of such polypeptides in imaging and therapy. The invention also relates to bispecific ligands having binding affinity for both HER3 and HER2, or HER3 and EGFR.

### Background

The epidermal growth factor family of transmembrane tyrosine kinase receptors, including EGFR (ErbB1 or HER1), ErbB2 (HER2), ErbB3 (ERBB3 or HER3) and ErbB4 (HER4) are involved in regulating key cellular functions (e.g. cell proliferation, survival, differentiation and migration) through a complex network of intracellular signaling pathways. Today, it is well established that abnormal expression and signaling of these receptors are associated with development and progression of several types of cancer, making them important targets for development of novel cancer therapeutics. So far, the EGFR and HER2 receptors have been the most extensively studied, with several targeting agents approved by regulatory authorities for diagnosis or treatment of human cancers. HER3 lacks tyrosine kinase activity and must therefore form heterodimers with other ErbB receptors (Citri et al, Exp Cell Res 284(1):54-65 (2003)), e.g. with HER2 to exert its signaling function. The HER2-HER3 heterodimer is considered to be a potent oncogenic unit (Holbro et al, Proc Natl Acad Sci 100(15):8933-8 (2003)) and HER3 has recently gained increased attention as the preferred heterodimerization partner for HER2. Dimerization with HER2 leads to activation of the PI3K/Akt pathway and promotion of tumor cell survival and proliferation (Hynes et al, Nat Rev Cancer 5(5):341-54 (2005)). HER3 is expressed in a number of human cancers, including breast (Bobrow et al, Eur J Cancer 33(11):1846-50 (1997)), ovarian and bladder cancer (Rajkumar et al, Clin Mol Pathol 49(4) (1996)), and play an important role in signaling in other cancers (Stove et al, Clin Exp Metastasis 21(8):665-84 (2004)), including some lung cancers (Engelman et al, Proc Natl Acad Sci 102(10):3788-93 (2005)) and prostate cancer (Gregory et al, Clin Cancer Res 11(5):1704-12 (2005)). In addition, HER3 expression has a prognostic value, since high levels of receptor expression are associated with significantly shorter survival time compared with patients that overexpress HER2 (Tanner et al, J Clin Oncol 24(26):4317-23 (2006), Reschke et al, Clin Cancer Res 14(16):5188-97 (2008)). HER3 has indeed been suggested to be a key node in ligand-induced activation of the ErbB receptor-PI3K signaling axis (Schoeberl et al, Sci Signal 2(77):ra 31 (2009)).

A relatively large fraction of the more recently approved therapies directed towards the EGFR and HER2 receptors is based on monoclonal antibodies. One of the reasons behind the success for this new class of biological agents in cancer therapy is that antibodies offer new and unique mechanisms of action previously unachievable using small chemical drugs. In addition to more traditional agonistic (e.g. stimulation of a cell surface receptor) and antagonistic (e.g. blocking of natural protein-protein interactions) therapeutic effects, antibodies can also be employed as targeting agents in order to specifically direct a range of immunological defense mechanisms against the cancerous cells, as well as to achieve specific delivery of various imaging or therapeutic conjugates (e.g. chemotherapeutic drugs, radionuclides and toxins). In contrast to the well investigated EGFR and HER2 receptor members of the ErbB-family, there are relatively few reports on the use of anti-HER3 antibodies.

Ullrich and co-workers have however reported that anti-HER3 monoclonal antibodies inhibit HER3 mediated signaling in cell models of breast cancer (van der Horst et al, Int J Cancer 115(4):519-27 (2005)), and there are currently two monoclonal anti-HER3 antibodies in Phase I clinical trials (AMG 888, Baselga et al, Nat Rev Cancer 9(7):463-75 (2009), and MM-121, Schoeberl *et al, supra*).

However, although several successful cancer therapy studies have been reported using full-length monoclonal antibodies, this class of agents is not always optimal for targeting solid tumors (neither for diagnostic nor for therapeutic pay-load purposes). Therapeutic effect is dependent on an efficient distribution of the drug throughout the tumor, and molecular imaging depends on a high ratio between tumor uptake and surrounding normal tissue. Since tumor penetration rate (including extravasation) is negatively associated with the size of the molecule, the relatively large IgG molecule inherently has poor tissue distribution and penetration capacity. Moreover, for molecular imaging, the extraordinarily long *in vivo* half-life of antibodies results in relatively high blood signals and thereby relatively poor tumor-to-blood contrasts.

Since HER3 may be expressed on the same tumor cell as other members of the EGF family, production of bispecific molecules targeting HER3 and another member of the EGF family has recently attracted some interest. Such bispecific molecules could for example be utilized as targeting vehicles for increasing the specificity of targeting in molecular imaging applications and simultaneously targeting HER3 and another antigen expressed on tumors.

It is however complicated to produce bispecific monoclonal antibodies which bind to two separate targets. When the genes encoding the four required polypeptide chains are produced in a cell as many as 10 different combinations are possible, and only one combination represents the desired bispecific antibody. Therefore, the concept of bispecific binding has not been fully explored using antibodies. Instead, antibody fragments and other binding molecules have been more widely utilized for making bispecific binding molecules, including bispecific Z variants (Friedman et al, Biotechnol Appl Biochem 54:121-31 (2009)).

### Description

It is an object of the present invention to provide novel HER3 binding agents which for example may be used in targeting HER3 expressing cells, molecular imaging of such HER3 expressing cells and treatment of HER3 related conditions.

A further object of the invention is to provide targeting agents with high specificity for lesions expressing both HER2 and HER3. The HER3 binding polypeptide of the invention comprises a HER3 binding motif, BM, which motif consists of the amino acid sequence

EX₂X₃X₄AYX₇EIWX₁₁LPNLX₁₆X₁₇X₁₈QX₂₀X₂₁AFIX₂₅X₂₆LX₂₈D,

wherein, independently of each other,
X₂X₃ is selected from KY and RY;
X₄ is selected from A, H, I, K, L, N, Q, R, S, T and V;
X₇ is selected from A, F, G, V, W and Y;
X₁₁ is Q;
X₁₆ is selected from N and T;
X₁₇X₁₈ is selected from RV, RT, VR and RR;
X₂₀ is K;
X₂₁ is A;
X₂₅ is selected from A, G, and S;
X₂₆ is selected from S and K;
X₂₈ is selected from A, E, F, Q, S and W;
and wherein the polypeptide binds to the extra-cellular domain of HER3.

The invention is defined by the claims.

Described herein is furthermore a HER3 binding polypeptide, comprising a HER3 binding motif, *BM,* which motif consists of the amino acid sequence selected from
i)

   EX₂X₃X₄A X₆X₇EIW X₁₁LPNL X₁₆X₁₇X₁₈QX₂₀ X₂₁AFIX₂₅ X₂₆LX₂₈D,

   wherein, independently of each other,
   X₂ is selected from R, K, L, W and V;
   X₃ is selected from R, H, K, M, S, W, Y and V;
   X₄ is selected from A, R, N, D, Q, E, G, H, I, L, K, M, S, T, W and V;
   X₆ is selected from A, R, Q, M, S, T and Y;
   X₇ is selected from A, E, G, H, K, F, S, T, W, Y and V;
   X₁₁ is selected from A, N, D, Q, E, I, L and T;
   X₁₆ is selected from N and T;
   X₁₇ is selected from A, R, N, Q, K, P, S, T and V;
   X₁₈ is selected from A, R, N, E, F, D, Q, G, H, I, L, K, S, T, W, Y and V;
   X₂₀ is selected from A, R and K;
   X₂₁ is selected from A, R, N, G, H, S and V;
   X₂₅ is selected from A, R, E, G, I, K, S, T and V;
   X₂₆ is selected from S and K;
   X₂₈ is selected from A, D, Q, E, F, P, S, T, W and Y;
   and
ii) an amino acid sequence which has at least 90 % identity to the sequence defined in i),
wherein the polypeptide binds to the extra-cellular domain of HER3 (HER3-ECD).

The polypeptides as described herein present good binding affinity for HER3 in that they bind well to HER3. The above definition of a class of sequence related HER3 binding polypeptides is based on an analysis of a number of random polypeptide variants of a parent scaffold that were selected for their interaction with HER3 in selection experiments. The identified HER3 binding motif, or *"BM",* corresponds to the target binding region of the parent scaffold, which region constitutes two alpha helices within a three-helical bundle protein domain. In the parent scaffold, the varied amino acid residues of the two *BM* helices constitute a binding surface for interaction with the constant Fc part of antibodies. By random variation of binding surface residues and subsequent selection of variants, the Fc interaction capacity of the binding surface has been replaced with a capacity for interaction with HER3.

As the skilled person will realize, the function of any polypeptide, such as the HER3 binding capacity of the polypeptides as defined herein, is dependent on the tertiary structure of the polypeptide. It is therefore possible to make minor changes to the amino acid sequence of a polypeptide without largely affecting the tertiary structure and the function thereof. Described is a polypeptide comprising modified variants of the *BM* of i), which are such that the resulting sequence is at least 90 % identical to a sequence belonging to the class defined by i), such as at least 93 % identical, such as at least 97 % identical to a sequence belonging to the class defined by i). In some embodiments, minor changes may be made in all positions of the sequences of the HER3 binding polypeptides as disclosed herein. In other embodiments, minor changes may be made only in the non-variable positions, also denoted as scaffold amino acid residues. In such cases, changes are not allowed in the variable positions, i.e. positions denoted with an "X" (e.g. X₂, X₃, X₄, X₆, X₇, X₁₁, X₁₆, X₁₇, X₁₈, X₂₀, X₂₁, X₂₅, X₂₆ and X₂₈ of the above defined *BM*). For example, it is possible that an amino acid residue belonging to a certain functional grouping of amino acid residues (e.g. hydrophobic, hydrophilic, polar etc) could be exchanged for another amino acid residue from the same functional group.

The term "% identity", as used throughout the specification and the appended claims, is calculated as follows. The query sequence is aligned to the target sequence using the CLUSTAL W algorithm (Thompson, J.D., Higgins, D.G. and Gibson, T.J., Nucleic Acids Research, 22: 4673-4680 (1994)). A comparison is made over the window corresponding to the shortest of the aligned sequences. The shortest of the aligned sequences may in some instances be the target sequence, such as the 29 amino acid residue HER3 binding motif. In other instances, the query sequence may constitute the shortest of the aligned sequences. The query sequence may for example consist of at least 10 amino acid residues, such as at least 20 amino acid residues. The amino acid residues at each position are compared, and the percentage of positions in the query sequence that have identical correspondences in the target sequence is reported as % identity.

The term "% similarity", as used throughout the specification and the appended claims, is calculated in the following way. Sequence alignment and comparison are basically performed as described in relation to the % identity calculation. "Similarity" should however be interpreted as follows. Two amino acid residues are considered similar if they belong to the same group of amino acid residues. Non-limiting examples of groups of amino acid residues are the hydrophobic group, comprising the amino acid residues Ala, Val, Phe, Pro, Leu, Ile, Trp, Met and Cys; the basic group, comprising the amino acid residues Lys, Arg and His; the acidic group, comprising the amino acid residues Glu and Asp; the hydrophilic group, comprising the uncharged amino acid residues Gln, Asn, Ser, Thr and Tyr; and the natural group, comprising the amino acid residue Gly. Thus, the amino acid residues at each position are compared, and the percentage of positions in the query sequence that have similar correspondences in the target sequence is reported as % similarity.

Throughout this description, alternative embodiments according to the description fulfill, instead of the specified percentage of identity, the corresponding percentage of similarity. Other alternative embodiments fulfill the specified percentage of identity as well as another, higher percentage of similarity, selected from the group of preferred percentages of identity for each sequence. For example, a sequence may be 70 % similar to another sequence; or it may be 70 % identical to another sequence; or it may be 70 % identical and 90 % similar to another sequence.

In one embodiment of the above disclosed polypeptide, X₂ is selected from R, K and W.

In a further embodiment of the above disclosed polypeptide, X₃ is selected from R, K, S and Y.

In a further embodiment of the above disclosed polypeptide, X₄ is selected from A, R, N, Q, H, I, L, K, S, T and V.

In a further embodiment of the above disclosed polypeptide, X₆ is selected from A, S, T and Y.

In a further embodiment of the above disclosed polypeptide, X₇ is selected from A, G, F, W, Y and V.

In a further embodiment of the above disclosed polypeptide, X₁₁ is selected from Q and E.

In a further embodiment of the above disclosed polypeptide, X₁₇ is selected from R, Q, T and V.

In a further embodiment of the above disclosed polypeptide, X₁₈ is selected from R, Q, G, H, I, L, S, T, W, Y and V.

In a further embodiment of the above disclosed polypeptide, X₂₀ is selected from R and K.

In a further embodiment of the above disclosed polypeptide, X₂₁ is selected from A and G.

In a further embodiment of the above disclosed polypeptide, X₂₅ is selected from A, R, G, K and S.

In a further embodiment of the above disclosed polypeptide, X₂₈ is selected from A, Q, E, F, S, W and Y.

In a further embodiment of the above disclosed polypeptide, X₂ is selected from R and K.

In a further embodiment of the above disclosed polypeptide, X₃ is selected from K and Y.

In a further embodiment of the above disclosed polypeptide, X₄ is selected from A, R, N, Q, H, L, K, S, T and V.

In a further embodiment of the above disclosed polypeptide, X₆ is selected from T and Y.

In a further embodiment of the above disclosed polypeptide, X₇ is selected from A, G, F, Y and V.

In a further embodiment of the above disclosed polypeptide, X₁₇ is selected from R, Q and V.

In a further embodiment of the above disclosed polypeptide, X₁₈ is selected from R, Q, I, L, T, Y and V.

In a further embodiment of the above disclosed polypeptide, X₂₅ is selected from A, G, K and S.

In a further embodiment of the above disclosed polypeptide, X₂₈ is selected from A, Q, E, F, S and W.

In one embodiment of the above disclosed HER3 binding polypeptide, the polypeptide comprises a HER3 binding motif, *BM,* which motif consists of the amino acid sequence selected from
i)

   EX₂X₃X₄A YX₇EIW X₁₁LPNL X₁₆X₁₇X₁₈QX₂₀ X₂₁AFIX₂₅ X₂₆LX₂₈D,

   wherein, independently of each other,
   X₂ is selected from K, R and W;
   X₃ is selected from K, R, and Y;
   X₄ is selected from A, D, E, G, H, I, K, L, M, N, Q, R, S, T, V and W;
   X₇ is selected from A, E, F, G, H, K, S, T, V, W and Y;
   X₁₁ is selected from E and Q;
   X₁₆ is selected from N and T;
   X₁₇ is selected from R, T and V;
   X₁₈ is selected from R, T and V;
   X₂₀ is selected from K and R;
   X₂₁ is selected from A and G;
   X₂₅ is selected from A, G, K, R and S;
   X₂₆ is selected from S and K;
   X₂₈ is selected from A, D, E, F, P, Q, S, T, W and Y;
   and
ii) an amino acid sequence which has at least 93 % identity to the sequence defined in i), wherein the polypeptide binds to the extra-cellular domain of HER3.

In a further embodiment of the above disclosed polypeptide, X₂X₃ is selected from KY, RY, WK and WR.

In a further embodiment of the above disclosed polypeptide, X₁₁ is Q.

In a further embodiment of the above disclosed polypeptide, X₁₇X₁₈ is selected from RV, RT, VR and RR.

In a further embodiment of the above disclosed polypeptide, X₂₀ is K.

In a further embodiment of the above disclosed polypeptide, X₂₁ is A.

In a further embodiment of the above disclosed polypeptide, X₂X₃ is selected from KY and RY.

In a further embodiment of the above disclosed polypeptide, X₄ is selected from K, L, S and T.

In a further embodiment of the above disclosed polypeptide, X₇ is selected from F, G and Y.

In a further embodiment of the above disclosed polypeptide, X₁₇X₁₈ is selected from RV, RT and VR.

In a further embodiment of the above disclosed polypeptide, X₂₅ is selected from A, G and S.

In yet a further embodiment of the above disclosed polypeptide, the amino acid sequence i) fulfils at least one of the following four conditions I, II, III and IV:
I) X₂X₃ is selected from KY and RY;
II) X₁₁ is Q;
III) X₂₀ is K;
IV) X₂₁ is A.
The amino acid sequence i) may for example fulfill at least two, such as at least three or all, of the four conditions I, II, III and IV

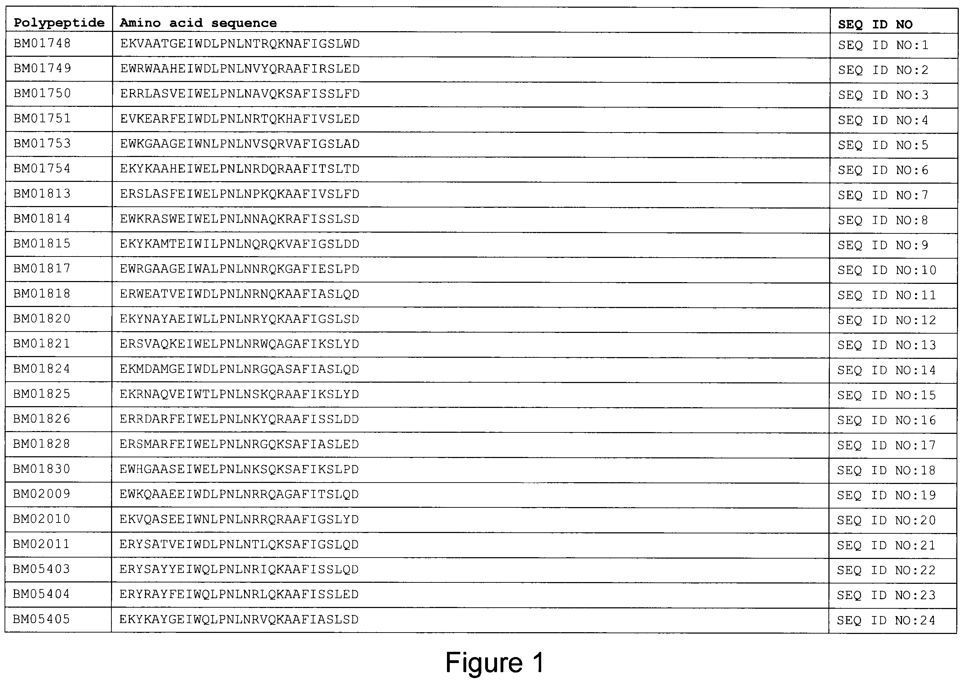

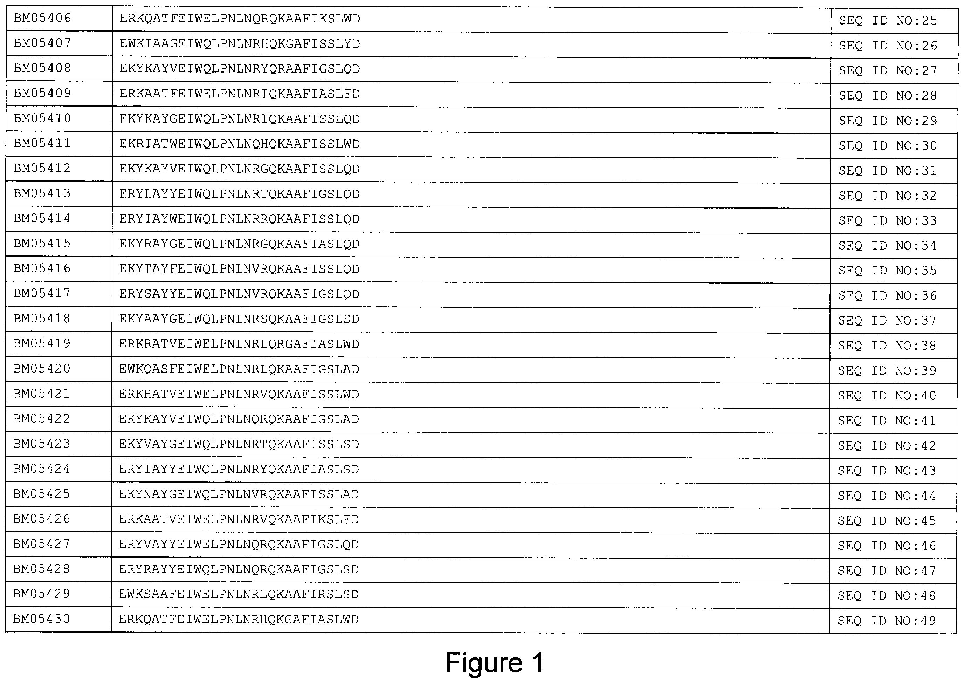

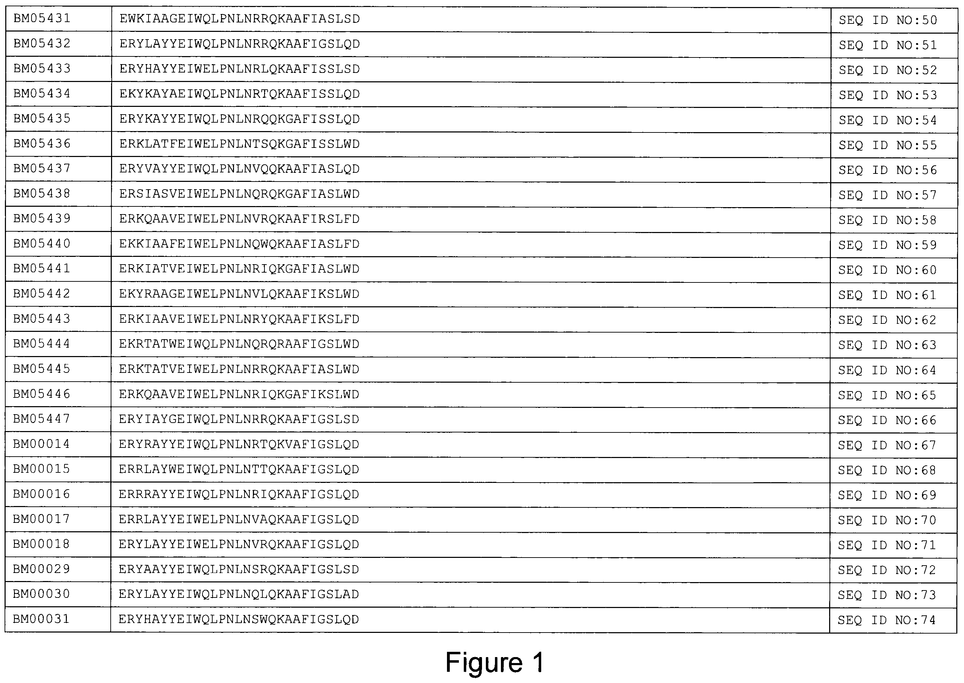

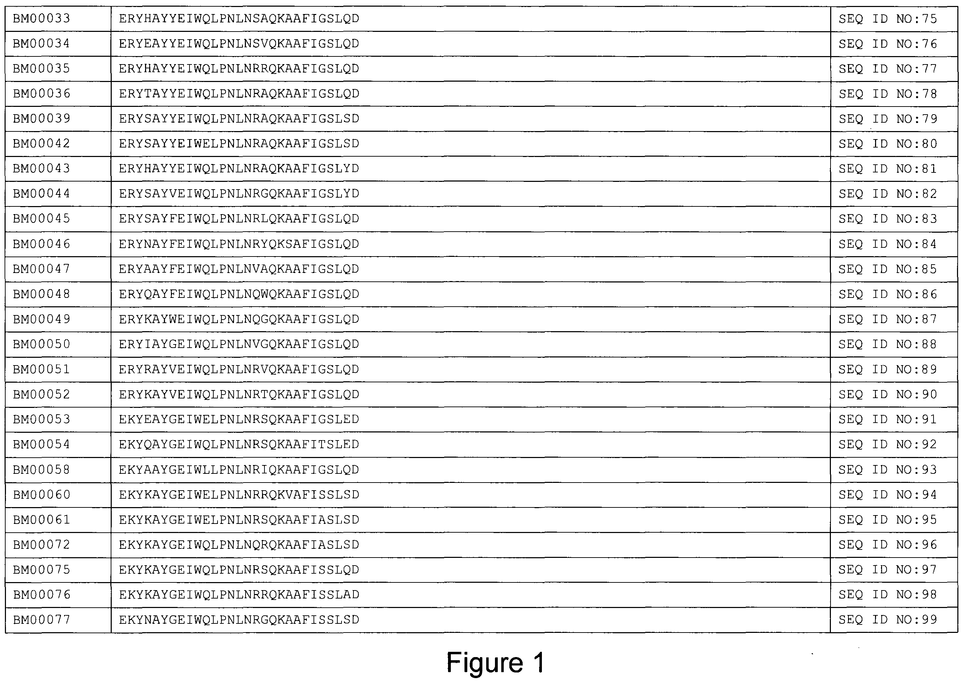

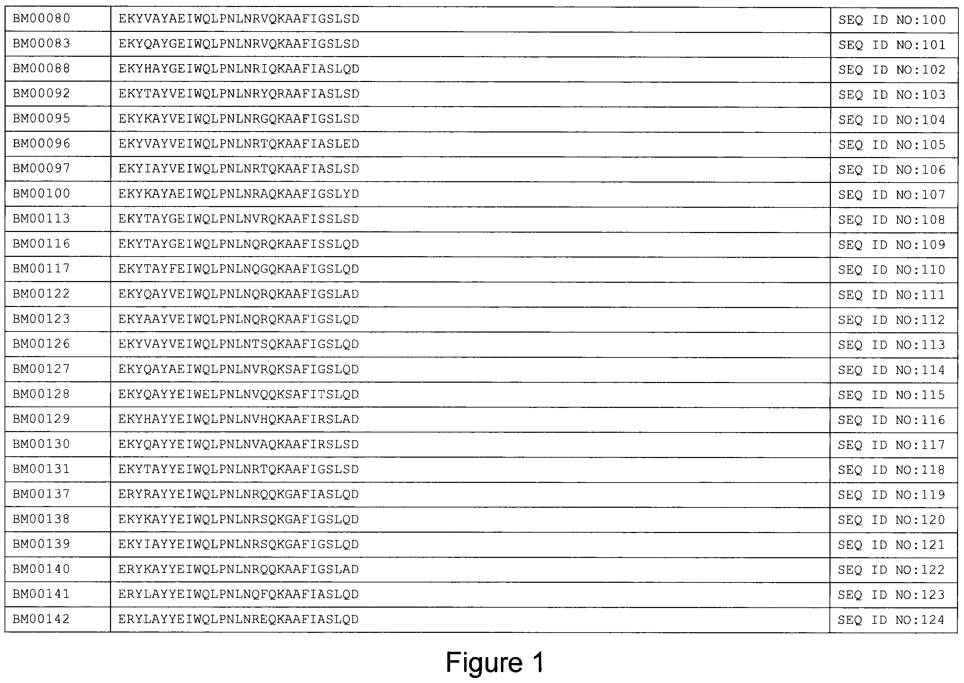

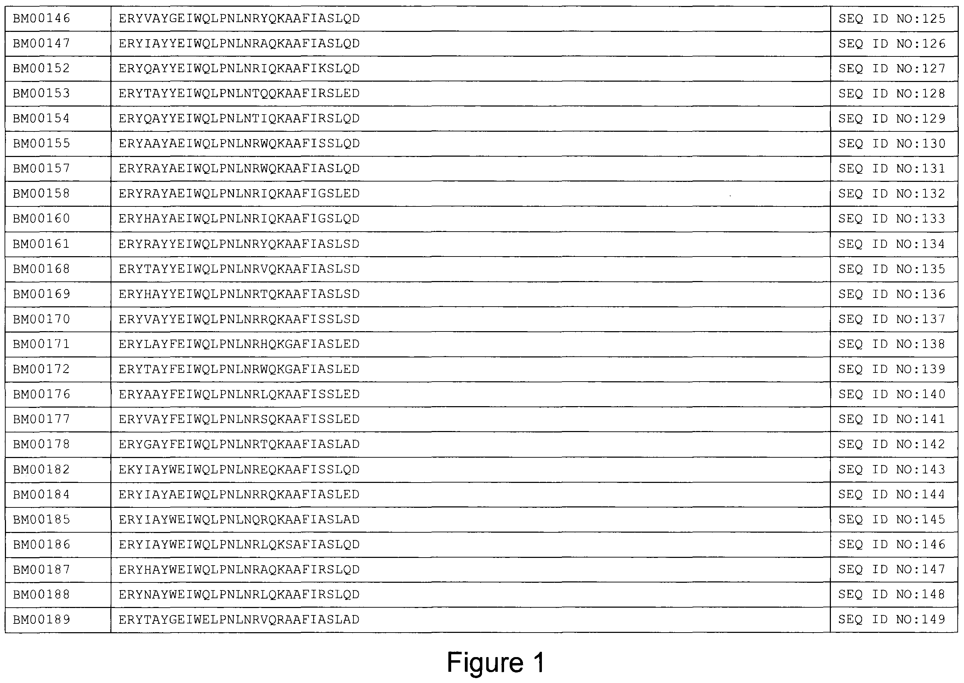

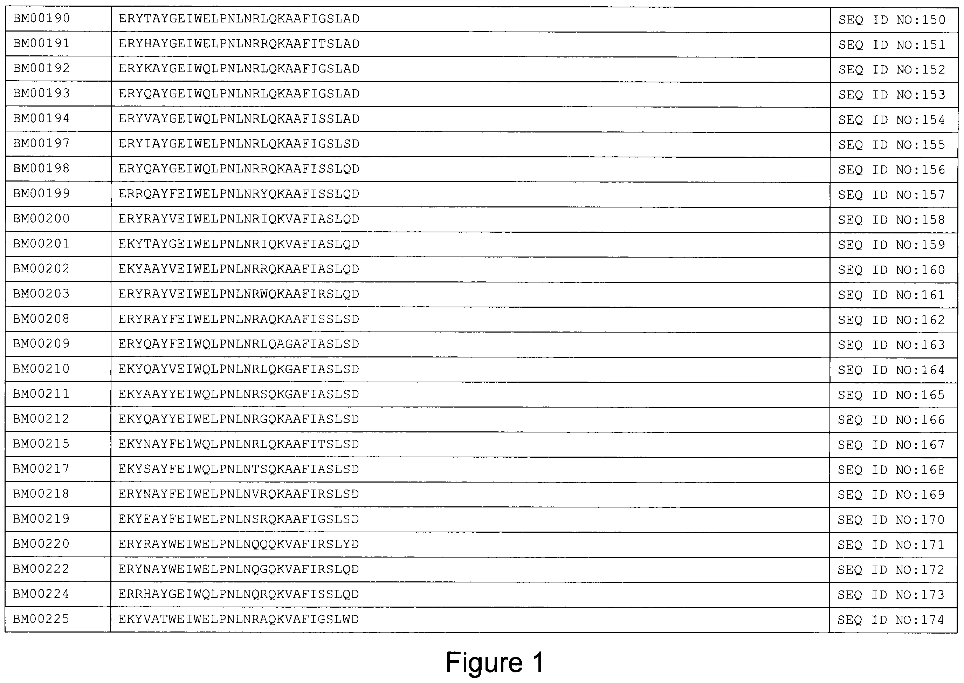

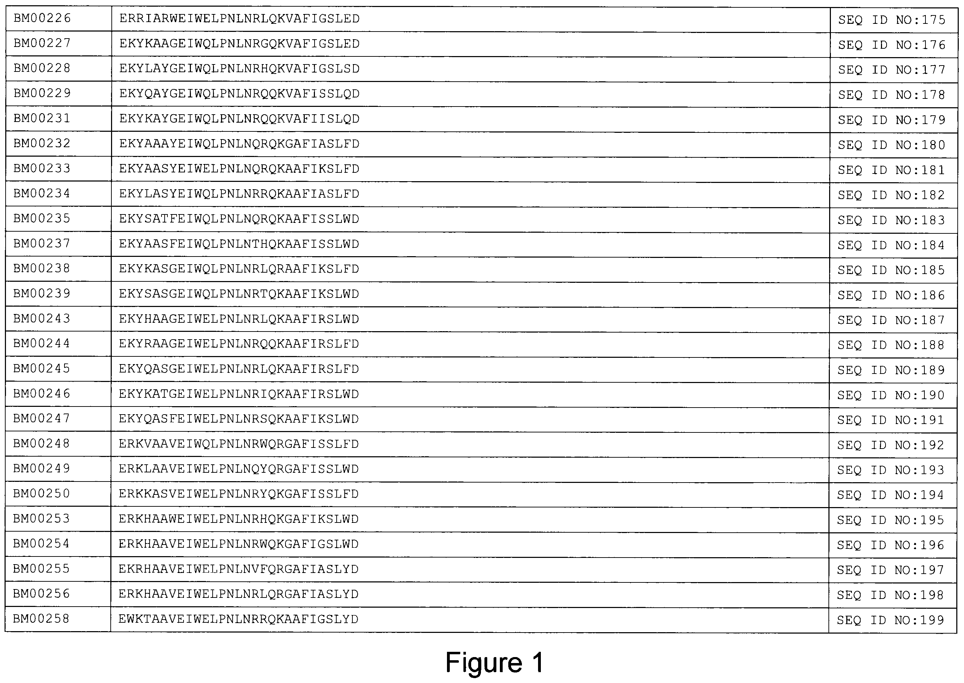

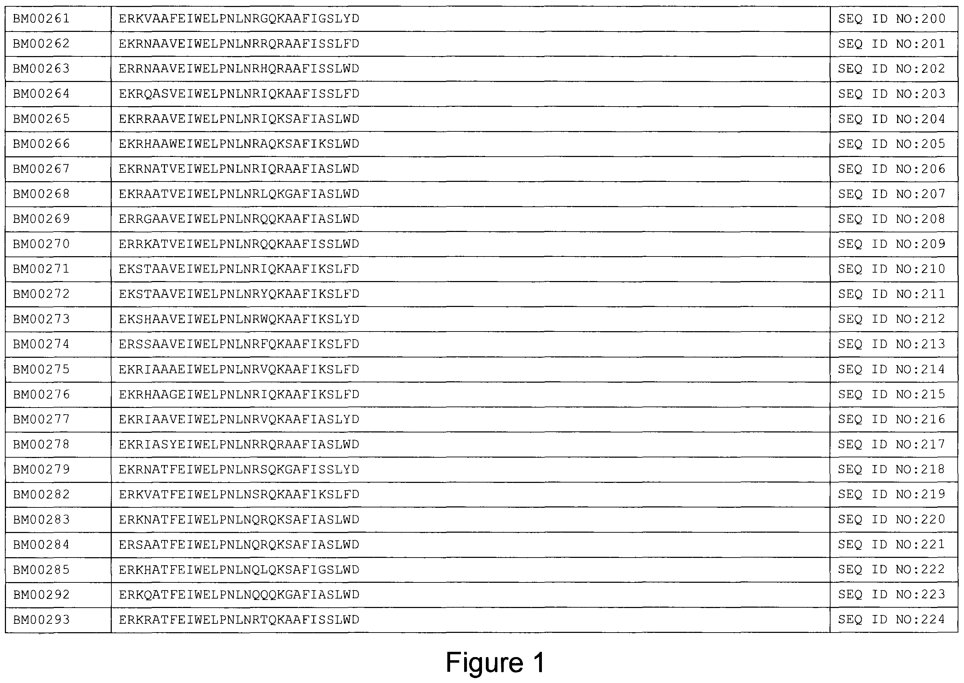

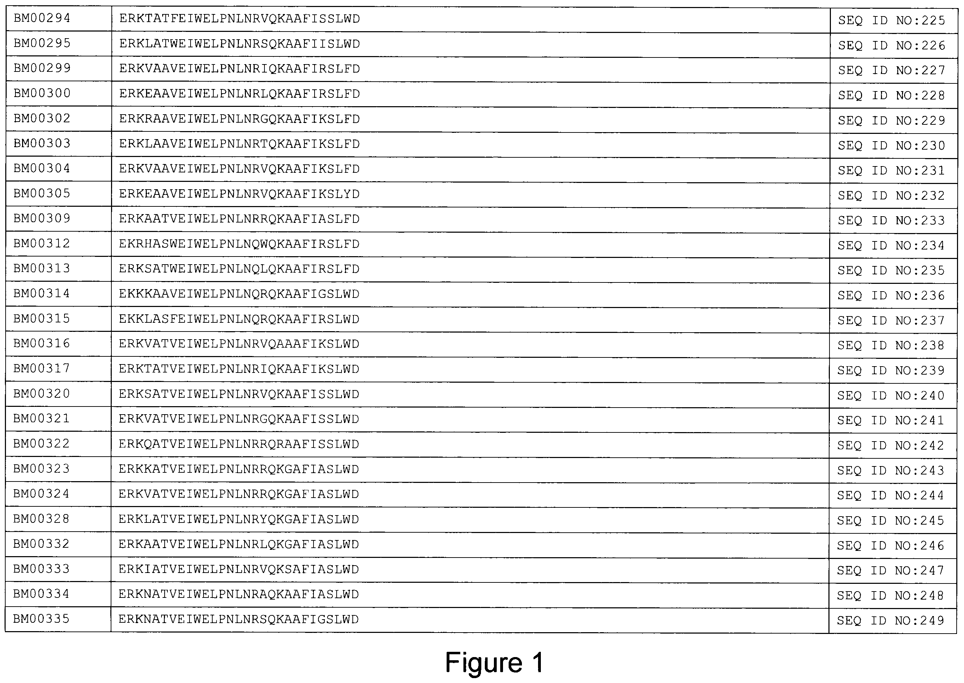

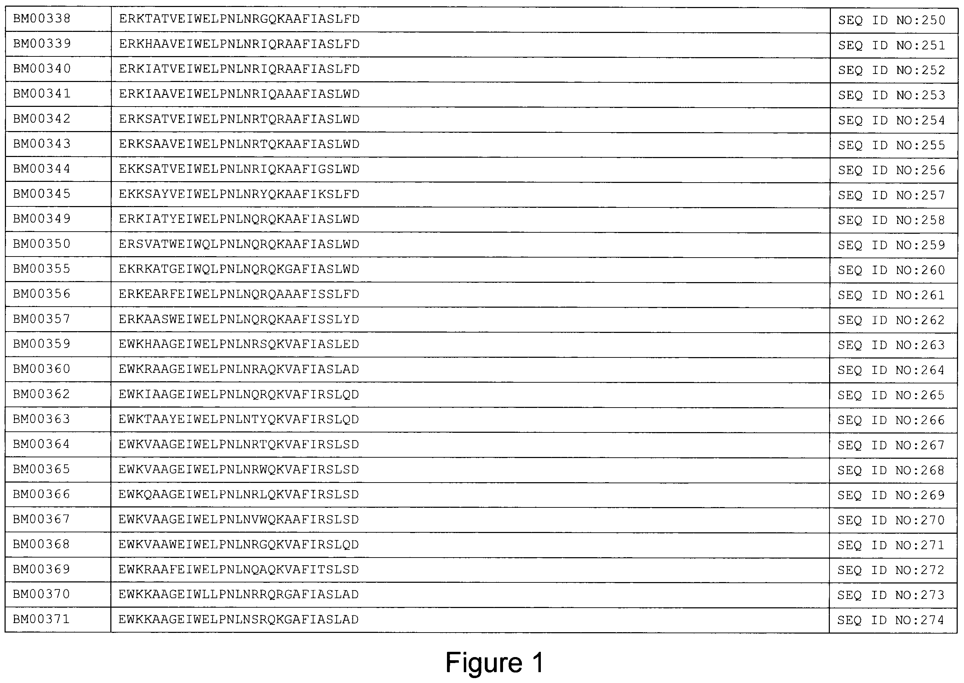

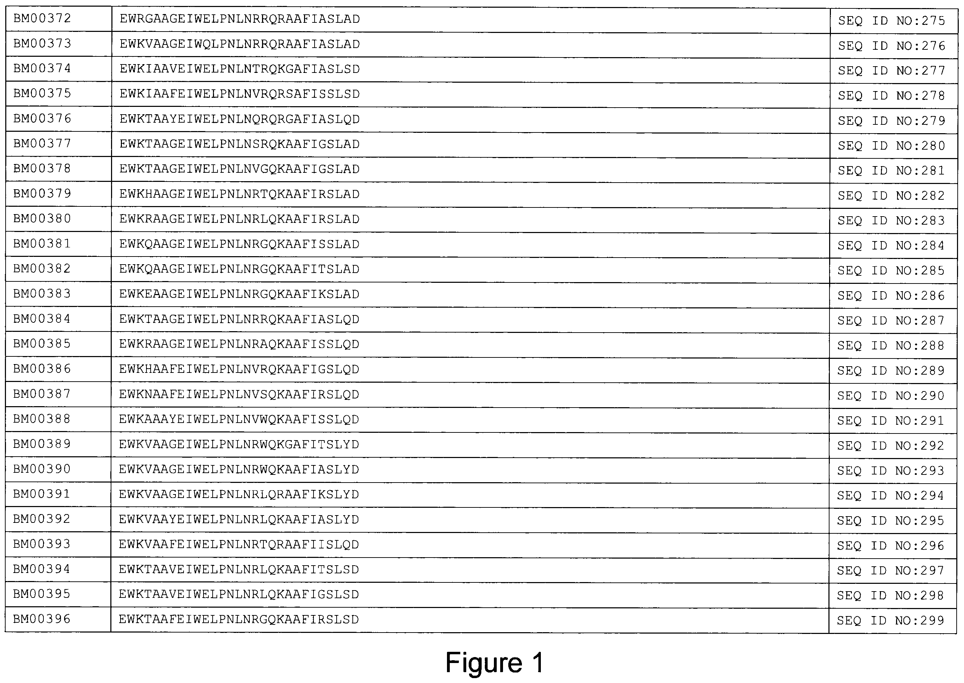

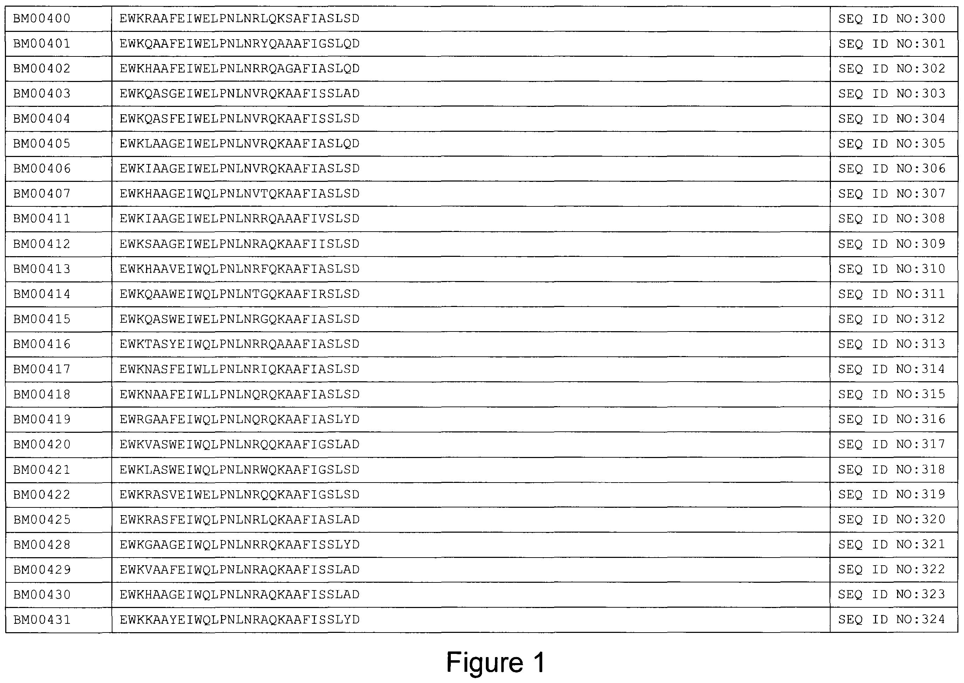

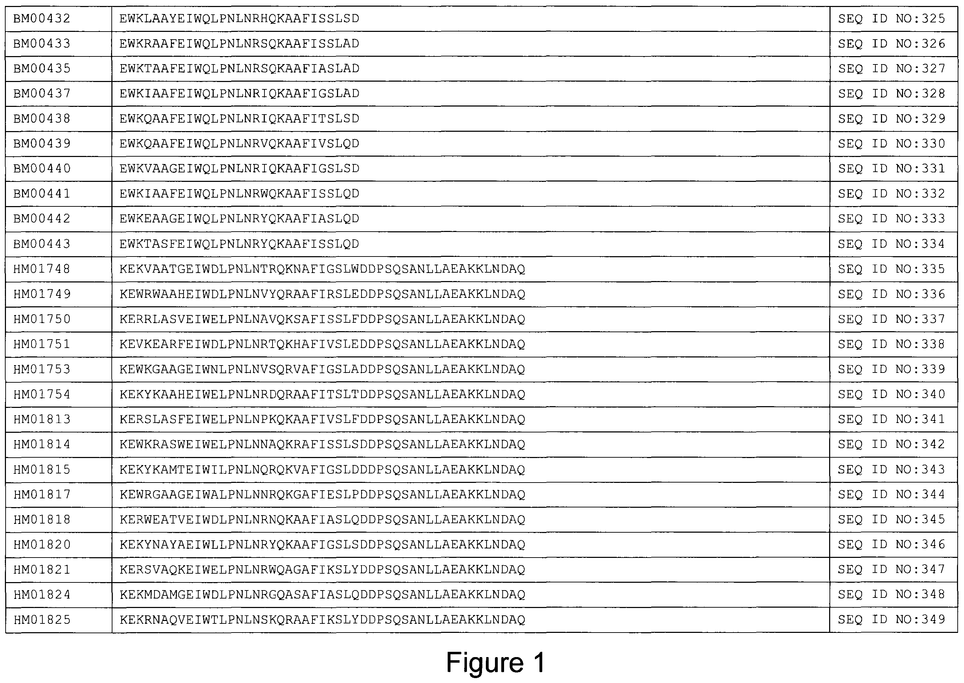

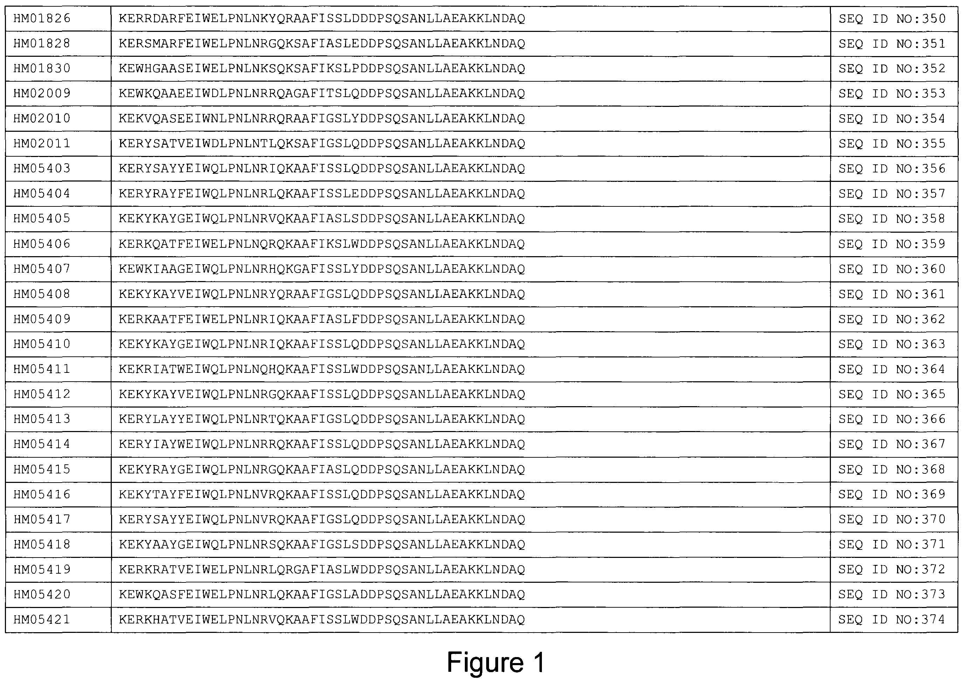

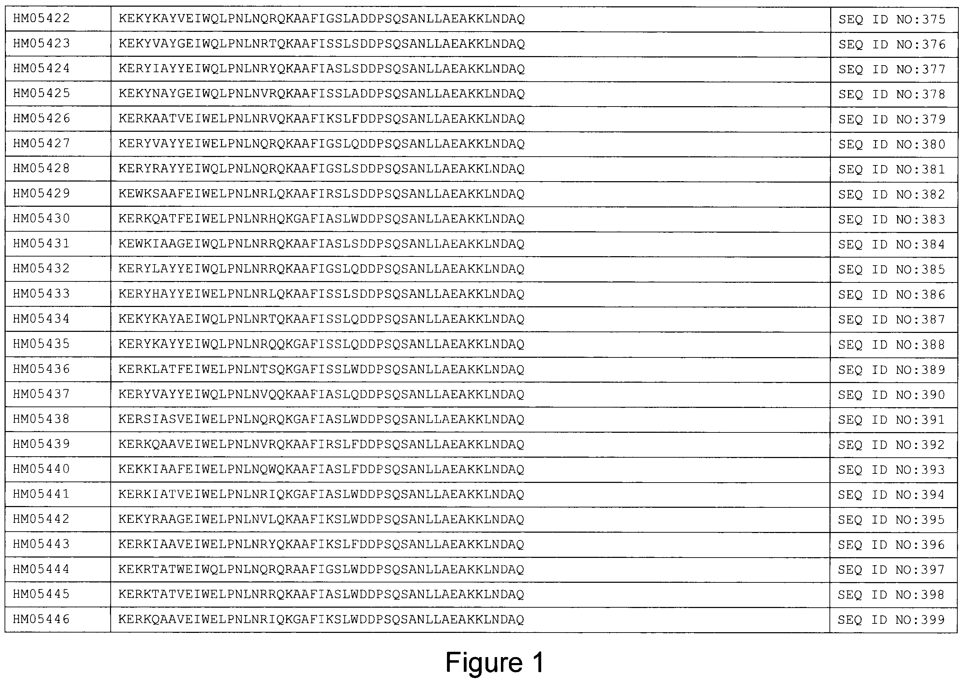

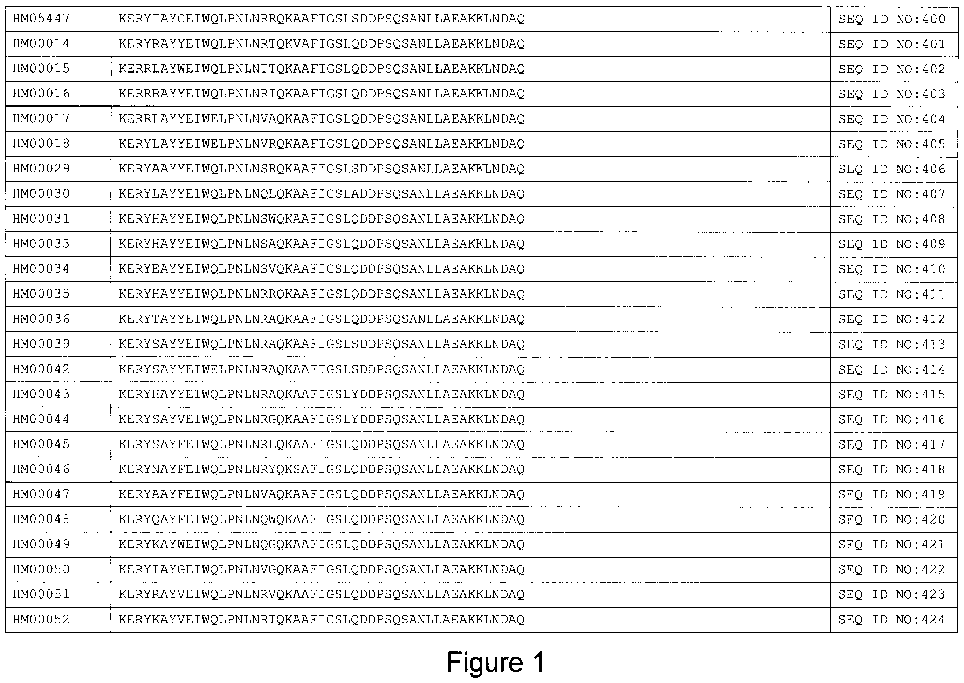

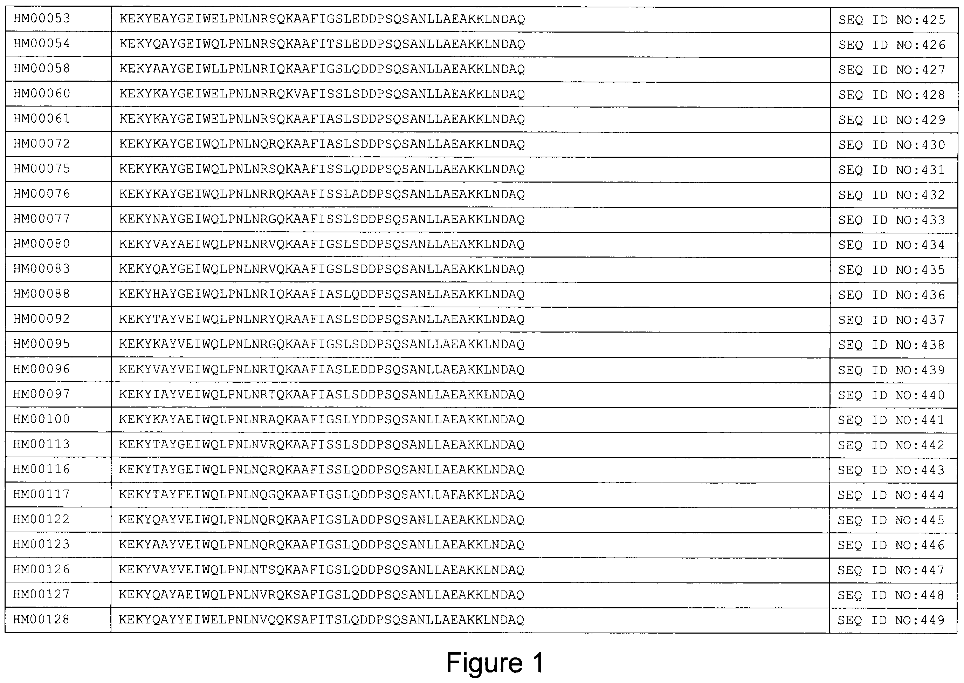

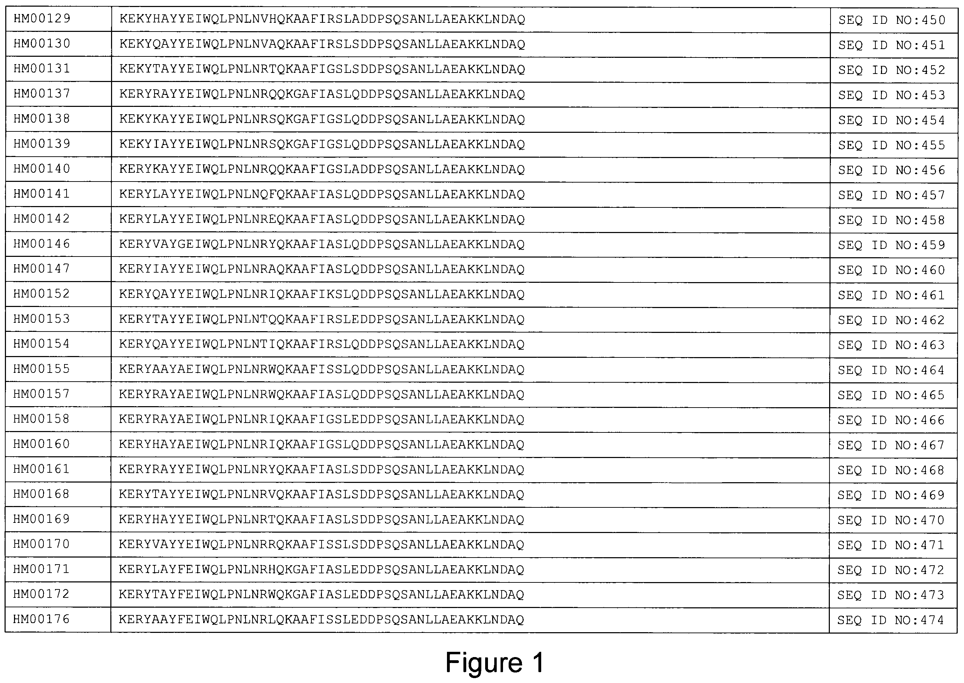

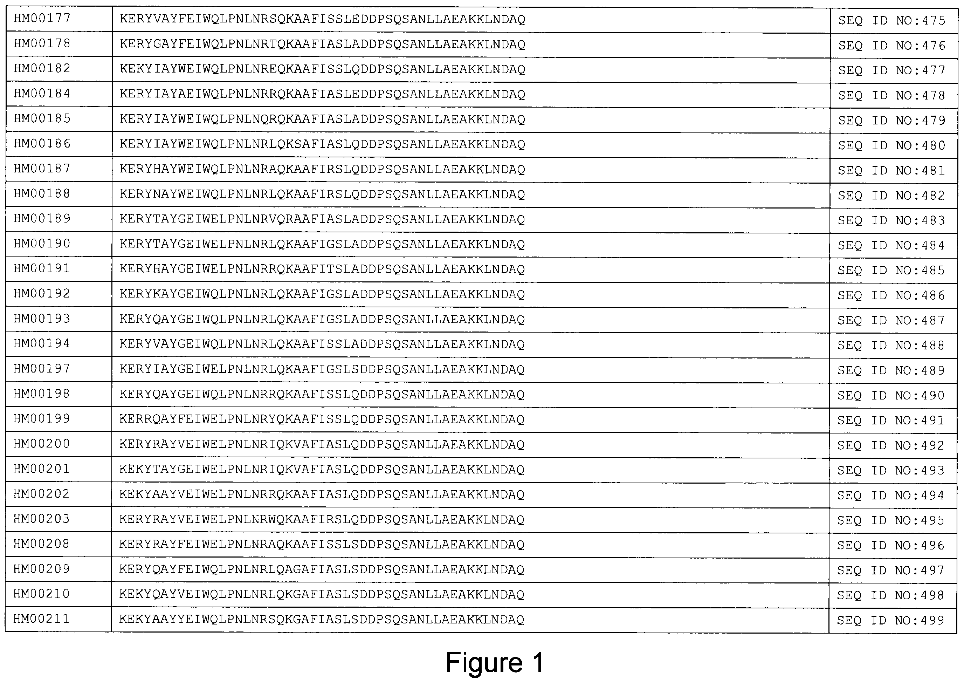

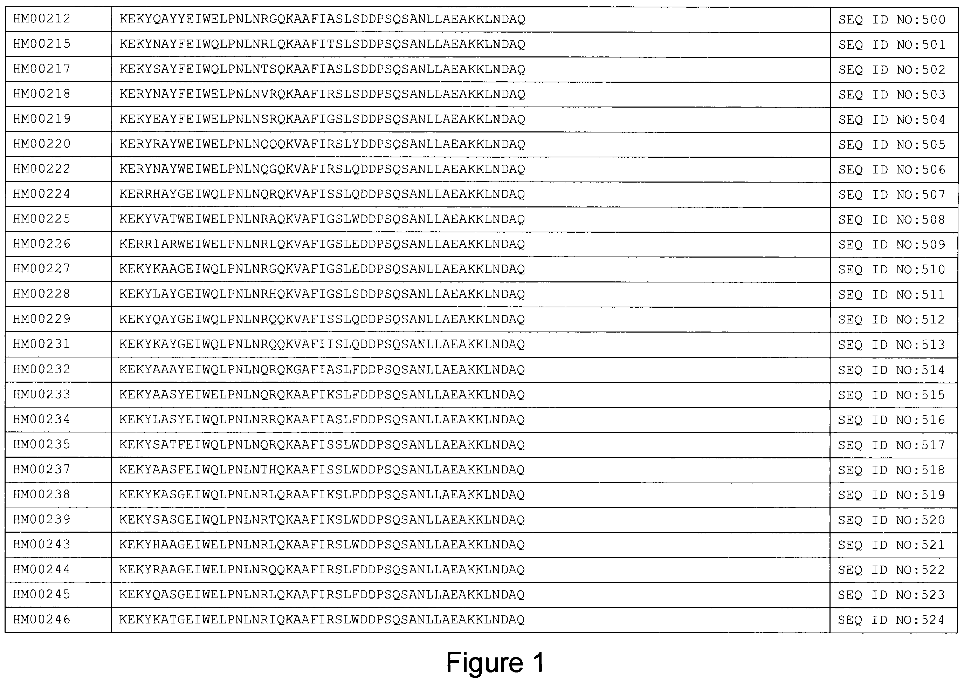

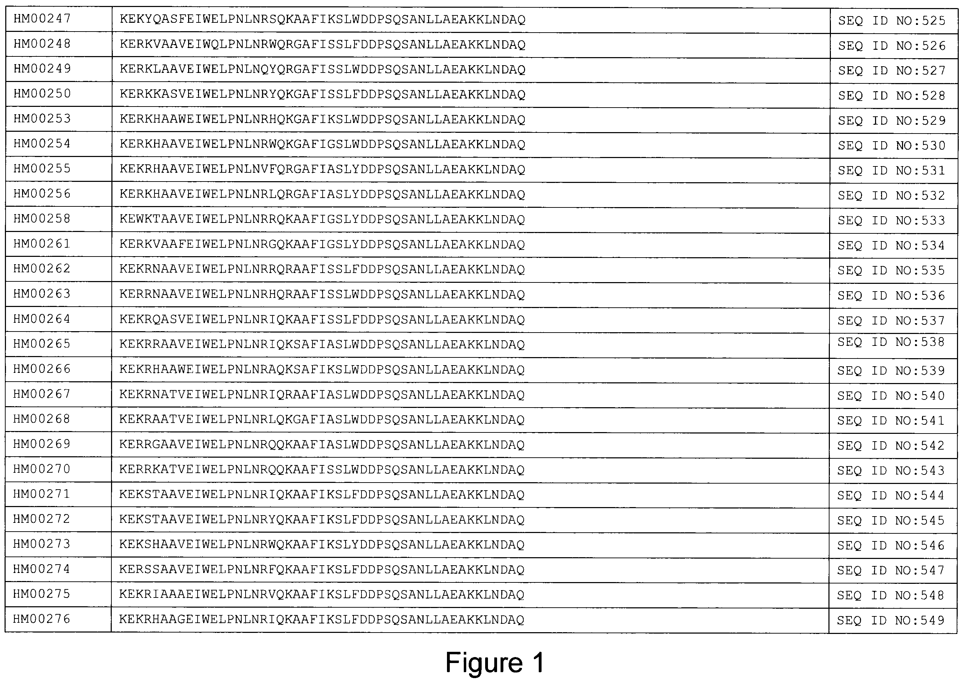

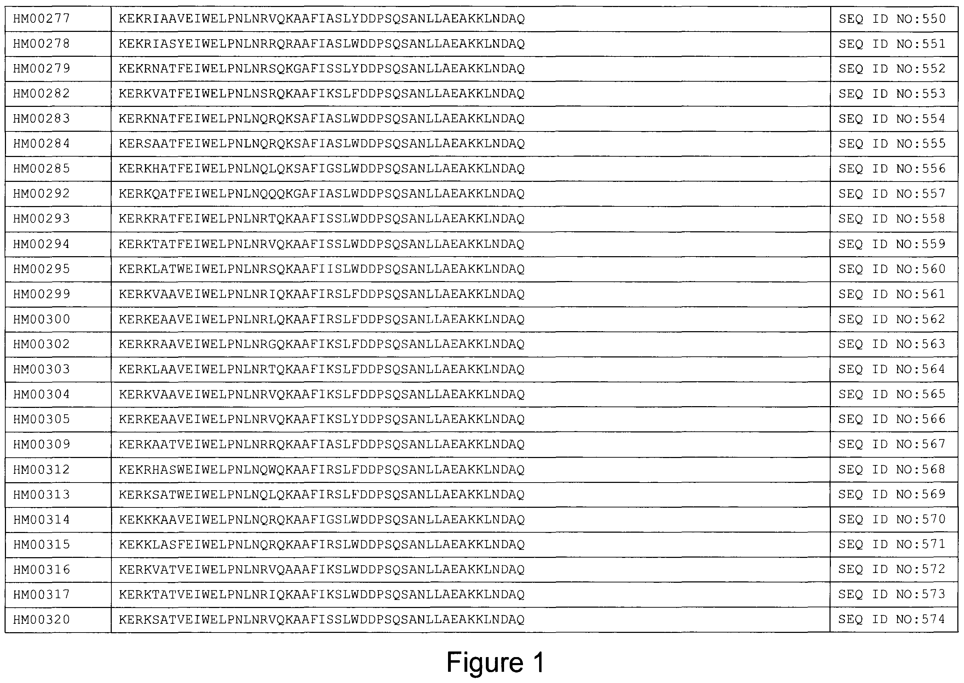

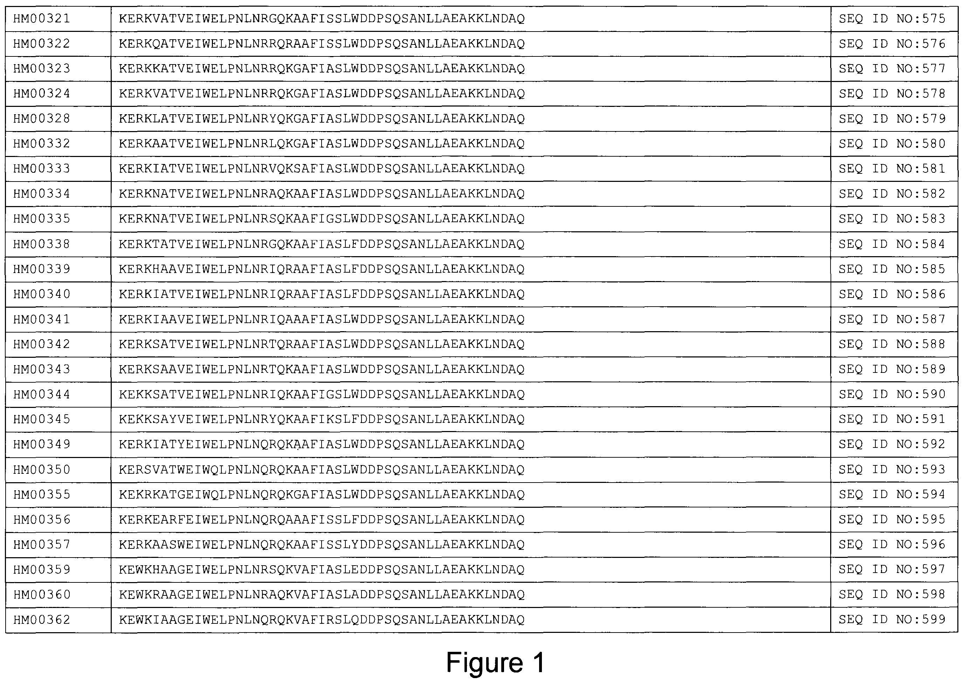

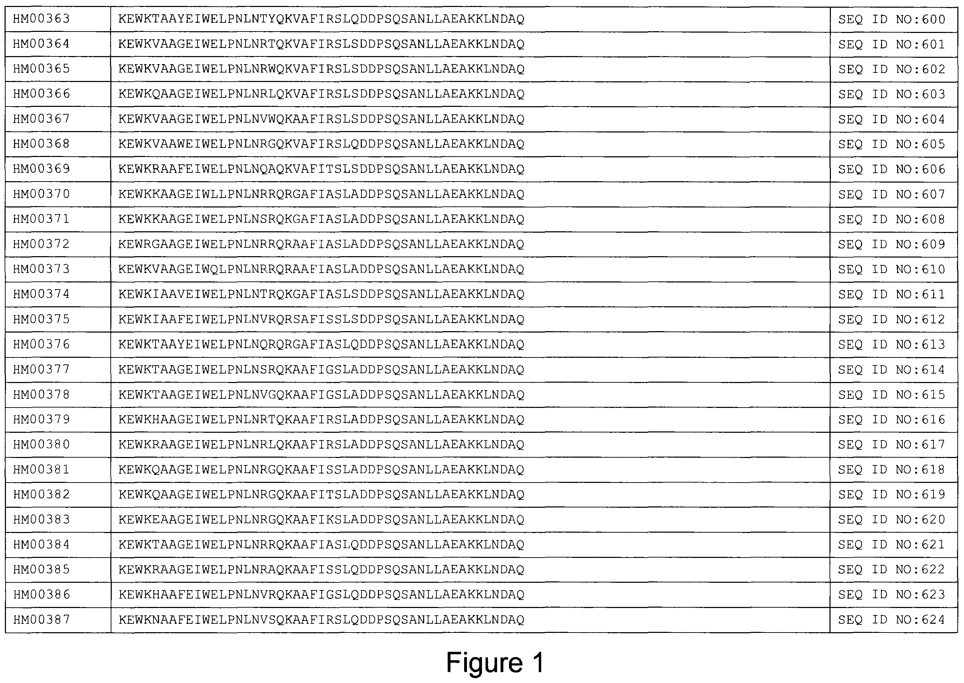

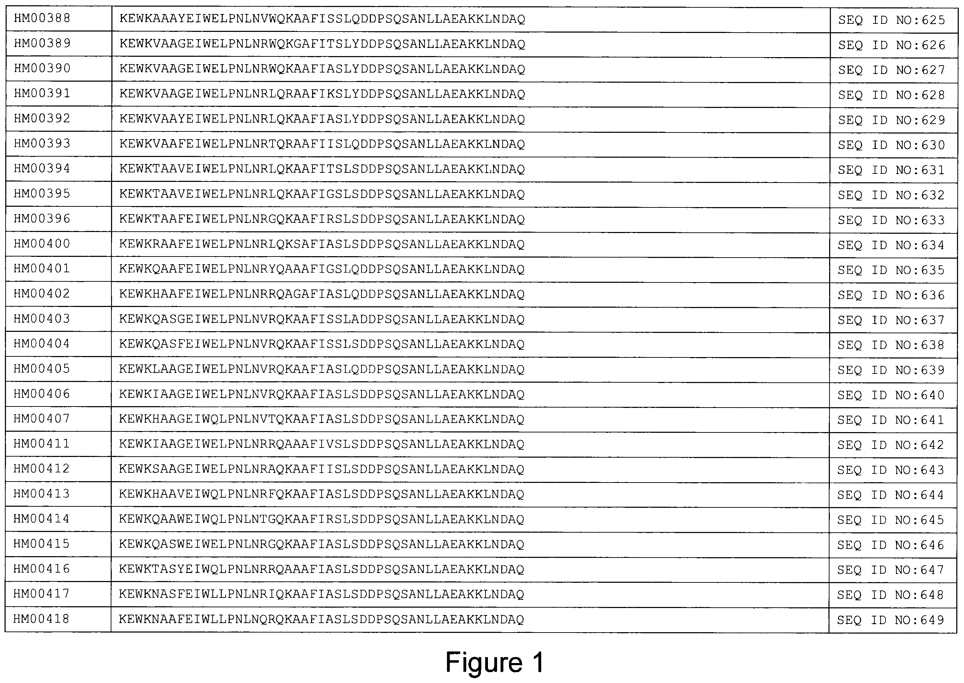

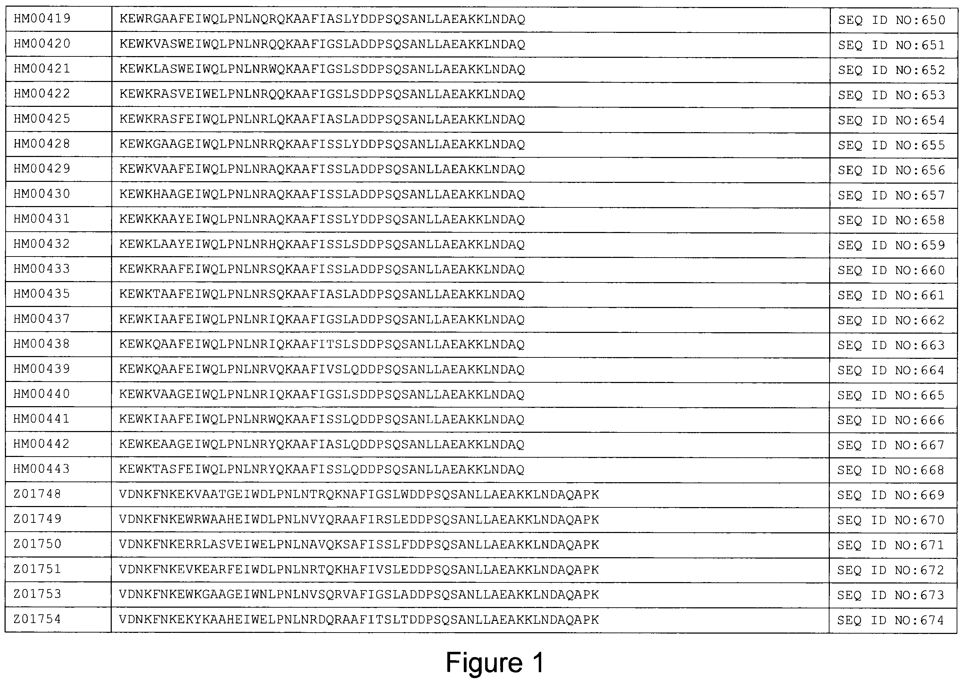

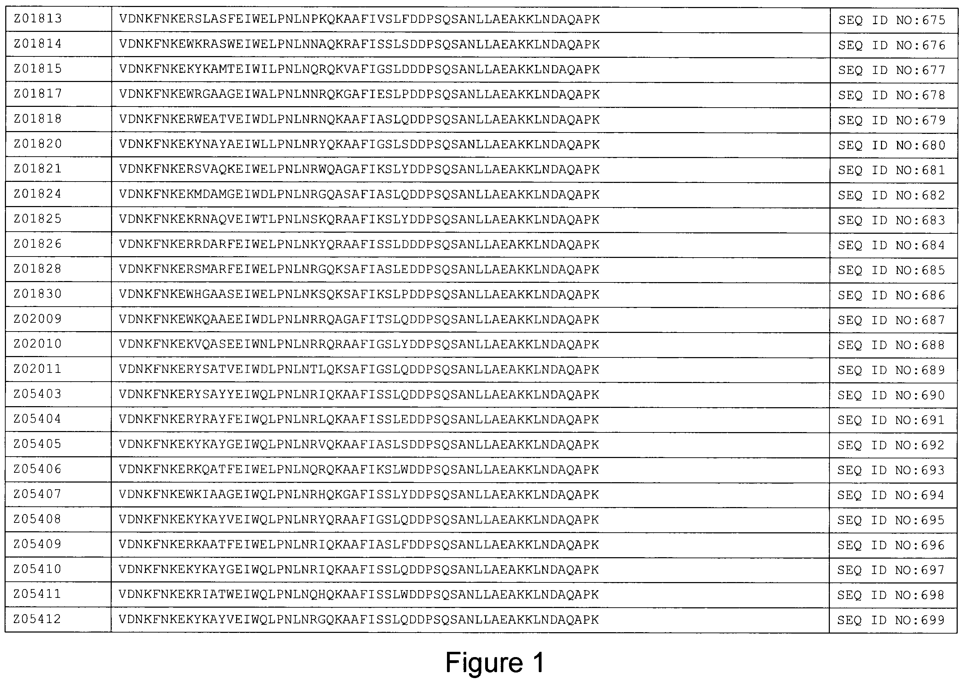

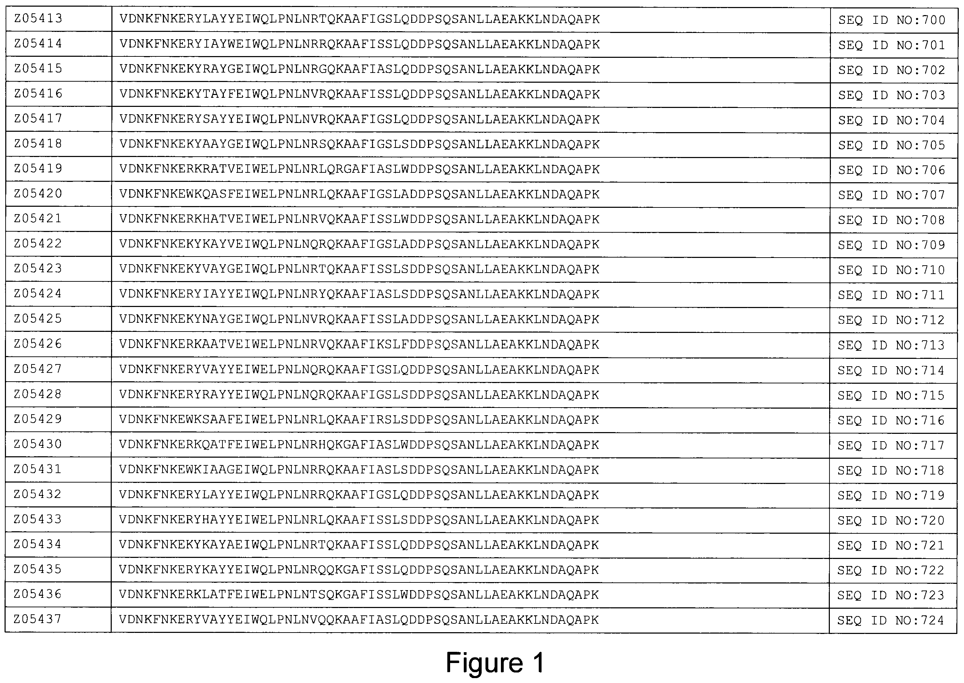

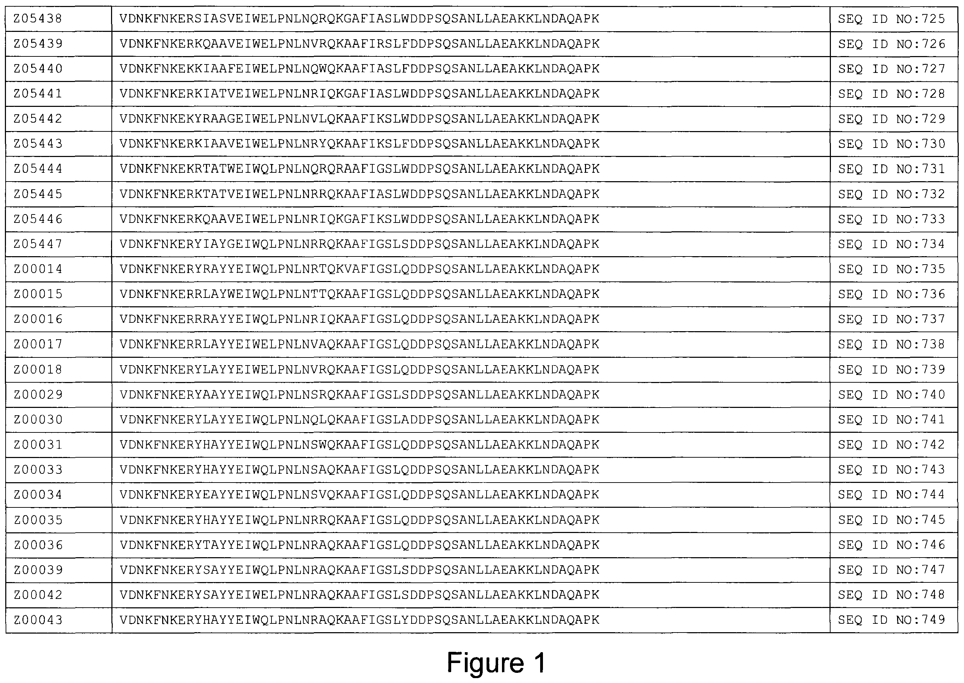

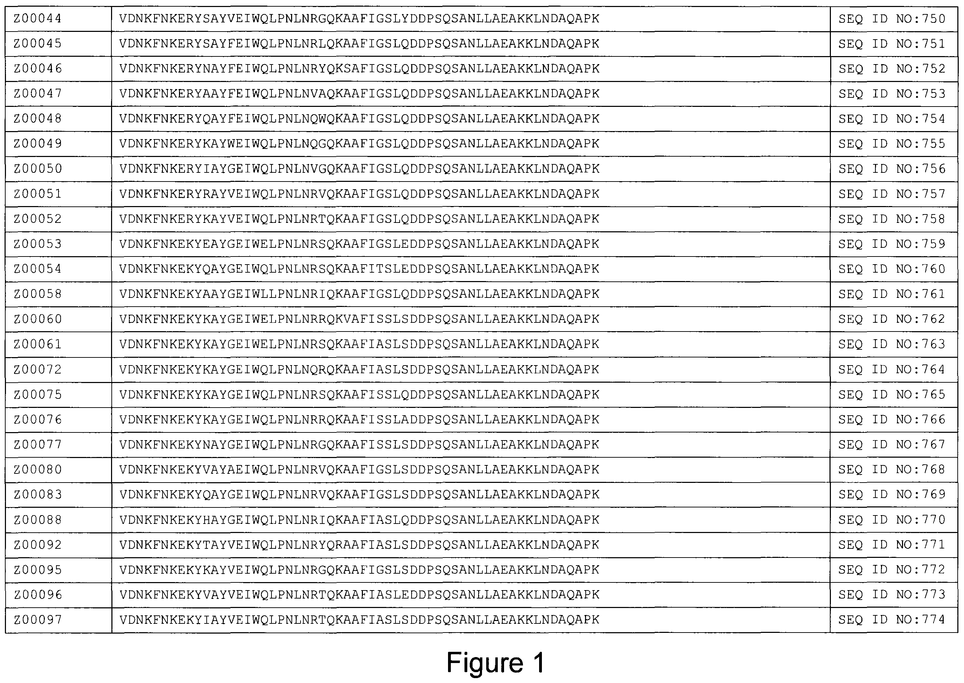

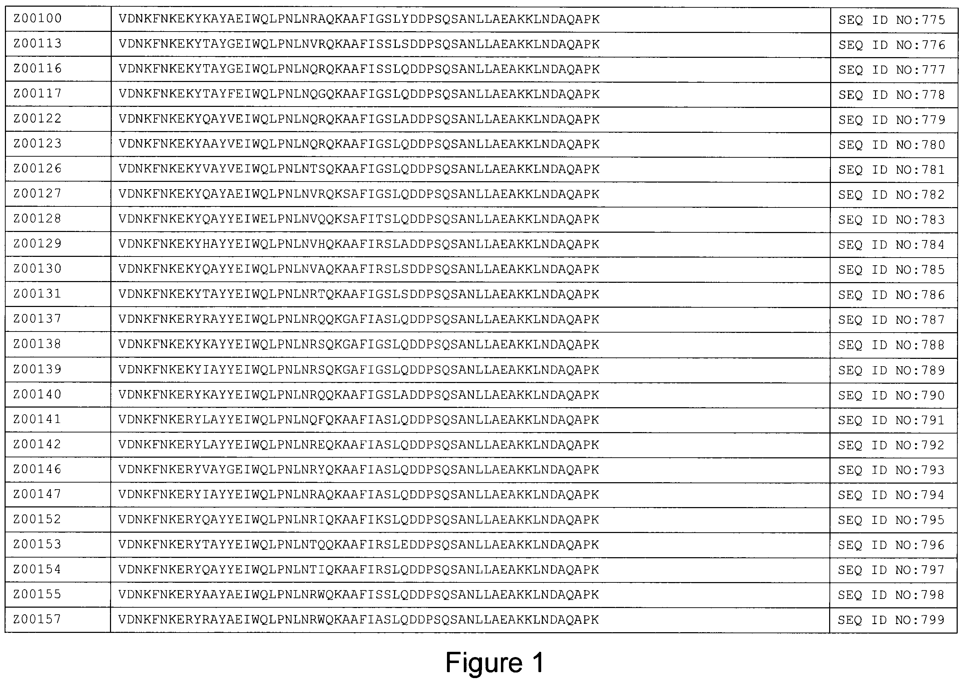

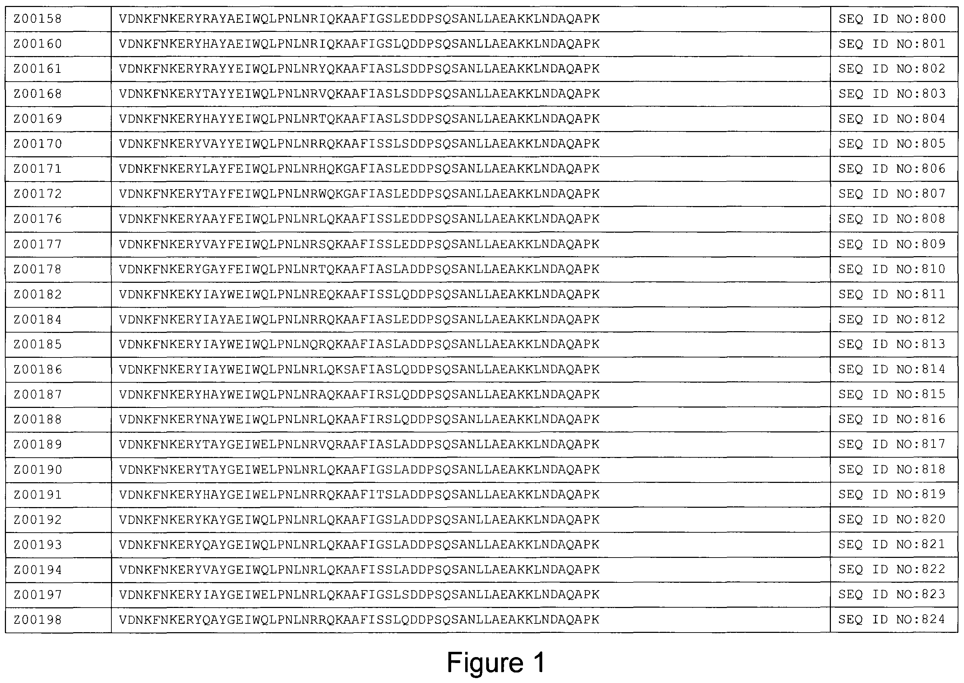

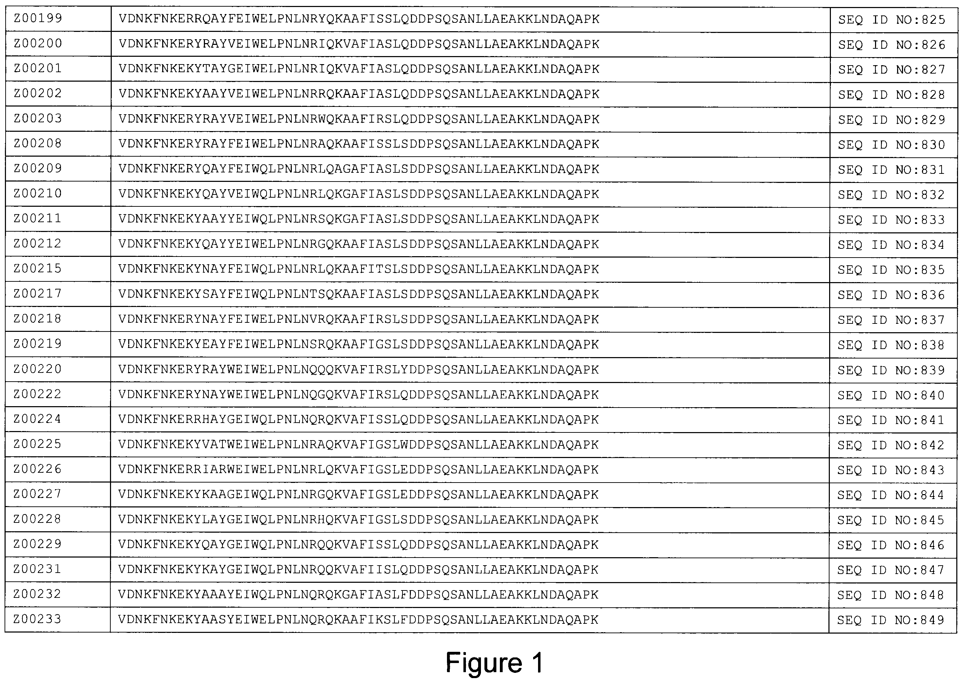

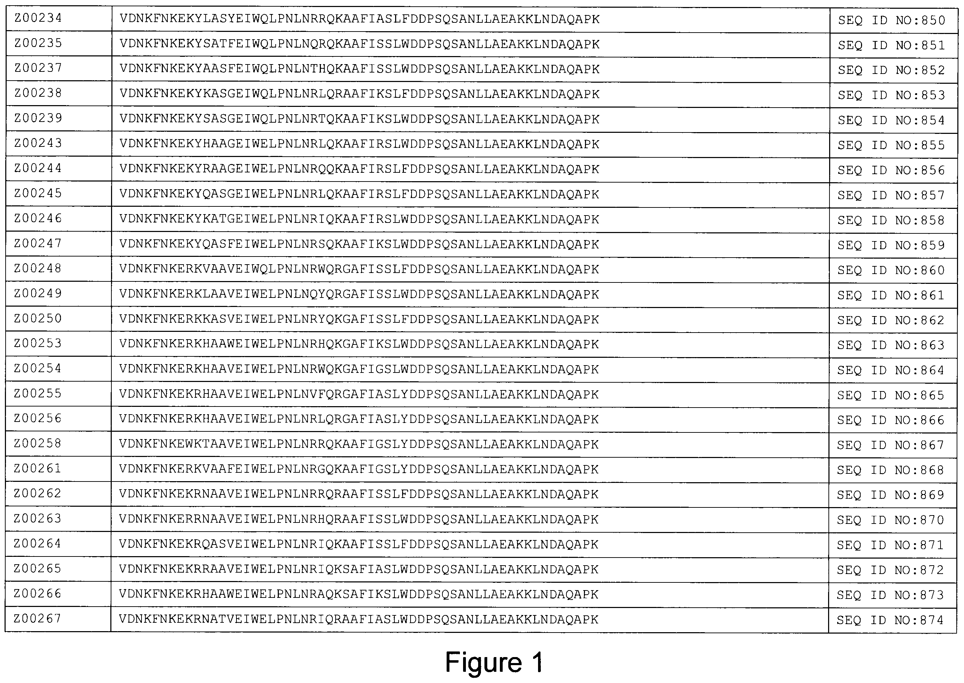

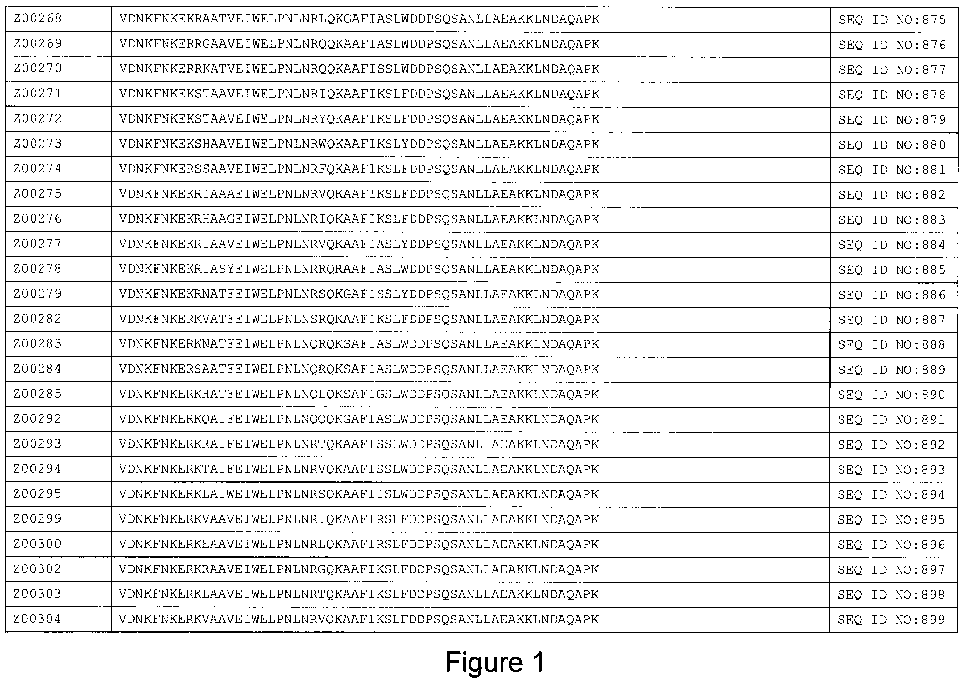

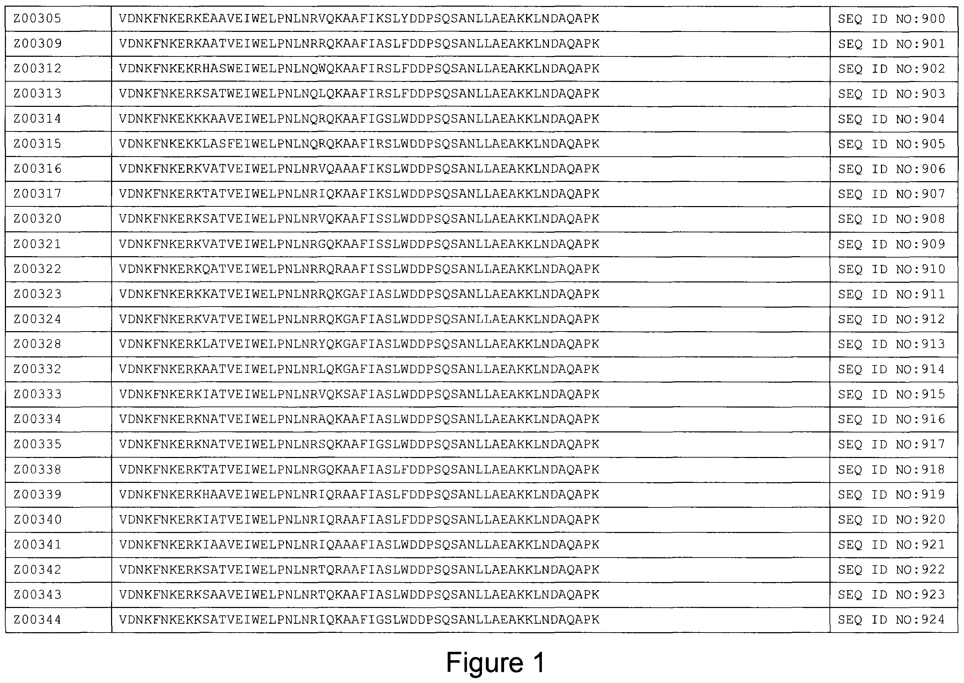

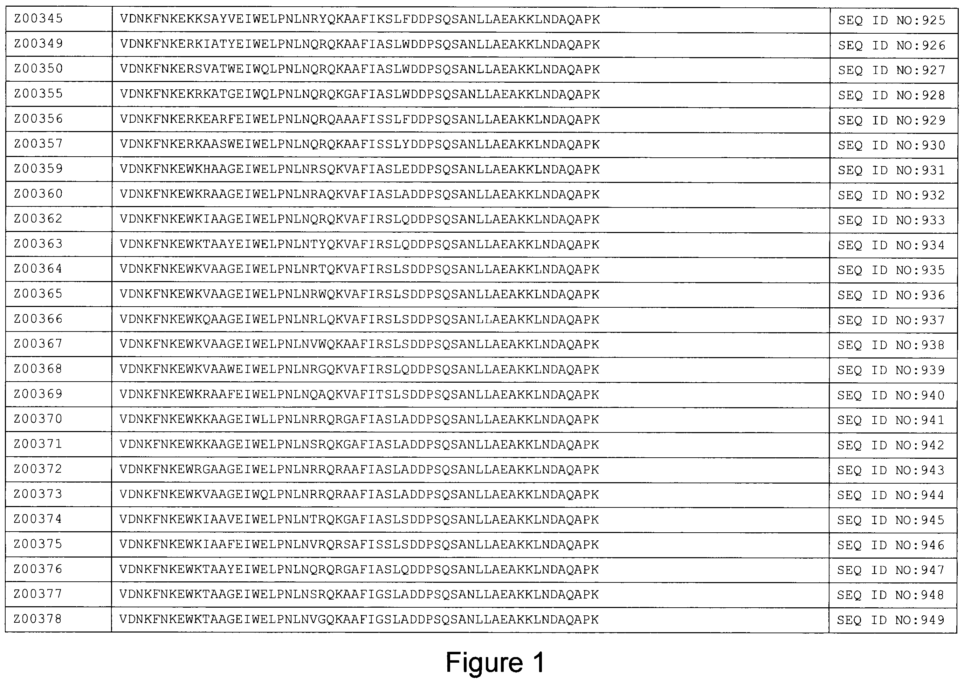

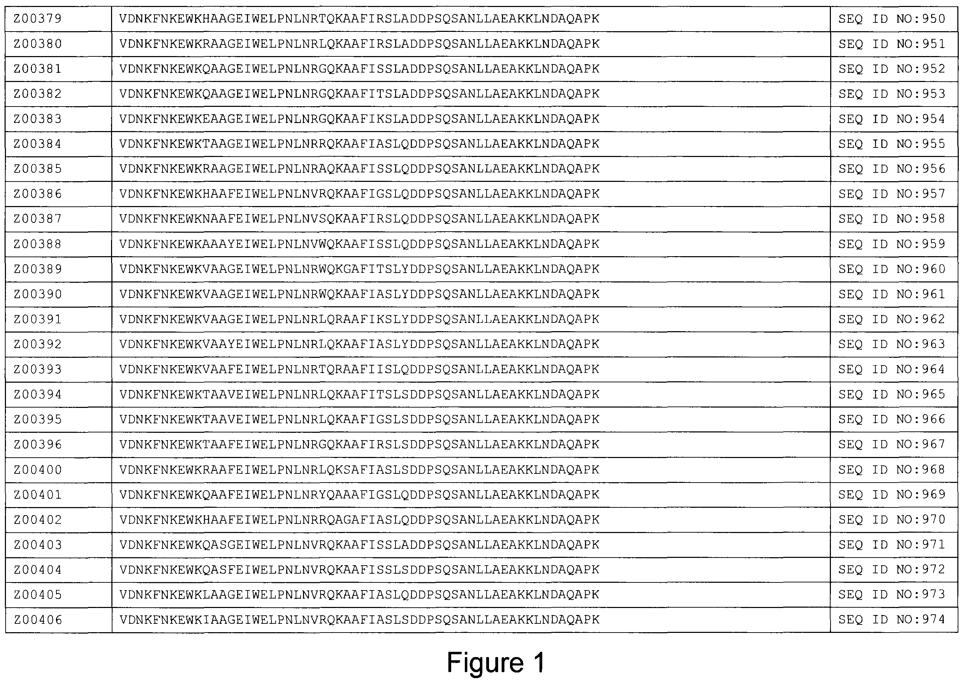

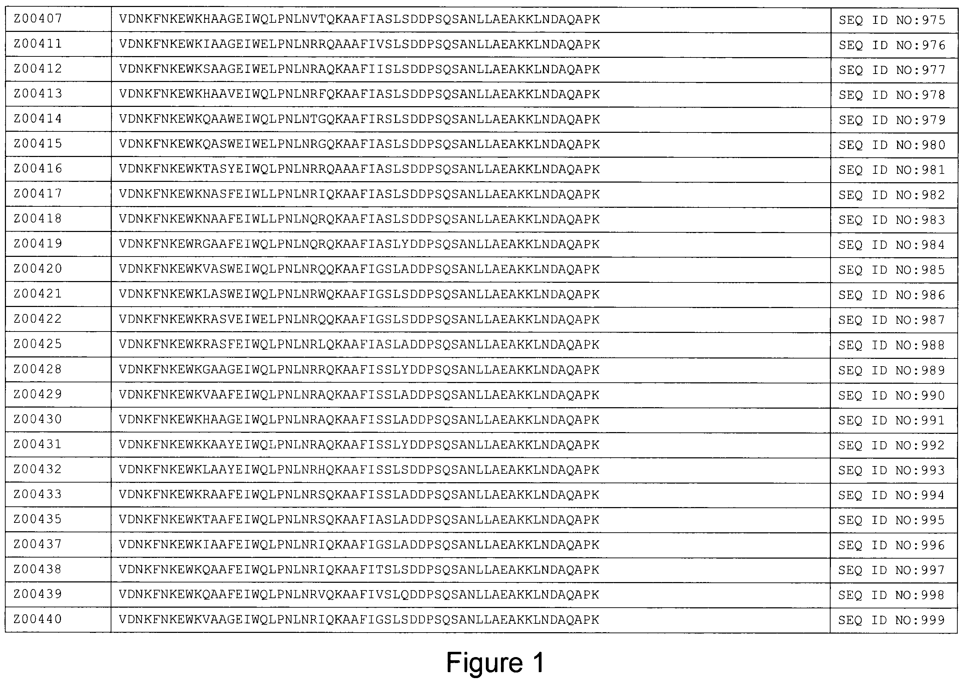

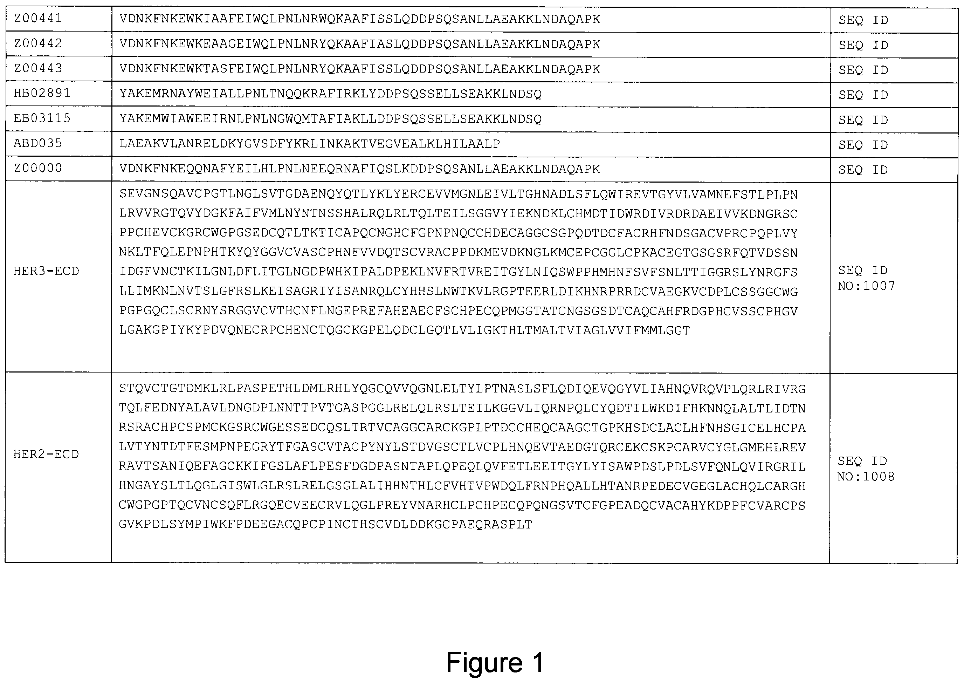

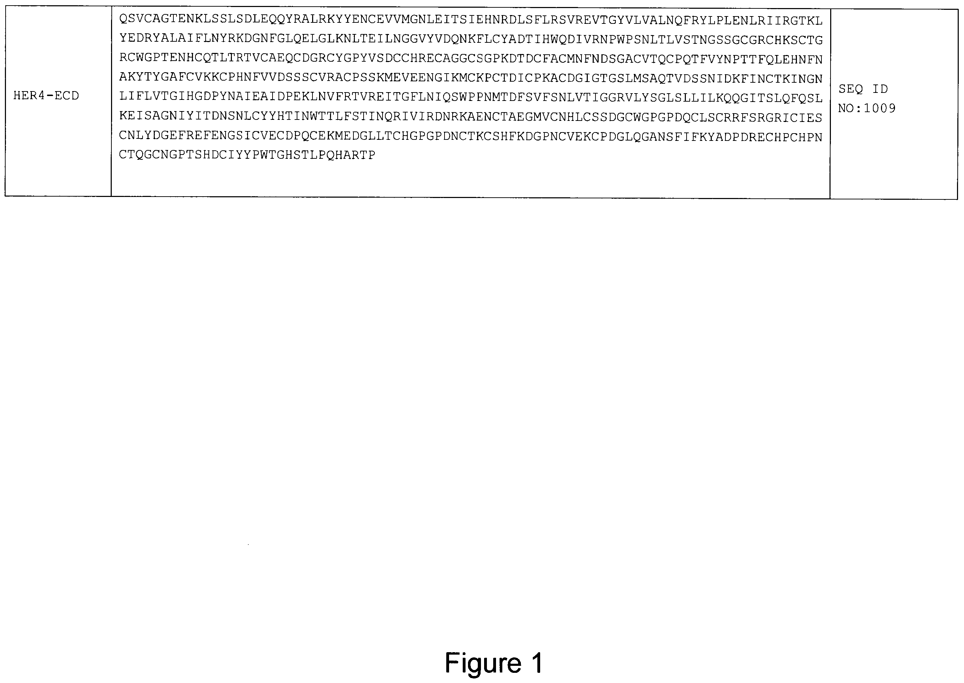

As described in detail in the experimental section to follow, the selection of HER3 binding variants has led to the identification of individual HER3 binding motif (*BM*) sequences. These sequences constitute individual embodiments of HER3 binding polypeptides according to this aspect. The sequences of individual HER3 binding motifs are presented in Figure 1 and as SEQ ID NO:1-334. In some embodiments of this aspect, the *BM* sequence i) is selected from any one of SEQ ID NO:1 to SEQ ID NO:66, such as from any one of SEQ ID NO:22-66, such as from SEQ ID NO:23-25, SEQ ID NO:27-28, SEQ ID NO:32, SEQ ID NO:35-36, SEQ ID NO:40, SEQ ID NO:42, SEQ ID NO:44-45, SEQ ID NO:53-54 and SEQ ID NO:56.

In particular embodiments, the HER3 binding motif *(BM)* thus forms part of a three-helix bundle protein domain. For example, the *BM* may essentially constitute two alpha helices with an interconnecting loop, within said three-helix bundle protein domain. In these embodiments, the HER3 binding polypeptide of the description may comprise an amino acid sequence selected from:
i)

   K-[*BM*]-DPSQS XₐX_{b}LLX_{c} EAKKL NDX_{d}Q;

   wherein
   [*BM*] is a HER3 binding motif as defined above;
   Xₐ is selected from A and S;
   X_{b} is selected from N and E;
   X_{c} is selected from A, S and C;
   X_{d} is selected from A and S;
   and
ii) an amino acid sequence which has at least 80 % identity to any one of the sequences defined above. Said amino acid sequence may have at least 82 %, such as at least 84 %, such as at least 86 %, such as at least 88 %, such as at least 90 %, such as at least 92 %, such as at least 94 %, such as at least 96 %, such as at least 98 % identity to any one of the sequences defined above.

In one embodiment of the HER3 binding polypeptide as described above, Xₐ is A; X_{b} is N; X_{c} is A and X_{d} is A.

In a further embodiment of the HER3 binding polypeptide as described above, Xₐ is A; X_{b} is N; X_{c} is C and X_{d} is A.

In a further embodiment of the HER3 binding polypeptide as described above, Xₐ is S; X_{b} is E; X_{c} is Sank X_{d} is S.

In a further embodiment of the HER3 binding polypeptide as described above, Xₐ is S; X_{b} is E; X_{c} is C and X_{d} is S.

In yet a further embodiment, the amino acid sequence of the HER3 binding polypeptide as defined above is selected from SEQ ID NO:335-668, in particular from SEQ ID NO:335-400, such as from SEQ ID NO:356-400,
such as from SEQ ID NO:357-359, SEQ ID NO:361-362, SEQ ID NO:366, SEQ ID NO:369-370, SEQ ID NO:374, SEQ ID NO:376, SEQ ID NO:378-379, SEQ ID NO:387-388 and SEQ ID NO:390.

In particular embodiments, said three-helix bundle protein domain is selected from domains of bacterial receptor proteins. Non-limiting examples of such domains are the five different three-helical domains of Protein A from *Staphylococcus aureus,* such as domain B, and derivatives thereof. In some embodiments, the three-helical bundle protein domain is a variant of protein Z, which is derived from said domain B of staphylococcal Protein A.

Thus, in a further embodiment, there is provided a HER3 binding polypeptide which comprises an amino acid sequence selected from:
i)

   YAK-[*BM*]-DPSQS SELLX_{c} EAKKL NDSQA P;

   wherein [*BM*] is a HER3 binding motif as defined above and
   X_{c} is selected from S and C; and
ii) an amino acid sequence which has at least 80 % identity to any one of the sequences defined in i) above.

Alternatively, there is provided a HER3 binding polypeptide which comprises an amino acid sequence selected from:
i)

   FNK-[*BM*]-DPSQS ANLLX_{c} EAKKL NDAQA P;

   wherein [*BM*] is a HER3 binding motif as defined above and
   X_{c} is selected from A and C; and
ii) an amino acid sequence which has at least 80 % identity to any one of the sequences defined in i) above.

In some embodiments, the HER3 binding polypeptides as defined above may for example have a sequence which is at least 82 %, at least 83 %, at least 85 %, at least 87 %, at least 89 %, at least 91 %, at least 92 % at least 94 %, at least 96 % or at least 98 % identical to the sequence described herein.

In some embodiments and as disclosed in the Experimental section below, the HER3 binding motif may form part of a 58 amino acid polypeptide. Such a polypeptide may e.g. comprise a sequence selected from any one of SEQ ID NO:669-1002, in particular a sequence selected from SEQ ID NO:669-734, such as selected from SEQ ID NO:690-734, such as from SEQ ID NO:691-693, SEQ ID NO:695-696, SEQ ID NO:700, SEQ ID NO:703-704, SEQ ID NO:708, SEQ ID NO:710, SEQ ID NO:712-713, SEQ ID NO:721, SEQ ID NO:722 and SEQ ID NO:724.

A HER3 binding polypeptide according to any aspect described herein may bind to HER3 such that the K_{D} value of the interaction is at most 1 x 10⁻⁶ M, or such as at most 1 x 10⁻⁷ M, such as at most 1 x 10⁻⁸ M. The terms HER3 binding" and "binding affinity for HER3" as used in this specification refers to a property of a polypeptide which may be tested for example by the use of surface plasmon resonance technology, such as in a Biacore instrument (GE Healthcare). HER3 binding affinity may e.g. be tested in an experiment in which HER3 is immobilized on a sensor chip of a Biacore instrument, and the sample containing the polypeptide to be tested is passed over the chip. Alternatively, and as described in the Examples below, HER3 binding affinity may be tested in an on-cell experiment. An on-cell experiment may for example be performed by incubating varying concentrations of labeled HER3 with cells displaying the polypeptide to be tested. The cell populations may thereafter be analyzed in a flow cytometer, such as in a fluorescence activated cell-sorting system (FACS), and the mean fluorescence intensity data may be determined. Mean fluorescence may subsequently be plotted against the HER3 concentrations and fitted to a one-site binding model in order to determine the apparent equilibrium dissociation constant, K_{D}. The HER3 or fragment thereof used in the K_{D} determination may for example comprise the amino acid sequence represented by SEQ ID NO:1007 (HER3-ECD).

As accounted for in the following Examples, selection of HER3 binding variants may for example be achieved by phage display for selection of naïve variants of a protein scaffold optionally followed by affinity maturation and cell display for selection of affinity maturated HER3 binding variants. It is however understood that any selection system, whether phage-based, cell-based or other, may be used for selection of HER3 binding polypeptides. In the following Examples, an expression system for cell surface display of recombinant proteins on the Gram-positive bacterium *Staphylococcus carnosus* was used (Jonsson et al, Biotechnol Applied Biochem 54:93-103 (2009)). As compared to the size of a phage particle, the larger size of a cell however generally enables use of fluorescence-activated cell sorting (FACS) for high-throughput screening and selection from cell-displayed protein libraries.

As compared to the IgG molecule, the HER3 binding polypeptides of the invention are relatively small, which, apart from rendering the HER3 binding polypeptides useful in a wide range of applications, may enable production using solid-phase peptide synthesis. A solid-phase synthesis route may e.g. facilitate site-specific conjugation of non-biological groups such as chelators used for radiolabeling in molecular imaging.

A HER3 binding polypeptide according to any aspect may comprise further C terminal and/or N terminal amino acids. Such a polypeptide should be comprehended as a polypeptide having additional amino acids residues at the very first and/or the very last position in the polypeptide chain, i.e. at the N- and/or C-terminus. Thus, a HER3 binding polypeptide may comprise any suitable number of additional amino acid residues, for example at least one additional amino acid residue. Each additional amino acid residue may individually or collectively be added in order to, for example, improve production, purification, stabilization *in vivo* or *in vitro,* coupling, or detection of the polypeptide. Such additional amino acid residues may comprise one or more amino acid residues added for the purpose of chemical coupling. One example of this is the addition of a cysteine residue. Such additional amino acid residues may also provide a "tag" for purification or detection of the polypeptide such as a His₆ tag or a "myc" (c-myc) tag or a "FLAG" tag for interaction with antibodies specific to the tag or immobilized metal affinity chromatography (IMAC) in the case of the His₆-tag.

The further amino acids as discussed above may be coupled to the HER3 binding polypeptide by means of chemical conjugation (using known organic chemistry methods) or by any other means, such as expression of the HER3 binding polypeptide as a fusion protein.

The further amino acids as discussed above may for example comprise one or more polypeptide domain(s). A further polypeptide domain may provide the HER3 binding polypeptide with another function, such as for example another binding function, or an enzymatic function, or a toxic function (e.g. an immunotoxin), or a fluorescent signaling function, or combinations thereof.

In some embodiments, the further polypeptide domain(s) may comprise a half life extending moiety which increases half life of the HER3 binding polypeptide *in vivo.* As understood by the skilled person, increased, or extended, half life means slowed clearance of a particular molecule from blood. A half life extending moiety may for example comprise a peptide or protein that will allow *in vivo* association to serum albumins. In particular, the half life extending moiety may be an albumin binding moiety. An albumin binding moiety may e.g. consist of a naturally occurring polypeptide, or an albumin binding fragment thereof, or an engineered polypeptide. An engineered polypeptide may be derived from a naturally occurring starting polypeptide through subjecting it to protein engineering techniques, such as mutations and alterations in a site-directed or randomized approach, with a view to create novel or enhanced properties, such as binding affinity for a molecule such as albumin.

Such an engineered albumin binding polypeptide may for example be a variant of a protein scaffold, which variant has been selected for its specific binding affinity for albumin. In a specific embodiment, the protein scaffold may be selected from domains of streptococcal Protein G or derivatives thereof, such as for example domain GA1, domain GA2 and domain GA3 of Protein G from *Streptococcus* strain G148, in particular domain GA3. Accordingly, in one embodiment of the HER3 binding polypeptide, the further amino acids comprise an albumin binding domain (ABD) of streptococcal Protein G, or a derivative thereof. One example of an albumin binding domain which may be comprised as a further polypeptide domain in the HER3 binding polypeptide of the invention is set out in SEQ ID N0:1005. Other examples of suitable albumin binding domains are disclosed in WO2009/016043. Such an ABD-extended polypeptide binds to serum albumin *in vivo,* and benefits from its longer half life, which increases the net half life of the polypeptide itself (see e.g. WO91/01743).

When a HER3 binding polypeptide according to the invention comprises an albumin binding moiety as defined above, the overall size of the HER3 bindnig polypeptide is relatively small. When administered for example to a mammalian subject, such as a human subject, the albumin binding part of the HER3 binding polypeptide will associate non-covalently with serum albumin and the polypeptide may thereby benefit from decreased renal clearance and increased recirculation in epithelial cells. Tissue penetration may however still be fast due to extravasating properties of serum albumin. Furthermore, a HER3 binding polypeptide comprising a half life extending moiety may not only display an extended half life *in vivo,* but also a reduced immunologic response *in vivo,* as compared to a polypeptide lacking a corresponding half life extending moiety (see e.g. WO2005/097202).

A further polypeptide domain may moreover provide the HER3 binding polypeptide with the same binding function. Thus, in a further embodiment, there is provided a HER3 binding polypeptide comprising at least two HER3 binding polypeptide monomer units, the amino acid sequences of which may be the same or different. Multimeric forms of the polypeptides may comprise a suitable number of domains, each having a HER3 binding motif, and each forming a "monomer" within the multimer. These domains may all have the same amino acid sequence, but alternatively, they may have different amino acid sequences. In particular, the HER3 binding polypeptide of the invention may form homo- or heterodimers.

The further polypeptide domain(s) as described above may be joined to the HER3 binding polypeptide by covalent coupling using known organic chemistry methods. Alternatively, the HER3 binding polypeptide comprising the further polypeptide domain(s) may be expressed as one or more fusion polypeptides, for example in a system for recombinant expression of polypeptides, or joined in any other fashion, either directly or via a linker, for example an amino acid linker.

In one embodiment of the HER3 binding polypeptide, the polypeptide binds to the same epitope as the naturally occurring HER3 ligands, or sufficiently close to it to block binding of the naturally occurring HER3 ligands, e.g. Neuregulins-1 and 2, to HER3. The HER3 binding polypeptide may thus be used as a HER3 blocking agent and/or to inhibit HER3 mediated cell signaling *in vivo.*

In a further aspect, there is provided a polynucleotide encoding a HER3 binding polypeptide as described above. An expression vector comprising such a polynucleotide may enable production of a HER3 binding polypeptide, for example by expression in a host cell.

A HER3 binding polypeptide may be used as an alternative to conventional antibodies or low molecular weight substances in various medical, veterinary and diagnostic applications. The skilled addressee will understand that the polypeptide of the invention may be useful in any method requiring affinity for HER3 of a reagent. Thus, the HER3 binding polypeptide may be used as a detection reagent, a capture reagent or a separation reagent in such methods. In addition, the HER3 binding polypeptide may be employed in diagnostics in order to reveal, diagnose and examine the presence of a disease characterized by HER3 expression on the cell surface in e.g. a mammalian subject. Thus, HER3 expression may diagnostically be monitored *in vivo* by use of a polypeptide of the invention. Diagnostic monitoring may for example be accomplished by delivery of diagnostic nuclides for assessment of HER3 expression by molecular imaging. A HER3 binding polypeptide according to the invention may also be useful as a therapeutic or diagnostic agent in its own right or as a means for targeting other therapeutic or diagnostic agents, with direct (e.g. toxic molecules, including radionuclides such as yttrium-90, lutetium-177 and thorium-227, toxins, such as maytansinoid) or indirect (e.g. cancer vaccines, immunostimulatory molecules) effects on the HER3 protein. The therapeutic effect may potentially be mediated by blocking the receptor interaction, or alternatively by using the HER3 binding polypeptide as a target for delivering a potent payload to a tumor cell.

In a further aspect, there is provided a ligand having binding affinity for HER3 and for HER2, comprising a HER3 binding polypeptide as defined above; a HER2-binding polypeptide; and a linking moiety for linking the HER3 binding polypeptide with the HER2 binding polypeptide. Such a ligand should be comprehended as an example of a bispecific heterodimer comprising two different binding functions: one binding function for HER3, provided by a polypeptide as disclosed above, and one binding function for HER2, provided by a HER2 binding polypeptide. It is to be understood that any HER3 binding polypeptide according to the invention may be comprised in the ligand. The two binding functions are comprised in the ligand as two separate monomer units.

Such a bispecific ligand according to the invention may be useful in detecting, characterizing and/or diagnosing cancer that involves both HER3 and HER2. Detection, characterization and/or diagnosis may for example be accomplished by molecular imaging using gamma or positron emitting nuclides or by using near infrared fluorescent probes. As described above, HER3 is a preferred heterodimerization partner for HER2, and critical for driving the proliferation of tumors characterized by over-expression of HER2. HER2 lacks a known ligand and is currently believed to be dependent on other receptors in the HER family for ligand induced signaling. A bispecific ligand according to the invention may however be used not only for diagnostic but also for targeted therapeutic payload applications. Compounds used in payload applications include, among others, radionuclides, prodrug enzymes, cytokines, chemical toxic molecules, toxins and photosensitizers. For targeted payload applications, high discrimination between normal and diseased tissue is extremely important.

By fine tuning the affinity of both binding polypeptides comprised in the bispecific ligand binding two different receptors, the ligand may preferentially locate to and accumulate only in lesions expressing both receptors simultaneously. Accordingly, HER2 and HER3 binding functions may provide the ligand with two equally strong binding functions for their individual targets. Thus, the binding affinity of the HER3 binding polypeptide for HER3 may be approximately of the same order as the binding affinity of the HER2 binding polypeptide for HER2. In one embodiment, the HER2 binding polypeptide may bind to HER2 such that the K_{D} value of the interaction is at most 1 x 10⁻⁶ M, such as at most 1 x 10⁻⁷ M, such as at most 1 x 10⁻⁸ M. In another embodiment, binding of the HER2 binding polypeptide to HER2 may be as strong or stronger than the corresponding binding of the HER3 binding polypeptide to HER3. Thus, the K_{D} value of the interaction between the HER2 binding polypeptide and HER2 is no more than the K_{D} value of the interaction between the HER3 binding polypeptide and HER3. The K_{D} value of the interaction between the HER3 binding polypeptide and HER3 may for example be 5 times, 10 times or 100 times the K_{D} value of the interaction between the HER2 binding polypeptide and HER2.

In one embodiment, the HER2 binding polypeptide of the bispecific ligand is an engineered polypeptide. The HER2 binding polypeptide may be an engineered polypeptide which is a variant of a protein scaffold, which variant has been selected for its specific binding affinity for HER2. Non-limiting examples of a protein scaffold are selected from the group consisting of staphylococcal protein A, and domains and derivatives thereof. In some embodiments, the HER2 binding polypeptide is an engineered protein derived from domain B of Protein A from *Staphylococcus aureus.* The engineered protein may in particular comprise a protein Z derivative, which is a variant of protein Z which is derived from said domain B of Protein A.

In some embodiments of the ligand, the engineered HER2 binding protein comprises an amino acid sequence selected from
i) YAKEM RNAYW EIALL PNLTN QQKRA FIRKL YDDPS QSSEL LSEAK KLNDS Q, corresponding to SEQ ID NO:1003; and
ii) an amino acid sequence which has at least 80 % identity to the sequence defined in i).

Linking of the HER3 binding polypeptide and the HER2 binding polypeptide may for example be provided by a peptide linker comprising from 1 up to 45 amino acids. A linking moiety may in particular be arranged for coupling the N- and/or C-terminals of the HER3 binding polypeptide and the HER2 binding polypeptide. The linking thus provided may be selected from C-to C-terminal linking, C- to N-terminal linking or N- to C-terminal linking. Coupling of the linking moiety to the HER3 binding polypeptide and the HER2 binding polypeptide may e.g. be accomplished by means of chemical conjugation (using known organic chemistry methods) or by any other means, such as expression of the ligand as a fusion protein.

The ligand may, as described above, further comprise a half life extending moiety for extension of ligand half life *in vivo.* Half life extension may be accomplished by a number of different half life extending moieties, and non-limiting examples of such moieties are selected from albumin, albumin binding moieties as defined above in connection with the HER3 binding polypeptide, the Fc-part from IgG, and polymeric compounds such as polyethylene glycol (PEG). Coupling of the ligand to serum albumin, which is the most abundant protein in mammalian sera, may provide extension of ligand half life *in vivo.*

In particular, the albumin binding moiety may be an engineered protein derived from domain GA3 of streptococcal Protein G, such as an albumin binding moiety comprising an amino acid sequence selected from
i) LAEAK VLANR ELDKY GVSDF YKRLI NKAKT VEGVE ALKLH ILAAL P; as set out in SEQ ID NO:1005; and
ii) an amino acid sequence which has at least 80 % identity to the sequence defined in i).

The half life extending moiety may for example be connected to the remainder of the ligand by covalent coupling using known organic chemistry methods, or joined in any other fashion, directly or mediated by a linker comprising a number of amino acids. When the half life extending moiety is represented by albumin or an albumin binding moiety, the ligand may in itself be expressed as one or more fusion polypeptides in a system for recombinant expression of polypeptides. In one embodiment, the half life extending moiety is attached to the remainder of the ligand via a cysteine residue present in the ligand. In particular, the half life extending moiety may be attached to a cysteine residue of the HER3 or the HER2 binding polypeptide, introduced at a site distant from the binding motif. In another embodiment, the half life extending moiety is attached to the remainder of the ligand via the linking moiety.

In a specific embodiment, the linking moiety comprises a half life extending moiety as defined above. Thus, albumin, albumin binding moieties or PEG may for example be employed for connecting the HER3 binding polypeptide with the HER2 binding polypeptide.

In some embodiments, a ligand according to the invention may comprise further C terminal or N terminal amino acids. Such further amino acids may, as described above as regards the HER3 binding polypeptide, individually or collectively be added in order to for example, improve production, purification, stabilization *in vivo* or *in vitro,* coupling, or detection of the polypeptide. In certain embodiments, the further amino acids may constitute one or more polypeptide domain(s). The function of such a further polypeptide domain may be the same as, or different from, the functions already comprised in the ligand. A further polypeptide domain may for example provide the ligand with another HER3 binding function and/or another HER2 binding function. Thus, a bispecific ligand according to the invention may comprise multimers of HER3 binding functions and/or multimers of HER2 binding functions. One example of bispecific ligand comprising multimeric binding functions is a ligand comprising two HER3 binding polypeptides and two HER2 binding polypeptides.

A ligand as defined above may, in one embodiment, prevent dimerization of HER2 and HER3 *in vivo.* More specifically, the ligand may, e.g. by binding to HER2 and/or HER3 expressed on a cell surface in a mammalian subject, inhibit HER3 mediated signaling in the subject. Due to the bispecific nature of the ligand, it may bind to and block the two receptors in a position such that they are no longer able to exert signaling. As the skilled addressee will understand, a ligand according to the invention may accordingly be utilized for the treatment of conditions associated with expression, and in particular over-expression, of HER2 and/or HER3 on the cell surface.

HER3 can however also form heterodimers with EGFR, thus forming an active signaling unit activating the PI3K/Akt pathway (Engelman et al, Proc Natl Acad Sci 102(10):3788-93 (2005)). Indeed, HER3 is over-expressed in non-small cell lung cancer (NSCLC), apparently because of up-regulation of ErbB ligands (Fujimoto et al, Cancer Res 65(24):11478-85 (2005)), whereas HER3 is not expressed in normal lung epithelium. Thus, in a related aspect, there is provided a ligand having binding affinity for HER3 and for EGFR, comprising a HER3 binding polypeptide as defined above; an EGFR binding polypeptide; and a linking moiety for linking the HER3 binding polypeptide with the EGFR binding polypeptide. In particular, such a bispecific ligand may target HER3 and EGFR and exert similar, or the same, modes of action as described above for the bispecific ligand binding to HER3 and HER2. Examples of a bispecific ligand binding to HER3 and EGFR may comprise an EGFR binding polypeptide selected from
i) YAKEM WIAWE EIRNL PNLNG WQMTA FIAKL LDDPS QSSEL LSEAK KLNDS Q, corresponding to SEQ ID NO:1004; and
ii) an amino acid sequence which has at least 80 % identity to the sequence defined in i).

Other examples of EGFR binding polypeptides that may be used in a HER3 and EGFR binding ligand are disclosed in WO2007/065635.

In other embodiments, the HER3 binding polypeptide or bispecific ligand according to the invention may be used in targeting therapeutic or diagnostic agents to cells expressing HER3, both *in vivo* and *in vitro,* particularly to cells which over-express HER3. As discussed above, a ligand according to the invention may be utilized in targeting cells that express, or over-express, HER3 and/or HER2, or HER3 and/or EGFR. In a related aspect, there is thus provided a combination of a HER3 binding polypeptide as defined herein, or a ligand as defined above, and a therapeutic agent. Non-limiting examples of therapeutic agents are selected from cytotoxic payloads, such as radionuclides, small molecular drugs or toxins. Such therapeutic agents may be chemically conjugated or otherwise bound to the HER3 binding polypeptide or ligand.

A combination, a HER3 binding polypeptide or a ligand according to the invention, may furthermore be used as a medicament in therapy, for example for the treatment of a HER3 related condition, such as a HER3 related condition in a mammal, such as a human subject, selected from cancer disease, such as colon cancer, endometrial cancer, gastric cancer, glioma, breast cancer, pancreas cancer, head and neck squamous carcinoma, lung cancer, melanoma, medulloblastoma, neuroepithelioma, ovarian cancer, Paget's disease, papillary thyroid cancer, prostate cancer, skin squamous cell carcinoma, transitional cell carcinoma and vestibular schwannoma.

In a related aspect of the disclosure, there is provided a method of treatment of a HER3 related condition, comprising administering of a HER3 binding polypeptide, or ligand or combination as described above to a mammalian subject in need thereof. Consequently, in the method of treatment, the subject is treated with a HER3 binding polypeptide, a ligand or a combination according to the invention. In a more specific embodiment of said method, the binding of the HER3 binding polypeptide, the ligand or the combination to a HER3 expressed on a cell surface in the subject inhibits receptor activation. Said binding may for example inhibit HER3 mediated signaling. In one embodiment of the method of treatment, said HER3 related condition is selected from cancer disease, such as colon cancer, endometrial cancer, gastric cancer, glioma, breast cancer, pancreas cancer, head and neck squamous carcinoma, lung cancer, melanoma, medulloblastoma, neuroepithelioma, ovarian cancer, Paget's disease, papillary thyroid cancer, prostate cancer, skin squamous cell carcinoma, transitional cell carcinoma and vestibular schwannoma.

In another embodiment, there is provided a method of treatment of a HER3 and/or HER2 related condition, comprising administering of a ligand according to the invention to a mammalian subject in need thereof.

As understood by the skilled person and as discussed above, a HER3 binding polypeptide or a ligand according to the invention may be used in diagnosis, such as in the diagnosis of a HER3 or a HER3 and/or HER2 related condition. A HER3 binding polypeptide according to the invention may be employed separately or in combination with a diagnostic agent. Such a combination may be used in the diagnosis of a HER3 related condition, for example in molecular imaging of cells over-expressing HER3.

The invention will now be further illustrated by the following non-limiting Examples.

### Brief description of the figures

Figure 1 is a listing of the amino acid sequences of examples of HER3 binding motifs comprised in HER3 binding polypeptides of the invention (SEQ ID NO:1-334), examples of 49-mer HER3 binding polypeptides according to the invention (SEQ ID NO:335-668) and examples of 58-mer HER3 binding polypeptides according to the invention (SEQ ID NO:669-1002), an example of a HER2 binding polypeptide (SEQ ID NO:1003) comprised in a ligand according to the invention, an example of an albumin binding moiety (SEQ ID NO:1005) as may be comprised in one embodiment of a ligand according of the invention, an example of an EGFR binding polypeptide (SEQ ID NO:1004) which may be comprised in one embodiment of a ligand according to the invention, as well as the sequences of protein Z (SEQ ID NO:1006), and the sequences of the extracellular domain of HER3 (SEQ ID NO:1007), the extracellular domain of HER2 (SEQ ID NO:1008) and the extracellular domain of HER4 (SEQ ID NO:1009).

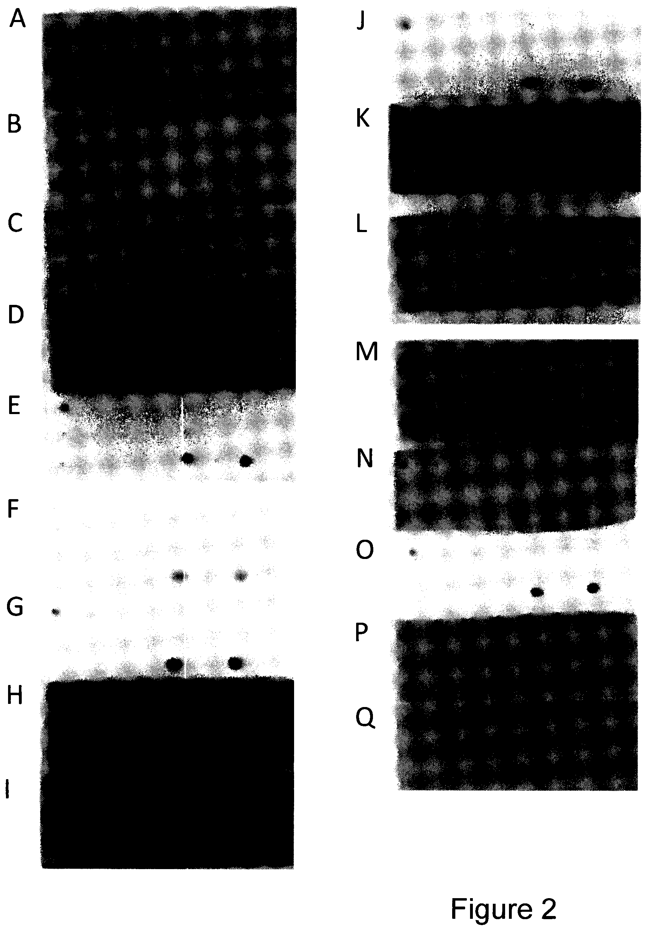

Figure 2A-Q show the result of a dot blot assay performed using periplasmic fractions from individual HER3 binding Z variants expressed as fusions to the HSA binding domain ABD. The potential background binding to 21 control proteins as well as binding to HER3-ECD and HER3-Fc were analyzed. Proteins were dotted on the membrane in three rows from left to right in the following order;
   Row 1: HSA, IgG, IgM, IgA, alpha-2 macroglobulin, fibrinogen, holo-transferrin, alpha-1-antitrypsin,
   Row 2: C3, haptoglobulin, alpha-1 acid glycoprotein, alpha-1 antichymotrypsin, C4, mouse IgE kappa, hemopexin, transthyretin,
   Row 3: streptavidin, neutravidin, HER2-ECD, HER3-Fc, HER4-Fc, HER3-ECD and HER4-ECD.

   The images show response of A) Z01748, B) Z01813, C) Z01814, D) Z01815, E) Z02009, F) Z01821, G) Z02010, H) Z01824, I) Z01825, J) Z01817, K) Z01818, L) Z01820, M) Z01826, N) Z01828, O) Z02011, P) Z01830, and Q) the product from pAY01247, which expresses only ABD, as a negative control.

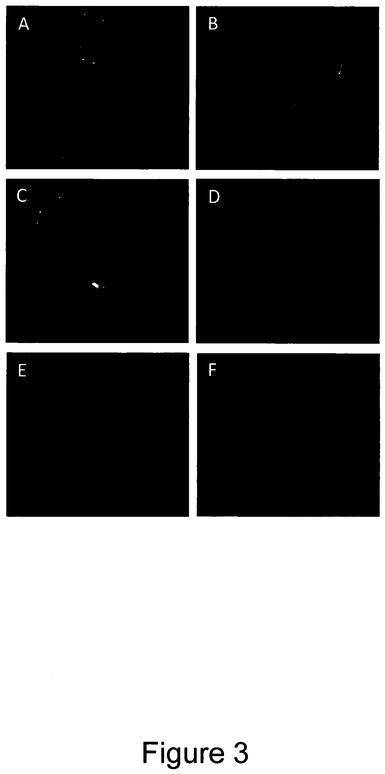

Figure 3A-F show images from the immunofluorescence experiment described in Example 2. HER3 positive AU566 cells (A-D) and HER2 positive and HER3 negative SKOV-3 cells (E-F) were stained with HER3 specific Z variants or controls according to the following: A) His₆-(Z01814)₂-cys, B) His₆-(Z01820)₂-cys, C) anti-HER3 antibody (positive control), D) (Z01154)₂ (negative control Z variant), E) Hi_{S6}-(Z01814)₂-cys, F) His₆-(Z01820)₂-cys.

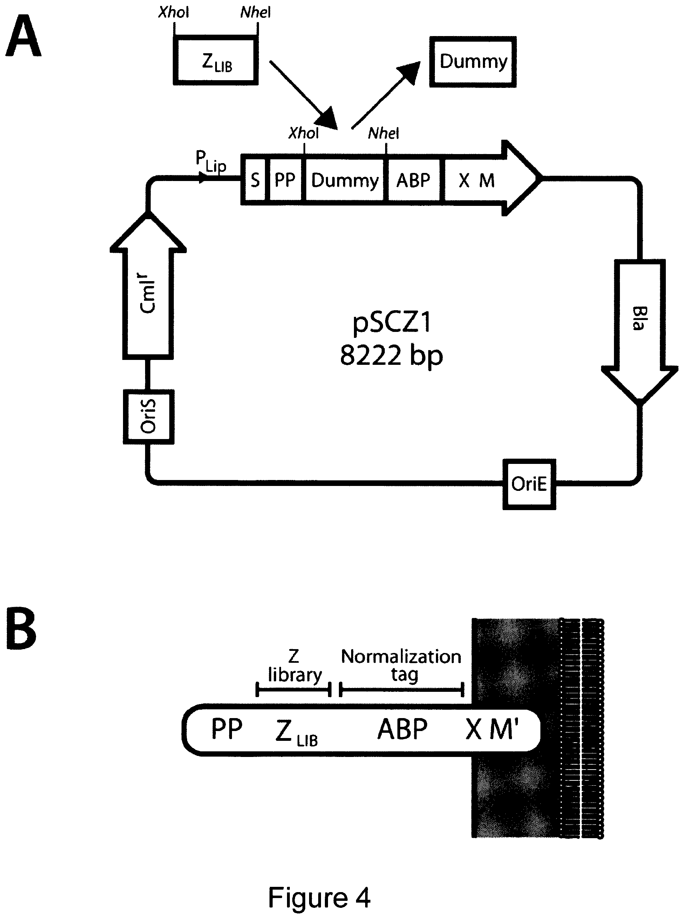

Figure 4 is a schematic representation of the staphylococcal display vector (A) used for cell display of HER3 binding polypeptides of the invention on the surface of *S*. *carnosus* (B). The vector pSCZ1 (A) contains: i) a promoter and secretion signal (S) from a lipase gene (expressed on the surface of the related *Staphylococcus hyicus),* ii) a gene fragment encoding a propeptide (PP) from the same lipase gene, which has been demonstrated to be important for efficient translocation of recombinant proteins, iii) a cell-wall anchoring region (XM), originating from staphylococcal Protein A, iv) an albumin binding protein (ABP, Samuelson et al, J Bacteriology 177(6):1470-1476, (1995)) from streptococcal Protein G introduced to provide surface expression level normalization of the target binding signal, v) a staphylococcal origin of replication and chloramphenicol acetyl transferase gene (Cml^{r}) for stable replication and expression in staphylococci, and vi) an origin of replication for *E. coli* (OriE) as well as a β-lactamase gene (Bla), facilitating subcloning work in *E. coli.*

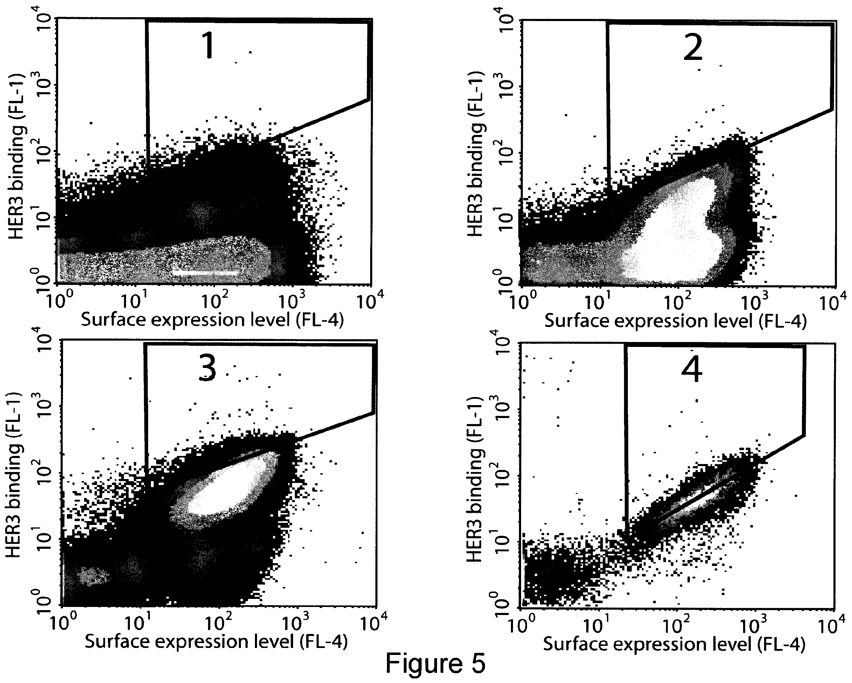

Figure 5 shows density plots as a result from flow-cytometric sortings of SC:Z_{HER3LIB}. The plots show FL-4 channel fluorescence intensity corresponding to surface expression level (monitored via HSA binding) on the x-axis and FL-1 channel fluorescence corresponding to HER3 binding on the y-axis. The density plots show the staphylococcal library before flow-cytometric sorting round 1, 2, 3 and 4, respectively, with the regions used in gating outlined as a box in each plot.

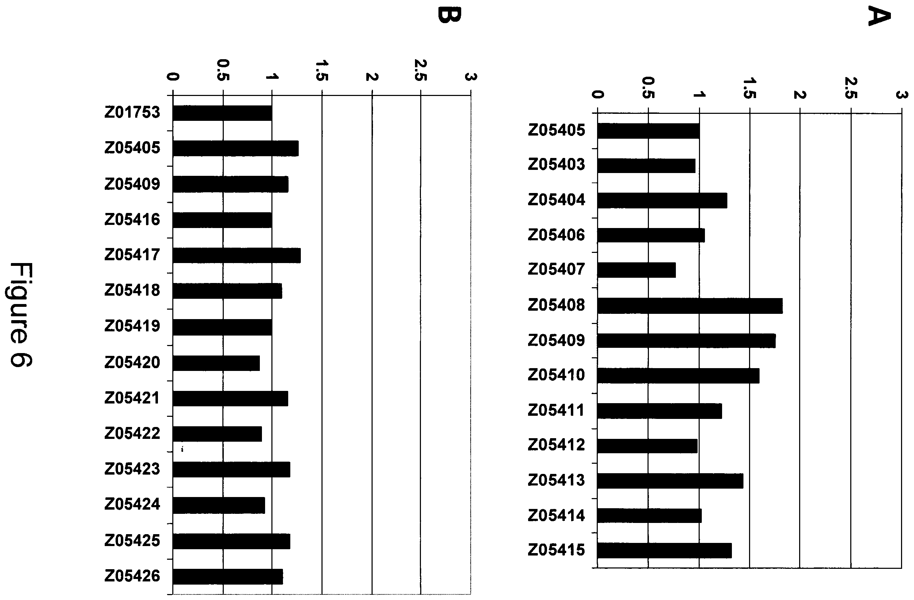

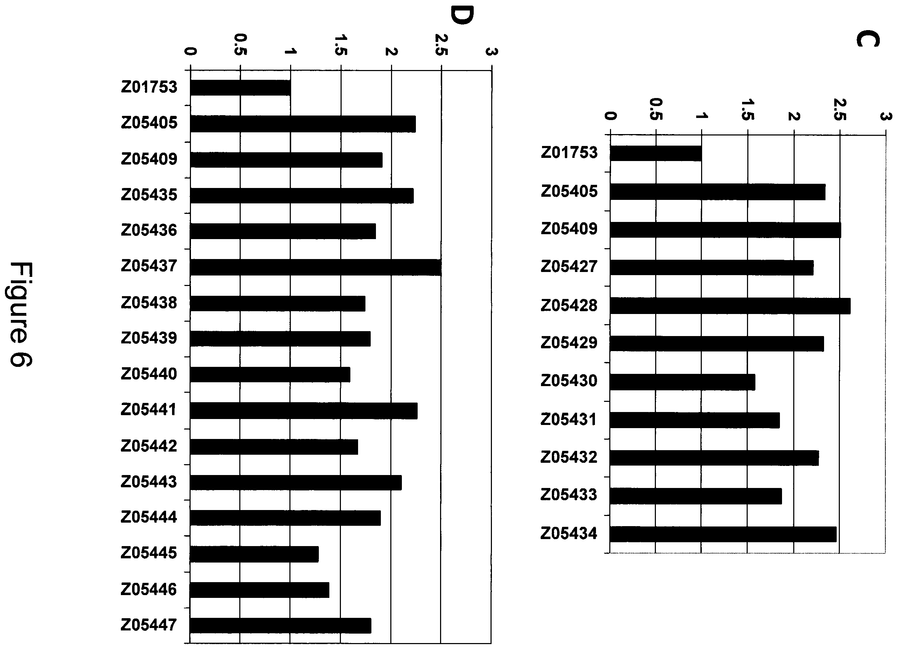

Figure 6A-D show histogram representations of a cell-based flow cytometry assay where FACS isolated cells displaying Z variants in fusion to an albumin binding protein (ABP) were incubated with fluorescently labeled HER3-Fc and fluorescently labeled HSA. The ratio of HER3 binding signal (MFI, FL-1) and HSA binding signal (MFI, FL-4) is on the y-axis. All FL-1/FL-4 ratios are normalized; A) with the FL-1/FL-4 ratio of Z05405 and B-D) with the FL-1/FL-4 ratio of a first generation binder (Z01753). 45 individual clones were analyzed in 4 separate assays (A-D) where Z05405 and Z05409 were included in all assays for comparison. A) shows cell-based flow cytometry assay results of Z05403-Z05415, B) shows cell-based flow cytometry assay results of Z05416-Z05426, C) shows cell-based flow cytometry assay results of Z05427-Z05434 and D) shows cell-based flow cytometry assay results of Z05435-Z05447.

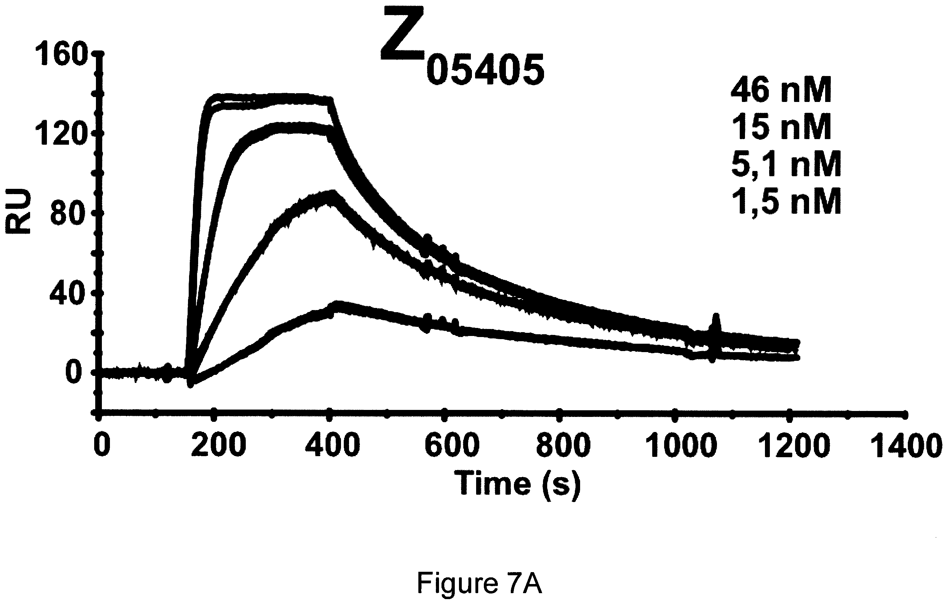

Figure 7A-D show sensorgrams from surface plasmon resonance (SPR) kinetic analyses of four different Z variants in four different concentrations. The uppermost curve in each sensorgram corresponds to the highest concentration of injected Z variant, the second curve from the top corresponds to the second highest concentration, the third curve from the top corresponds to the third highest concentration etc. The Z variants Z05405 (A) in concentrations of 46, 15, 1.5 and 1.5 nM, Z05413 (B), in concentrations of 57, 19, 6.3 and 1.9 nM, Z05416 (C), in concentrations of 67, 22, 7.5 and 2.2 nM and Z05417 (D), in concentrations of 59, 20, 6.5 and 2 nM, were injected over a surface immobilized with human HER3-Fc and dissociation constants based on kₒₙ and k_{off} were determined.

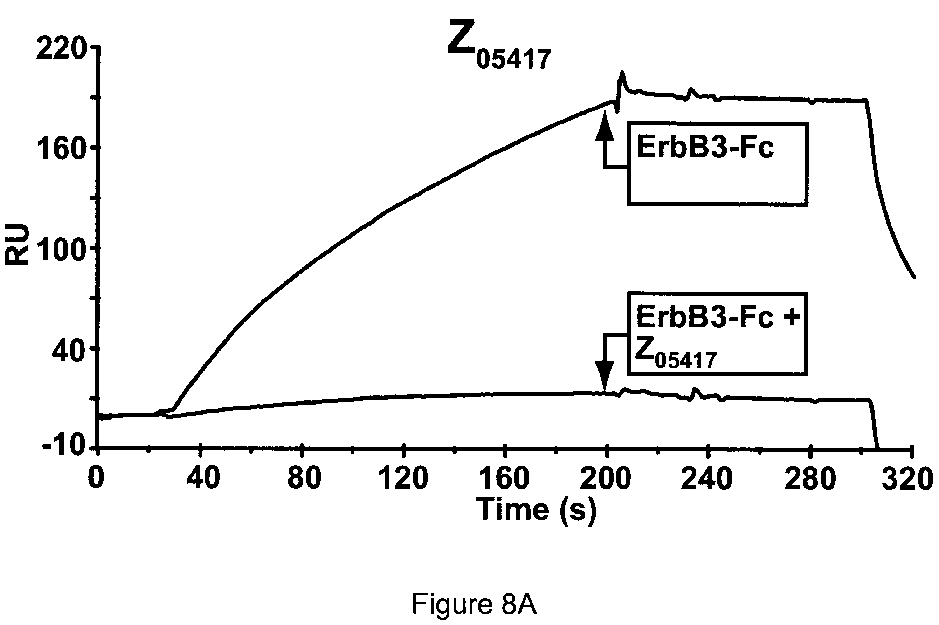

Figure 8A-B show sensorgrams from SPR competition analyses of Z05417 with heregulin. A) shows a sensorgram resulting from injections of HER3-Fc, alone and preincubated with 40-fold molar excess of Z05417, over a surface immobilized with human heregulin. B) shows a sensorgram resulting from co-injection of Z05417 with heregulin over a surface immobilized with human HER3-Fc. Z05417 was injected in duplicates in five different concentrations, 10 nM, 5 nM, 1 nM, 0.5 nM and 0 nM. The uppermost curve corresponds to the highest concentration of injected Z05417, the second curve from the top corresponds to the second highest concentration, the third curve from the top corresponds to the third highest concentration etc.

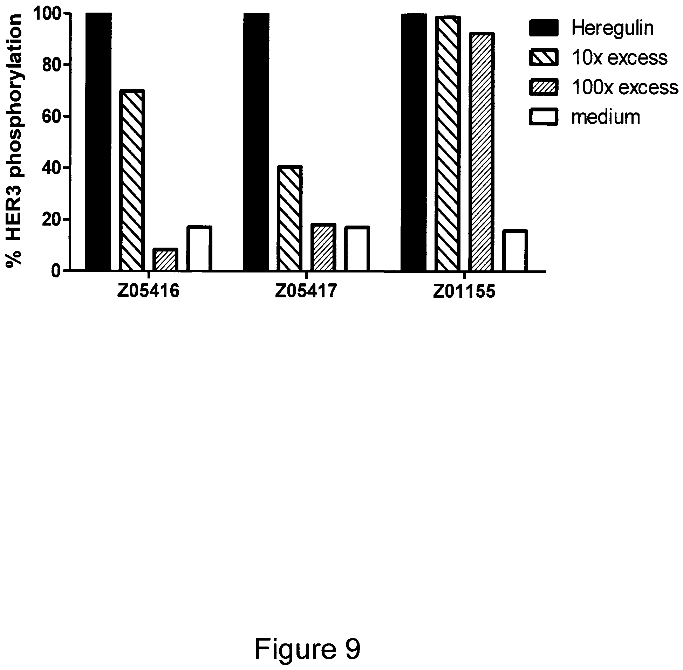

Figure 9 shows a diagram over the percentage of HER3 phosphorylation in the absence and presence of HER3 binding Z variants. MCF-7 cells were stimulated with 5 nM heregulin in the absence of Z variants (100 % phosphorylation, filled bars) and in the presence of the HER3 binding Z variants Z05416 and Z05417 and the negative control Z01155 (taq polymerase binding Z variant, striped bars) or with medium alone (open bars).The diagram shows the percentage of HER3 phosphorylation in the presence of Z variants or medium compared to heregulin-induced HER3 phosphorylation (100 %).

### Examples

### Example 1: Selection and screening of HER3 binding polypeptides

### Material and methods

Labeling of HER3 and HSA: Biotinylation of recombinant human HER3/Fc chimera (R&D Systems, #348-RB-050), here denoted HER3-Fc, was performed using the Biotin-XX Microscale Protein Labeling Kit (Invitrogen, #B30010) according to the supplier's recommendations. The extracellular domain of HER3 (SEQ ID NO:1007), here denoted HER3-ECD, was biotinylated using EZ-Link™-Sulfo-NHS-LC-LC-Biotin (Pierce, #21338) according to the supplier's recommendations. Human serum albumin (HSA; Sigma, #A-3782) was fluorescently labeled using Alexa Fluor® 647 succinimidyl ester (Invitrogen, #A20006) according to the supplier's recommendations. The protein buffer was changed to PBS (10 mM phosphate, 137 mM NaCl, 2.68 mM KCI, pH 7.4) or PBS supplemented with 0.1% Pluronic® F108 NF Surfactant (PBSP; BASF Corporation, #30085231) in order to remove any excess biotin or fluorophore.

Phage display selection of HER3 binding polypeptides: A library of random variants of Z molecules displayed on bacteriophage, denoted Zlib2002, constructed in phagemid pAffi1/pAY00065 as described in Grönwall et al, J Biotechnol 128:162-183 (2007), was used to select HER3-ECD-binding polypeptides. Preparation of phage stocks from the phagemid library, as well as between rounds of selection, was performed according to previously described procedures (Nord et al, Nat Biotech 15:772-777 (1997); Hansson et al, Immunotechnology 4:237-252 (1999)) using the helper phage M13K07 (New England Biolabs, #NO315S). The *Escherichia coli* amber suppressor strain RR1ΔM15 (Rüther, Nucleic Acids Res 10:5765-5772, 1982) was used for all cloning procedures and as host for phage production.

The phage selection was performed in solution using biotinylated HER3 protein and streptavidin-coated paramagnetic beads (Dynabeads® M-280 Streptavidin, Dynal #112.06) for capture of bound phages. In addition, phage selection was performed on solid phase using HER3-ECD immobilized on MaxiSorp immunotubes (Nunc). Unspecific binding of phage particles was minimized by pre-treatment of all tubes and beads with PBST 0.1-gelatin (PBS supplemented with 0.1 % Tween-20 and 0.1 % gelatin). Selection was performed in four cycles. All selections were performed in PBST 0.1-gelatin and in a volume of 1 ml.

For the selection performed in solution, four cycles of selection were performed at room temperature (RT) using different concentrations of biotinylated recombinant HER3-ECD as target protein. Phage stocks used in cycle 1 and 2 were pre-incubated with 0.1 mg strepavidin coated beads for 1 h to remove streptavidin binding phages. In cycle 1, phage particles from the Zlib2002 library were incubated with 100 nM of biotinylated HER3-ECD in PBST 0.1-gelatin for 2 hours. The subsequent selection cycles were divided into two selection tracks for which the target concentration was lowered to 50 (track 1) or 20 nM (track 2) for cycle 2, 20 (track 1) or 10 nM (track 2) for cycle 3 and 20 (track 1) or 5 nM (track 2) for cycle 4. The bound phages were captured with streptavidin-coated M-280 Dynabeads®, allowing an immobilization of 4 µg of HER3-ECD per milligram of beads.

For selection on solid phase, immunotubes were immobilized with 1 ml HER3-ECD, 10 µg/ml in 50 mM sodium carbonat buffer, pH 9.6 for 1.5 h at RT and thereafter blocked with 3 ml PBST 0.1-gelatin for 1 h at RT. Phage stock was added to the tube and incubated for 2 h.

For both selection strategies, the number of washes with PBST 0.1-gelatin was increased between rounds in order to increase selection stringency. Therefore, washes were performed twice in cycle 1, three times in cycle 2, six times in cycle 3 and 12 times in cycle 4. The bound phage particles were eluted with 0.1 M glycine-HCl (pH 2.2) followed by immediate neutralization with 1 M Tris-HCl (pH 8). The phage-containing eluate was used to infect log phase RR1ΔM15 cells and phagemid particles were rescued from infected cells using helper phage M13K07. The selection process was monitored by titration of phage stocks before each selection round and after elution from target protein. Serial dilutions of phage solutions were used for infection of log phase RR1ΔM15 cells.
ELISA screening of Z variants: To test if the Z variant molecules could indeed interact with HER3, two different ELISA were performed, one using biotinylated HER3-ECD and the second using HER3-Fc as target protein. The Z variants were produced by inoculating single colonies, prepared as described above, in 1 ml TSB-YE medium (30 g/l TSB, 5 g/l yeast extract) supplemented with 100 µg/ml ampicillin and 0.1 mM isopropyl-β-D-1-thiogalactopyranoside (IPTG) in deep-well plates (Nunc #278752). As a negative control, an insulin-binding Z variant (Z00801) was inoculated and grown in each plate. The plates were incubated for 18-24 h at 37 °C. After incubation, replica plates were made by transferring a small fraction of each culture to 96-well plates with 15 % glycerol for storage at -20 °C. Remaining cells were pelleted by centrifugation, re-suspended in 300 µl PBST 0.05 (PBS supplemented with 0.05 % Tween 20) and frozen at -80 °C to release the periplasmic fraction of the cells. Frozen samples were thawed in a water bath and cells were pelleted by centrifugation. The periplasmic supernatant contained the Z variants as fusions to the albumin binding domain from GA3 of Protein G from *Streptococcus* strain G148, expressed as AQHDEALE-[Z#####]-VDYV-[ABD]-YVPG (Grönwall *et al, supra*). Z##### refers to individual Z variants.

Half area 96 well ELISA plates (Costar #3690) were coated with 50 µl/well of coating buffer (50 mM sodium carbonate, pH 9.6) containing 6 µg/ml human serum albumin (HSA, Sigma #A3782), and incubated over night (ON). The HSA solution was poured off and the wells were blocked with 100 µl of PBST 0.1 supplemented with 2 % non-fat dry milk solution (Semper AB) for 1 h at RT. The blocking solution was discarded and 50 µl of periplasmic solution was added to each well and incubated for 1.5 h at RT under slow shaking. The supernatants were poured off and the wells were washed 4 times with PBST 0.05. 50 µl of HER3-Fc at a concentration of 0.5 µg/ml in PBST 0.05 or biotinylated HER3-ECD at a concentration of 1 µg/ml in PBS were added to each well. The plates were incubated for 1.5 h at RT followed by wash 4x in PBST 0.05. In HER3-Fc plates, an antibody against human Fc (DAKO Cytomation, #P0214), labeled with horseradish peroxidase (HRP) and diluted 1:4000 in PBST 0.05, was added to the wells and incubated for 1 h at RT. In the plates with biotinylated HER3-ECD, streptavidin conjugated with HRP (DAKO, #P0397) was added to each well diluted 1:5000 in PBST 0.05. After washing as described above, 50 µl ImmunoPure TMB substrate (Pierce #34021) was added to the wells and the plates were treated according to the manufacturer's recommendations. All steps from blocking to reading were performed in a Tecan Genesis Freedom 200 robot (Tecan Group LTD). Absorbance of the wells was read at 450 nm in an ELISA reader Tecan Ultra 384 (Tecan) and evaluated with Magellan v. 5.0 software (Tecan).
Sequencing: Based on the ELISA screening, a part of all clones regarded as positive were picked for sequencing. PCR fragments were amplified from single colonies using a standard PCR program and the primers AFFI-21 (5'-tgcttccggctcgtatgttgtgtg) and AFFI-22 (5'-cggaaccagagccaccaccgg). Sequencing of amplified fragments was performed using the biotinylated oligonucleotide AFFI-72 (5'-biotin-cggaaccagagccaccaccgg) and a BigDye® Terminator v3.1 Cycle Sequencing Kit (Applied Biosystems), used in accordance with the manufacturer's protocol. The sequencing reactions were purified by binding to magnetic streptavidin coated beads using a Magnatrix 8000 (Magnetic Biosolution), and analyzed on ABI PRISM® 3100 Genetic Analyzer (PE Applied Biosystems). The sequencing results were imported and analyzed with an ALD LIMS Nautilus™ 2003 R2 B3 software (Thermo Electronics Corp.).
Dot blot analysis: The produced HER3 binding Z variants were tested for specificity by dot blot analysis against alpha-2 macroglobulin (MP biomedicals/Cappel, #55833), alpha-1 acid glycoprotein (RDI, #RDI-SCP-153-1), alpha-1 antichymotrypsin (RDI, #RDI-SCP-159-0), alpha-1-antitrypsin (RDI, #RDI-SCP-165-5), C3 complement (RDI, #RDI-SCP-150-0), C4 complement (RDI, #RDI-SCP-151-0), fibrinogen (Enzyme research, #031015E), haptoglobulin (RDI, #RDI-SCP-119-1), hemopexin (Agilent), holo-transferrin (Sigma, #T0665), human IgA (Bethyl, #P80-102), mouse IgE kappa (BD, #55079), human IgG (Sigma, #G4386), human IgM (Sigma, #18260), human serum albumin (HSA, Sigma, #A3782), neutravidin (Pierce, #31000), streptavidin (Pierce, #21122), transthyretin (Sigma, #P1742), HER2-ECD (SEQ ID NO:1008), HER3-ECD (SEQ ID NO:1007), HER3-Fc (R&D Systems), HER4-ECD (SEQ ID NO:1009), HER4-Fc (R&D Systems).

The Z variants were produced in essentially the same manner as described under ELISA screening in order to obtain periplasmic supernatants containing soluble protein. The supernatants were filtered using 0.45 µm membranes.

Nitrocellulose membranes (Invitrogen) were dotted with 1 µl of each protein at a concentration of 0.25 mg/ml, except for HER3-ECD and HER4-ECD that had a concentration of 0.29 mg/ml and 0.14 mg/ml, respectively. The membranes were blocked ON in PBST 0.1 supplemented with 0.5 % casein (blocking solution) at 4 °C. After removal of the solution, the membranes were incubated for 1 h with periplasmic supernatants supplemented with 0.5 % casein. The membranes were washed 3 times very briefly and 4 x 5 minutes in PBST 0.1. The Z variants were detected with a ABD fused hyperimmune polyclonal rabbit Ig against an epitope common for all Z variants (produced in house) diluted 1:5000 in PBST blocking solution by incubation for 1 h at RT. After washing as above an antibody against rabbit IgG conjugated with HRP (DAKO Cytomation, #P0448), diluted 1:5000 in blocking solution, was added to the membranes, followed by incubation of the membranes for 1 h at RT. The membranes were washed, rinsed in PBS and soaked with Supersignal (Pierce #34075). Light emissions were photographed with a Chemilmager 5500 (Alpha Innotech Corp.)
Subcloning of Z variants: Dimeric Z variants were amplified from pAffi1/pAY00065 vectors. A PCR was performed using different primer pairs and the resulting gene fragments were purified and hybridized in ligase buffer. The hybridized gene fragments were subcloned in the pAY00430 vector, providing an N-terminal His₆ tag and a C-terminal cysteine (His₆-(Z#####)₂-Cys). The HER3 binding Z variants were subcloned as dimers and the constructs encoded by the expression vectors were MGSSHHHHHHLQ-[Z#####][Z#####]-VDC. A three parts ligation was used for insertion of both insert fragments into the vector at the same step. Hybridized gene fragments and *Acc*I-digested and dephosphorylated expression vectors were ligated in ligase buffer and electroporated into electrocompetent *E. coli* TOP10 cells. The transformed cells were spread on TBAB plates (30 g/l tryptose blood agar base) supplemented with 50 µg/ml of kanamycin, followed by incubation at 37 °C overnight. The colonies were screened using PCR and the lengths of the PCR fragments were verified on agarose gels. To verify the sequences, sequencing was performed as described above. Plasmid DNA stock was prepared from the sequenced clones and deposited in -80 °C. In addition, *E. coli* BL21 (DE3) cells were transformed with the plasmids through electroporation.

### Results

Phage display selection of HER3 binding polypeptides: Four rounds of phage display selection were run against biotinylated or non-biotinylated human HER3-ECD. The four selection cycles were performed in solution with biotinylated target at two different target concentrations or on solid phase with non-biotinylated target. In solution, phage particle target complexes were captured onto streptavidin-coated beads. For each selection cycle, the number of washes was increased. The phage particle titers and yields were calculated after each selection cycle. The phage particle yield (phage particles out/phage particles in) increased for the third or last cycle indicating an enrichment in target binding clones.

ELISA screening of Z variants: The clones obtained after four cycles of selection were produced in 96-well plates and screened for HER3-ECD and HER3-Fc-binding activity in an ELISA. In total, 93 clones from each selection track were screened. The absorbance measurements showed that, independently of selection strategy, approximately 90 % of the clones were positive for HER3, defined as a response two times the signal for the negative control. A Z variant molecule binding to insulin was used in negative and positive control experiments. The positive control was detected with biotinylated insulin and for the negative control the target protein was omitted.

Sequencing: Sequencing was performed for clones selected on the basis of having variying signal absorbance values against HER3-Fc in ELISA screening, thus obtaining many different representative binders for the sequencing. In total, 135 HER3 positive clones were sequenced. Half of the clones were found in several copies resulting in total 23 new clones whereof two were identified as background binders. Each variant was given a unique identification number #####, and individual variants are referred to as Z#####. The amino acid sequences of the 58-mer Z variants are listed in Figure 1 and in the sequence listing as SEQ ID NO:669-689. The deduced HER3 binding motifs of these Z variants are listed in Figure 1 and in the sequence listing as SEQ ID NO:1-21. The amino acid sequences of the 49-mer polypeptides predicted to constitute the complete three-helix bundle within each of these Z variants are listed in Figure 1 and in the sequence listing as SEQ ID NO:335-355.

Dot blot analysis: The specificities of 16 of the sequenced unique HER3-binding polypeptides (in the form AQHDEALE-[Z#####]-VDYV-[ABD]-YVPG) were tested using dot blot analysis. 23 different proteins were blotted onto nitrocellulose membranes. The 23 proteins included 16 highly abundant human serum proteins, HER2-ECD, HER3-ECD and HER3-Fc and HER4-ECD and HER4-Fc. All Z variant fusion proteins except Z01824 bound to human to HSA as well as HER3-ECD and HER3-Fc in a specific way. Thus, the specificity of the tested Z variants was satisfying (Figure 2).

Subcloning of Z variants: 16 of the unique clones were chosen for subcloning in the expression vector pAY004308 as dimers. The cloning resulted in 15 dimers of HER3-binding Z variants (dimers of Z01748, Z01749, Z01751, Z01753, Z01814, Z01815, Z01817, Z01820, Z01821, Z01826, Z01828, Z01830, Z02009, Z02010 and Z02011).

### Example 2: Production and characterization of Z variants

### Materials and methods

Protein expression and purification: Transformed *E. coli* BL21 (DE3) cultures as subcloned in Example 1 were grown in TSB-YE to an optical density of approximately 1. The protein expression was then induced by addition of 1 M IPTG to a final concentration of 0.5 mM. Cultures were harvested 5 h after induction by centrifugation. The supernatants were discarded and the cell pellets were collected and stored at -20 °C.

The HER3 binding Z variants were purified from cell pellets under denatured conditions on 1.5 ml Ni-NTA Superflow Columns (Qiagen) and buffer was exchanged to PBS using PD-10 columns (GE Healthcare). Purified Z variants were aliquoted and stored at -80 °C.
Protein characterization: The concentration of purified Z variants (in His₆-(Z#####)₂-Cys form) was determined by absorbance measurements at 280 nm. The purity was estimated by SDS-PAGE analysis on 10 wells 4-12 % NuPAGET™ gels (Invitrogen) using Coomassie blue staining. To verify the identity and to determine the molecular weights of purified Z variants, LC/MS-analyses were performed on an Agilent 1100 LC/MSD system (Agilent Technologies).
CD analysis: The purified Z variants were thawed and diluted to 0.5 mg/ml in PBS. For each diluted Z variant, a CD spectrum at 250-195 nm was obtained at 20 °C. In addition, a variable temperature measurement (VTM) was performed to determine the melting temperature (Tm). In the VTM, the absorbance was measured at 220 nm while the temperature was raised from 20 to 90 °C, with a temperature slope of 5 °C/min. The CD measurements were performed on a Jasco J-810 spectropolarimeter (Jasco Scandinavia AB) using a cell with an optical path-length of 1 mm.
Immunofluorescence staining: The HER3 positive human mammary gland cell line AU565 (ATCC, #CRL-2351) and HER3 negative human ovary carcinoma cell line SKOV-3 (Ecacc, #91091004) were cultured as recommended by the supplier. The day before assay, 25000 cells were added to each well of multi well slides (eight wells per slide, Histolab #ER-201-1B) and allowed to grow ON in a CO₂ incubator to take on a flattened morphology. The slides were gently washed in PBS by adding droplets onto the slide with a Pasteur pipette.

Two experiments were performed. In the first, AU565 cells were stained with 11 different HER3 specific Z variants; His₆-(Z01748)₂-Cys, His₆-(Z01749)₂-Cys, His₆-(Z01751)₂-Cys, His₆-(Z01753)₂-Cys, His₆-(Z01814)₂-Cys, His₆-(Z01815)₂-Cys, His₆-(Z01817)₂-Cys, His₆-(Z01820)₂-Cys, His₆-(Z01828)₂-Cys, His₆-(Z01830)₂-Cys and His₆-(Z02011)₂-Cys. The second experiment was performed with His₆-(Z01814)₂-Cys and His₆-(Z01820)₂-Cys, and with (Z01154)₂ as a negative control Z variant. The staining was performed on slides with HER3 positive AU565 and HER3 negative SKOV-3 cells. An anti-HER3 antibody from R&D Systems (#AF234) was included as a positive control in both experiments.

In both experiments, the Z variants were added to the wells at a concentration of 15 µg/ml in PBS and all dilutions of reagents were made in PBS as well as all washes. After 1 hour of incubation at RT, the multiwell slides were gently washed as described above and goat anti-Affibody molecule Ig (Affibody AB, #20.1000.01.0005) was added to each well at a concentration of 5 µg/ml. After 45 minutes of incubation at RT, the slides were gently washed as described above and 5 µg/ml of a chicken anti-goat Alexa Fluor488 (Invitrogen, #A21467) was added to each well. After an additional 45 minutes of incubation, the slides were gently washed. The positive control, goat anti-HER3 antibody was used at a concentration of 15 µg/ml followed by 5 µg/ml of chicken anti-goat Alexa Fluor488. After completed staining, the cells were fixed in 3 % formaldehyde (Sigma, #1365) in PBS for 10 minutes at RT. Slides were then rinsed twice, dried and mounted with anti-fading solution containing DAPI (4',6-diamidine-2-phenyl indole, Vector laboratories, #H1200). The staining was documented using a LEICA DMLA microscope equipped with a video camera for live imaging.
On-cell affinity ranking using flow cytometry: The full gene sequences encoding Z variants Z01751, Z01753, Z01814 and Z01820 were PCR amplified from their corresponding pAY00430 vector constructs and ligated to the staphylococcal display vector pSCZ1 (Kronqvist et al, Protein Eng Des Sel 21:247-255 (2008)), previously digested with restriction enzymes *Xho*I and *Sal*I (New England Biolabs). The *E. coli* strain RR1ΔM15 was used as host for plasmid construction and preparation and the constructs were transformed to electrocompetent *Staphylococcus carnosus* TM300 (Götz, Soc Appl Bacteriol Symp Ser. 19:49-53 (1990)) according to a previously described protocol (Löfblom et al, J Appl Microbiol 102:736-747 (2007)). Staphylococcal cells individually displaying the four different Z variants were inoculated to 10 ml TSB-YE supplemented with 20 µg/ml chloramphenicol and grown ON at 37 °C and 150 rpm. From the cultures, 10⁶ cells were washed with 1 ml PBSP. The cells were pelleted by centrifugation (3500 x g, 4 °C, 6 min) and resuspended in 100 µl of PBSP containing different concentrations of biotinylated HER3-Fc (5, 20, 50 and 100 nM; see Example 1 for biotinylation). Equilibrium binding was reached by incubation at RT for 1 h with gentle mixing. The cells were washed once with 1 ml ice-cold PBSP, followed by incubation on ice in 100 µl ice-cold PBSP containing 1.25 µg/ml Alexa Fluor® 488-conjugated strepavidin (Invitrogen, #S32354) and 225 nM Alexa Fluor® 647-conjugated HSA (see Example 1) for 40 min. Following one wash with 1 ml ice-cold PBSP, cells were resuspended in 300 µl ice-cold PBSP prior to flow-cytometric analysis. The mean fluorescence intensity (MFI) was measured using a FACS Vantage SE (BD Biosciences) flow cytometer.

### Results

Protein expression and purification: The 15 dimeric Z variant molecules (in His₆-(Z#####)₂-Cys form) yielded acceptable production levels of soluble product and the purity of produced batches was estimated to exceed 90 % by SDS-PAGE analysis, except for two Z variants (His₆-(Z01826)₂-Cys and His₆-(Z02009)₂-Cys) that did not meet that criteria. The LC/MS analysis verified the correct molecular weight for all pure Z variant molecules.

CD analysis: The CD spectrums showed that the Z variant molecules had α-helical structures at 20 °C. This result was also verified in the variable temperature measurements where the melting temperatures (Tm) were determined (Table 1).

**Table 1. Melting temperatures for the Z variants**

| Z variant | Tm (°C) |
|---|---|
| His₆-(Z01748)₂-Cys | 44 |
| His₆-(Z01749)₂-Cys | 63 |
| His₆-(Z01751)₂-Cys | 52 |
| His₆-(Z01753)₂-Cys | 56 |
| His₆-(Z01814)₂-Cys | 58 |
| His₆-(Z01815)₂-Cys | 56 |
| His₆-(Z01817)₂-Cys | 40 |
| His₆-(Z01820)₂-Cys | 55 |
| His₆-(Z01821)₂-Cys | 44 |
| His₆-(Z01828)₂-Cys | 49 |
| His₆-(Z01830)₂-Cys | 46 |
| His₆-(Z02010)₂-Cys | 51 |
| His₆-(Z02011)₂-Cys | 46 |

Immunofluorescence staining: Immunofluorescence microscopy was employed for characterization of specificity and binding activity to native HER3 expressed on human cancer cells. Eleven clones, showing positive signals in the ELISA screening of Example 1, were produced as dimers fused to an N-terminal His₆-tag and a C-terminal cysteine. Each Z variant was incubated on HER3 positive AU565 cells (human mammary mammary gland cell line). An anti-HER3 antibody was used as positive control. The cells were fixed after the staining procedure. Of eleven binders, 6 gave a membrane bound staining pattern. The background was high for some Z variants and in some cases it was difficult to judge if they were specific or not. Two of the HER3 specific molecules, His₆-(Z01814)₂-Cys and His₆-(Z01820)₂-Cys were selected for additional analysis on both HER3 positive AU565 cells and HER2 positive- and HER3 negative SKOV-3 cells (human ovary carcinoma cell line). Distinct cell membrane staining was obtained on AU565 but not on SKOV-3 cells demonstrating selectivity of the Z variants for HER3 over HER2 (Figure 3).

On-cell affinity ranking using flow cytometry: To further characterize the HER3 binding molecules and to verify functional expression on bacterial cells, four of the HER3 binding molecules showing specificity for HER3 in immunofluorescence staining were employed for an on-cell affinity ranking experiment using staphylococcal cell display and flow cytometry. Monomer constructs of the four variants were subcloned to the staphylococcal display vector for subsequent transformation to the staphylococcal host. Staphylococcal cells displaying the four Z variants, respectively, were incubated with four concentrations of biotinylated HER3-Fc ranging from 5-100 nM. Cells were analyzed using flow cytometry, revealing efficient expression on the cell surface and specific binding to HER3 for all four variants with no significant difference in relative affinity among the clones (data not shown). Biotinylated HER2 was used as negative control in the experiments and no cross-specificity was observed, confirming the results from the dot blot made with periplasmic supernatants in Example 1 and the immunofluorescence staining in this Example.

### Example 3: Design and construction of a maturated library of HER3 binding Z variants

In this Example, a maturated library was constructed. The library was used for selections of HER3 binding polypeptides. Selections from maturated libraries are usually expected to result in binders with increased affinity (Orlova et al, Cancer Res 66(8):4339-48 (2006). Traditional oligonucleotide synthesis techniques using mononucleotides and degenerate codons may however limit the design since staphylococcal strains are not able to suppress amber stop codons. In this study, randomized double stranded linkers were instead generated by the Slonomics® technique which enables incorporation of randomized sets of trinucleotide building blocks using ligations and restrictions of the subsequently built up double stranded DNA.

### Materials and Methods

Library design: The library was based on the sequences of the HER3 binding Z variants described in Examples 1 and 2. In the new library, 13 variable positions in the Z molecule scaffold were biased towards certain amino acid residues, according to a strategy based on the binding motifs of the Z variant sequences defined in SEQ ID NO:1-21. A SlonoMax® library containing a mixture of 131 bp double stranded linkers, encoding partially randomized positions in helix 1 and 2 of the HER3 binding polypeptide, e.g. 5'-GAA NNN NNN NNN GCG NNN NNN GAG ATC TGG NNN TTA CCT AAC TTA AAC NNN NNN CAA NNN NNN GCC TTC ATC NNN AGT TTA NNN GAT GAC CCA AGC CAA AGC GCT AAC TT-3' (randomized codons are illustrated as NNN) flanked with restriction sites *Xho*I and *Nhe*I*,* was ordered from Sloning BioTechnology GmbH. The theoretical distributions of amino acid residues in the new library for the 13 variable Z positions are given in Table 3:

**Table 3: Library design**

| Amino acid position in the Z variant molecule | Randomization (amino acid abbreviations) | No of amino acids | Proportion |
|---|---|---|---|
| 2 | W,R,K | 3 | 1/3 |
| 3 | Y,R,K,S | 4 | ¼ |
| 4 | A,V,I,L,G,S,T,K,R,E,Q,N,H | 13 | 1/13 |
| 6 | A,S,R,Y,T | 5 | 1/5 |
| 7 | W,Y,F,A,V,G | 6 | 1/6 |
| 10 | W | 1 | 1/1 |
| 11 | E,L,Q | 3 | 1/3 |
| 17 | R,Q,V,S,T | 5 | 1/5 |
| 18 | A,V,I,L,G,S,T,R,E,Q,H,W,F,Y | 14 | 1/14 |
| 20 | K,R,A | 3 | 1/3 |
| 21 | A,S,G,V | 4 | ¼ |
| 25 | A,V,I,L,G,S,T,K,R | 9 | 1/9 |
| 28 | E,Q,F,Y,W,A,S | 7 | 1/7 |

Library construction and cloning: The library was amplified using Phusion DNA polymerase (Finnzymes, #F530L) during 8 cycles of PCR and pooled products were purified with QIAquick PCR Purification Kit (QIAGEN) according to the supplier's recommendations. The purified pool of randomized library fragments was digested with restriction enzymes *Xho*I and *Nhe*I (New England Biolabs) and purified using preparative gel electrophoresis on a 2 % agarose gel. The *E. coli* strain RR1ΔM15 was used as host strain for plasmid production of the staphylococcal display vector pSCZ1 (Figure 4A) with Jetstar Maxi Kit (Genomed, #220020).

The vector was digested with the same enzymes, *Xho*I and *Nhe*I, and purified using preparative gel electrophoresis on a 1 % agarose gel. Ligation of pSCZ1 with the randomized library fragments was performed at a 1:4 molar ratio of vector to fragment using T4 DNA ligase (New England Biolabs, #M0202T). The ligation mixture was purified using QIAquick PCR Purification Kit according to the supplier's recommendations prior to transformation to electrocompetent *E. coli* DH5α cells (Invitrogen, #18263-012). Individual clones, plated directly after transformation as well as after ON amplification, were PCR amplified for sequence verification using BigDye Thermo Cycle Sequencing reactions and an ABI Prism 3700 instrument (PE Applied Biosystems). Plasmids were prepared from ON cultures of *E. coli* using Jetstar Maxi Kit and transformed to electrocompetent S. *carnosus* as described previously (Löfblom *et al,* supra). The staphylococcal-displayed library is hereinafter denoted Sc:Z_{HER3LIB}.
Library quality analysis: An aliquot of Sc:Z_{HER3LIB} (at least ten times the library size, i.e. more than 1.3 x 10⁸) was inoculated to 100 ml TSB-YE with 20 µg/ml chloramphenicol and grown ON at 37 °C and 150 rpm. After 16 hours, 10⁷ cells were washed once with 1 ml PBSP. The cells were pelleted by centrifugation (3500 x g, 4 °C, 6 min) and resuspended in PBSP containing 225 nM Alexa Fluor® 647-conjugated HSA and incubated for 1 hour at RT in the dark. Following one wash with 1 ml ice-cold PBSP, cells were resuspended in 300 µl ice-cold PBSP prior to flow-cytometric analysis. The mean fluorescence intensity (MFI) was measured using a FACS Vantage SE (BD Biosciences) flow cytometer.

### Results

Library construction and cloning: The new library was designed based on a set of HER3 binding Z variants (SEQ ID NO:669-689) with verified binding properties (Example 1 and 2). The theoretical size of the designed library was 7.4 x 10⁸ Z variants. The library of DNA fragments was cloned into the staphylococcal expression vector and transformed into *S*. *carnosus* to generate a cell-displayed library containing around 1.3 x 10⁷ individual clones. Sequence analysis of individual library members revealed a distribution of codons in accordance with the theoretical design and a low proportion of unexpected codons, multiple inserts and frame shifts.

Library quality analysis: In order to verify that the Z variant maturation library was functionally displayed on the bacterial surface, staphylococcal cells from the library were incubated with fluorescently labeled HSA and analyzed using flow cytometry. The result showed that around 72 % of the library expressed full-length proteins with functional ABP fusions on the cell surface (data not shown). A maturated library of HER3 binding polypeptides was thus successfully constructed.

### Example 4: Selection, screening and characterization of Z variants from a staphylococcal surface display library

### Materials and Methods

Cell labeling and staphylococcal cell sorting using FACS: An aliquot of SC:Z_{HER3LIB} (at least ten times the library size, i.e. more than 1.3 x 10⁸) was inoculated to 100 ml TSB-YE with 20 µg/ml chloramphenicol and grown ON at 37 °C and 150 rpm. After 16 hours, cells (at least four times the subsequent sampling number) were washed with 1 ml PBSP. The cells were pelleted by centrifugation (3500 x g, 4 °C, 6 min) and resuspended in PBSP containing biotinylated HER3-Fc and incubated at RT with gentle mixing for 2 hours to reach equilibrium binding. The cells were thereafter washed with ice-cold PBSP followed by incubation in 1 ml PBSP containing 1.25 µg/ml Alexa Fluor® 488-conjugated streptavidin (Invitrogen) and 225 nM Alexa Fluor® 647-conjugated HSA for 1 hour on ice in the dark. After a final washing step in 1 ml of ice-cold PBSP, the cells were resuspended in ice-cold PBSP before sorting. Cells were sorted using a FACSVantage SE (BD Biosciences) flow cytometer. The sort gate was set to sort out the top fraction of Z variant displaying cells (typically 0.1 %) showing the highest Alexa Fluor® 488 to Alexa Fluor® 647 fluorescence intensity ratio. The cells were sorted directly into 0.5 ml TSB-YE medium and thereafter inoculated to TSB containing 10 µg/ml chloramphenicol and incubated at 37 °C for 16 hours in order to amplify isolated cells by growth for the next round of labeling and FACS. The procedure was repeated four times.

Sequencing: Sequencing of individual staphylococcal clones was performed after cell sorting cycle 3 and 4 as described in under Library construction in Example 3.

On-cell affinity ranking and K_{D} determination: HER3-Fc was biotinylated and HSA conjugated with Alexa Fluor® 647 as described in Example 1. Staphylococcal cells displaying the different Z variants, respectively, were inoculated to 10 ml TSB-YE and 20 µg/ml chloramphenicol and grown ON at 37 °C and 150 rpm. From the cultures, 10⁶ cells were washed with 1 ml PBSP. The cells were pelleted by centrifugation (3500 x g, 4 °C, 6 min) and resuspended in PBSP containing different concentrations of biotinylated HER3-Fc (135, 90, 27, 9, 2.7, 0.9, 0.45, 0.22, 0.11 and 0.054 nM for Z05405, Z05413, Z05416 and Z05417; 670, 337, 110, 45, 18, 6.7, 2.2, 1.1, 0.37 and 0.27 nM for Z01820) spanning the estimated K_{D}. Equilibrium binding was reached by incubation at RT for one hour with gentle mixing. The cells were washed with 1 ml ice-cold PBSP, followed by incubation on ice in 100 µl ice-cold PBSP containing 1.25 µg/ml Alexa Fluor® 488-conjugated streptavidin, (Invitrogen) and, in the ranking experiment, 225 nM Alexa Fluor® 647-conjugated HSA, for 40 min. Following a wash with 1 ml ice-cold PBSP, cells were resuspended in 300 µl ice-cold PBSP prior to flow cytometric analysis. The mean fluorescence intensity (MFI) was measured using a FACS Vantage SE (BD Biosciences) flow cytometer.

### Results

Flow-cytometric sorting for isolation of improved Z variants: For isolation of matured HER3 binding Z variants, the staphylococcal library was subjected to four rounds of fluorescence-activated cell sorting (FACS) with alternating rounds of amplification by cell growth.

Briefly cells were incubated with biotinylated HER3-Fc at concentrations around 10-fold lower compared to the estimated K_{D} of the Z variants from Example 1 and 2. Cells were thereafter washed and incubated with fluorescently labeled streptavidin for subsequent fluorescence-mediated detection of cell-bound HER3 as well as fluorescently labeled HSA for monitoring of surface expression levels. The incubation of secondary reagents and HSA was performed on ice in order to reduce the dissociation rate of bound HER3. After an additional washing, the labeled cell library was screened and sorted in a flow cytometer. Selection stringency in terms of target concentration, sorting parameters and sorting gates was increased with each sorting round and typically, the top 0.1 % of the library, demonstrating highest target binding to surface expression ratio, was gated and isolated for amplification and subsequent rounds of sorting. The visualization of the target-binding properties of the library in the flow cytometer revealed an enrichment of HER3-positive clones in each sorting round, and essentially only HER3-positive clones in the last round (Figure 5). After up to four rounds of FACS, isolated cells were spread on semi-solid medium for sequencing and characterization of individual candidates.
Sequencing: 576 individual clones were sequenced resulting in 443 readable sequences, out of which 45 clones appeared more than once in the same sorting round (SEQ ID NO:690-734). Each variant was given a unique identification number #####, and individual variants are referred to as Z#####. The amino acid sequences of the 58-mer Z variants are listed in Figure 1 and in the sequence listing as SEQ ID NO:690-1002. The deduced HER3 binding motifs of these Z variants are listed in Figure 1 and in the sequence listing as SEQ ID NO:22-334. The amino acid sequences of the 49-mer polypeptides predicted to constitute the complete three-helix bundle within each of these Z variants are listed in Figure 1 and in the sequence listing as SEQ ID NO:356-668.
On-cell affinity ranking: On the basis of the sequencing results, the 45 clones that appeared more than once were subjected to a whole-cell ELISA assay in order to rank the variants with respect to affinity for HER3. The staphylococcal cell populations were analyzed using flow cytometry and one Z variant from Example 1 and 2 (Z01753) surface displayed on staphylococcal cells was included in the analysis for comparison. All 45 candidates were positive for HER3 binding in the assay and, moreover, 43 demonstrated a higher signal compared to the Z variant from Example 1 and 2 (Figure 6A-D). The highest signals in the assay were seen with the four clones that appeared most frequently after sequencing, demonstrating quantitative isolation of Z variants in the flow-cytometric sorting. K_{D}-determination: The apparent equilibrium dissociation constant (K_{D}) was determined on-cell for four Z variants (Figure 4B) and one HER3 binding Z variant from Example 1 and 2 (Z01820). Staphylococcal cells displaying the four variants and Z01820 were incubated in varying concentrations of labeled HER3-Fc spanning the estimated K_{D}. The cell populations were analyzed in the flow cytometer and the mean fluorescence intensity data was subsequently plotted against the HER3-Fc concentrations and fitted to a one-site binding model in order to determine the K_{D}. All four maturated Z variants demonstrated low nanomolar apparent K_{D}. The strongest binder demonstrated a 15-fold improvement in affinity for HER3-Fc compared to the Z variant from Example 1 and 2 (Table 3).

**Table 3. Affinities of HER3-Fc binding Z variants on cell using flow cytometry**

| Z variant | K_{D} (on-cell analysis, nM, mean ± SD) |
|---|---|
| | |
| Z05405 | 2.7 ± 0.6 |
| Z05413 | 2.3 ± 0.7 |
| Z05416 | 3.1 ± 0.6 |
| Z05417 | 3.2 ± 0.2 |
| Z01820 | 33.7 ± 1.3 |

### Example 5: Surface plasmon resonance K_{D} determination of Z variants

### Materials and Methods

Surface plasmon resonance analysis: Surface plasmon resonance (SPR) analysis was performed for the HER3 binding Z variants Z05405, Z05413, Z05416 and Z05417 on a BIAcore^{™} 2000 instrument (GE Healthcare). Human HER3-Fc (R&D Systems) and mouse HER3-Fc (R&D systems) were immobilized by NHS/ECD amine-coupling chemistry on a CM-5 sensor chip (GE Healthcare). The immobilization was performed in 10 mM NaAc (pH 4.5) at a flow rate of 30 µl/min and with receptor concentrations of 10 µg/ml, aiming for an immobilization level of 4000 RU. PBSP was used as running buffer at a flow rate of 20 µl/min (unless stated otherwise) and 5 mM NaOH for regeneration.

For determination of the kinetics, 250 µl of four different concentrations (1.5-67 nM diluted in PBSP) of the four HER3 binding Z variants were injected over HER3-Fc immobilized on the CM-5 chip surface. The surfaces were regenerated by four injections of 15 µl of 5 mM NaOH and extensive washing with running buffer. Each sample was measured in duplicates and the response from the blank surface was subtracted from the response from each Z variant at each concentration. The obtained sensorgrams were fitted to a one-site binding model for determination of K_{D} values based on mean association (kₒₙ) and dissociation rates (k_{off}).
Control experiments: To verify that the Z variants were not selected for affinity to the Fc part of HER3-Fc, each Z variant (50 nM) was incubated with a 10-fold molar excess of human polyclonal IgG (500 nM) for 1 h at room temperature prior to injection of 100 µl of each sample (in the above concentrations) over HER3-Fc immobilized on the biosensor chip surface. In an additional control experiment, the Z variants were injected over HER3-ECD immobilized on the sensor chip surface.

### Results

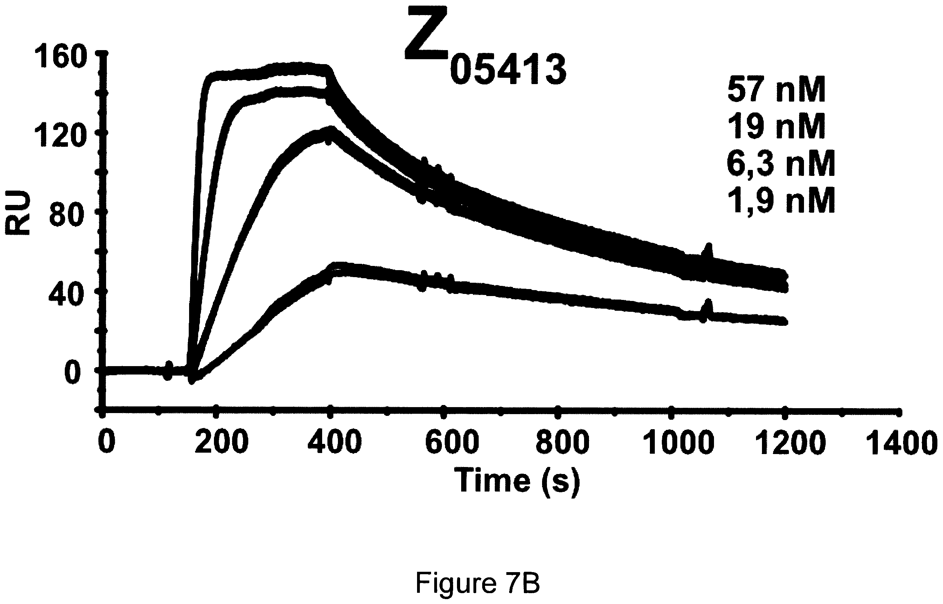

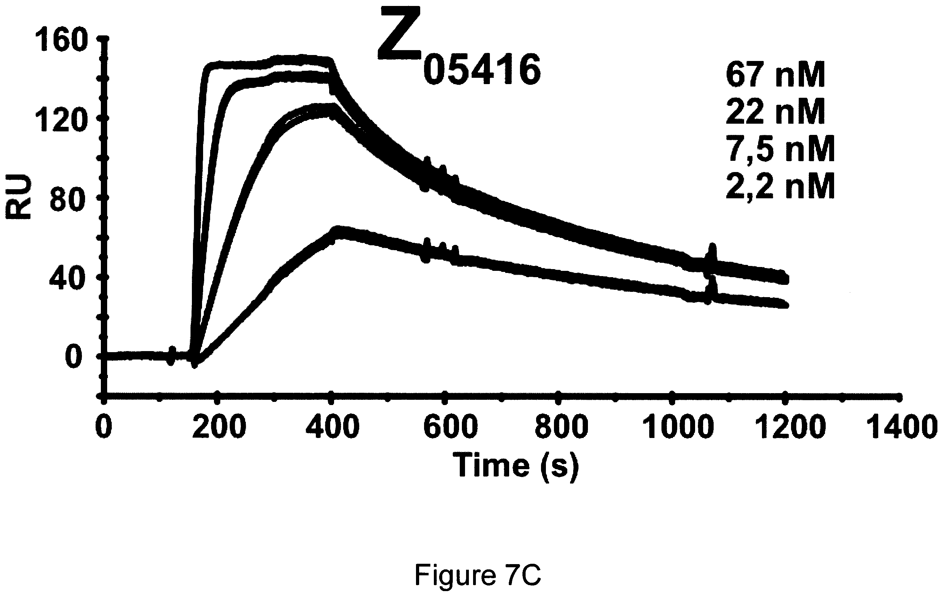

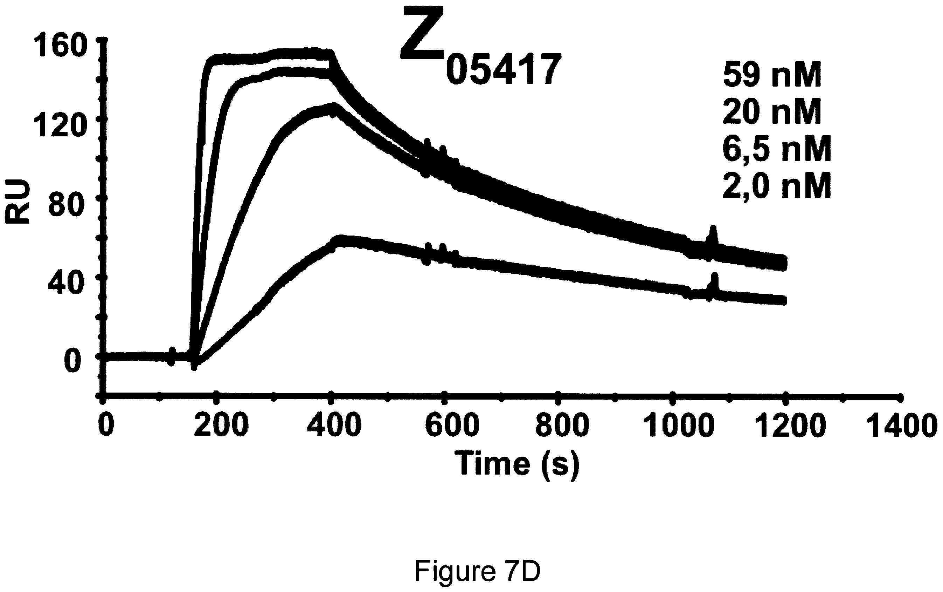

Surface plasmon resonance analysis: The dissociation equilibrium constant (K_{D}) of the four Z variants Z05405, Z05413, Z05416 and Z05417 was determined by SPR technology. The affinity was determined from the association and dissociation rates of the Z variants using non-linear regression to a one-site binding model, resulting in dissociation constants of 1.61 nM for Z05405, 0.78 nM for Z05413, 0.78 nM for Z05416 and 0.69 nM for Z05417 (Figure 7, Table 4). In Table 4, all values are means of duplicates of each concentration performed on the same day.

**Table 4: Affinities of five HER3 binding Z variants for human/mouse HER3**

| Z variant | Human HER3 | | | Mouse HER3 | | |
|---|---|---|---|---|---|---|
| | K_{D} (nM) | kₐ (M⁻¹s⁻¹) | k_{d} (s⁻¹) | K_{D} (nM) | kₐ (M⁻¹s⁻¹) | k_{d} (s⁻¹) |
| Z05405 | 1.6 | 1.8 x 10⁶ | 2.8 x 10⁻³ | 2.4 | 1.4 x 10⁶ | 3.3 x 10⁻³ |
| Z05413 | 0.8 | 1.7 x 10⁶ | 1.3 x 10⁻³ | 2.0 | 1.4 x 10⁶ | 3.0 x 10⁻³ |
| Z05416 | 0.8 | 1.9 x 10⁶ | 1.5 x 10⁻³ | 2.0 | 1.6 x 10⁶ | 3.2 x 10⁻³ |
| Z05417 | 0.7 | 1.9 x 10⁶ | 1.3 x 10⁻³ | 1.7 | 1.6 x 10⁶ | 2.7 x 10⁻³ |

Control experiments: Since the analysis of the HER3 binding Z variants was performed with HER3 fused to the human IgG Fc-region, two control experiments were performed in order to verify that the Z variants were not selected for affinity to the Fc part of HER3-Fc. No difference in response could be observed between Z variants pre-incubated with IgG and the samples containing only Z variants (data not shown). Moreover, the Z variants injected over a surface with HER3-ECD showed specific binding for all four variants (data not shown). Taken together, the two experiments clearly demonstrate that Fc is not part of the Z variants' binding site on HER3-Fc.

### Example 6: Competition assay for heregulin and HER3 binding Z variants

In this Example, the ability of HER3 binding Z variants to block binding of the ligand heregulin to HER3 was investigated by SPR in a competition assay.

### Materials and Method

Competition between the natural ligand heregulin (HRG1-β1, EGF-like domain, R&D Systems) and the four HER3 specific Z variants Z05405, Z05413, Z05416 and Z05417 was analyzed using SPR in two different assays.

In a first assay, 2.5 nM HER3-Fc was pre-incubated with 100 nM of each of the four HER3 specific Z variants (40-fold molar excess) for 1 h at room temperature. Thereafter, 75 µl of each mixture of HER3-Fc and Z variant was injected over a sensor chip surface with immobilized heregulin. Additionally, HER3-Fc not pre-incubated with Z variants was injected over the chip surface with immobilized heregulin.

In a second assay, 100 µl of each Z variant at five different concentrations (0-10 nM) was injected over the sensor chip surface with immobilized HER3, immediately followed by injection of 100 µl of 250 nM heregulin (using the BIAcore COINJECT command). All samples were injected in duplicates and an HER2 specific Z variant was injected using the same setup as a negative control. The surfaces were regenerated with two injections of 5 mM NaOH and extensive washing with running buffer.

### Results

Competition of the HER3 specific Z variants Z05405, Z05413, Z05416 and Z05417 with the natural HER3 ligand heregulin for HER3 was investigated by SPR technology.

The results of the first assay, using heregulin immobilized on the chip surface, showed a nearly complete reduction in response level when injecting HER3-Fc pre-incubated with Z variants compared to injecting HER3-Fc not pre-incubated with Z variants (Figure 8A).

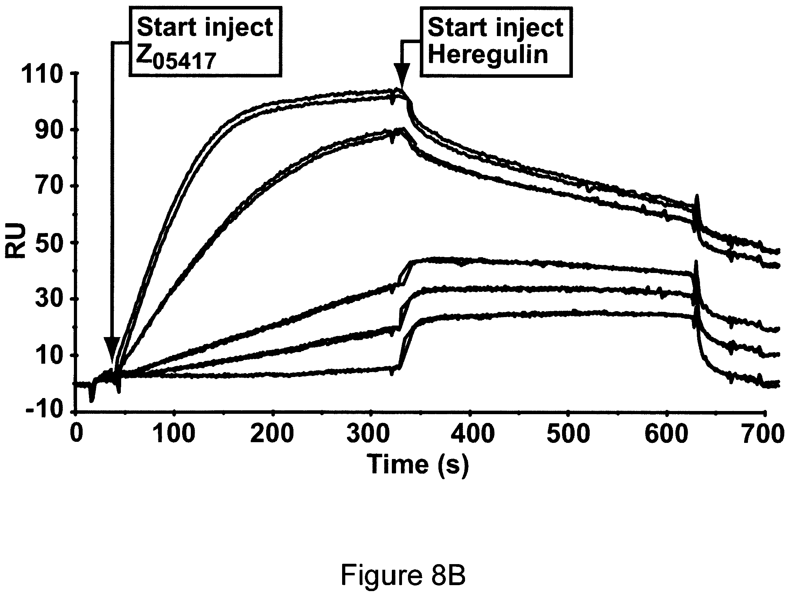

The results of the second assay, using HER3-Fc immobilized on the chip surface, indicated a concentration-dependent competition with the interaction between the HER3 receptor and its natural ligand (Figure 8B). The same pattern was observed for mouse HER3-Fc (data not shown). No competition between the negative control and heregulin could be observed for either human or mouse HER3 (data not shown).

The results demonstrate that the HER3 specific Z variants interact with the same binding site on HER3 as the natural ligand heregulin and can hence compete with the binding between the ligand and the receptor *in vitro.* This effect may be exploited in future therapeutic *in vivo* applications.

### Example 7: Inhibition of heregulin-induced HER3 phosphorylation in vitro

In this Example, the ability of HER3 binding Z variants to prevent heregulin-induced HER3 phosphorylation was investigated in a cell assay.

### Materials and Methods

Stimulation: MCF-7 (ACC115, DSMZ) breast carcinoma cells were cultured in RPMI medium (BE12-167F, Lonza) supplemented with L-glutamine (BE17-605E, Cambrex), non-essential amino acids (BE13-114E, Cambrex), sodium pyruvate (BE13-115E, Cambrex) and 10 % fetal bovine serum (FBS, 10108-165, Gibco). The day before stimulation, 1 x 10⁶ cells were seeded in 6 mm Petri dishes (430168, BD Biosciences) in 5 ml of the above medium.

One hour prior to stimulation, the medium was changed to a medium consisiting of RPMI + L-glutamine + 2 % dialyzed FBS (26400-036, Gibco). Phosphorylation of HER3 was induced with 5 nM heregulin (HRG1-β, 396-HB, R&D systems). HER3 binding Z variants Z05416 and Z05417, each comprising an N-terminal his-tag and a C-terminal cysteine (His₆-Z05416-cys and His₆-Z05417-cys), were added simultaneously with heregulin in 10 or 100 times molar excess. A taq polymerase specific Z variant (His₆-Z01155-cys) was used as a negative control and was added according to the same procedure as described above.

Following incubation for 10 minutes at 37 °C, cellular processes were stopped by placing the petri dishes on ice and by subsequent washing with ice-cold PBS. After washing, 2 ml PBS containing 1 mM activated orthovanadate (450243, Sigma) was added to each dish and the cells were detached using a cell scraper. The cell solution was transferred to a 10 ml tube and the cells were pelleted by spinning in a pre-cooled centrifuge at 1000 rpm for 3 minutes. The dry pellet was dissolved in 100 µl ice cold lysing buffer (1 % NP-40 (Sigma 13021), 20 mM Tris (pH 8.0), 137 mM NaCl, 10 % glycerol, 2 mM EDTA, 1 mM activated sodium orthovanadate) to lyse the cells. The lysate was incubated end-over-end at 4 °C for 30 minutes and centrifuged at 13000 rpm for 15 minutes. The supernatant was collected and stored at -80 °C until further analysis.
pHER3-ELISA: The presence of phosphorylated HER3 protein (pHER3) in cell lysates was analyzed using a Duo-Set IC phospo ErbB3 sandwich ELISA (DYC-1769, R&D Systems).

96 well half area plates (Costar 3690) were coated over night with 50 µl/well of 2 µg/ml capture antibody diluted in PBS. The next day, the plate was washed four times with PBS + 0.05% Tween (PBST) in an automated ELISA washer. The wells were blocked with PBS containing 1 % BSA (100 µl/well) for 2 hours at RT and washed as previously described.

50 µl/well of cell lysates, diluted two or four times in IC Diluent #12 (1 % NP-40, 20 mM Tris (pH 8.0), 137 mM NaCl, 10 % glycerol, 2 mM EDTA, and 1 mM activated sodium orthovanadate), were added. Positive controls, provided by the manufacturer, were also added to the wells. The plate was subsequently incubated for 2 hours.

After washing, 50 µl/well of detection antibody was added, diluted according to manufacturer protocol in IC diluent #14 (20 mM Tris, 137 mM NaCl, 0.05 % Tween 20, 0.1 % BSA, pH 7.2 - 7.4), and the plate was incubated for another 2 hours. Following incubation, the plate was washed and 50 µl/well of TMB (Immunopure, Termo Fisher Scientific) was added. The reaction was stopped after 20 minutes by addition of 50 µl/well of 2 M H₂SO₄ and the absorbance was measured at 450 nm using a Victor³ ELISA plate reader.

### Results

The HER3-specific Z variants Z05416 and Z05417 were, together with the negative control (taq polymerase specific Z01155), assessed for their ability to block heregulin-induced phosphorylation of HER3. The relative content of pHER3 (%) was determined as the relation of blocked culture (OD-value obtained from a culture containing a Z variant) to heregulin-stimulated culture (OD-value obtained from heregulin-stimulated cultures). Figure 9 shows that the HER3 binding variants Z05416 and Z05417 inhibited heregulin-induced phosphorylation in a dose dependent manner, whereas the taq polymerase binding variant Z01155 did not have any effect.

### Example 8: Bispecific Z variants binding to HER3 and HER2

As described in the Background section, HER3 is a preferred heterodimerization partner for HER2, and HER2 is dependent on other receptors in the HER family for ligand induced signaling. A bispecific HER3 and HER2 targeting ligand (for example a HER3-HER2 binding Z variant), might block the receptor signal driving tumor growth on tumors expressing both receptors.

Different molecular constructs for the bispecific molecules, optionally comprising various linker lengths, are contemplated. Examples of constructs are N-or C-terminal positioning of the HER3 binding polypeptide to the HER2 binding polypeptide. If a linker is introduced between the two binding polypeptides, it can comprise 1-60 amino acid residues, such as 1-45 amino acids. In addition, the bispecific construct may be provided with a half life extension moiety, e.g. by albumin fusion, coupling to an albumin binding moiety or a molecule such as PEG, at the N- or C-terminal part of the molecule or within the linker region. Alternatively, the half life extending moiety may in itself be utilized as a linker, or a spacer, between the two binding polypeptides.

### Material and Methods

A dimeric bispecific Z variant molecule is engineered as previously reported in Friedman et al, Biotechnol Appl Biochem, 54(2):121-31 (2009). Although Friedman *et al* describes production of tetrameric Z variants specific for two members of the HER family, the construction of a HER3 and HER2 bispecific Z variant in principle follows the same protocol. A person skilled in the field will easily understand how to apply the procedure based on Friedman *et al.* For construction of a heterodimeric Z variant binding to HER3 and HER2, a gene fragment encoding a HER3 binding variant is genetically fused in-frame with a gene fragment encoding a HER2 binding Z variant using a 40 aa peptide linker between the two binding polypeptides by following the procedure described by Jonsson, *et al, supra.*

The gene encoding the bispecific protein is constructed by cloning of vectors encoding the HER3-HER2 binding Z variants. The HER3 binding Z variant is selected from SEQ ID NO:669-1002, in particular a sequence selected from SEQ ID NO:669-734, such as selected from SEQ ID NO:690-734, such as from SEQ ID NO:691-693, SEQ ID NO:695-696, SEQ ID NO:700, SEQ ID NO:703-704, SEQ ID NO:708, SEQ ID NO:710, SEQ ID NO:712-713, SEQ ID NO:721, SEQ ID NO:722 and SEQ ID NO:724, and the HER2 binding Z variant is selected from SEQ ID NO:1003. The HER2 and the HER3 binding Z variants can have different affinities for the different receptors. Constructs may also be made from vectors encoding Z variants having various affinity for the same receptor. The vectors encoding the bispecific HER3-HER2 binding Z variants are cleaved using suitable endonucleases (e.g. *Sfi*I, *Acc*I, *Pst*I, *BamH*I, New England Biolabs) and the gene fragments encoding HER3-HER2 specific Z variants are recovered and ligated to form the gene encoding the bispecific molecule. The HER3 and HER2 binding polypeptides may individually form a monomeric unit or a multimeric unit, such as a dimer, trimer or tetramer, within the gene fragment.

For introduction of a peptide linker, multiples of gene fragments encoding a linker region (e.g. GGGS or GGGGSGGGGLVGLGSGGGGS) are introduced to create constructs with different linker lengths. The linker region can also contain an albumin gene or the gene for an albumin binding domain (ABD). Here, the ABD gene is amplified using PCR, and a linker encoding unit is introduced. This is followed by *BamH*I restriction digestion and ligation into the dimeric HER3-HER2 bispecific gene between the HER binding polypeptides. This is essentially conducted as described for creating trimeric TNF constructs by Jonsson *et al, supra,* however replacing the central TNF molecules described in Jonsson with an ABD. Alternatively, the gene fragments encoding HER3-HER2 binding Z variants are amplified using suitable PCR primers that also introduce the desired linker regions and restriction sites for cloning of the bispecific molecule in various formats. Different formats of the bispecific molecule are for example N-or C-terminal positioning, various lengths of linkers between the binding Z variants and/or the ABD gene in between. Another possibility for construction of genes encoding for the desired bispecific molecule is simply to purchase the fully synthetic gene from companies offering such services, such as e.g. Geneart AG.

The resulting gene fragments encoding the HER3 binding Z variant, the HER2 binding Z variant, the peptide linker and optionally ABD, are ligated into an expression vector, transformed into expression cells (e.g. *E. coli*). Proteins are expressed for example as described in Example 2. Expression vectors are used that encode an additional his-tag and/or a unique cysteine for labeling purposes, and the expressed protein is purified as for example described in Example 2. The methods used for protein expression and purification are standard methods known to the person skilled in the field.

The resulting bispecific proteins are then tested for simultaneous binding *in vitro,* as described in Friedman *et al, supra,* by using Biosensor analysis (Biacore, GE Healthcare). Human HER3-Fc (hHER3-Fc) and human HER2-Fc (hHER2-Fc, both from R&D Systems) are immobilized by amine coupling on the carboxylated dextran layer of a CM5 sensor chip (GE Healthcare), according to the manufacturer's instructions. Another flow-cell surface is activated and de-activated as a reference surface. The bispecific HER3-HER2 molecule is diluted in a running buffer, HBS (10 mM Hepes, 150 mM NaCl, 3.4 mM EDTA and 0.005% surfactant P20, pH 7.4), before binding analysis is performed at 25 °C. In a first experiment, the bispecific HER3-HER2 molecule is injected at concentrations ranging from 125 nM to 2 µM over all surfaces with a flow rate of 30 µl/min. A HER3 binding Z variant and a HER2 binding Z variant are also injected as controls. After each injection the flow cells are regenerated by the injection of 10 ml of 10 mM HCl.

In a second experiment, 5 µM HER3-HER2 molecule is injected over flow-cell surfaces having immobilized hHER3-Fc or hHER2-Fc (R&D Systems) by amine coupling on the carboxylated dextran layer of a CM5 sensor chip. Following a short dissociation time of 1 min, 115 nM hHER3-Fc and hHER2-Fc (diluted in the running buffer) is injected and the ability of the bispecific molecules to simultaneously bind both their targets is monitored.

By incubating a fluorescently labeled bispecific HER3-HER2 molecule with cells expressing either HER3 and not HER2, or HER2 and not HER3, binding ability of the HER3-HER2 molecule on live cells is monitored using fluorescence microscopy and flow cytometry analysis, as described in Friedman *et al, supra.* Differences in specificity are investigated at the same time, to find out if different constructs, having different affinities for their respective receptors, will differentiate between different receptor expression levels on cells.

The biological effect of binding bispecific Z variants to cells is tested by exposing cell cultures to various amounts, ranging from 0.05 nM up to 5000 nM, of the bispecific Z variants and analyzing cell growth rate and survival, and by analyzing phosphorylation patterns of important cell signaling pathways using western blot analysis, as described for a HER2 specific Z variant by Ekerljung et al (in Tumour Biol 27(4):201-10 (2006) and in Biochem Biophys Res Commun 377(2):489-94 (2008)). Cell lines that can be tested include MDA-MB-361, NCI-N87, CALU-3 and SKOV-3. Also HER3 transduced BT-474 and ZR75-cells can be used.

To test the effect of the HER3-HER2 Z variants *in vivo,* the bispecific constructs are evaluated for tumor targeting and for biological effect in a tumor. The bispecific HER3-HER2 binding Z variants are radiolabeled as previously described (Ahlgren et al, Bioconjug Chem 19(1):235-43 (2008)), and characterized for *in vitro* cellular uptake, retention and internalization using described methods (Steffen et al, Cancer Biother Radiopharm 20(3):239-48(2005)). Animals bearing xenografted tumors expressing HER3 and HER2 receptors (e.g. MDA-MB-361, NCI-N87, CALU-3 and SKOV-3) are in a biodistribution study injected with the radiolabeled HER3-HER2 binding Z variants and monitored for tumor uptake and contrast to other organs. For constructs not having a half life extending moiety, the distribution of the radioactivity in the xenografted mice is typically assessed at 1, 2, 4,8, 24, 48 and 72 hours following injection of the radiolabeled bispecific HER3-HER2 binding Z variant. For constructs having a half life extending moiety, the distribution of the radioactivity is typically assessed at 1, 4, 12, 24, 48, 72, 168 and 332 hours following injection of the radiolabeled bispecific HER3-HER2 binding Z variant, essentially as described in Tolmachev et al (Cancer Res 67(6):2773-82 (2007)). The uptake of the radiolabeled bispecific Z variants is compared to the uptake of HER2 and/or HER3 binding Z variants alone. In cases where the bispecific Z variants have superior uptake and retention and/or specificity in tumors *in vivo* as compared to the monospecific counterparts, the bispecific variants may be suitable for targeted payload therapy. Such therapy will utilize a potent effector function, e.g. radionuclides, toxic chemical molecules, toxins, cytokines or photosensitizers, fused or conjugated to the bispecific molecule.

The bispecific Z variants are furthermore characterized for intrinsic effect *in vivo.* Mice are grafted with tumor cells expressing both receptors, or as control, only one or none of the receptors, and subjected to a single or multiple injections of the bispecific Z variants. Tumor growth is monitored over time and versus control groups, including a vehicle group receiving only the injection buffer but no active compound, i.e. the bispecific HER3-HER2 binding molecule.

### Example 9: Z variants with bispecific binding to HER3 and EGFR

As described in the introduction, HER3 is a preferred heterodimerization partner for HER2. It is however, in certain conditions, also an important dimerization partner for the EGF-receptor (EGFR or HER1). By using a bispecific HER3 and EGFR targeting ligand, tumors expressing both receptors will be targeted, and the tumor driving receptor signaling may be blocked.

Bispecific HER3 and EGFR binding Z variants are engineered essentially as described above in Example 8. The resulting construct can be tested in cell lines expressing HER3 and EGFR receptors, such as A431-cells, in a similar fashion as described in Example 8 for bispecific HER3 and HER2 binding Z variants.

## Claims

1. HER3 binding polypeptide, comprising a HER3 binding motif, *BM,* which motif consists of the amino acid sequence
EX₂X₃X₄A YX₇EIW X₁₁LPNL X₁₆X₁₇X₁₈QX₂₀ X₂₁AFIX₂₅ X₂₆LX₂₈D,
wherein, independently of each other,
X₂X₃ is selected from KY and RY;
X₄ is selected from A, H, I, K, L, N, Q, R, S, T and V ;
X₇ is selected from A, F, G, V, W and Y;
X₁₁ is Q;
X₁₆ is selected from N and T;
X₁₇X₁₈ is selected from RV, RT, VR and RR;
X₂₀ is K;
X₂₁ is A;
X₂₅ is selected from A, G, and S;
X₂₆ is selected from S and K;
X₂₈ is selected from A, E, F, Q, S and W;
and
wherein the polypeptide binds to the extra-cellular domain of HER3.

2. HER3 binding polypeptide according to claim 1, wherein the amino acid sequence is selected from any one of SEQ ID NO:24, 32, 33, 35, 36, 42, 44, 51, 53, 66, 77, 89, 90, 100, 101, 105, 106, 108, 118, 135, 136, 137, 144 and 156.

3. HER3 binding polypeptide according to claim 2, wherein the amino acid sequence is selected from any one of SEQ ID NO:24, SEQ ID NO:32, SEQ ID NO:35-36, SEQ ID NO:42, SEQ ID NO:44 and SEQ ID NO:53.

4. HER3 binding polypeptide according to any preceding claim, in which said HER3 binding motif forms part of a three-helix bundle protein domain.

5. HER3 binding polypeptide according to any preceding claim, which comprises the amino acid sequence
K-[*BM*]-DPSQS XₐX_{b}LLX_{c} EAKKL NDX_{d}Q;
wherein
[*BM*] is a HER3 binding motif as defined in any one of claims 1-3;
Xₐ is selected from A and S;
X_{b} is selected from N and E;
X_{c} is selected from A, S and C;
X_{d} is selected from A and S.

6. HER3 binding polypeptide according to claim 5, wherein the amino acid sequence is selected from any one of SEQ ID NO:358, 366, 367, 369, 370, 376, 378, 385, 387, 400, 411, 423, 424, 434, 435, 439, 440, 442, 452, 469, 470, 471, 478 and 490.

7. HER3 binding polypeptide according to claim 6, wherein the amino acid sequence is selected from any one of SEQ ID NO:358, SEQ ID NO:366, SEQ ID NO:369-370, SEQ ID NO:376, SEQ ID NO:378 and SEQ ID NO:387.

8. HER3 binding polypeptide according to any one of claims 1-4, wherein the amino acid sequence is selected from SEQ ID NO:692, 700, 701, 703, 704, 710, 712, 719, 721, 734, 745, 757, 758, 768, 769, 773, 774, 776, 786, 803, 804, 805, 812 and 824.

9. HER3 binding polypeptide according to claim 8, wherein the amino acid sequence is selected from SEQ ID NO:692, SEQ ID NO:700, SEQ ID NO:703-704, SEQ ID NO:710, SEQ ID NO:712 and SEQ ID NO:721.

10. HER3 binding polypeptide according to any preceding claim, wherein the HER3 binding polypeptide binds to HER3 such that the K_{D} value of the interaction is at most 1 x 10⁻⁶ M, such as at most 1 x 10⁻⁷ M, such as at most 1 x 10⁻⁸ M as determined by surface plasmon resonance technology.

11. HER3 binding polypeptide according to any preceding claim comprising further C terminal and/or N terminal amino acids, in which each further C terminal and/or N terminal amino acid for example improves production, purification, stabilization *in vivo* or *in vitro,* coupling, or detection of the polypeptide.

12. HER3 binding polypeptide according to any preceding claim in multimeric form, comprising at least two HER3 binding polypeptide monomer units, the amino acid sequences of which may be the same or different.

13. Polynucleotide encoding a polypeptide according to any preceding claim.

14. Ligand having binding affinity for HER3 and for HER2, comprising a HER3 binding polypeptide according to any one of claims 1-12; a HER2-binding polypeptide; and a linking moiety for linking the HER3 binding polypeptide with the HER2 binding polypeptide.

15. Ligand according to claim 14, wherein the HER2 binding polypeptide binds to HER2 such that the K_{D} value of the interaction is at most 1 x 10⁻⁶ M, such as at most 1 x 10⁻⁷ M, such as at most 1 x 10⁻⁸ M as determined by surface plasmon resonance technology.

16. Ligand having binding affinity for HER3 and for EGFR, comprising a HER3 binding polypeptide according to any one of claims 1-12; an EGFR-binding polypeptide; and a linking moiety for linking the HER3 binding polypeptide with the EGFR binding polypeptide.

17. HER3 binding polypeptide according to any one of claims 1-12 or ligand according to any one of claims 14-16, further comprising a half life extending moiety for extension of polypeptide or ligand half life *in vivo.*

18. Combination of a HER3 binding polypeptide according to any one of claims 1-12 or a ligand according to any one of claims 14-17 with a therapeutic agent.

19. HER3 binding polypeptide according to any one of claims 1-12, ligand according to any one of claims 14-17 or combination according to claim 18 for use in therapy.

20. HER3 binding polypeptide according to any one of claims 1-12, a ligand according to any one of claims 14-17 or a combination according to claim 18 for use in the treatment of a HER3 related condition selected from colon cancer, endometrial cancer, gastric cancer, glioma, breast cancer, pancreas cancer, head and neck squamous carcinoma, lung cancer, melanoma, medulloblastoma, neuroepithelioma, ovarian cancer, Paget's disease, papillary thyroid cancer, prostate cancer, skin squamous cell carcinoma, transitional cell carcinoma and vestibular schwannoma.

## Patentansprüche

1. HER3-bindendes Polypeptid, umfassend ein HER3-bindendes Motiv, *BM*, das Motiv bestehend aus der Aminosäuresequenz
EX₂X₃X₄A YX₇EIW X₁₁LPNL X₁₆X₁₇X₁₈QX₂₀ X₂₁AFIX₂₅ X₂₆LX₂₈D,
wobei, unabhängig voneinander,
X₂X₃ ausgewählt ist aus KY und RY;
X₄ ausgewählt ist aus A, H, I, K, L, N, Q, R, S, T und V;
X₇ ausgewählt ist aus A, F, G, V, W und Y;
X₁₁ Q ist;
X₁₆ ausgewählt ist aus N und T;
X₁₇X₁₈ ausgewählt ist aus RV, RT, VR und RR;
X₂₀ K ist;
X₂₁ A ist;
X₂₅ ausgewählt ist aus A, G und S;
X₂₆ ausgewählt ist aus S und K;
X₂₈ ausgewählt ist aus A, E, F, Q, S und W
und
wobei das Polypeptid an die extrazelluläre Domäne von HER3 bindet.

2. HER3-bindendes Polypeptid nach Anspruch 1, wobei die Aminosäuresequenz ausgewählt ist aus SEQ ID NO:24, 32, 33, 35, 36, 42, 44, 51, 53, 66, 77, 89, 90, 100, 101, 105, 106, 108, 118, 135, 136, 137, 144 und 156.

3. HER3-bindendes Polypeptid nach Anspruch 2, wobei die Aminosäuresequenz ausgewählt ist aus SEQ ID NO:24, SEQ ID NO:32, SEQ ID NO:35-36, SEQ ID NO:42, SEQ ID NO:44 und SEQ ID NO:53.

4. HER3-bindendes Polypeptid nach einem der vorstehenden Ansprüche, wobei das HER3-bindende Motiv einen Teil einer 3-Helix-Bündel-Proteindomäne bildet.

5. HER3-bindendes Polypeptid nach einem der vorstehenden Ansprüche, umfassend die Aminosäuresequenz
K-[*BM]*-DPSQS XₐX_{b}LLX_{c} EAKKL NDX_{d}Q;
wobei
[*BM*] ein HER3-bindendes Motiv nach einem der Ansprüche 1 bis 3 ist;
Xₐ ausgewählt ist aus A und S;
X_{b} ausgewählt ist aus N und E;
X_{c} ausgewählt ist aus A, S und C;
X_{d} ausgewählt ist aus A und S.

6. HER3-bindendes Polypeptid nach Anspruch 5, wobei die Aminosäuresequenz ausgewählt ist aus SEQ ID NO:358, 366, 367, 369, 370, 376, 378, 385, 387, 400, 411, 423, 424, 434, 435, 439, 440, 442, 452, 469, 470, 471, 478 und 490.

7. HER3-bindendes Polypeptid nach Anspruch 6, wobei die Aminosäuresequenz ausgewählt ist aus SEQ ID NO:358, SEQ ID NO:366, SEQ ID NO:369-370, SEQ ID NO:376, SEQ ID NO:378 und SEQ ID NO:387.

8. HER3-bindendes Polypeptid nach einem der Ansprüche 1 bis 4, wobei die Aminosäuresequenz ausgewählt ist aus SEQ ID NO:692, 700, 701, 703, 704, 710, 712, 719, 721, 734, 745, 757, 758, 768, 769, 773, 774, 776, 786, 803, 804, 805, 812 und 824.

9. HER3-bindendes Polypeptid nach Anspruch 8, wobei die Aminosäuresequenz ausgewählt ist aus SEQ ID NO:692, SEQ ID NO:700, SEQ ID NO:703-704, SEQ ID NO:710, SEQ ID NO:712 und SEQ ID NO:721.

10. HER3-bindendes Polypeptid nach einem der vorstehenden Ansprüche, wobei das HER3-bindende Polypeptid derart an HER3 bindet, dass der Wert K_{D} der Interaktion höchstens 1 x 10⁻⁶ M ist, wie höchstens 1 x 10⁻⁷ M, wie höchstens 1 x 10⁻⁸ M gemäß Bestimmung über die Oberflächenplasmonenresonanztechnologie.

11. HER3-bindendes Polypeptid nach einem der vorstehenden Ansprüche, umfassend weitere C-terminale und/oder N-terminale Aminosäuren, wobei jede weitere C-terminale und/oder N-terminale Aminosäure zum Beispiel die Erzeugung, Reinigung, Stabilisierung *in vivo* oder *in vitro,* Kopplung oder Erkennung des Polypeptids verbessert.

12. HER3-bindendes Polypeptid nach einem der vorstehenden Ansprüche in multimerer Form, umfassend wenigstens zwei Monomereinheiten des HER3-bindenden Polypeptids, deren Aminosäuresequenzen gleich oder verschieden sein können.

13. Polynucleotid, das ein Polypeptid codiert, nach einem der vorstehenden Ansprüche.

14. Ligand mit Bindungsaffinität für HER3 und für HER2, umfassend ein HER3-bindendes Polypeptid nach einem der Ansprüche 1 bis 12; ein HER2-bindendes Polypeptid und einen verbindenden Anteil zum Verbinden des HER3-bindenden Polypeptids mit dem HER2-bindenden Polypeptid.

15. Ligand nach Anspruch 14, wobei das HER2-bindende Polypeptid derart an HER2 bindet, dass der Wert K_{D} der Interaktion höchstens 1 x 10⁻⁶ M ist, wie höchstens 1 x 10⁻⁷ M, wie höchstens 1 x 10⁻⁸ M gemäß Bestimmung über die Oberflächenplasmonenresonanztechnologie.

16. Ligand mit Bindungsaffinität für HER3 und für EGFR, umfassend ein HER3-bindendes Polypeptid nach einem der Ansprüche 1 bis 12; ein EGFRbindendes Polypeptid und einen verbindenden Anteil zum Verbinden des HER3-bindenden Polypeptids mit dem EGFR-bindenden Polypeptid.

17. HER3-bindendes Polypeptid nach einem der Ansprüche 1 bis 12 oder Ligand nach einem der Ansprüche 14 bis 16, ferner umfassend einen halbwertszeitverlängernden Anteil zum Verlängern der Halbwertszeit des Polypeptids oder Liganden *in vivo.*

18. Kombination eines HER3-bindenden Polypeptids nach einem der Ansprüche 1 bis 12 oder eines Liganden nach einem der Ansprüche 14 bis 17 mit einem Therapeutikum.

19. HER3-bindendes Polypeptid nach einem der Ansprüche 1 bis 12, Ligand nach einem der Ansprüche 14 bis 17 oder Kombination nach Anspruch 18 zum therapeutischen Gebrauch.

20. HER3-bindendes Polypeptid nach einem der Ansprüche 1 bis 12, Ligand nach einem der Ansprüche 14 bis 17 oder Kombination nach Anspruch 18 zum Gebrauch bei der Behandlung einer HER3-bezogenen Erkrankung ausgewählt aus Darmkrebs, Endometriumkarzinom, Magenkrebs, Gliom, Brustkrebs, Bauchspeicheldrüsenkrebs, Plattenepithelkarzinom des Kopf-Hals-Bereichs, Lungenkrebs, Melanom, Medulloblastom, Neuroepitheliom, Eierstockkrebs, Paget-Krankheit, Schilddrüsenkrebs, Prostatakrebs, Plattenepithelkarzinom der Haut, Urothelkarzinom und Vestibularisschwannom.

## Revendications

1. Polypeptide de liaison à HER3, comprenant un motif de liaison à HER3, *BM,* lequel motif consiste en la séquence d'acides aminés
EX₂X₃X₄A YX₇EIW X₁₁LPNL X₁₆X₁₇X₁₈QX₂₀ X₂₁AFIX₂₅ X₂₆LX₂₈D,
où, indépendamment les uns des autres,
X₂X₃ est choisi parmi KY et RY ;
X₄ est choisi parmi A, H, I, K, L, N, Q, R, S, T et V ;
X₇ est choisi parmi A, F, G, V, W, et Y ;
X₁₁ est Q ;
X₁₆ est choisi parmi N et T ;
X₁₇X₁₈ est choisi parmi RV, RT, VR et RR ;
X₂₀ est K ;
X₂₁ est A ;
X₂₅ est choisi parmi A, G, et S ;
X₂₆ est choisi parmi S et K ;
X₂₈ est choisi parmi A, E, F, Q, S et W ;
et
où le polypeptide se lie au domaine extracellulaire de HER3.

2. Polypeptide de liaison à HER3 selon la revendication 1, dans lequel la séquence d'acides aminés est choisie parmi l'une quelconque des SEQ ID NO : 24, 32, 33, 35, 36, 42, 44, 51, 53, 66, 77, 89, 90, 100, 101, 105, 106, 108, 118, 135, 136, 137, 144 et 156.

3. Polypeptide de liaison à HER3 selon la revendication 2, dans lequel la séquence d'acides aminés est choisie parmi l'une quelconque des SEQ ID NO : 24, SEQ ID NO : 32, SEQ ID NO : 35-36, SEQ ID NO : 42, SEQ ID NO : 44 et SEQ ID NO : 53.

4. Polypeptide de liaison à HER3 selon n'importe quelle revendication précédente, dans lequel ledit motif de liaison à HER3 fait partie d'un domaine protéique comportant un faisceau de trois hélices.

5. Polypeptide de liaison à HER3 selon n'importe quelle revendication précédente, qui comprend la séquence d'acides aminés
K-[*BM*]-DPSQS XₐX_{b}LLX_{c} EAKKL NDX_{d}Q;
où
[*BM*] est un motif de liaison à HER3 tel que défini dans l'une quelconque des revendications 1 à 3 ;
Xₐ est choisi parmi A et S ;
X_{b} est choisi parmi N et E ;
X_{c} est choisi parmi A, S et C ;
X_{d} est choisi parmi A et S.

6. Polypeptide de liaison à HER3 selon la revendication 5, dans lequel la séquence d'acides aminés est choisie parmi l'une quelconque des SEQ ID NO : 358, 366, 367, 369, 370, 376, 378, 385, 387, 400, 411, 423, 424, 434, 435, 439, 440, 442, 452, 469, 470, 471, 478 et 490.

7. Polypeptide de liaison à HER3 selon la revendication 6, dans lequel la séquence d'acides aminés est choisie parmi l'une quelconque des SEQ ID NO : 358, SEQ ID NO : 366, SEQ ID NO : 369-370, SEQ ID NO : 376, SEQ ID NO : 378 et SEQ ID NO : 387.

8. Polypeptide de liaison à HER3 selon l'une quelconque des revendications 1 à 4, dans lequel la séquence d'acides aminés est choisie parmi les SEQ ID NO : 692, 700, 701, 703, 704, 710, 712, 719, 721, 734, 745, 757, 758, 768, 769, 773, 774, 776, 786, 803, 804, 805, 812 et 824.

9. Polypeptide de liaison à HER3 selon la revendication 8, dans lequel la séquence d'acides aminés est choisie parmi les SEQ ID NO : 692, SEQ ID NO : 700, SEQ ID NO : 703-704, SEQ ID NO : 710, SEQ ID NO : 712 et SEQ ID NO : 721.

10. Polypeptide de liaison à HER3 selon n'importe quelle revendication précédente, lequel polypeptide de liaison à HER3 se lie à HER3 de sorte que la valeur K_{D} de l'interaction est au plus de 1x10⁻⁶ M, telle que au plus 1x10⁻⁷ M, telle que au plus 1x10⁻⁸ M, déterminée par la technologie de résonance des plasmons de surface.

11. Polypeptide de liaison à HER3 selon n'importe quelle revendication précédente comprenant des acides aminés C-terminaux et/ou N-terminaux supplémentaires, dans lequel chaque acide aminé C-terminal et/ou N-terminal supplémentaire améliore par exemple la production, la purification, la stabilisation *in vivo* ou *in vitro,* le couplage, ou la détection du polypeptide.

12. Polypeptide de liaison à HER3 selon n'importe quelle revendication précédente sous forme multimérique, comprenant au moins deux unités monomériques de polypeptide de liaison à HER3, dont les séquences d'acides aminés peuvent être identiques ou différentes.

13. Polynucléotide codant un polypeptide selon n'importe quelle revendication précédente.

14. Ligand ayant une affinité de liaison pour HER3 et pour HER2, comprenant un polypeptide de liaison à HER3 selon l'une quelconque des revendications 1 à 12 ; un polypeptide de liaison à HER2 ; et un groupement liant permettant de lier le polypeptide de liaison à HER3 avec le polypeptide de liaison à HER2.

15. Ligand selon la revendication 14, dans lequel le polypeptide de liaison à HER2 se lie à HER2 de sorte que la valeur K_{D} de l'interaction est au plus de 1x10⁻⁶ M, telle que au plus 1x10⁻⁷ M, telle que au plus 1x10⁻⁸M, déterminée par la technologie de résonance des plasmons de surface.

16. Ligand ayant une affinité de liaison pour HER3 et pour EGFR, comprenant un polypeptide de liaison à HER3 selon l'une quelconque des revendications 1 à 12 ; un polypeptide de liaison à EGFR ; et un groupement liant permettant de lier le polypeptide de liaison à HER3 avec le polypeptide de liaison à EGFR.

17. Polypeptide de liaison à HER3 selon l'une quelconque des revendications 1 à 12 ou ligand selon l'une quelconque des revendications 14 à 16, comprenant en outre un groupement allongeant la demi-vie permettant l'allongement de la demi-vie du polypeptide ou du ligand *in vivo.*

18. Association d'un polypeptide de liaison à HER3 selon l'une quelconque des revendications 1 à 12 ou d'un ligand selon l'une quelconque des revendications 14 à 17 avec un agent thérapeutique.

19. Polypeptide de liaison à HER3 selon l'une quelconque des revendications 1 à 12, ligand selon l'une quelconque des revendications 14 à 17 ou association selon la revendication 18 pour utilisation en thérapeutique.

20. Polypeptide de liaison à HER3 selon l'une quelconque des revendications 1 à 12, ligand selon l'une quelconque des revendications 14 à 17 ou association selon la revendication 18 pour utilisation dans le traitement d'une affection liée à HER3 choisie parmi le cancer du côlon, le cancer de l'endomètre, le cancer de l'estomac, le gliome, le cancer du sein, le cancer du pancréas, le carcinome épidermoïde de la tête et du cou, le cancer du poumon, le mélanome, le médulloblastome, le neuroépithéliome, le cancer de l'ovaire, la maladie de Paget, le cancer papillaire de la thyroïde, le cancer de la prostate, le carcinome épidermoïde cutané, le carcinome à cellules transitionnelles et le schwannome vestibulaire.
